# EUROPÄISCHE PATENTANMELDUNG

(11) **EP 2 377 944 A1**
(43) Veröffentlichungstag der Anmeldung: **19.10.2011**
(21) Anmeldenummer: 11173560.1
(22) Anmeldetag: 27.10.2010
(51) Int. Cl.: C12P 7/42, C12N 9/02, C12N 1/16

(54) **Candida tropicalis Zellen und deren Verwendung**

(30) Priorität: 11.11.2009 DE 102009046626
(62) Teilanmeldung aus: 10188993.9
(71) Anmelder: Evonik Degussa GmbH, 45128 Essen (DE)
(72) Erfinder: Pötter, Markus, 48149 Münster (DE); Hennemann, Hans-Georg, 50181 Bedburg (DE); Schaffer, Steffen, 45699 Herten (DE); Haas, Thomas, 48161 Münster (DE)

(57) **Zusammenfassung**

Gegenstand der Erfindung sind gentechnisch modifizierte *Candida tropicalis* Zellen, deren Verwendung sowie ein Verfahren zur Herstellung von ω-Hydroxy-Carbonsäuren und ω-Hydroxy-Carbonsäureestern.

## Beschreibung

### Gebiet der Erfindung

Gegenstand der Erfindung sind gentechnisch modifizierte *Candida tropicalis* Zellen, deren Verwendung sowie ein Verfahren zur Herstellung von ω-Hydroxy-Carbonsäuren und ω-Hydroxy-Carbonsäureestern.

### Stand der Technik

*Candida tropicalis* ist aufgrund seiner Fähigkeit, Dicarbonsäuren aus Alkanen zu bilden, ein gut charakterisierter Schlauchpilz.

WO91/006660 beschreibt durch Unterbrechung der POX4 und/oder POX5 Gene in der β-Oxidation vollständig inhibierte *Candida tropicalis* Zellen, welche erhöhte Ausbeuten an α, ω-Dicarbonsäuren erzielen.

WO00/020566 beschreibt Cytochrom P450 Monooxygenasen und NADPH Cytochrom P450 Oxidoreduktasen von *Candida tropicalis* und deren Verwendung zur Beeinflussung der ω-Hydroxylierung, dem ersten Schritt der ω-Oxidation.

WO03/089610 beschreibt Enzyme aus *Candida tropicalis,* die den zweiten Schritt der ω-Oxidation, die Umsetzung eines Fettalkohols zu einem Aldehyd, katalysieren, sowie deren Verwendung zur verbesserten Produktion von Dicarbonsäuren.

Die bisher beschriebenen Zellen und Verfahren sind zur Herstellung von ω-Hydroxy-Carbonsäuren oder deren Ester ungeeignet, da die ω-Hydroxy-Carbonsäuren immer nur als kurzfristiges Intermediat vorliegen und sofort weiter verstoffwechselt werden.

ω-Hydroxy-Carbonsäuren und deren Ester sind als Vorläuferstoffe von Polymeren wirtschaftlich bedeutende Verbindungen, wodurch die gewerbliche Anwendbarkeit der vorliegenden Erfindung begründet ist.

Aufgabe der Erfindung war es, eine Möglichkeit zu finden, ω-Hydroxy-Carbonsäuren bzw. ω-Hydroxy-Carbonsäureestern fermentativ in ausreichenden Mengen, insbesondere in dem die Zellen umgebenden Medium, bereitzustellen.

### Beschreibung der Erfindung

Überraschenderweise wurde gefunden, dass die im Folgenden beschriebenen Zellen einen Beitrag zur Lösung der gestellten Aufgabe leisten.

Gegenstand der vorliegenden Erfindung ist daher eine Zelle wie in Anspruch 1 beschrieben.

Ein weiterer Gegenstand der Erfindung ist die Verwendung der erfindungsgemäßen Zelle sowie ein Verfahren zur Herstellung von ω-Hydroxy-Carbonsäuren und ω-Hydroxy-Carbonsäureestern unter Einsatz der erfindungsgemäßen Zellen.

Vorteile der Erfindung sind die schonende Umsetzung des eingesetzten Eduktes zu den ω-Hydroxy-Carbonsäuren und korrespondierenden Estern sowie eine hohe Spezifität des Verfahrens und somit einhergehender hohen Ausbeute bezogen auf das eingesetzte Edukt.

Ein Gegenstand der vorliegenden Erfindung ist eine *Candida tropicalis* Zelle, insbesondere eine vom Stamm ATCC 20336, welche dadurch gekennzeichnet ist, dass die Zelle im Vergleich zu Ihrem Wildtyp eine verminderte Aktivität mindestens eines der Enzyme aufweist, welche kodiert werden durch die intronfreien Nukleinsäuresequenzen ausgewählt aus den beiden Gruppe bestehend aus
A) Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49, Seq ID Nr. 51, Seq ID Nr. 53, Seq ID Nr. 55, Seq ID Nr. 57, Seq ID Nr. 59, Seq ID Nr. 61, Seq ID Nr. 63, Seq ID Nr. 65 und Seq ID Nr. 67; insbesondere Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49 und Seq ID Nr. 51; ganz besonders Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25 und Seq ID Nr. 27,
B) eine Sequenz, die zu mindestens 80 %, besonders bevorzugt zu mindestens 90 %, darüber hinaus bevorzugt zu mindestens 95 % und am meisten bevorzugt zu mindestens 99 % identisch zu einer der Sequenzen Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49, Seq ID Nr. 51, Seq ID Nr. 53, Seq ID Nr. 55, Seq ID Nr. 57, Seq ID Nr. 59, Seq ID Nr. 61, Seq ID Nr. 63, Seq ID Nr. 65 und Seq ID Nr. 67; insbesondere zu Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49 und Seq ID Nr. 51; ganz besonders zu Seq ID Nr. 1, Seq ID Nr. 3, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25 und Seq ID Nr. 27 ist.

In diesem Zusammenhang erfindungsgemäß bevorzugte Nukleinsäuresequenzgruppe ist Gruppe A).

Unter einem "Wildtyp" einer Zelle wird im Zusammenhang mit der vorliegenden Erfindung bevorzugt der Ausgangsstamm verstanden, aus dem die erfindungsgemäße Zelle durch Manipulation an den Elementen (wie beispielsweise die Gene umfassend die genannten Nukleinsäuresequenzen kodierend für entsprechendes Enzym oder die im entsprechenden Gen enthaltenen Promotoren, die mit den genannten Nukleinsäuresequenzen funktional verknüpft sind), die die Aktivitäten der Enzyme kodiert durch die genannten Nukleinsäure Seq ID Nr. beeinflussen, hervorgegangen ist.

Wird beispielsweise die Aktivität des Enzyms kodiert durch die Seq ID Nr. 1 in dem Stamm ATCC 20336 durch Unterbrechung des korrespondierenden Gens vermindert, so ist als "Wildtyp" der unveränderte und zur entsprechenden Manipulation eingesetzte Stamm ATCC 20336 zu betrachten.

Unter dem Begriff "Gen" wird im Zusammenhang mit der vorliegenden Erfindung nicht nur der kodierende bzw. in mRNA transkribierte DNA-Bereich, das "Strukturgen", verstanden, sondern außerdem Promotor-, mögliche Intron-, Enhancer- und andere Regulatorsequenz-, sowie Terminator-Bereiche, verstanden.

Unter dem Begriff "Aktivität eines Enzyms" im Zusammenhang mit der Erfindung ist immer die enzymatische Aktivität zu verstehen, die die Umsetzungen von 12-Hydroxy-Dodekansäure zu 1,12-Dodekandisäure durch die gesamte Zelle katalysiert.

Diese Aktivität wird bevorzugt bestimmt nach dem folgenden Verfahren:
Ausgehend von einer Einzelkolonie wird ein 100 ml Erlenmeyerkolben mit 10 ml YM Medium (0,3% Hefe Extrakt, 0,3% Malz Extrakt, 0,5% Pepton und 1,0% (w/w) Glucose) bei 30 °C und 90 rpm über 24 h kultiviert. Anschließend werden ausgehend von dieser Kultur 10 ml in einen 1 l Erlenmeyerkolben mit 100 ml Produktionsmedium (für 1 l : 25 g Glucose, 7,6 g NH₄Cl, 1,5 g Na₂SO₄, 300 ml eines 1 mM Kaliumphosphatpuffers (pH 7,0), 20 mg ZnSO₄ x 7 H₂O, 20 mg MnSO₄ x 4 H₂O, 20 mg Nikotinsäure, 20 mg Pyridoxin, 8 mg Thiamin und 6 mg Pantothenat) angeimpft. Die Kultivierung erfolgt für 24 h bei 30 °C.

Nach 24 h wird der Zellsuspension 12-Hydroxy-Dodekansäure zudosiert, so dass die Konzentration nicht größer als 0,5 g/l beträgt. Zudem wird Glucose oder Glycerin als Co-Substrat zugegeben, so dass die Konzentration des Co-Substrates nicht unter 0,2 g/l sinkt. Nach 0h, 0,5 h, 1h, und dann bis zu 24 h Kultivierungsdauer stündlich werden Proben (1 ml) für die Messung von 12-Hydroxy-Dodekansäure, 12-Oxo-Dodekansäure und der 1, 12-Dodekandisäure und der korrespondierenden Methylester, sowie der Überprüfung der Zellzahl entnommen. Der pH Wert wird nach jeder Messung mit 6 N NaOH bzw. 4 N H₂SO₄ zwischen 5,0 und 6,5 gehalten. Während der Kultivierungen wird das Zellwachstum durch Überprüfung der "Colony Forming Units"(CFU) überprüft. Die Abnahme von 12-Hydroxy-Dodekansäure Und die Produktion von 1,12-Dodekandisäure bzw. der korrespondierenden Methylester werden durch LC-MS überprüft. Dafür werden 500 µl Kulturbrühe auf pH 1 eingestellt und anschließend mit dem gleichen Volumen Diethylether oder Ethylacetat extrahiert und mittels LC-MS analysiert.

Das Messsystem besteht aus einer HP1100 HPLC (Agilent Technologies, Waldbronn, D) mit Degasser, Autosampler und Säulenofen, gekoppelt an einen massenselektiven Quadrupol Detektor MSD (Agilent Technologies, Waldbronn, D). Die chromatographische Trennung wird auf einer Umkehrphase z.B. 125x2 mm Luna C18(2) Säule (Phenomenex, Aschaffenburg, D) bei 40°C erzielt. Die Gradientenelution erfolgt bei einem Fluss von 0,3 ml/min (A: 0.02% Ameisensäure in Wasser und B: 0.02% Ameisensäure in Acetonitril). Alternativ werden die organischen Extrakte mittels GC-FID (Perkin Elmer, Rodgau-Jügesheim, D) analysiert. Die chromatographische Trennung erfolgt auf einer Methylpolysiloxan (5% Phenyl) Phase z.B. Elite 5, 30 m, 0,25 mm ID, 0,25 µm FD (Perkin Elmer, Rodgau-Jügesheim, D). Freie Säuren werden vor der Messung mit einem Methylierungsreagenz z.B. Trimethylsulphoniumhydroxid "TMSH" (Macherey-Nagel GmbH & Co. KG, Düren, D) versetzt und bei der Injektion in die entsprechenden Methylester überführt.

Durch Berechnung der gemessenen Konzentration an 1,12-Dodekandisäure und der Zellzahl zu dem Probenzeitpunkt, kann die spezifische Produktionsrate der 1,12-Dodekandisäure aus 12-Hydroxy-Dodekansäure bestimmt und somit die oben definierte "Aktivität eines Enzyms" in einer Zelle bestimmt werden.

Unter der Formulierung "im Vergleich zu ihrem Wildtyp verminderte Aktivität" wird vorzugsweise eine um mindestens 50% verminderte, besonders bevorzugt um mindestens 90%, darüber hinaus bevorzugt um mindestens 99.9%, darüber hinaus noch mehr bevorzugt um mindestens 99,99% und am meisten bevorzugt um mindestens 99,999% verminderte Aktivität bezogen auf die Wildtyp-Aktivität verstanden.

Die Verminderung der Aktivität der erfindungsgemäßen Zelle im Vergleich zu ihrem Wildtyp wird bestimmt nach oben beschriebenen Verfahren zur Bestimmung der Aktivität unter Einsatz von möglichst gleichen Zellzahlen/-konzentrationen, wobei die Zellen unter gleichen Bedingungen wie beispielsweise Medium, Begasung, Agitation angezogen wurden.

Die "Nukleotid-Identität" relativ zu den angebenden Sequenzen, kann mit Hilfe bekannter Verfahren bestimmt werden. Generell werden besondere Computerprogramme mit Algorithmen unter Berücksichtigung spezieller Erfordernisse verwendet.

Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
- GAP (Deveroy, J. et al., Nucleic Acid Research 12 (1984), Seite 387, Genetics Computer Group University of Wisconsin, Medicine (Wi), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der bekannte Smith-Waterman Algorithmus kann ebenso zur Bestimmung der Nukleotid-Identität verwendet werden.

Bevorzugte Parameter für die Bestimmung der "Nukleotid-Identität" sind bei Verwendung des BLASTN-Programms (Altschul, S. et al., Journal of Molecular Biology 215 (1990), Seiten 403-410:
- Expect Threshold:: 10
- Word size:: 28
- Match Score:: 1
- Mismatch Score:: -2
- Gap costs:: Linear
Die vorstehenden Parameter sind die default-Parameter im Nukleotidsequenzvergleich.

Das GAP-Programm ist ebenso zur Verwendung mit den vorstehenden Parametern geeignet.

Eine Identität von 80 % gemäß dem vorstehenden Algorithmus bedeutet im Zusammenhang mit der vorliegenden Erfindung 80 % Identität. Das gleiche gilt für höhere Identitäten.

Durch den Begriff "welche kodiert werden durch die intronfreien Nukleinsäuresequenzen" ist klar herausgestellt, dass bei einem Sequenzvergleich mit den hier angegebenen Sequenzen die zu vergleichenden Nukleinsäuresequenzen zuvor um etwaige Introns bereinigt werden müssen.

Alle angegebenen Prozent (%) sind wenn nicht anders angegeben Massenprozent.

Verfahren zur Verminderung von enzymatischen Aktivitäten in Mikroorganismen sind dem Fachmann bekannt.

Insbesondere molekularbiologische Techniken bieten sich hier an. Anleitung zur Modifikation und Verminderung von Proteinexpressionen und damit einhergehender

Enzymaktivitätsverringerung speziell für *Candida tropicalis,* insbesondere zum Unterbrechen spezieller Gene findet der Fachmann in der WO91/006660; WO03/100013; Picataggio et al. Mol Cell Biol. 1991 Sep;11(9):4333-9; Rohrer et al. Appl Microbiol Biotechnol. 1992 Feb;36(5):650-4; Picataggio et al. Biotechnology (N Y). 1992 Aug; 10(8):894-8; Ueda et al. Biochim Biophys Acta. 2003 Mar 17;1631(2):160-8; Ko et al. Appl Environ Microbiol. 2006 Jun;72(6):4207-13; Hara et al. Arch Microbiol. 2001 Nov;176(5):364-9; Kanayama et al. J Bacteriol. 1998 Feb;180(3):690-8.

Erfindungsgemäß bevorzugte Zellen sind dadurch gekennzeichnet, dass die Verminderung der enzymatischen Aktivität erreicht wird durch Modifikation mindestens eines Genes umfassend eine der Sequenzen ausgewählt aus den zuvor genannten Nukleinsäuresequenzgruppen A) und B), wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz und Aussetzen des Gens unter den Einfluss von RNA-Interferenz oder Austausch von Teilen des Gens mit Fremd-DNA, insbesondere des Promoterbereichs.

Unter Fremd-DNA ist in diesem Zusammenhang jegliche DNA-Sequenz zu verstehe, die dem Gen (und nicht dem Organismus) "fremd" ist, d.h. auch *Candida tropicalis* endogene DNA-Sequenzen können in diesem Zusammenhang als "Fremd-DNA" fungieren.

In diesem Zusammenhang ist es insbesondere bevorzugt, dass das Gen durch Insertion eines Selektionsmarkergens unterbrochen wird, somit die Fremd-DNA ein Selektionsmarkergen ist, wobei bevorzugt die Insertion durch homologe Rekombination in den Genlocus erfolgte.

In diesem Zusammenhang kann es vorteilhaft sein, wenn das Selektionsmarkergen durch weitere Funktionalitäten erweitert wird, die wiederum eine anschließende Entfernung aus dem Gen ermöglichen, dies kann beispielsweise durch ein Cre/loxP-System, durch *Flippase Recognition Targets* (FRT) oder homologe Rekombination erreicht werden.

Erfindungsgemäß bevorzugte Zellen sind dadurch gekennzeichnet, dass sie in ihrer β-Oxidation mindestens teilweise, bevorzugt vollständig, blockiert sind, da dies den Abfluss von Substrat verhindert und somit höhere Titer ermöglicht werden.

Beispiele für teilweise in Ihrer β-Oxidation blockierten *Candida tropicalis* Zellen sind in der EP0499622 als Stämme H41, H41B, H51, H45, H43, H53, H534, H534B und H435 beschrieben, von denen eine erfindungsgemäß bevorzugte *Candida tropicalis* Zelle sich ableitent.

Weitere, in der β-Oxidation blockierte *Candida tropicalis* Zellen werden beispielsweise in der WO03/100013 beschrieben.

In diesem Zusammenhang sind Zellen bevorzugt, bei denen die β-Oxidation durch eine induzierte Missfunktion mindestens eines der Gene POX2, POX4 oder POX5 hervorgerufen wird.

Somit sind in diesem Zusammenhang Zellen bevorzugt, die dadurch gekennzeichnet sind, dass eine erfindungsgemäß bevorzugte *Candida tropicalis* Zelle sich ableitet von Stämmen ausgewählt aus der Gruppe bestehend aus
ATCC 20962 und dem in US2004/0014198 beschriebenen *Candida tropicalis* HDC100.

Ein weiteren Beitrag zur Lösung der der Erfindung gestellten Aufgabe leistet die Verwendung der erfindungsgemäßen Zellen zur Herstellung von ω-Hydroxy-Carbonsäuren oder ω-Hydroxy-Carbonsäureestern.

Insbesondere die Verwendung der erfindungsgemäßen Zellen zur Herstellung von ω-Hydroxy-Carbonsäuren oder ω-Hydroxy-Carbonsäureestern mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4, insbesondere von 1 oder 2 Kohlenstoffatomen, ist vorteilhaft. In diesem Zusammenhang ist es bevorzugt, dass es sich bei der ω-Hydroxy-Carbonsäuren um 12-Hydroxy-Dodekansäure und bei dem ω-Hydroxy-Carbonsäureester um 12-Hydroxy-Dodekansäuremethylester handelt.

Bevorzugte Verwendung ist erfindungsgemäß dadurch gekennzeichnet, dass erfindungsgemäß bevorzugte Zellen wie oben beschrieben verwendet werden.

Ein weiteren Beitrag zur Lösung der der Erfindung gestellten Aufgabe leistet ein Verfahren zur Herstellung der zuvor beschriebenen erfindungsgemäßen C. *tropicalis* Zelle umfassend die Verfahrensschritte:
I) Bereitstellen einer *C. tropicalis* Zelle, bevorzugt einer in ihrer β-Oxidation mindestens teilweise, bevorzugt vollständig, blockierten Zelle
II) Modifikation mindestens eines Genes umfassend eine der Intronfreien Nukleinsäuresequenzenausgewählt aus den zuvor genannten Nukleinsäuresequenzgruppen A) und B) durch Insertion von Fremd-DNA, insbesondere von DNA kodierend für ein Selektionsmarkergen, in das Gen, Deletion mindestens von Teilen des Gens, Punktmutationen in der Gensequenz und Aussetzen des Gens unter den Einfluss von RNA-Interferenz oder Austausch von Teilen des Gens mit Fremd-DNA, insbesondere des Promoterbereichs.

Ein weiteren Beitrag zur Lösung der der Erfindung gestellten Aufgabe leistet ein Verfahren zur Herstellung von ω-Hydroxy-Carbonsäuren oder ω-Hydroxy-Carbonsäureestern, insbesondere von ω-Hydroxy-Carbonsäuren oder ω-Hydroxy-Carbonsäureestern mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4, insbesondere von 1 oder 2 Kohlenstoffatomen, insbesondere von 12-Hydroxy-Dodekansäure oder 12-Hydroxy-Dodekansäuremethylester umfassend die Verfahrensschritte
A) In Kontakt bringen einer zuvor beschriebenen erfindungsgemäßen Zelle mit einem Medium beinhaltend eine Carbonsäure bzw. einen Carbonsäureester, insbesondere eine Carbonsäure oder einen Carbonsäureester mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4 Kohlenstoffatomen, insbesondere Dodekansäure oder Dodekansäuremethylester,
B) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden und
C) gegebenenfalls Isolierung der gebildeten ω-Hydroxy-Carbonsäure oder ω-Hydroxy-Carbonsäureester.

Erfindungsgemäß bevorzugte Verfahren setzen oben als erfindungsgemäß bevorzugt genannte Zellen ein.

Somit ist beispielsweise ein Verfahren zur Herstellung von 12-Hydroxy-Dodekansäure oder 12-Hydroxy-Dodekansäuremethylester umfassend die Verfahrensschritte
a) In Kontakt bringen einer in ihrer β-Oxidation mindestens teilweise blockierten *Candida tropicalis* Zelle vom Stamm ATTC 20336, die im Vergleich zu Ihrem Wildtyp eine verminderte Aktivität mindestens eines der Enzyme aufweist, welche kodiert werden durch die intronfreien Nukleinsäuresequenzen ausgewählt aus den zuvor genannten Nukleinsäuresequenzgruppen A) und B) wobei die Verminderung der enzymatischen Aktivität erreicht wird durch Modifikation eines Genes umfassend eine der Nukleinsäuresequenzen ausgewählt aus den zuvor genannten Nukleinsäuresequenzgruppen A) und B), wobei die Modifikation aus Insertion eines Selektionsmarkergens in das Gen besteht, mit einem Medium beinhaltend Dodekansäure oder Dodekansäuremethylester,
b) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden und
c) gegebenenfalls Isolierung der gebildeten ω-Hydroxy-Carbonsäure oder ω-Hydroxy-Carbonsäureester ganz besonders bevorzugt.

Geeignete Kultivierungsbedingungen für *Candida tropicalis* sind dem Fachmann bekannt. Insbesondere für Verfahrensschritt b) geeignete Bedingungen sind solche, die dem Fachmann aus Biokonversionsverfahren zur Herstellung von Dicarbonsäuren mit *Candida tropicalis* bekannt sind.

Solche Kultivierungsbedingungen sind beispielsweise in der WO00/017380 und WO00/015828 beschrieben.

Zur Isolierung der gebildeten ω-Hydroxy-Carbonsäure oder ω-Hydroxy-Carbonsäureester sind dem Fachmann Verfahren bekannt. Dies sind Standardverfahren zur Isolierung von langkettigen Carbonsäuren aus wässrigen Lösung, wie beispielsweise Destillation oder Extraktion und können beispielsweise auch der W02009/077461 entnommen werden.

Es ist vorteilhaft, die durch erfindungsgemäßes Verfahren erhaltenen ω-Hydroxy-Carbonsäuren oder ω-Hydroxy-Carbonsäureester zur Herstellung von Polymeren, insbesondere Polyestern, zu verwenden. Des Weiteren können aus den ω-Hydroxy-Carbonsäuren auch Lactone hergestellt werden, die dann zum Beispiel wiederum zur Herstellung von Polyestern eingesetzt werden können.

Eine weitere vorteilhafte Verwendung ist es, die ω-Hydroxy-Carbonsäuren oder ω-Hydroxy-Carbonsäureester zu ω-Amino-Carbonsäuren oder ω-Amino-Carbonsäureestern umzuwandeln, um als Polymer Polyamide zu erhalten. Die ω-Amino-Carbonsäuren oder ω-Amino-Carbonsäureestern können auch erst zu den entsprechenden Lactamen umgesetzt werden, die dann wiederum anionisch, oder auch säurekatalysiert zu einem Polyamid umgesetzt werden können. Ganz besonders vorteilhaft ist es, in einem ersten Reaktionsschritt die ω-Hydroxy-Carbonsäuren oder entsprechenden Ester in die ω-oxo-Carbonsäuren oder den entsprechenden Ester umzuwandeln und anschließend die Aminierung der Oxo-Gruppe z.B. im Zuge einer reduktiven Aminierung durchzuführen.

In diesem Zusammenhang ist die Verwendung von 12-Hydroxy-Dodekansäure oder 12-Hydroxy-Dodekansäuremethylester zur Herstellung von Polymeren, insbesondere von Polyamid 12 besonders bevorzugt.

## Patentansprüche

1. Verwendung von ω-Hydroxy-Carbonsäuren oder ω-Hydroxy-Carbonsäureestern hergestellt nach einem Verfahren umfassend die Verfahrensschritte
a) in Kontakt Bringen einer *Candida tropicalis* Zelle mit einem Medium beinhaltend eine Carbonsäure bzw. einen Carbonsäureester,
b) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden, und
c) gegebenenfalls Isolierung der gebildeten ω-Hydroxy-Carbonsäure oder ω-Hydroxy-Carbonsäureester
zur Herstellung von ω-Amino-Carbonsäuren oder ω-Amino-Carbonsäureestern,
**dadurch gekennzeichnet,**
**dass** die *Candida tropicalis* Zelle im Vergleich zu Ihrem Wildtyp eine verminderte Aktivität mindestens eines der Enzyme aufweist,
welche kodiert werden durch die intronfreien Nukleinsäuresequenzen ausgewählt aus den beiden Gruppen A) und B) bestehend aus
A) Seq ID Nr. 3, Seq ID Nr. 1, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49, Seq ID Nr. 51, Seq ID Nr. 53, Seq ID Nr. 55, Seq ID Nr. 57, Seq ID Nr. 59, Seq ID Nr. 61, Seq ID Nr. 63, Seq ID Nr. 65 und Seq ID Nr. 67
B) eine Sequenz, die zu mindestens 80 % identisch zu einer der Sequenzen Seq ID Nr. 3, Seq ID Nr. 1, Seq ID Nr. 5, Seq ID Nr. 7, Seq ID Nr. 9, Seq ID Nr. 11, Seq ID Nr. 13, Seq ID Nr. 15, Seq ID Nr. 17, Seq ID Nr. 19, Seq ID Nr. 21, Seq ID Nr. 23, Seq ID Nr. 25, Seq ID Nr. 27, Seq ID Nr. 29, Seq ID Nr. 31, Seq ID Nr. 33, Seq ID Nr. 35, Seq ID Nr. 37, Seq ID Nr. 39, Seq ID Nr. 41, Seq ID Nr. 43, Seq ID Nr. 45, Seq ID Nr. 47, Seq ID Nr. 49, Seq ID Nr. 51, Seq ID Nr. 53, Seq ID Nr. 55, Seq ID Nr. 57, Seq ID Nr. 59, Seq ID Nr. 61, Seq ID Nr. 63, Seq ID Nr. 65 und Seq ID Nr. 67 ist.

2. Verwendung gemäß Anspruch 1, **dadurch gekennzeichnet, dass** die Verminderung der enzymatischen Aktivität erreicht wird durch Modifikation eines Genes umfassend eine der in Anspruch 1 genannten Nukleinsäuresequenzen, wobei die Modifikation ausgewählt ist aus der Gruppe umfassend
Insertion von Fremd-DNA in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

3. Verwendung gemäß Anspruch 2, **dadurch gekennzeichnet, dass** die Fremd-DNA ein Selektionsmarkergen ist.

4. Verwendung gemäß mindestens einem der vorherigen Ansprüche, **dadurch gekennzeichnet, dass** die Zelle in ihrer β-Oxidation mindestens teilweise blockiert ist.

5. Verwendung gemäß mindestens einem der vorherigen Ansprüche, **dadurch gekennzeichnet, dass** die *Candida tropicalis* Zelle sich ableitet von Stämmen ausgewählt aus der Gruppe bestehend aus
*Candida tropicalis* H41, *Candida tropicalis* H41B, *Candida tropicalis* H51, *Candida tropicalis* H45, *Candida tropicalis* H43, *Candida tropicalis* H53, *Candida tropicalis* H534, *Candida tropicalis* 534B, *Candida tropicalis* H435, *Candida tropicalis* ATCC20962 und *Candida tropicalis* HDC100, insbesondere bestehend aus *Candida tropicalis* ATCC20962 und *Candida tropicalis* HDC100.

6. Verwendung gemäß mindestens einem der vorherigen Ansprüche, **dadurch gekennzeichnet, dass** die ω-Hydroxy-Carbonsäure oder der ω-Hydroxy-Carbonsäureester, eine Kettenlänge der Carbonsäure von 6 bis 24 Kohlenstoffatomen und eine Kettenlänge der Alkoholkomponente des Esters von 1 bis 4 Kohlenstoffatomen aufweisen.

7. Verwendung gemäß mindestens einem der vorherigen Ansprüche, **dadurch gekennzeichnet, dass** die ω-Hydroxy-Carbonsäure oder der ω-Hydroxy-Carbonsäureester 12-Hydroxy-Dodekansäure oder 12-Hydroxy-Dodekansäuremethylester darstellen.

8. Verwendung der ω-Hydroxy-Carbonsäure oder des ω-Hydroxy-Carbonsäureesters erhalten durch das Verfahren wie in einem der vorherigen Ansprüche beschrieben zur Herstellung eines Polymers.


SEQUENCE LISTING

<110>  Evonik Degussa GmbH

<120>  Candida tropicalis Zellen und deren Verwendung

<130>  200900334

<160>  68

<170>  PatentIn version 3.4

<210>  1
<211>  1575
<212>  DNA
<213>  Candida tropicalis

<220>
<221>  CDS
<222>  (1)..(1575)

<400>  1
atg gcc aca caa gaa att att gat tct gca ctt ccg tac ttg aca aag       48
Met Ala Thr Gln Glu Ile Ile Asp Ser Ala Leu Pro Tyr Leu Thr Lys
1               5                   10                  15

tgg tat act gtt atc act tta gca gct ttg gtt ttc tta att tca tct       96
Trp Tyr Thr Val Ile Thr Leu Ala Ala Leu Val Phe Leu Ile Ser Ser
            20                  25                  30

aat att aaa aat tac gtc aag gct aag aag ttg aaa tgc aga gat cct      144
Asn Ile Lys Asn Tyr Val Lys Ala Lys Lys Leu Lys Cys Arg Asp Pro
        35                  40                  45

cca tat ttc aaa gga gcc ggt tgg aca ggt att agt cca tta att gaa      192
Pro Tyr Phe Lys Gly Ala Gly Trp Thr Gly Ile Ser Pro Leu Ile Glu
    50                  55                  60

att att aaa gtt aaa ggt aat ggt aga ttg gca aga ttt tgg ccg ata      240
Ile Ile Lys Val Lys Gly Asn Gly Arg Leu Ala Arg Phe Trp Pro Ile
65                  70                  75                  80

aaa aca ttc gac gac tat cca aac cat act ttt tac atg tct att att      288
Lys Thr Phe Asp Asp Tyr Pro Asn His Thr Phe Tyr Met Ser Ile Ile
                85                  90                  95

ggt gct ttg aaa atc gtc ttg act gtg atc caa gaa aat att aaa gct      336
Gly Ala Leu Lys Ile Val Leu Thr Val Ile Gln Glu Asn Ile Lys Ala
            100                 105                 110

gtt ttg gct act caa ttt act gat ttc tcc tta ggt act aga cat gcc      384
Val Leu Ala Thr Gln Phe Thr Asp Phe Ser Leu Gly Thr Arg His Ala
        115                 120                 125

cat ttc tat cca tta tta ggt gat ggt att ttt act ttg gat ggt gaa      432
His Phe Tyr Pro Leu Leu Gly Asp Gly Ile Phe Thr Leu Asp Gly Glu
    130                 135                 140

ggt tgg aaa cat agt aga gct atg ttg aga cca caa ttt gct aga gat      480
Gly Trp Lys His Ser Arg Ala Met Leu Arg Pro Gln Phe Ala Arg Asp
145                 150                 155                 160

caa att ggt cat gtt aaa gct ttg gaa cca cat att caa atc ttg gct      528

Gln Ile Gly His Val Lys Ala Leu Glu Pro His Ile Gln Ile Leu Ala
                165                 170                 175

aaa caa atc aaa ttg aat aaa ggt aaa act ttt gat att caa gaa ttg      576
Lys Gln Ile Lys Leu Asn Lys Gly Lys Thr Phe Asp Ile Gln Glu Leu
            180                 185                 190

ttt ttc aga ttt act gtt gat act gct act gaa ttc ttg ttt ggt gaa      624
Phe Phe Arg Phe Thr Val Asp Thr Ala Thr Glu Phe Leu Phe Gly Glu
        195                 200                 205

tct gtt cac tct ttg tat gat gaa aaa tta ggt att cct act cca aat      672
Ser Val His Ser Leu Tyr Asp Glu Lys Leu Gly Ile Pro Thr Pro Asn
    210                 215                 220

gaa att cca ggt aga gat aat ttt gca act gct ttt aac act tct caa      720
Glu Ile Pro Gly Arg Asp Asn Phe Ala Thr Ala Phe Asn Thr Ser Gln
225                 230                 235                 240

cat tat ttg gct acc aga aca tac tcc caa act ttc tac ttt tta act      768
His Tyr Leu Ala Thr Arg Thr Tyr Ser Gln Thr Phe Tyr Phe Leu Thr
                245                 250                 255

aac cct aag gaa ttt aga gac tgt aat gct aaa gtt cat tac ttg gct      816
Asn Pro Lys Glu Phe Arg Asp Cys Asn Ala Lys Val His Tyr Leu Ala
            260                 265                 270

aaa tat ttt gtc aat aaa gct ttg aat ttc act ccg gaa gaa att gaa      864
Lys Tyr Phe Val Asn Lys Ala Leu Asn Phe Thr Pro Glu Glu Ile Glu
        275                 280                 285

gaa aag tcc aaa tct ggt tat gtt ttc ttg tat gaa ttg gtt aaa caa      912
Glu Lys Ser Lys Ser Gly Tyr Val Phe Leu Tyr Glu Leu Val Lys Gln
    290                 295                 300

acc aga gat cca aaa gtt tta caa gat caa tta ttg aac att atg gtt      960
Thr Arg Asp Pro Lys Val Leu Gln Asp Gln Leu Leu Asn Ile Met Val
305                 310                 315                 320

gcc ggt aga gat acc act gct ggt tta tta tca ttt gca atg ttt gaa     1008
Ala Gly Arg Asp Thr Thr Ala Gly Leu Leu Ser Phe Ala Met Phe Glu
                325                 330                 335

tta gct aga cat cca gaa att tgg tct aaa tta aga gaa gaa att gaa     1056
Leu Ala Arg His Pro Glu Ile Trp Ser Lys Leu Arg Glu Glu Ile Glu
            340                 345                 350

gtt aac ttt ggt gtt ggt gaa gaa tct cgt gtt gaa gaa att act ttt     1104
Val Asn Phe Gly Val Gly Glu Glu Ser Arg Val Glu Glu Ile Thr Phe
        355                 360                 365

gaa tct ttg aag aga tgt gaa tac ttg aaa gct att ctt aat gaa act     1152
Glu Ser Leu Lys Arg Cys Glu Tyr Leu Lys Ala Ile Leu Asn Glu Thr
    370                 375                 380

ttg cgt atg tat cct tct gtt cca gtt aat tcc aga aca gcc act aga     1200
Leu Arg Met Tyr Pro Ser Val Pro Val Asn Ser Arg Thr Ala Thr Arg
385                 390                 395                 400

gat acc aca tta cca aga ggt ggt ggt cca aat ggt act gat cca att     1248
Asp Thr Thr Leu Pro Arg Gly Gly Gly Pro Asn Gly Thr Asp Pro Ile
                405                 410                 415

ttt att cca aag ggt tcc act gtt gct tat att gtt tac aaa act cat     1296

Phe Ile Pro Lys Gly Ser Thr Val Ala Tyr Ile Val Tyr Lys Thr His
            420                 425                 430

cgt tta gaa gaa tat tat ggt aaa gat gct gat gat ttc aga cca gaa      1344
Arg Leu Glu Glu Tyr Tyr Gly Lys Asp Ala Asp Asp Phe Arg Pro Glu
        435                 440                 445

aga tgg ttt gaa cca tca act aaa aag tta ggt tgg gct tat gtt cca      1392
Arg Trp Phe Glu Pro Ser Thr Lys Lys Leu Gly Trp Ala Tyr Val Pro
    450                 455                 460

ttt aat ggt ggt cca aga att tgt tta ggc caa caa ttt gct tta act      1440
Phe Asn Gly Gly Pro Arg Ile Cys Leu Gly Gln Gln Phe Ala Leu Thr
465                 470                 475                 480

gaa gct tct tat gtt att acc aga ttg gta caa atg ttt gaa act gtt      1488
Glu Ala Ser Tyr Val Ile Thr Arg Leu Val Gln Met Phe Glu Thr Val
                485                 490                 495

tct tct ccc cca gat gtt gaa tac cct cca cca aaa tgt att cat ttg      1536
Ser Ser Pro Pro Asp Val Glu Tyr Pro Pro Pro Lys Cys Ile His Leu
            500                 505                 510

act atg agt cat gat gat ggt gtt ttc gtt aaa atg taa                  1575
Thr Met Ser His Asp Asp Gly Val Phe Val Lys Met
        515                 520


<210>  2
<211>  524
<212>  PRT
<213>  Candida tropicalis

<400>  2

Met Ala Thr Gln Glu Ile Ile Asp Ser Ala Leu Pro Tyr Leu Thr Lys
1               5                   10                  15


Trp Tyr Thr Val Ile Thr Leu Ala Ala Leu Val Phe Leu Ile Ser Ser
            20                  25                  30


Asn Ile Lys Asn Tyr Val Lys Ala Lys Lys Leu Lys Cys Arg Asp Pro
        35                  40                  45


Pro Tyr Phe Lys Gly Ala Gly Trp Thr Gly Ile Ser Pro Leu Ile Glu
    50                  55                  60


Ile Ile Lys Val Lys Gly Asn Gly Arg Leu Ala Arg Phe Trp Pro Ile
65                  70                  75                  80


Lys Thr Phe Asp Asp Tyr Pro Asn His Thr Phe Tyr Met Ser Ile Ile
                85                  90                  95


Gly Ala Leu Lys Ile Val Leu Thr Val Ile Gln Glu Asn Ile Lys Ala
            100                 105                 110


Val Leu Ala Thr Gln Phe Thr Asp Phe Ser Leu Gly Thr Arg His Ala

115                 120                 125

His Phe Tyr Pro Leu Leu Gly Asp Gly Ile Phe Thr Leu Asp Gly Glu
    130                 135                 140

Gly Trp Lys His Ser Arg Ala Met Leu Arg Pro Gln Phe Ala Arg Asp
145                 150                 155                 160

Gln Ile Gly His Val Lys Ala Leu Glu Pro His Ile Gln Ile Leu Ala
                165                 170                 175

Lys Gln Ile Lys Leu Asn Lys Gly Lys Thr Phe Asp Ile Gln Glu Leu
            180                 185                 190

Phe Phe Arg Phe Thr Val Asp Thr Ala Thr Glu Phe Leu Phe Gly Glu
        195                 200                 205

Ser Val His Ser Leu Tyr Asp Glu Lys Leu Gly Ile Pro Thr Pro Asn
    210                 215                 220

Glu Ile Pro Gly Arg Asp Asn Phe Ala Thr Ala Phe Asn Thr Ser Gln
225                 230                 235                 240

His Tyr Leu Ala Thr Arg Thr Tyr Ser Gln Thr Phe Tyr Phe Leu Thr
                245                 250                 255

Asn Pro Lys Glu Phe Arg Asp Cys Asn Ala Lys Val His Tyr Leu Ala
            260                 265                 270

Lys Tyr Phe Val Asn Lys Ala Leu Asn Phe Thr Pro Glu Glu Ile Glu
        275                 280                 285

Glu Lys Ser Lys Ser Gly Tyr Val Phe Leu Tyr Glu Leu Val Lys Gln
    290                 295                 300

Thr Arg Asp Pro Lys Val Leu Gln Asp Gln Leu Leu Asn Ile Met Val
305                 310                 315                 320

Ala Gly Arg Asp Thr Thr Ala Gly Leu Leu Ser Phe Ala Met Phe Glu
                325                 330                 335

Leu Ala Arg His Pro Glu Ile Trp Ser Lys Leu Arg Glu Glu Ile Glu
            340                 345                 350

Val Asn Phe Gly Val Gly Glu Glu Ser Arg Val Glu Glu Ile Thr Phe
        355                 360                 365

Glu Ser Leu Lys Arg Cys Glu Tyr Leu Lys Ala Ile Leu Asn Glu Thr

370                 375                 380

Leu Arg Met Tyr Pro Ser Val Pro Val Asn Ser Arg Thr Ala Thr Arg
385                 390                 395                 400

Asp Thr Thr Leu Pro Arg Gly Gly Gly Pro Asn Gly Thr Asp Pro Ile
                405                 410                 415

Phe Ile Pro Lys Gly Ser Thr Val Ala Tyr Ile Val Tyr Lys Thr His
            420                 425                 430

Arg Leu Glu Glu Tyr Tyr Gly Lys Asp Ala Asp Asp Phe Arg Pro Glu
        435                 440                 445

Arg Trp Phe Glu Pro Ser Thr Lys Lys Leu Gly Trp Ala Tyr Val Pro
    450                 455                 460

Phe Asn Gly Gly Pro Arg Ile Cys Leu Gly Gln Gln Phe Ala Leu Thr
465                 470                 475                 480

Glu Ala Ser Tyr Val Ile Thr Arg Leu Val Gln Met Phe Glu Thr Val
                485                 490                 495

Ser Ser Pro Pro Asp Val Glu Tyr Pro Pro Pro Lys Cys Ile His Leu
            500                 505                 510

Thr Met Ser His Asp Asp Gly Val Phe Val Lys Met
        515                 520

<210>  3
<211>  1572
<212>  DNA
<213>  Candida tropicalis

<220>
<221>  CDS
<222>  (1)..(1572)

<400>  3
atg gcc aca caa gaa atc atc gat tct gta ctt ccg tac ttg acc aaa       48
Met Ala Thr Gln Glu Ile Ile Asp Ser Val Leu Pro Tyr Leu Thr Lys
1               5                   10                  15

tgg tac act gtg att act gca gca gta tta gtc ttc ctt atc tcc aca       96
Trp Tyr Thr Val Ile Thr Ala Ala Val Leu Val Phe Leu Ile Ser Thr
            20                  25                  30

aac atc aag aac tac gtc aag gca aag aaa ttg aaa tgt gtc gat cca      144
Asn Ile Lys Asn Tyr Val Lys Ala Lys Lys Leu Lys Cys Val Asp Pro
        35                  40                  45

cca tac ttg aag gat gcc ggt ctc act ggt att ctg tct ttg atc gcc      192
Pro Tyr Leu Lys Asp Ala Gly Leu Thr Gly Ile Leu Ser Leu Ile Ala

50                  55                  60

gcc atc aag gcc aag aac gac ggt aga ttg gct aac ttt gcc gat gaa      240
Ala Ile Lys Ala Lys Asn Asp Gly Arg Leu Ala Asn Phe Ala Asp Glu
65                  70                  75                  80

gtt ttc gac gag tac cca aac cac acc ttc tac ttg tct gtt gcc ggt      288
Val Phe Asp Glu Tyr Pro Asn His Thr Phe Tyr Leu Ser Val Ala Gly
                85                  90                  95

gct ttg aag att gtc atg act gtt gac cca gaa aac atc aag gct gtc      336
Ala Leu Lys Ile Val Met Thr Val Asp Pro Glu Asn Ile Lys Ala Val
            100                 105                 110

ttg gcc acc caa ttc act gac ttc tcc ttg ggt acc aga cac gcc cac      384
Leu Ala Thr Gln Phe Thr Asp Phe Ser Leu Gly Thr Arg His Ala His
        115                 120                 125

ttt gct cct ttg ttg ggt gac ggt atc ttc acc ttg gac gga gaa ggt      432
Phe Ala Pro Leu Leu Gly Asp Gly Ile Phe Thr Leu Asp Gly Glu Gly
    130                 135                 140

tgg aag cac tcc aga gct atg ttg aga cca cag ttt gct aga gac cag      480
Trp Lys His Ser Arg Ala Met Leu Arg Pro Gln Phe Ala Arg Asp Gln
145                 150                 155                 160

att gga cac gtt aaa gcc ttg gaa cca cac atc caa atc atg gct aag      528
Ile Gly His Val Lys Ala Leu Glu Pro His Ile Gln Ile Met Ala Lys
                165                 170                 175

cag atc aag ttg aac cag gga aag act ttc gat atc caa gaa ttg ttc      576
Gln Ile Lys Leu Asn Gln Gly Lys Thr Phe Asp Ile Gln Glu Leu Phe
            180                 185                 190

ttt aga ttt acc gtc gac acc gct act gag ttc ttg ttt ggt gaa tcc      624
Phe Arg Phe Thr Val Asp Thr Ala Thr Glu Phe Leu Phe Gly Glu Ser
        195                 200                 205

gtt cac tcc ttg tac gat gaa aaa ttg ggc atc cca act cca aac gaa      672
Val His Ser Leu Tyr Asp Glu Lys Leu Gly Ile Pro Thr Pro Asn Glu
    210                 215                 220

atc cca gga aga gaa aac ttt gcc gct gct ttc aac gtt tcc caa cac      720
Ile Pro Gly Arg Glu Asn Phe Ala Ala Ala Phe Asn Val Ser Gln His
225                 230                 235                 240

tac ttg gcc acc aga agt tac tcc cag act ttt tac ttt ttg acc aac      768
Tyr Leu Ala Thr Arg Ser Tyr Ser Gln Thr Phe Tyr Phe Leu Thr Asn
                245                 250                 255

cct aag gaa ttc aga gac tgt aac gcc aag gtc cac cac ttg gcc aag      816
Pro Lys Glu Phe Arg Asp Cys Asn Ala Lys Val His His Leu Ala Lys
            260                 265                 270

tac ttt gtc aac aag gcc ttg aac ttt act cct gaa gaa ctc gaa gag      864
Tyr Phe Val Asn Lys Ala Leu Asn Phe Thr Pro Glu Glu Leu Glu Glu
        275                 280                 285

aaa tcc aag tcc ggt tac gtt ttc ttg tac gaa ttg gtt aag caa acc      912
Lys Ser Lys Ser Gly Tyr Val Phe Leu Tyr Glu Leu Val Lys Gln Thr
    290                 295                 300

aga gat cca aag gtc ttg caa gat caa ttg ttg aac att atg gtt gcc      960
Arg Asp Pro Lys Val Leu Gln Asp Gln Leu Leu Asn Ile Met Val Ala

305                 310                 315                 320

gga aga gac acc act gcc ggt ttg ttg tcc ttt gct ttg ttt gaa ttg      1008
Gly Arg Asp Thr Thr Ala Gly Leu Leu Ser Phe Ala Leu Phe Glu Leu
                325                 330                 335

gct aga cac cca gag atg tgg tcc aag ttg aga gaa gaa atc gaa gtt      1056
Ala Arg His Pro Glu Met Trp Ser Lys Leu Arg Glu Glu Ile Glu Val
            340                 345                 350

aac ttt ggt gtt ggt gaa gac tcc cgc gtt gaa gaa att acc ttc gaa      1104
Asn Phe Gly Val Gly Glu Asp Ser Arg Val Glu Glu Ile Thr Phe Glu
        355                 360                 365

gcc ttg aag aga tgt gaa tac ttg aag gct atc ctt aac gaa acc ttg      1152
Ala Leu Lys Arg Cys Glu Tyr Leu Lys Ala Ile Leu Asn Glu Thr Leu
    370                 375                 380

cgt atg tac cca tct gtt cct gtc aac ttt aga acc gcc acc aga gac      1200
Arg Met Tyr Pro Ser Val Pro Val Asn Phe Arg Thr Ala Thr Arg Asp
385                 390                 395                 400

acc act ttg cca aga ggt ggt ggt gct aac ggt acc gac cca atc tac      1248
Thr Thr Leu Pro Arg Gly Gly Gly Ala Asn Gly Thr Asp Pro Ile Tyr
                405                 410                 415

att cct aaa ggc tcc act gtt gct tac gtt gtc tac aag acc cac cgt      1296
Ile Pro Lys Gly Ser Thr Val Ala Tyr Val Val Tyr Lys Thr His Arg
            420                 425                 430

ttg gaa gaa tac tac ggt aag gac gct aac gac ttc aga cca gaa aga      1344
Leu Glu Glu Tyr Tyr Gly Lys Asp Ala Asn Asp Phe Arg Pro Glu Arg
        435                 440                 445

tgg ttt gaa cca tct act aag aag ttg ggc tgg gct tat gtt cca ttc      1392
Trp Phe Glu Pro Ser Thr Lys Lys Leu Gly Trp Ala Tyr Val Pro Phe
    450                 455                 460

aac ggt ggt cca aga gtc tgc ttg ggt caa caa ttc gcc ttg act gaa      1440
Asn Gly Gly Pro Arg Val Cys Leu Gly Gln Gln Phe Ala Leu Thr Glu
465                 470                 475                 480

gct tct tat gtg atc act aga ttg gcc cag atg ttt gaa act gtc tca      1488
Ala Ser Tyr Val Ile Thr Arg Leu Ala Gln Met Phe Glu Thr Val Ser
                485                 490                 495

tct gat cca ggt ctc gaa tac cct cca cca aag tgt att cac ttg acc      1536
Ser Asp Pro Gly Leu Glu Tyr Pro Pro Pro Lys Cys Ile His Leu Thr
            500                 505                 510

atg agt cac aac gat ggt gtc ttt gtc aag atg taa                      1572
Met Ser His Asn Asp Gly Val Phe Val Lys Met
        515                 520


<210>  4
<211>  523
<212>  PRT
<213>  Candida tropicalis

<400>  4

Met Ala Thr Gln Glu Ile Ile Asp Ser Val Leu Pro Tyr Leu Thr Lys
1               5                   10                  15

Trp Tyr Thr Val Ile Thr Ala Ala Val Leu Val Phe Leu Ile Ser Thr
            20                  25                  30

Asn Ile Lys Asn Tyr Val Lys Ala Lys Lys Leu Lys Cys Val Asp Pro
        35                  40                  45

Pro Tyr Leu Lys Asp Ala Gly Leu Thr Gly Ile Leu Ser Leu Ile Ala
    50                  55                  60

Ala Ile Lys Ala Lys Asn Asp Gly Arg Leu Ala Asn Phe Ala Asp Glu
65                  70                  75                  80

Val Phe Asp Glu Tyr Pro Asn His Thr Phe Tyr Leu Ser Val Ala Gly
                85                  90                  95

Ala Leu Lys Ile Val Met Thr Val Asp Pro Glu Asn Ile Lys Ala Val
            100                 105                 110

Leu Ala Thr Gln Phe Thr Asp Phe Ser Leu Gly Thr Arg His Ala His
        115                 120                 125

Phe Ala Pro Leu Leu Gly Asp Gly Ile Phe Thr Leu Asp Gly Glu Gly
    130                 135                 140

Trp Lys His Ser Arg Ala Met Leu Arg Pro Gln Phe Ala Arg Asp Gln
145                 150                 155                 160

Ile Gly His Val Lys Ala Leu Glu Pro His Ile Gln Ile Met Ala Lys
                165                 170                 175

Gln Ile Lys Leu Asn Gln Gly Lys Thr Phe Asp Ile Gln Glu Leu Phe
            180                 185                 190

Phe Arg Phe Thr Val Asp Thr Ala Thr Glu Phe Leu Phe Gly Glu Ser
        195                 200                 205

Val His Ser Leu Tyr Asp Glu Lys Leu Gly Ile Pro Thr Pro Asn Glu
    210                 215                 220

Ile Pro Gly Arg Glu Asn Phe Ala Ala Ala Phe Asn Val Ser Gln His
225                 230                 235                 240

Tyr Leu Ala Thr Arg Ser Tyr Ser Gln Thr Phe Tyr Phe Leu Thr Asn
                245                 250                 255

Pro Lys Glu Phe Arg Asp Cys Asn Ala Lys Val His His Leu Ala Lys
            260                 265                 270

Tyr Phe Val Asn Lys Ala Leu Asn Phe Thr Pro Glu Glu Leu Glu Glu
        275                 280                 285

Lys Ser Lys Ser Gly Tyr Val Phe Leu Tyr Glu Leu Val Lys Gln Thr
    290                 295                 300

Arg Asp Pro Lys Val Leu Gln Asp Gln Leu Leu Asn Ile Met Val Ala
305                 310                 315                 320

Gly Arg Asp Thr Thr Ala Gly Leu Leu Ser Phe Ala Leu Phe Glu Leu
                325                 330                 335

Ala Arg His Pro Glu Met Trp Ser Lys Leu Arg Glu Glu Ile Glu Val
            340                 345                 350

Asn Phe Gly Val Gly Glu Asp Ser Arg Val Glu Glu Ile Thr Phe Glu
        355                 360                 365

Ala Leu Lys Arg Cys Glu Tyr Leu Lys Ala Ile Leu Asn Glu Thr Leu
    370                 375                 380

Arg Met Tyr Pro Ser Val Pro Val Asn Phe Arg Thr Ala Thr Arg Asp
385                 390                 395                 400

Thr Thr Leu Pro Arg Gly Gly Gly Ala Asn Gly Thr Asp Pro Ile Tyr
                405                 410                 415

Ile Pro Lys Gly Ser Thr Val Ala Tyr Val Val Tyr Lys Thr His Arg
            420                 425                 430

Leu Glu Glu Tyr Tyr Gly Lys Asp Ala Asn Asp Phe Arg Pro Glu Arg
        435                 440                 445

Trp Phe Glu Pro Ser Thr Lys Lys Leu Gly Trp Ala Tyr Val Pro Phe
    450                 455                 460

Asn Gly Gly Pro Arg Val Cys Leu Gly Gln Gln Phe Ala Leu Thr Glu
465                 470                 475                 480

Ala Ser Tyr Val Ile Thr Arg Leu Ala Gln Met Phe Glu Thr Val Ser
                485                 490                 495

Ser Asp Pro Gly Leu Glu Tyr Pro Pro Pro Lys Cys Ile His Leu Thr
            500                 505                 510

Met Ser His Asn Asp Gly Val Phe Val Lys Met
        515                 520

<210>  5
<211>  1569
<212>  DNA
<213>  Candida tropicalis

<220>
<221>  CDS
<222>  (1)..(1569)

<400>  5
atg act gta cac gat att atc gcc aca tac ttc acc aaa tgg tac gtg       48
Met Thr Val His Asp Ile Ile Ala Thr Tyr Phe Thr Lys Trp Tyr Val
1               5                   10                  15

ata gta cca ctc gct ttg att gct tat aga gtc ctc gac tac ttc tat       96
Ile Val Pro Leu Ala Leu Ile Ala Tyr Arg Val Leu Asp Tyr Phe Tyr
            20                  25                  30

ggc aga tac ttg atg tac aag ctt ggt gct aaa cca ttt ttc cag aaa      144
Gly Arg Tyr Leu Met Tyr Lys Leu Gly Ala Lys Pro Phe Phe Gln Lys
        35                  40                  45

cag aca gac ggc tgt ttc gga ttc aaa gct ccg ctt gaa ttg ttg aag      192
Gln Thr Asp Gly Cys Phe Gly Phe Lys Ala Pro Leu Glu Leu Leu Lys
    50                  55                  60

aag aag agc gac ggt acc ctc ata gac ttc aca ctc cag cgt atc cac      240
Lys Lys Ser Asp Gly Thr Leu Ile Asp Phe Thr Leu Gln Arg Ile His
65                  70                  75                  80

gat ctc gat cgt ccc gat atc cca act ttc aca ttc ccg gtc ttt tcc      288
Asp Leu Asp Arg Pro Asp Ile Pro Thr Phe Thr Phe Pro Val Phe Ser
                85                  90                  95

atc aac ctt gtc aat acc ctt gag ccg gag aac atc aag gcc atc ttg      336
Ile Asn Leu Val Asn Thr Leu Glu Pro Glu Asn Ile Lys Ala Ile Leu
            100                 105                 110

gcc act cag ttc aac gat ttc tcc ttg ggt acc aga cac tcg cac ttt      384
Ala Thr Gln Phe Asn Asp Phe Ser Leu Gly Thr Arg His Ser His Phe
        115                 120                 125

gct cct ttg ttg ggt gat ggt atc ttt acg ttg gat ggc gcc ggc tgg      432
Ala Pro Leu Leu Gly Asp Gly Ile Phe Thr Leu Asp Gly Ala Gly Trp
    130                 135                 140

aag cac agc aga tct atg ttg aga cca cag ttt gcc aga gaa cag att      480
Lys His Ser Arg Ser Met Leu Arg Pro Gln Phe Ala Arg Glu Gln Ile
145                 150                 155                 160

tcc cac gtc aag ttg ttg gag cca cac gtt cag gtg ttc ttc aaa cac      528
Ser His Val Lys Leu Leu Glu Pro His Val Gln Val Phe Phe Lys His
                165                 170                 175

gtc aga aag gca cag ggc aag act ttt gac atc cag gaa ttg ttt ttc      576
Val Arg Lys Ala Gln Gly Lys Thr Phe Asp Ile Gln Glu Leu Phe Phe
            180                 185                 190

aga ttg acc gtc gac tcc gcc acc gag ttt ttg ttt ggt gaa tcc gtt      624
Arg Leu Thr Val Asp Ser Ala Thr Glu Phe Leu Phe Gly Glu Ser Val
        195                 200                 205

gag tcc ttg aga gat gaa tct atc ggc atg tcc atc aat gcg ctt gac 672
Glu Ser Leu Arg Asp Glu Ser Ile Gly Met Ser Ile Asn Ala Leu Asp
    210                 215                 220

ttt gac ggc aag gct ggc ttt gct gat gct ttt aac tat tcg cag aat 720
Phe Asp Gly Lys Ala Gly Phe Ala Asp Ala Phe Asn Tyr Ser Gln Asn
225                 230                 235                 240

tat ttg gct tcg aga gcg gtt atg caa caa ttg tac tgg gtg ttg aac 768
Tyr Leu Ala Ser Arg Ala Val Met Gln Gln Leu Tyr Trp Val Leu Asn
                245                 250                 255

ggg aaa aag ttt aag gag tgc aac gct aaa gtg cac aag ttt gct gac 816
Gly Lys Lys Phe Lys Glu Cys Asn Ala Lys Val His Lys Phe Ala Asp
            260                 265                 270

tac tac gtc aac aag gct ttg gac ttg acg cct gaa caa ttg gaa aag 864
Tyr Tyr Val Asn Lys Ala Leu Asp Leu Thr Pro Glu Gln Leu Glu Lys
        275                 280                 285

cag gat ggt tat gtg ttt ttg tac gaa ttg gtc aag caa acc aga gac 912
Gln Asp Gly Tyr Val Phe Leu Tyr Glu Leu Val Lys Gln Thr Arg Asp
    290                 295                 300

aag caa gtg ttg aga gac caa ttg ttg aac atc atg gtt gct ggt aga 960
Lys Gln Val Leu Arg Asp Gln Leu Leu Asn Ile Met Val Ala Gly Arg
305                 310                 315                 320

gac acc acc gcc ggt ttg ttg tcg ttt gtt ttc ttt gaa ttg gcc aga 1008
Asp Thr Thr Ala Gly Leu Leu Ser Phe Val Phe Phe Glu Leu Ala Arg
                325                 330                 335

aac cca gaa gtt acc aac aag ttg aga gaa gaa att gag gac aag ttt 1056
Asn Pro Glu Val Thr Asn Lys Leu Arg Glu Glu Ile Glu Asp Lys Phe
            340                 345                 350

gga ctc ggt gag aat gct agt gtt gaa gac att tcc ttt gag tcg ttg 1104
Gly Leu Gly Glu Asn Ala Ser Val Glu Asp Ile Ser Phe Glu Ser Leu
        355                 360                 365

aag tcc tgt gaa tac ttg aag gct gtt ctc aac gaa acc ttg aga ttg 1152
Lys Ser Cys Glu Tyr Leu Lys Ala Val Leu Asn Glu Thr Leu Arg Leu
    370                 375                 380

tac cca tcc gtg cca cag aat ttc aga gtt gcc acc aag aac act acc 1200
Tyr Pro Ser Val Pro Gln Asn Phe Arg Val Ala Thr Lys Asn Thr Thr
385                 390                 395                 400

ctc cca aga ggt ggt ggt aag gac ggg ttg tct cct gtt ttg gtg aga 1248
Leu Pro Arg Gly Gly Gly Lys Asp Gly Leu Ser Pro Val Leu Val Arg
                405                 410                 415

aag ggt cag acc gtt att tac ggt gtc tac gca gcc cac aga aac cca 1296
Lys Gly Gln Thr Val Ile Tyr Gly Val Tyr Ala Ala His Arg Asn Pro
            420                 425                 430

gct gtt tac ggt aag gac gct ctt gag ttt aga cca gag aga tgg ttt 1344
Ala Val Tyr Gly Lys Asp Ala Leu Glu Phe Arg Pro Glu Arg Trp Phe
        435                 440                 445

gag cca gag aca aag aag ctt ggc tgg gcc ttc ctc cca ttc aac ggt 1392
Glu Pro Glu Thr Lys Lys Leu Gly Trp Ala Phe Leu Pro Phe Asn Gly
    450                 455                 460

ggt cca aga atc tgt ttg gga cag cag ttt gcc ttg aca gaa gct tcg      1440
Gly Pro Arg Ile Cys Leu Gly Gln Gln Phe Ala Leu Thr Glu Ala Ser
465                 470                 475                 480

tat gtc act gtc agg ttg ctc cag gag ttt gca cac ttg tct atg gac      1488
Tyr Val Thr Val Arg Leu Leu Gln Glu Phe Ala His Leu Ser Met Asp
                485                 490                 495

cca gac acc gaa tat cca cct aag aaa atg tcg cat ttg acc atg tcg      1536
Pro Asp Thr Glu Tyr Pro Pro Lys Lys Met Ser His Leu Thr Met Ser
            500                 505                 510

ctt ttc gac ggt gcc aat att gag atg tat tag                          1569
Leu Phe Asp Gly Ala Asn Ile Glu Met Tyr
        515                 520

<210>  6
<211>  522
<212>  PRT
<213>  Candida tropicalis

<400>  6

Met Thr Val His Asp Ile Ile Ala Thr Tyr Phe Thr Lys Trp Tyr Val
1               5                   10                  15

Ile Val Pro Leu Ala Leu Ile Ala Tyr Arg Val Leu Asp Tyr Phe Tyr
            20                  25                  30

Gly Arg Tyr Leu Met Tyr Lys Leu Gly Ala Lys Pro Phe Phe Gln Lys
        35                  40                  45

Gln Thr Asp Gly Cys Phe Gly Phe Lys Ala Pro Leu Glu Leu Leu Lys
    50                  55                  60

Lys Lys Ser Asp Gly Thr Leu Ile Asp Phe Thr Leu Gln Arg Ile His
65                  70                  75                  80

Asp Leu Asp Arg Pro Asp Ile Pro Thr Phe Thr Phe Pro Val Phe Ser
                85                  90                  95

Ile Asn Leu Val Asn Thr Leu Glu Pro Glu Asn Ile Lys Ala Ile Leu
            100                 105                 110

Ala Thr Gln Phe Asn Asp Phe Ser Leu Gly Thr Arg His Ser His Phe
        115                 120                 125

Ala Pro Leu Leu Gly Asp Gly Ile Phe Thr Leu Asp Gly Ala Gly Trp
    130                 135                 140

Lys His Ser Arg Ser Met Leu Arg Pro Gln Phe Ala Arg Glu Gln Ile
145                 150                 155                 160

165 170 175

Val Arg Lys Ala Gln Gly Lys Thr Phe Asp Ile Gln Glu Leu Phe Phe
180 185 190

Arg Leu Thr Val Asp Ser Ala Thr Glu Phe Leu Phe Gly Glu Ser Val
195 200 205

Glu Ser Leu Arg Asp Glu Ser Ile Gly Met Ser Ile Asn Ala Leu Asp
210 215 220

Phe Asp Gly Lys Ala Gly Phe Ala Asp Ala Phe Asn Tyr Ser Gln Asn
225 230 235 240

Tyr Leu Ala Ser Arg Ala Val Met Gln Gln Leu Tyr Trp Val Leu Asn
245 250 255

Gly Lys Lys Phe Lys Glu Cys Asn Ala Lys Val His Lys Phe Ala Asp
260 265 270

Tyr Tyr Val Asn Lys Ala Leu Asp Leu Thr Pro Glu Gln Leu Glu Lys
275 280 285

Gln Asp Gly Tyr Val Phe Leu Tyr Glu Leu Val Lys Gln Thr Arg Asp
290 295 300

Lys Gln Val Leu Arg Asp Gln Leu Leu Asn Ile Met Val Ala Gly Arg
305 310 315 320

Asp Thr Thr Ala Gly Leu Leu Ser Phe Val Phe Phe Glu Leu Ala Arg
325 330 335

Asn Pro Glu Val Thr Asn Lys Leu Arg Glu Glu Ile Glu Asp Lys Phe
340 345 350

Gly Leu Gly Glu Asn Ala Ser Val Glu Asp Ile Ser Phe Glu Ser Leu
355 360 365

Lys Ser Cys Glu Tyr Leu Lys Ala Val Leu Asn Glu Thr Leu Arg Leu
370 375 380

Tyr Pro Ser Val Pro Gln Asn Phe Arg Val Ala Thr Lys Asn Thr Thr
385 390 395 400

Leu Pro Arg Gly Gly Gly Lys Asp Gly Leu Ser Pro Val Leu Val Arg
405 410 415

Lys Gly Gln Thr Val Ile Tyr Gly Val Tyr Ala Ala His Arg Asn Pro

420 425 430

Ala Val Tyr Gly Lys Asp Ala Leu Glu Phe Arg Pro Glu Arg Trp Phe
435 440 445

Glu Pro Glu Thr Lys Lys Leu Gly Trp Ala Phe Leu Pro Phe Asn Gly
450 455 460

Gly Pro Arg Ile Cys Leu Gly Gln Gln Phe Ala Leu Thr Glu Ala Ser
465 470 475 480

Tyr Val Thr Val Arg Leu Leu Gln Glu Phe Ala His Leu Ser Met Asp
485 490 495

Pro Asp Thr Glu Tyr Pro Pro Lys Lys Met Ser His Leu Thr Met Ser
500 505 510

Leu Phe Asp Gly Ala Asn Ile Glu Met Tyr
515 520

<210> 7
<211> 1569
<212> DNA
<213> Candida tropicalis

<220>
<221> CDS
<222> (1)..(1569)

<400> 7
atg act gca cag gat att atc gcc aca tac atc acc aaa tgg tac gtg 48
Met Thr Ala Gln Asp Ile Ile Ala Thr Tyr Ile Thr Lys Trp Tyr Val
1               5                   10                  15

ata gta cca ctc gct ttg att gct tat agg gtc ctc gac tac ttt tac 96
Ile Val Pro Leu Ala Leu Ile Ala Tyr Arg Val Leu Asp Tyr Phe Tyr
            20                  25                  30

ggc aga tac ttg atg tac aag ctt ggt gct aaa ccg ttt ttc cag aaa 144
Gly Arg Tyr Leu Met Tyr Lys Leu Gly Ala Lys Pro Phe Phe Gln Lys
        35                  40                  45

caa aca gac ggt tat ttc gga ttc aaa gct cca ctt gaa ttg tta aaa 192
Gln Thr Asp Gly Tyr Phe Gly Phe Lys Ala Pro Leu Glu Leu Leu Lys
    50                  55                  60

aag aag agt gac ggt acc ctc ata gac ttc act ctc gag cgt atc caa 240
Lys Lys Ser Asp Gly Thr Leu Ile Asp Phe Thr Leu Glu Arg Ile Gln
65                  70                  75                  80

gcg ctc aat cgt cca gat atc cca act ttt aca ttc cca atc ttt tcc 288
Ala Leu Asn Arg Pro Asp Ile Pro Thr Phe Thr Phe Pro Ile Phe Ser
                85                  90                  95

atc aac ctt atc agc acc ctt gag ccg gag aac atc aag gct atc ttg 336
Ile Asn Leu Ile Ser Thr Leu Glu Pro Glu Asn Ile Lys Ala Ile Leu

                100                 105                 110

gcc acc cag ttc aac gat ttc tcc ttg ggc acc aga cac tcg cac ttt       384
Ala Thr Gln Phe Asn Asp Phe Ser Leu Gly Thr Arg His Ser His Phe
        115                 120                 125

gct cct ttg ttg ggc gat ggt atc ttt acc ttg gac ggt gcc ggc tgg       432
Ala Pro Leu Leu Gly Asp Gly Ile Phe Thr Leu Asp Gly Ala Gly Trp
    130                 135                 140

aag cac agc aga tct atg ttg aga cca cag ttt gcc aga gaa cag att       480
Lys His Ser Arg Ser Met Leu Arg Pro Gln Phe Ala Arg Glu Gln Ile
145                 150                 155                 160

tcc cac gtc aag ttg ttg gag cca cac atg cag gtg ttc ttc aag cac       528
Ser His Val Lys Leu Leu Glu Pro His Met Gln Val Phe Phe Lys His
                165                 170                 175

gtc aga aag gca cag ggc aag act ttt gac atc caa gaa ttg ttt ttc       576
Val Arg Lys Ala Gln Gly Lys Thr Phe Asp Ile Gln Glu Leu Phe Phe
            180                 185                 190

aga ttg acc gtc gac tcc gcc act gag ttt ttg ttt ggt gaa tcc gtt       624
Arg Leu Thr Val Asp Ser Ala Thr Glu Phe Leu Phe Gly Glu Ser Val
        195                 200                 205

gag tcc ttg aga gat gaa tct att ggg atg tcc atc aat gca ctt gac       672
Glu Ser Leu Arg Asp Glu Ser Ile Gly Met Ser Ile Asn Ala Leu Asp
    210                 215                 220

ttt gac ggc aag gct ggc ttt gct gat gct ttt aac tac tcg cag aac       720
Phe Asp Gly Lys Ala Gly Phe Ala Asp Ala Phe Asn Tyr Ser Gln Asn
225                 230                 235                 240

tat ttg gct tcg aga gcg gtt atg caa caa ttg tac tgg gtg ttg aac       768
Tyr Leu Ala Ser Arg Ala Val Met Gln Gln Leu Tyr Trp Val Leu Asn
                245                 250                 255

ggg aaa aag ttt aag gag tgc aac gct aaa gtg cac aag ttt gct gac       816
Gly Lys Lys Phe Lys Glu Cys Asn Ala Lys Val His Lys Phe Ala Asp
            260                 265                 270

tat tac gtc agc aag gct ttg gac ttg aca cct gaa caa ttg gaa aag       864
Tyr Tyr Val Ser Lys Ala Leu Asp Leu Thr Pro Glu Gln Leu Glu Lys
        275                 280                 285

cag gat ggt tat gtg ttc ttg tac gag ttg gtc aag caa acc aga gac       912
Gln Asp Gly Tyr Val Phe Leu Tyr Glu Leu Val Lys Gln Thr Arg Asp
    290                 295                 300

agg caa gtg ttg aga gac cag ttg ttg aac atc atg gtt gcc ggt aga       960
Arg Gln Val Leu Arg Asp Gln Leu Leu Asn Ile Met Val Ala Gly Arg
305                 310                 315                 320

gac acc acc gcc ggt ttg ttg tcg ttt gtt ttc ttt gaa ttg gcc aga      1008
Asp Thr Thr Ala Gly Leu Leu Ser Phe Val Phe Phe Glu Leu Ala Arg
                325                 330                 335

aac cca gag gtg acc aac aag ttg aga gaa gaa atc gag gac aag ttt      1056
Asn Pro Glu Val Thr Asn Lys Leu Arg Glu Glu Ile Glu Asp Lys Phe
            340                 345                 350

ggt ctt ggt gag aat gct cgt gtt gaa gac att tcc ttt gag tcg ttg      1104
Gly Leu Gly Glu Asn Ala Arg Val Glu Asp Ile Ser Phe Glu Ser Leu

355                 360                 365

aag tca tgt gaa tac ttg aag gct gtt ctc aac gaa act ttg aga ttg      1152
Lys Ser Cys Glu Tyr Leu Lys Ala Val Leu Asn Glu Thr Leu Arg Leu
    370                 375                 380

tac cca tcc gtg cca cag aat ttc aga gtt gcc acc aaa aac act acc      1200
Tyr Pro Ser Val Pro Gln Asn Phe Arg Val Ala Thr Lys Asn Thr Thr
385                 390                 395                 400

ctt cca agg gga ggt ggt aag gac ggg tta tct cct gtt ttg gtc aga      1248
Leu Pro Arg Gly Gly Gly Lys Asp Gly Leu Ser Pro Val Leu Val Arg
                405                 410                 415

aag ggt caa acc gtt atg tac ggt gtc tac gct gcc cac aga aac cca      1296
Lys Gly Gln Thr Val Met Tyr Gly Val Tyr Ala Ala His Arg Asn Pro
            420                 425                 430

gct gtc tac ggt aag gac gcc ctt gag ttt aga cca gag agg tgg ttt      1344
Ala Val Tyr Gly Lys Asp Ala Leu Glu Phe Arg Pro Glu Arg Trp Phe
        435                 440                 445

gag cca gag aca aag aag ctt ggc tgg gcc ttc ctt cca ttc aac ggt      1392
Glu Pro Glu Thr Lys Lys Leu Gly Trp Ala Phe Leu Pro Phe Asn Gly
    450                 455                 460

ggt cca aga att tgc ttg gga cag cag ttt gcc ttg aca gaa gct tcg      1440
Gly Pro Arg Ile Cys Leu Gly Gln Gln Phe Ala Leu Thr Glu Ala Ser
465                 470                 475                 480

tat gtc act gtc aga ttg ctc caa gag ttt gga cac ttg tct atg gac      1488
Tyr Val Thr Val Arg Leu Leu Gln Glu Phe Gly His Leu Ser Met Asp
                485                 490                 495

ccc aac acc gaa tat cca cct agg aaa atg tcg cat ttg acc atg tcc      1536
Pro Asn Thr Glu Tyr Pro Pro Arg Lys Met Ser His Leu Thr Met Ser
            500                 505                 510

ctt ttc gac ggt gcc aac att gag atg tat tag                          1569
Leu Phe Asp Gly Ala Asn Ile Glu Met Tyr
        515                 520


<210>  8
<211>  522
<212>  PRT
<213>  Candida tropicalis

<400>  8

Met Thr Ala Gln Asp Ile Ile Ala Thr Tyr Ile Thr Lys Trp Tyr Val
1               5                   10                  15


Ile Val Pro Leu Ala Leu Ile Ala Tyr Arg Val Leu Asp Tyr Phe Tyr
            20                  25                  30


Gly Arg Tyr Leu Met Tyr Lys Leu Gly Ala Lys Pro Phe Phe Gln Lys
        35                  40                  45


Gln Thr Asp Gly Tyr Phe Gly Phe Lys Ala Pro Leu Glu Leu Leu Lys
    50                  55                  60

Lys Lys Ser Asp Gly Thr Leu Ile Asp Phe Thr Leu Glu Arg Ile Gln
65                  70                  75                  80

Ala Leu Asn Arg Pro Asp Ile Pro Thr Phe Thr Phe Pro Ile Phe Ser
                85                  90                  95

Ile Asn Leu Ile Ser Thr Leu Glu Pro Glu Asn Ile Lys Ala Ile Leu
            100                 105                 110

Ala Thr Gln Phe Asn Asp Phe Ser Leu Gly Thr Arg His Ser His Phe
        115                 120                 125

Ala Pro Leu Leu Gly Asp Gly Ile Phe Thr Leu Asp Gly Ala Gly Trp
    130                 135                 140

Lys His Ser Arg Ser Met Leu Arg Pro Gln Phe Ala Arg Glu Gln Ile
145                 150                 155                 160

Ser His Val Lys Leu Leu Glu Pro His Met Gln Val Phe Phe Lys His
                165                 170                 175

Val Arg Lys Ala Gln Gly Lys Thr Phe Asp Ile Gln Glu Leu Phe Phe
            180                 185                 190

Arg Leu Thr Val Asp Ser Ala Thr Glu Phe Leu Phe Gly Glu Ser Val
        195                 200                 205

Glu Ser Leu Arg Asp Glu Ser Ile Gly Met Ser Ile Asn Ala Leu Asp
    210                 215                 220

Phe Asp Gly Lys Ala Gly Phe Ala Asp Ala Phe Asn Tyr Ser Gln Asn
225                 230                 235                 240

Tyr Leu Ala Ser Arg Ala Val Met Gln Gln Leu Tyr Trp Val Leu Asn
                245                 250                 255

Gly Lys Lys Phe Lys Glu Cys Asn Ala Lys Val His Lys Phe Ala Asp
            260                 265                 270

Tyr Tyr Val Ser Lys Ala Leu Asp Leu Thr Pro Glu Gln Leu Glu Lys
        275                 280                 285

Gln Asp Gly Tyr Val Phe Leu Tyr Glu Leu Val Lys Gln Thr Arg Asp
    290                 295                 300

Arg Gln Val Leu Arg Asp Gln Leu Leu Asn Ile Met Val Ala Gly Arg
305                 310                 315                 320

Asp Thr Thr Ala Gly Leu Leu Ser Phe Val Phe Phe Glu Leu Ala Arg
                325                 330                 335

Asn Pro Glu Val Thr Asn Lys Leu Arg Glu Glu Ile Glu Asp Lys Phe
            340                 345                 350

Gly Leu Gly Glu Asn Ala Arg Val Glu Asp Ile Ser Phe Glu Ser Leu
        355                 360                 365

Lys Ser Cys Glu Tyr Leu Lys Ala Val Leu Asn Glu Thr Leu Arg Leu
    370                 375                 380

Tyr Pro Ser Val Pro Gln Asn Phe Arg Val Ala Thr Lys Asn Thr Thr
385                 390                 395                 400

Leu Pro Arg Gly Gly Gly Lys Asp Gly Leu Ser Pro Val Leu Val Arg
                405                 410                 415

Lys Gly Gln Thr Val Met Tyr Gly Val Tyr Ala Ala His Arg Asn Pro
            420                 425                 430

Ala Val Tyr Gly Lys Asp Ala Leu Glu Phe Arg Pro Glu Arg Trp Phe
        435                 440                 445

Glu Pro Glu Thr Lys Lys Leu Gly Trp Ala Phe Leu Pro Phe Asn Gly
    450                 455                 460

Gly Pro Arg Ile Cys Leu Gly Gln Gln Phe Ala Leu Thr Glu Ala Ser
465                 470                 475                 480

Tyr Val Thr Val Arg Leu Leu Gln Glu Phe Gly His Leu Ser Met Asp
                485                 490                 495

Pro Asn Thr Glu Tyr Pro Pro Arg Lys Met Ser His Leu Thr Met Ser
            500                 505                 510

Leu Phe Asp Gly Ala Asn Ile Glu Met Tyr
        515                 520

<210>  9
<211>  1623
<212>  DNA
<213>  Candida tropicalis

<220>
<221>  CDS
<222>  (1)..(1623)

<400>  9

atg tcg tct tct cca tcg ttt gcc caa gag gtt ctc gct acc act agt 48
Met Ser Ser Ser Pro Ser Phe Ala Gln Glu Val Leu Ala Thr Thr Ser
1               5                   10                  15

cct tac atc gag tac ttt ctt gac aac tac acc aga tgg tac tac ttc 96
Pro Tyr Ile Glu Tyr Phe Leu Asp Asn Tyr Thr Arg Trp Tyr Tyr Phe
            20                  25                  30

ata cct ttg gtg ctt ctt tcg ttg aac ttt ata agt ttg ctc cac aca 144
Ile Pro Leu Val Leu Leu Ser Leu Asn Phe Ile Ser Leu Leu His Thr
        35                  40                  45

agg tac ttg gaa cgc agg ttc cac gcc aag cca ctc ggt aac ttt gtc 192
Arg Tyr Leu Glu Arg Arg Phe His Ala Lys Pro Leu Gly Asn Phe Val
    50                  55                  60

agg gac cct acg ttt ggt atc gct act ccg ttg ctt ttg atc tac ttg 240
Arg Asp Pro Thr Phe Gly Ile Ala Thr Pro Leu Leu Leu Ile Tyr Leu
65                  70                  75                  80

aag tcg aaa ggt acg gtc atg aag ttt gct tgg ggc ctc tgg aac aac 288
Lys Ser Lys Gly Thr Val Met Lys Phe Ala Trp Gly Leu Trp Asn Asn
                85                  90                  95

aag tac atc gtc aga gac cca aag tac aag aca act ggg ctc agg att 336
Lys Tyr Ile Val Arg Asp Pro Lys Tyr Lys Thr Thr Gly Leu Arg Ile
            100                 105                 110

gtt ggc ctc cca ttg att gaa acc atg gac cca gag aac atc aag gct 384
Val Gly Leu Pro Leu Ile Glu Thr Met Asp Pro Glu Asn Ile Lys Ala
        115                 120                 125

gtt ttg gct act cag ttc aat gat ttc tct ttg gga acc aga cac gat 432
Val Leu Ala Thr Gln Phe Asn Asp Phe Ser Leu Gly Thr Arg His Asp
    130                 135                 140

ttc ttg tac tcc ttg ttg ggt gac ggt att ttc acc ttg gac ggt gct 480
Phe Leu Tyr Ser Leu Leu Gly Asp Gly Ile Phe Thr Leu Asp Gly Ala
145                 150                 155                 160

ggc tgg aaa cat agt aga act atg ttg aga cca cag ttt gct aga gaa 528
Gly Trp Lys His Ser Arg Thr Met Leu Arg Pro Gln Phe Ala Arg Glu
                165                 170                 175

cag gtt tct cac gtc aag ttg ttg gag cca cac gtt cag gtg ttc ttc 576
Gln Val Ser His Val Lys Leu Leu Glu Pro His Val Gln Val Phe Phe
            180                 185                 190

aag cac gtt aga aag cac cgc ggt caa acg ttc gac atc caa gaa ttg 624
Lys His Val Arg Lys His Arg Gly Gln Thr Phe Asp Ile Gln Glu Leu
        195                 200                 205

ttc ttc agg ttg acc gtc gac tcc gcc acc gag ttc ttg ttt ggt gag 672
Phe Phe Arg Leu Thr Val Asp Ser Ala Thr Glu Phe Leu Phe Gly Glu
    210                 215                 220

tct gct gaa tcc ttg agg gac gaa tct att gga ttg acc cca acc acc 720
Ser Ala Glu Ser Leu Arg Asp Glu Ser Ile Gly Leu Thr Pro Thr Thr
225                 230                 235                 240

aag gat ttc gat ggc aga aga gat ttc gct gac gct ttc aac tat tcg 768
Lys Asp Phe Asp Gly Arg Arg Asp Phe Ala Asp Ala Phe Asn Tyr Ser
                245                 250                 255

cag act tac cag gcc tac aga ttt ttg ttg caa caa atg tac tgg atc 816

Gln Thr Tyr Gln Ala Tyr Arg Phe Leu Leu Gln Gln Met Tyr Trp Ile
            260                 265                 270

ttg aat ggc tcg gaa ttc aga aag tcg att gct gtc gtg cac aag ttt      864
Leu Asn Gly Ser Glu Phe Arg Lys Ser Ile Ala Val Val His Lys Phe
        275                 280                 285

gct gac cac tat gtg caa aag gct ttg gag ttg acc gac gat gac ttg      912
Ala Asp His Tyr Val Gln Lys Ala Leu Glu Leu Thr Asp Asp Asp Leu
    290                 295                 300

cag aaa caa gac ggc tat gtg ttc ttg tac gag ttg gct aag caa acc      960
Gln Lys Gln Asp Gly Tyr Val Phe Leu Tyr Glu Leu Ala Lys Gln Thr
305                 310                 315                 320

aga gac cca aag gtc ttg aga gac cag tta ttg aac att ttg gtt gcc     1008
Arg Asp Pro Lys Val Leu Arg Asp Gln Leu Leu Asn Ile Leu Val Ala
                325                 330                 335

ggt aga gac acg acc gcc ggt ttg ttg tca ttt gtt ttc tac gag ttg     1056
Gly Arg Asp Thr Thr Ala Gly Leu Leu Ser Phe Val Phe Tyr Glu Leu
            340                 345                 350

tca aga aac cct gag gtg ttt gct aag ttg aga gag gag gtg gaa aac     1104
Ser Arg Asn Pro Glu Val Phe Ala Lys Leu Arg Glu Glu Val Glu Asn
        355                 360                 365

aga ttt gga ctc ggt gaa gaa gct cgt gtt gaa gag atc tcg ttt gag     1152
Arg Phe Gly Leu Gly Glu Glu Ala Arg Val Glu Glu Ile Ser Phe Glu
    370                 375                 380

tcc ttg aag tct tgt gag tac ttg aag gct gtc atc aat gaa acc ttg     1200
Ser Leu Lys Ser Cys Glu Tyr Leu Lys Ala Val Ile Asn Glu Thr Leu
385                 390                 395                 400

aga ttg tac cca tcg gtt cca cac aac ttt aga gtt gct acc aga aac     1248
Arg Leu Tyr Pro Ser Val Pro His Asn Phe Arg Val Ala Thr Arg Asn
                405                 410                 415

act acc ctc cca aga ggt ggt ggt gaa gat gga tac tcg cca att gtc     1296
Thr Thr Leu Pro Arg Gly Gly Gly Glu Asp Gly Tyr Ser Pro Ile Val
            420                 425                 430

gtc aag aag ggt caa gtt gtc atg tac act gtt att gct acc cac aga     1344
Val Lys Lys Gly Gln Val Val Met Tyr Thr Val Ile Ala Thr His Arg
        435                 440                 445

gac cca agt atc tac ggt gcc gac gct gac gtc ttc aga cca gaa aga     1392
Asp Pro Ser Ile Tyr Gly Ala Asp Ala Asp Val Phe Arg Pro Glu Arg
    450                 455                 460

tgg ttt gaa cca gaa act aga aag ttg ggc tgg gca tac gtt cca ttc     1440
Trp Phe Glu Pro Glu Thr Arg Lys Leu Gly Trp Ala Tyr Val Pro Phe
465                 470                 475                 480

aat ggt ggt cca aga atc tgt ttg ggt caa cag ttt gcc ttg acc gaa     1488
Asn Gly Gly Pro Arg Ile Cys Leu Gly Gln Gln Phe Ala Leu Thr Glu
                485                 490                 495

gct tca tac gtc act gtc aga ttg ctc cag gag ttt gca cac ttg tct     1536
Ala Ser Tyr Val Thr Val Arg Leu Leu Gln Glu Phe Ala His Leu Ser
            500                 505                 510

atg gac cca gac acc gaa tat cca cca aaa ttg cag aac acc ttg acc     1584

Met Asp Pro Asp Thr Glu Tyr Pro Pro Lys Leu Gln Asn Thr Leu Thr
        515                 520                 525

ttg tcg ctc ttt gat ggt gct gat gtt aga atg tac taa                1623
Leu Ser Leu Phe Asp Gly Ala Asp Val Arg Met Tyr
    530                 535                 540

<210>  10
<211>  540
<212>  PRT
<213>  Candida tropicalis

<400>  10

Met Ser Ser Ser Pro Ser Phe Ala Gln Glu Val Leu Ala Thr Thr Ser
1               5                   10                  15

Pro Tyr Ile Glu Tyr Phe Leu Asp Asn Tyr Thr Arg Trp Tyr Tyr Phe
            20                  25                  30

Ile Pro Leu Val Leu Leu Ser Leu Asn Phe Ile Ser Leu Leu His Thr
        35                  40                  45

Arg Tyr Leu Glu Arg Arg Phe His Ala Lys Pro Leu Gly Asn Phe Val
    50                  55                  60

Arg Asp Pro Thr Phe Gly Ile Ala Thr Pro Leu Leu Leu Ile Tyr Leu
65                  70                  75                  80

Lys Ser Lys Gly Thr Val Met Lys Phe Ala Trp Gly Leu Trp Asn Asn
                85                  90                  95

Lys Tyr Ile Val Arg Asp Pro Lys Tyr Lys Thr Thr Gly Leu Arg Ile
            100                 105                 110

Val Gly Leu Pro Leu Ile Glu Thr Met Asp Pro Glu Asn Ile Lys Ala
        115                 120                 125

Val Leu Ala Thr Gln Phe Asn Asp Phe Ser Leu Gly Thr Arg His Asp
    130                 135                 140

Phe Leu Tyr Ser Leu Leu Gly Asp Gly Ile Phe Thr Leu Asp Gly Ala
145                 150                 155                 160

Gly Trp Lys His Ser Arg Thr Met Leu Arg Pro Gln Phe Ala Arg Glu
                165                 170                 175

Gln Val Ser His Val Lys Leu Leu Glu Pro His Val Gln Val Phe Phe
            180                 185                 190

Lys His Val Arg Lys His Arg Gly Gln Thr Phe Asp Ile Gln Glu Leu

195                 200                 205

Phe Phe Arg Leu Thr Val Asp Ser Ala Thr Glu Phe Leu Phe Gly Glu
    210                 215                 220

Ser Ala Glu Ser Leu Arg Asp Glu Ser Ile Gly Leu Thr Pro Thr Thr
225                 230                 235                 240

Lys Asp Phe Asp Gly Arg Arg Asp Phe Ala Asp Ala Phe Asn Tyr Ser
                245                 250                 255

Gln Thr Tyr Gln Ala Tyr Arg Phe Leu Leu Gln Gln Met Tyr Trp Ile
            260                 265                 270

Leu Asn Gly Ser Glu Phe Arg Lys Ser Ile Ala Val Val His Lys Phe
        275                 280                 285

Ala Asp His Tyr Val Gln Lys Ala Leu Glu Leu Thr Asp Asp Asp Leu
    290                 295                 300

Gln Lys Gln Asp Gly Tyr Val Phe Leu Tyr Glu Leu Ala Lys Gln Thr
305                 310                 315                 320

Arg Asp Pro Lys Val Leu Arg Asp Gln Leu Leu Asn Ile Leu Val Ala
                325                 330                 335

Gly Arg Asp Thr Thr Ala Gly Leu Leu Ser Phe Val Phe Tyr Glu Leu
            340                 345                 350

Ser Arg Asn Pro Glu Val Phe Ala Lys Leu Arg Glu Glu Val Glu Asn
        355                 360                 365

Arg Phe Gly Leu Gly Glu Glu Ala Arg Val Glu Glu Ile Ser Phe Glu
    370                 375                 380

Ser Leu Lys Ser Cys Glu Tyr Leu Lys Ala Val Ile Asn Glu Thr Leu
385                 390                 395                 400

Arg Leu Tyr Pro Ser Val Pro His Asn Phe Arg Val Ala Thr Arg Asn
                405                 410                 415

Thr Thr Leu Pro Arg Gly Gly Gly Glu Asp Gly Tyr Ser Pro Ile Val
            420                 425                 430

Val Lys Lys Gly Gln Val Val Met Tyr Thr Val Ile Ala Thr His Arg
        435                 440                 445

Asp Pro Ser Ile Tyr Gly Ala Asp Ala Asp Val Phe Arg Pro Glu Arg

450                 455                 460

Trp Phe Glu Pro Glu Thr Arg Lys Leu Gly Trp Ala Tyr Val Pro Phe
465                 470                 475                 480

Asn Gly Gly Pro Arg Ile Cys Leu Gly Gln Gln Phe Ala Leu Thr Glu
                485                 490                 495

Ala Ser Tyr Val Thr Val Arg Leu Leu Gln Glu Phe Ala His Leu Ser
            500                 505                 510

Met Asp Pro Asp Thr Glu Tyr Pro Pro Lys Leu Gln Asn Thr Leu Thr
        515                 520                 525

Leu Ser Leu Phe Asp Gly Ala Asp Val Arg Met Tyr
    530                 535                 540

<210>  11
<211>  1623
<212>  DNA
<213>  Candida tropicalis

<220>
<221>  CDS
<222>  (1)..(1623)

<400>  11
atg tcg tct tct cca tcg ttt gct cag gag gtt ctc gct acc act agt       48
Met Ser Ser Ser Pro Ser Phe Ala Gln Glu Val Leu Ala Thr Thr Ser
1               5                   10                  15

cct tac atc gag tac ttt ctt gac aac tac acc aga tgg tac tac ttc       96
Pro Tyr Ile Glu Tyr Phe Leu Asp Asn Tyr Thr Arg Trp Tyr Tyr Phe
            20                  25                  30

atc cct ttg gtg ctt ctt tcg ttg aac ttc atc agc ttg ctc cac aca      144
Ile Pro Leu Val Leu Leu Ser Leu Asn Phe Ile Ser Leu Leu His Thr
        35                  40                  45

aag tac ttg gaa cgc agg ttc cac gcc aag ccg ctc ggt aac gtc gtg      192
Lys Tyr Leu Glu Arg Arg Phe His Ala Lys Pro Leu Gly Asn Val Val
    50                  55                  60

ttg gat cct acg ttt ggt atc gct act ccg ttg atc ttg atc tac tta      240
Leu Asp Pro Thr Phe Gly Ile Ala Thr Pro Leu Ile Leu Ile Tyr Leu
65                  70                  75                  80

aag tcg aaa ggt aca gtc atg aag ttt gcc tgg agc ttc tgg aac aac      288
Lys Ser Lys Gly Thr Val Met Lys Phe Ala Trp Ser Phe Trp Asn Asn
                85                  90                  95

aag tac att gtc aaa gac cca aag tac aag acc act ggc ctt aga att      336
Lys Tyr Ile Val Lys Asp Pro Lys Tyr Lys Thr Thr Gly Leu Arg Ile
            100                 105                 110

gtc ggc ctc cca ttg att gaa acc ata gac cca gag aac atc aaa gct      384
Val Gly Leu Pro Leu Ile Glu Thr Ile Asp Pro Glu Asn Ile Lys Ala

115                 120                 125

gtg ttg gct act cag ttc aac gat ttc tcc ttg gga act aga cac gat        432
Val Leu Ala Thr Gln Phe Asn Asp Phe Ser Leu Gly Thr Arg His Asp
    130                 135                 140

ttc ttg tac tcc ttg ttg ggc gat ggt att ttt acc ttg gac ggt gct        480
Phe Leu Tyr Ser Leu Leu Gly Asp Gly Ile Phe Thr Leu Asp Gly Ala
145                 150                 155                 160

ggc tgg aaa cac agt aga act atg ttg aga cca cag ttt gct aga gaa        528
Gly Trp Lys His Ser Arg Thr Met Leu Arg Pro Gln Phe Ala Arg Glu
                165                 170                 175

cag gtt tcc cac gtc aag ttg ttg gaa cca cac gtt cag gtg ttc ttc        576
Gln Val Ser His Val Lys Leu Leu Glu Pro His Val Gln Val Phe Phe
            180                 185                 190

aag cac gtt aga aaa cac cgc ggt cag act ttt gac atc caa gaa ttg        624
Lys His Val Arg Lys His Arg Gly Gln Thr Phe Asp Ile Gln Glu Leu
        195                 200                 205

ttc ttc aga ttg acc gtc gac tcc gcc acc gag ttc ttg ttt ggt gag        672
Phe Phe Arg Leu Thr Val Asp Ser Ala Thr Glu Phe Leu Phe Gly Glu
    210                 215                 220

tct gct gaa tcc ttg aga gac gac tct gtt ggt ttg acc cca acc acc        720
Ser Ala Glu Ser Leu Arg Asp Asp Ser Val Gly Leu Thr Pro Thr Thr
225                 230                 235                 240

aag gat ttc gaa ggc aga gga gat ttc gct gac gct ttc aac tac tcg        768
Lys Asp Phe Glu Gly Arg Gly Asp Phe Ala Asp Ala Phe Asn Tyr Ser
                245                 250                 255

cag act tac cag gcc tac aga ttt ttg ttg caa caa atg tac tgg att        816
Gln Thr Tyr Gln Ala Tyr Arg Phe Leu Leu Gln Gln Met Tyr Trp Ile
            260                 265                 270

ttg aat ggc gcg gaa ttc aga aag tcg att gcc atc gtg cac aag ttt        864
Leu Asn Gly Ala Glu Phe Arg Lys Ser Ile Ala Ile Val His Lys Phe
        275                 280                 285

gct gac cac tat gtg caa aag gct ttg gag ttg acc gac gat gac ttg        912
Ala Asp His Tyr Val Gln Lys Ala Leu Glu Leu Thr Asp Asp Asp Leu
    290                 295                 300

cag aaa caa gac ggc tat gtg ttc ttg tac gag ttg gct aag caa act        960
Gln Lys Gln Asp Gly Tyr Val Phe Leu Tyr Glu Leu Ala Lys Gln Thr
305                 310                 315                 320

aga gac cca aag gtc ttg aga gac cag ttg ttg aac att ttg gtt gcc       1008
Arg Asp Pro Lys Val Leu Arg Asp Gln Leu Leu Asn Ile Leu Val Ala
                325                 330                 335

ggt aga gac acg acc gcc ggt ttg ttg tcg ttt gtg ttc tac gag ttg       1056
Gly Arg Asp Thr Thr Ala Gly Leu Leu Ser Phe Val Phe Tyr Glu Leu
            340                 345                 350

tcg aga aac cct gaa gtg ttt gcc aag ttg aga gag gag gtg gaa aac       1104
Ser Arg Asn Pro Glu Val Phe Ala Lys Leu Arg Glu Glu Val Glu Asn
        355                 360                 365

aga ttt gga ctc ggc gaa gag gct cgt gtt gaa gag atc tct ttt gag       1152
Arg Phe Gly Leu Gly Glu Glu Ala Arg Val Glu Glu Ile Ser Phe Glu

370                 375                 380

tcc ttg aag tcc tgt gag tac ttg aag gct gtc atc aat gaa gcc ttg      1200
Ser Leu Lys Ser Cys Glu Tyr Leu Lys Ala Val Ile Asn Glu Ala Leu
385                 390                 395                 400

aga ttg tac cca tct gtt cca cac aac ttc aga gtt gcc acc aga aac      1248
Arg Leu Tyr Pro Ser Val Pro His Asn Phe Arg Val Ala Thr Arg Asn
                405                 410                 415

act acc ctt cca aga ggc ggt ggt aaa gac gga tgc tcg cca att gtt      1296
Thr Thr Leu Pro Arg Gly Gly Gly Lys Asp Gly Cys Ser Pro Ile Val
            420                 425                 430

gtc aag aag ggt caa gtt gtc atg tac act gtc att ggt acc cac aga      1344
Val Lys Lys Gly Gln Val Val Met Tyr Thr Val Ile Gly Thr His Arg
        435                 440                 445

gac cca agt atc tac ggt gcc gac gcc gac gtc ttc aga cca gaa aga      1392
Asp Pro Ser Ile Tyr Gly Ala Asp Ala Asp Val Phe Arg Pro Glu Arg
    450                 455                 460

tgg ttc gag cca gaa act aga aag ttg ggc tgg gca tat gtt cca ttc      1440
Trp Phe Glu Pro Glu Thr Arg Lys Leu Gly Trp Ala Tyr Val Pro Phe
465                 470                 475                 480

aat ggt ggt cca aga atc tgt ttg ggt cag cag ttt gcc ttg act gaa      1488
Asn Gly Gly Pro Arg Ile Cys Leu Gly Gln Gln Phe Ala Leu Thr Glu
                485                 490                 495

gct tca tac gtc act gtc aga ttg ctc caa gag ttt gga aac ttg tcc      1536
Ala Ser Tyr Val Thr Val Arg Leu Leu Gln Glu Phe Gly Asn Leu Ser
            500                 505                 510

ctg gat cca aac gct gag tac cca cca aaa ttg cag aac acc ttg acc      1584
Leu Asp Pro Asn Ala Glu Tyr Pro Pro Lys Leu Gln Asn Thr Leu Thr
        515                 520                 525

ttg tca ctc ttt gat ggt gct gac gtt aga atg ttc taa                  1623
Leu Ser Leu Phe Asp Gly Ala Asp Val Arg Met Phe
    530                 535                 540

<210>  12
<211>  540
<212>  PRT
<213>  Candida tropicalis

<400>  12

Met Ser Ser Ser Pro Ser Phe Ala Gln Glu Val Leu Ala Thr Thr Ser
1               5                   10                  15

Pro Tyr Ile Glu Tyr Phe Leu Asp Asn Tyr Thr Arg Trp Tyr Tyr Phe
            20                  25                  30

Ile Pro Leu Val Leu Leu Ser Leu Asn Phe Ile Ser Leu Leu His Thr
        35                  40                  45

Lys Tyr Leu Glu Arg Arg Phe His Ala Lys Pro Leu Gly Asn Val Val
    50                  55                  60

Leu Asp Pro Thr Phe Gly Ile Ala Thr Pro Leu Ile Leu Ile Tyr Leu
65                  70                  75                  80

Lys Ser Lys Gly Thr Val Met Lys Phe Ala Trp Ser Phe Trp Asn Asn
                85                  90                  95

Lys Tyr Ile Val Lys Asp Pro Lys Tyr Lys Thr Thr Gly Leu Arg Ile
            100                 105                 110

Val Gly Leu Pro Leu Ile Glu Thr Ile Asp Pro Glu Asn Ile Lys Ala
        115                 120                 125

Val Leu Ala Thr Gln Phe Asn Asp Phe Ser Leu Gly Thr Arg His Asp
    130                 135                 140

Phe Leu Tyr Ser Leu Leu Gly Asp Gly Ile Phe Thr Leu Asp Gly Ala
145                 150                 155                 160

Gly Trp Lys His Ser Arg Thr Met Leu Arg Pro Gln Phe Ala Arg Glu
                165                 170                 175

Gln Val Ser His Val Lys Leu Leu Glu Pro His Val Gln Val Phe Phe
            180                 185                 190

Lys His Val Arg Lys His Arg Gly Gln Thr Phe Asp Ile Gln Glu Leu
        195                 200                 205

Phe Phe Arg Leu Thr Val Asp Ser Ala Thr Glu Phe Leu Phe Gly Glu
    210                 215                 220

Ser Ala Glu Ser Leu Arg Asp Asp Ser Val Gly Leu Thr Pro Thr Thr
225                 230                 235                 240

Lys Asp Phe Glu Gly Arg Gly Asp Phe Ala Asp Ala Phe Asn Tyr Ser
                245                 250                 255

Gln Thr Tyr Gln Ala Tyr Arg Phe Leu Leu Gln Gln Met Tyr Trp Ile
            260                 265                 270

Leu Asn Gly Ala Glu Phe Arg Lys Ser Ile Ala Ile Val His Lys Phe
        275                 280                 285

Ala Asp His Tyr Val Gln Lys Ala Leu Glu Leu Thr Asp Asp Asp Leu
    290                 295                 300

Gln Lys Gln Asp Gly Tyr Val Phe Leu Tyr Glu Leu Ala Lys Gln Thr
305                 310                 315                 320

Arg Asp Pro Lys Val Leu Arg Asp Gln Leu Leu Asn Ile Leu Val Ala
                325                 330                 335

Gly Arg Asp Thr Thr Ala Gly Leu Leu Ser Phe Val Phe Tyr Glu Leu
            340                 345                 350

Ser Arg Asn Pro Glu Val Phe Ala Lys Leu Arg Glu Glu Val Glu Asn
        355                 360                 365

Arg Phe Gly Leu Gly Glu Glu Ala Arg Val Glu Glu Ile Ser Phe Glu
    370                 375                 380

Ser Leu Lys Ser Cys Glu Tyr Leu Lys Ala Val Ile Asn Glu Ala Leu
385                 390                 395                 400

Arg Leu Tyr Pro Ser Val Pro His Asn Phe Arg Val Ala Thr Arg Asn
                405                 410                 415

Thr Thr Leu Pro Arg Gly Gly Gly Lys Asp Gly Cys Ser Pro Ile Val
            420                 425                 430

Val Lys Lys Gly Gln Val Val Met Tyr Thr Val Ile Gly Thr His Arg
        435                 440                 445

Asp Pro Ser Ile Tyr Gly Ala Asp Ala Asp Val Phe Arg Pro Glu Arg
    450                 455                 460

Trp Phe Glu Pro Glu Thr Arg Lys Leu Gly Trp Ala Tyr Val Pro Phe
465                 470                 475                 480

Asn Gly Gly Pro Arg Ile Cys Leu Gly Gln Gln Phe Ala Leu Thr Glu
                485                 490                 495

Ala Ser Tyr Val Thr Val Arg Leu Leu Gln Glu Phe Gly Asn Leu Ser
            500                 505                 510

Leu Asp Pro Asn Ala Glu Tyr Pro Pro Lys Leu Gln Asn Thr Leu Thr
        515                 520                 525

Leu Ser Leu Phe Asp Gly Ala Asp Val Arg Met Phe
    530                 535                 540

<210>  13
<211>  1554
<212>  DNA
<213>  Candida tropicalis

<220>

<221>  CDS
<222>  (1)..(1554)

<400>  13
atg att gaa caa ctc cta gaa tat tgg tat gtc gtt gtg cca gtg ttg       48
Met Ile Glu Gln Leu Leu Glu Tyr Trp Tyr Val Val Val Pro Val Leu
1               5                   10                  15

tac atc atc aaa caa ctc ctt gca tac aca aag act cgc gtc ttg atg       96
Tyr Ile Ile Lys Gln Leu Leu Ala Tyr Thr Lys Thr Arg Val Leu Met
            20                  25                  30

aaa aag ttg ggt gct gct cca gtc aca aac aag ttg tac gac aac gct      144
Lys Lys Leu Gly Ala Ala Pro Val Thr Asn Lys Leu Tyr Asp Asn Ala
        35                  40                  45

ttc ggt atc gtc aat gga tgg aag gct ctc cag ttc aag aaa gag ggc      192
Phe Gly Ile Val Asn Gly Trp Lys Ala Leu Gln Phe Lys Lys Glu Gly
    50                  55                  60

agg gct caa gag tac aac gat tac aag ttt gac cac tcc aag aac cca      240
Arg Ala Gln Glu Tyr Asn Asp Tyr Lys Phe Asp His Ser Lys Asn Pro
65                  70                  75                  80

agc gtg ggc acc tac gtc agt att ctt ttc ggc acc agg atc gtc gtg      288
Ser Val Gly Thr Tyr Val Ser Ile Leu Phe Gly Thr Arg Ile Val Val
                85                  90                  95

acc aaa gat cca gag aat atc aaa gct att ttg gca acc cag ttt ggt      336
Thr Lys Asp Pro Glu Asn Ile Lys Ala Ile Leu Ala Thr Gln Phe Gly
            100                 105                 110

gat ttt tct ttg ggc aag agg cac act ctt ttt aag cct ttg tta ggt      384
Asp Phe Ser Leu Gly Lys Arg His Thr Leu Phe Lys Pro Leu Leu Gly
        115                 120                 125

gat ggg atc ttc aca ttg gac ggc gaa ggc tgg aag cac agc aga gcc      432
Asp Gly Ile Phe Thr Leu Asp Gly Glu Gly Trp Lys His Ser Arg Ala
    130                 135                 140

atg ttg aga cca cag ttt gcc aga gaa caa gtt gct cat gtg acg tcg      480
Met Leu Arg Pro Gln Phe Ala Arg Glu Gln Val Ala His Val Thr Ser
145                 150                 155                 160

ttg gaa cca cac ttc cag ttg ttg aag aag cat att ctt aag cac aag      528
Leu Glu Pro His Phe Gln Leu Leu Lys Lys His Ile Leu Lys His Lys
                165                 170                 175

ggt gaa tac ttt gat atc cag gaa ttg ttc ttt aga ttt acc gtt gat      576
Gly Glu Tyr Phe Asp Ile Gln Glu Leu Phe Phe Arg Phe Thr Val Asp
            180                 185                 190

tcg gcc acg gag ttc tta ttt ggt gag tcc gtg cac tcc tta aag gac      624
Ser Ala Thr Glu Phe Leu Phe Gly Glu Ser Val His Ser Leu Lys Asp
        195                 200                 205

gaa tct att ggt atc aac caa gac gat ata gat ttt gct ggt aga aag      672
Glu Ser Ile Gly Ile Asn Gln Asp Asp Ile Asp Phe Ala Gly Arg Lys
    210                 215                 220

gac ttt gct gag tcg ttc aac aaa gcc cag gaa tac ttg gct att aga      720
Asp Phe Ala Glu Ser Phe Asn Lys Ala Gln Glu Tyr Leu Ala Ile Arg
225                 230                 235                 240

acc ttg gtg cag acg ttc tac tgg ttg gtc aac aac aag gag ttt aga      768

Thr Leu Val Gln Thr Phe Tyr Trp Leu Val Asn Asn Lys Glu Phe Arg
                245                 250                 255

gac tgt acc aag ctg gtg cac aag ttc acc aac tac tat gtt cag aaa 816
Asp Cys Thr Lys Leu Val His Lys Phe Thr Asn Tyr Tyr Val Gln Lys
            260                 265                 270

gct ttg gat gct agc cca gaa gag ctt gaa aag caa agt ggg tat gtg 864
Ala Leu Asp Ala Ser Pro Glu Glu Leu Glu Lys Gln Ser Gly Tyr Val
        275                 280                 285

ttc ttg tac gag ctt gtc aag cag aca aga gac ccc aat gtg ttg cgt 912
Phe Leu Tyr Glu Leu Val Lys Gln Thr Arg Asp Pro Asn Val Leu Arg
    290                 295                 300

gac cag tct ttg aac atc ttg ttg gcc gga aga gac acc act gct ggg 960
Asp Gln Ser Leu Asn Ile Leu Leu Ala Gly Arg Asp Thr Thr Ala Gly
305                 310                 315                 320

ttg ttg tcg ttt gct gtc ttt gag ttg gcc aga cac cca gag atc tgg 1008
Leu Leu Ser Phe Ala Val Phe Glu Leu Ala Arg His Pro Glu Ile Trp
                325                 330                 335

gcc aag ttg aga gag gaa att gaa caa cag ttt ggt ctt gga gaa gac 1056
Ala Lys Leu Arg Glu Glu Ile Glu Gln Gln Phe Gly Leu Gly Glu Asp
            340                 345                 350

tct cgt gtt gaa gag att acc ttt gag agc ttg aag aga tgt gag tac 1104
Ser Arg Val Glu Glu Ile Thr Phe Glu Ser Leu Lys Arg Cys Glu Tyr
        355                 360                 365

ttg aaa gcg ttc ctt aat gaa acc ttg cgt att tac cca agt gtc cca 1152
Leu Lys Ala Phe Leu Asn Glu Thr Leu Arg Ile Tyr Pro Ser Val Pro
    370                 375                 380

aga aac ttc aga atc gcc acc aag aac acg aca ttg cca agg ggc ggt 1200
Arg Asn Phe Arg Ile Ala Thr Lys Asn Thr Thr Leu Pro Arg Gly Gly
385                 390                 395                 400

ggt tca gac ggt acc tcg cca atc ttg atc caa aag gga gaa gct gtg 1248
Gly Ser Asp Gly Thr Ser Pro Ile Leu Ile Gln Lys Gly Glu Ala Val
                405                 410                 415

tcg tat ggt atc aac tct act cat ttg gac cct gtc tat tac ggc cct 1296
Ser Tyr Gly Ile Asn Ser Thr His Leu Asp Pro Val Tyr Tyr Gly Pro
            420                 425                 430

gat gct gct gag ttc aga cca gag aga tgg ttt gag cca tca acc aaa 1344
Asp Ala Ala Glu Phe Arg Pro Glu Arg Trp Phe Glu Pro Ser Thr Lys
        435                 440                 445

aag ctc ggc tgg gct tac ttg cca ttc aac ggt ggt cca aga atc tgt 1392
Lys Leu Gly Trp Ala Tyr Leu Pro Phe Asn Gly Gly Pro Arg Ile Cys
    450                 455                 460

ttg ggt cag cag ttt gcc ttg acg gaa gct ggc tat gtg ttg gtt aga 1440
Leu Gly Gln Gln Phe Ala Leu Thr Glu Ala Gly Tyr Val Leu Val Arg
465                 470                 475                 480

ttg gtg caa gag ttc tcc cac gtt agg ctg gac cca gac gag gtg tac 1488
Leu Val Gln Glu Phe Ser His Val Arg Leu Asp Pro Asp Glu Val Tyr
                485                 490                 495

ccg cca aag agg ttg acc aac ttg acc atg tgt ttg cag gat ggt gct 1536

Pro Pro Lys Arg Leu Thr Asn Leu Thr Met Cys Leu Gln Asp Gly Ala
            500                 505                 510

att gtc aag ttt gac tag                                           1554
Ile Val Lys Phe Asp
        515

<210>  14
<211>  517
<212>  PRT
<213>  Candida tropicalis

<400>  14

Met Ile Glu Gln Leu Leu Glu Tyr Trp Tyr Val Val Val Pro Val Leu
1               5                   10                  15

Tyr Ile Ile Lys Gln Leu Leu Ala Tyr Thr Lys Thr Arg Val Leu Met
            20                  25                  30

Lys Lys Leu Gly Ala Ala Pro Val Thr Asn Lys Leu Tyr Asp Asn Ala
        35                  40                  45

Phe Gly Ile Val Asn Gly Trp Lys Ala Leu Gln Phe Lys Lys Glu Gly
    50                  55                  60

Arg Ala Gln Glu Tyr Asn Asp Tyr Lys Phe Asp His Ser Lys Asn Pro
65                  70                  75                  80

Ser Val Gly Thr Tyr Val Ser Ile Leu Phe Gly Thr Arg Ile Val Val
                85                  90                  95

Thr Lys Asp Pro Glu Asn Ile Lys Ala Ile Leu Ala Thr Gln Phe Gly
            100                 105                 110

Asp Phe Ser Leu Gly Lys Arg His Thr Leu Phe Lys Pro Leu Leu Gly
        115                 120                 125

Asp Gly Ile Phe Thr Leu Asp Gly Glu Gly Trp Lys His Ser Arg Ala
    130                 135                 140

Met Leu Arg Pro Gln Phe Ala Arg Glu Gln Val Ala His Val Thr Ser
145                 150                 155                 160

Leu Glu Pro His Phe Gln Leu Leu Lys Lys His Ile Leu Lys His Lys
                165                 170                 175

Gly Glu Tyr Phe Asp Ile Gln Glu Leu Phe Phe Arg Phe Thr Val Asp
            180                 185                 190

Ser Ala Thr Glu Phe Leu Phe Gly Glu Ser Val His Ser Leu Lys Asp

195                 200                 205

Glu Ser Ile Gly Ile Asn Gln Asp Asp Ile Asp Phe Ala Gly Arg Lys
    210                 215                 220

Asp Phe Ala Glu Ser Phe Asn Lys Ala Gln Glu Tyr Leu Ala Ile Arg
225                 230                 235                 240

Thr Leu Val Gln Thr Phe Tyr Trp Leu Val Asn Asn Lys Glu Phe Arg
                245                 250                 255

Asp Cys Thr Lys Leu Val His Lys Phe Thr Asn Tyr Tyr Val Gln Lys
            260                 265                 270

Ala Leu Asp Ala Ser Pro Glu Glu Leu Glu Lys Gln Ser Gly Tyr Val
        275                 280                 285

Phe Leu Tyr Glu Leu Val Lys Gln Thr Arg Asp Pro Asn Val Leu Arg
    290                 295                 300

Asp Gln Ser Leu Asn Ile Leu Leu Ala Gly Arg Asp Thr Thr Ala Gly
305                 310                 315                 320

Leu Leu Ser Phe Ala Val Phe Glu Leu Ala Arg His Pro Glu Ile Trp
                325                 330                 335

Ala Lys Leu Arg Glu Glu Ile Glu Gln Gln Phe Gly Leu Gly Glu Asp
            340                 345                 350

Ser Arg Val Glu Glu Ile Thr Phe Glu Ser Leu Lys Arg Cys Glu Tyr
        355                 360                 365

Leu Lys Ala Phe Leu Asn Glu Thr Leu Arg Ile Tyr Pro Ser Val Pro
    370                 375                 380

Arg Asn Phe Arg Ile Ala Thr Lys Asn Thr Thr Leu Pro Arg Gly Gly
385                 390                 395                 400

Gly Ser Asp Gly Thr Ser Pro Ile Leu Ile Gln Lys Gly Glu Ala Val
                405                 410                 415

Ser Tyr Gly Ile Asn Ser Thr His Leu Asp Pro Val Tyr Tyr Gly Pro
            420                 425                 430

Asp Ala Ala Glu Phe Arg Pro Glu Arg Trp Phe Glu Pro Ser Thr Lys
        435                 440                 445

Lys Leu Gly Trp Ala Tyr Leu Pro Phe Asn Gly Gly Pro Arg Ile Cys

450                 455                 460

Leu Gly Gln Gln Phe Ala Leu Thr Glu Ala Gly Tyr Val Leu Val Arg
465                 470                 475                 480

Leu Val Gln Glu Phe Ser His Val Arg Leu Asp Pro Asp Glu Val Tyr
                485                 490                 495

Pro Pro Lys Arg Leu Thr Asn Leu Thr Met Cys Leu Gln Asp Gly Ala
            500                 505                 510

Ile Val Lys Phe Asp
        515

<210>  15
<211>  1554
<212>  DNA
<213>  Candida tropicalis

<220>
<221>  CDS
<222>  (1)..(1554)

<400>  15
atg att gaa caa atc cta gaa tat tgg tat att gtt gtg cct gtg ttg       48
Met Ile Glu Gln Ile Leu Glu Tyr Trp Tyr Ile Val Val Pro Val Leu
1               5                   10                  15

tac atc atc aaa caa ctc att gcc tac agc aag act cgc gtc ttg atg       96
Tyr Ile Ile Lys Gln Leu Ile Ala Tyr Ser Lys Thr Arg Val Leu Met
            20                  25                  30

aaa cag ttg ggt gct gct cca atc aca aac cag ttg tac gac aac gtt      144
Lys Gln Leu Gly Ala Ala Pro Ile Thr Asn Gln Leu Tyr Asp Asn Val
        35                  40                  45

ttc ggt atc gtc aac gga tgg aag gct ctc cag ttc aag aaa gag ggc      192
Phe Gly Ile Val Asn Gly Trp Lys Ala Leu Gln Phe Lys Lys Glu Gly
    50                  55                  60

aga gct caa gag tac aac gat cac aag ttt gac agc tcc aag aac cca      240
Arg Ala Gln Glu Tyr Asn Asp His Lys Phe Asp Ser Ser Lys Asn Pro
65                  70                  75                  80

agc gtc ggc acc tat gtc agt att ctt ttt ggc acc aag att gtc gtg      288
Ser Val Gly Thr Tyr Val Ser Ile Leu Phe Gly Thr Lys Ile Val Val
                85                  90                  95

acc aag gat cca gag aat atc aaa gct att ttg gca acc cag ttt ggc      336
Thr Lys Asp Pro Glu Asn Ile Lys Ala Ile Leu Ala Thr Gln Phe Gly
            100                 105                 110

gat ttt tct ttg ggc aag aga cac gct ctt ttt aaa cct ttg tta ggt      384
Asp Phe Ser Leu Gly Lys Arg His Ala Leu Phe Lys Pro Leu Leu Gly
        115                 120                 125

gat ggg atc ttc acc ttg gac ggc gaa ggc tgg aag cat agc aga tcc      432
Asp Gly Ile Phe Thr Leu Asp Gly Glu Gly Trp Lys His Ser Arg Ser

130                 135                 140

atg tta aga cca cag ttt gcc aga gaa caa gtt gct cat gtg acg tcg        480
Met Leu Arg Pro Gln Phe Ala Arg Glu Gln Val Ala His Val Thr Ser
145                 150                 155                 160

ttg gaa cca cac ttc cag ttg ttg aag aag cat atc ctt aaa cac aag        528
Leu Glu Pro His Phe Gln Leu Leu Lys Lys His Ile Leu Lys His Lys
                165                 170                 175

ggt gag tac ttt gat atc cag gaa ttg ttc ttt aga ttt act gtc gac        576
Gly Glu Tyr Phe Asp Ile Gln Glu Leu Phe Phe Arg Phe Thr Val Asp
            180                 185                 190

tcg gcc acg gag ttc tta ttt ggt gag tcc gtg cac tcc tta aag gac        624
Ser Ala Thr Glu Phe Leu Phe Gly Glu Ser Val His Ser Leu Lys Asp
        195                 200                 205

gaa act atc ggt atc aac caa gac gat ata gat ttt gct ggt aga aag        672
Glu Thr Ile Gly Ile Asn Gln Asp Asp Ile Asp Phe Ala Gly Arg Lys
    210                 215                 220

gac ttt gct gag tcg ttc aac aaa gcc cag gag tat ttg tct att aga        720
Asp Phe Ala Glu Ser Phe Asn Lys Ala Gln Glu Tyr Leu Ser Ile Arg
225                 230                 235                 240

att ttg gtg cag acc ttc tac tgg ttg atc aac aac aag gag ttt aga        768
Ile Leu Val Gln Thr Phe Tyr Trp Leu Ile Asn Asn Lys Glu Phe Arg
                245                 250                 255

gac tgt acc aag ctg gtg cac aag ttt acc aac tac tat gtt cag aaa        816
Asp Cys Thr Lys Leu Val His Lys Phe Thr Asn Tyr Tyr Val Gln Lys
            260                 265                 270

gct ttg gat gct acc cca gag gaa ctt gaa aag caa ggc ggg tat gtg        864
Ala Leu Asp Ala Thr Pro Glu Glu Leu Glu Lys Gln Gly Gly Tyr Val
        275                 280                 285

ttc ttg tat gag ctt gtc aag cag acg aga gac ccc aag gtg ttg cgt        912
Phe Leu Tyr Glu Leu Val Lys Gln Thr Arg Asp Pro Lys Val Leu Arg
    290                 295                 300

gac cag tct ttg aac atc ttg ttg gca gga aga gac acc act gct ggg        960
Asp Gln Ser Leu Asn Ile Leu Leu Ala Gly Arg Asp Thr Thr Ala Gly
305                 310                 315                 320

ttg ttg tcc ttt gct gtg ttt gag ttg gcc aga aac cca cac atc tgg       1008
Leu Leu Ser Phe Ala Val Phe Glu Leu Ala Arg Asn Pro His Ile Trp
                325                 330                 335

gcc aag ttg aga gag gaa att gaa cag cag ttt ggt ctt gga gaa gac       1056
Ala Lys Leu Arg Glu Glu Ile Glu Gln Gln Phe Gly Leu Gly Glu Asp
            340                 345                 350

tct cgt gtt gaa gag att acc ttt gag agc ttg aag aga tgt gag tac       1104
Ser Arg Val Glu Glu Ile Thr Phe Glu Ser Leu Lys Arg Cys Glu Tyr
        355                 360                 365

ttg aaa gcg ttc ctt aac gaa acc ttg cgt gtt tac cca agt gtc cca       1152
Leu Lys Ala Phe Leu Asn Glu Thr Leu Arg Val Tyr Pro Ser Val Pro
    370                 375                 380

aga aac ttc aga atc gcc acc aag aat aca aca ttg cca agg ggt ggt       1200
Arg Asn Phe Arg Ile Ala Thr Lys Asn Thr Thr Leu Pro Arg Gly Gly

385                 390                 395                 400

ggt cca gac ggt acc cag cca atc ttg atc caa aag gga gaa ggt gtg      1248
Gly Pro Asp Gly Thr Gln Pro Ile Leu Ile Gln Lys Gly Glu Gly Val
                405                 410                 415

tcg tat ggt atc aac tct acc cac tta gat cct gtc tat tat ggc cct      1296
Ser Tyr Gly Ile Asn Ser Thr His Leu Asp Pro Val Tyr Tyr Gly Pro
            420                 425                 430

gat gct gct gag ttc aga cca gag aga tgg ttt gag cca tca acc aga      1344
Asp Ala Ala Glu Phe Arg Pro Glu Arg Trp Phe Glu Pro Ser Thr Arg
        435                 440                 445

aag ctc ggc tgg gct tac ttg cca ttc aac ggt ggg cca cga atc tgt      1392
Lys Leu Gly Trp Ala Tyr Leu Pro Phe Asn Gly Gly Pro Arg Ile Cys
    450                 455                 460

ttg ggt cag cag ttt gcc ttg acc gaa gct ggt tac gtt ttg gtc aga      1440
Leu Gly Gln Gln Phe Ala Leu Thr Glu Ala Gly Tyr Val Leu Val Arg
465                 470                 475                 480

ttg gtg caa gag ttc tcc cac att agg ctg gac cca gat gaa gtg tat      1488
Leu Val Gln Glu Phe Ser His Ile Arg Leu Asp Pro Asp Glu Val Tyr
                485                 490                 495

cca cca aag agg ttg acc aac ttg acc atg tgt ttg cag gat ggt gct      1536
Pro Pro Lys Arg Leu Thr Asn Leu Thr Met Cys Leu Gln Asp Gly Ala
            500                 505                 510

att gtc aag ttt gac tag                                              1554
Ile Val Lys Phe Asp
        515


<210>  16
<211>  517
<212>  PRT
<213>  Candida tropicalis

<400>  16

Met Ile Glu Gln Ile Leu Glu Tyr Trp Tyr Ile Val Val Pro Val Leu
1               5                   10                  15


Tyr Ile Ile Lys Gln Leu Ile Ala Tyr Ser Lys Thr Arg Val Leu Met
            20                  25                  30


Lys Gln Leu Gly Ala Ala Pro Ile Thr Asn Gln Leu Tyr Asp Asn Val
        35                  40                  45


Phe Gly Ile Val Asn Gly Trp Lys Ala Leu Gln Phe Lys Lys Glu Gly
    50                  55                  60


Arg Ala Gln Glu Tyr Asn Asp His Lys Phe Asp Ser Ser Lys Asn Pro
65                  70                  75                  80


Ser Val Gly Thr Tyr Val Ser Ile Leu Phe Gly Thr Lys Ile Val Val
                85                  90                  95

Thr Lys Asp Pro Glu Asn Ile Lys Ala Ile Leu Ala Thr Gln Phe Gly
            100                 105                 110

Asp Phe Ser Leu Gly Lys Arg His Ala Leu Phe Lys Pro Leu Leu Gly
        115                 120                 125

Asp Gly Ile Phe Thr Leu Asp Gly Glu Gly Trp Lys His Ser Arg Ser
    130                 135                 140

Met Leu Arg Pro Gln Phe Ala Arg Glu Gln Val Ala His Val Thr Ser
145                 150                 155                 160

Leu Glu Pro His Phe Gln Leu Leu Lys Lys His Ile Leu Lys His Lys
                165                 170                 175

Gly Glu Tyr Phe Asp Ile Gln Glu Leu Phe Phe Arg Phe Thr Val Asp
            180                 185                 190

Ser Ala Thr Glu Phe Leu Phe Gly Glu Ser Val His Ser Leu Lys Asp
        195                 200                 205

Glu Thr Ile Gly Ile Asn Gln Asp Asp Ile Asp Phe Ala Gly Arg Lys
    210                 215                 220

Asp Phe Ala Glu Ser Phe Asn Lys Ala Gln Glu Tyr Leu Ser Ile Arg
225                 230                 235                 240

Ile Leu Val Gln Thr Phe Tyr Trp Leu Ile Asn Asn Lys Glu Phe Arg
                245                 250                 255

Asp Cys Thr Lys Leu Val His Lys Phe Thr Asn Tyr Tyr Val Gln Lys
            260                 265                 270

Ala Leu Asp Ala Thr Pro Glu Glu Leu Glu Lys Gln Gly Gly Tyr Val
        275                 280                 285

Phe Leu Tyr Glu Leu Val Lys Gln Thr Arg Asp Pro Lys Val Leu Arg
    290                 295                 300

Asp Gln Ser Leu Asn Ile Leu Leu Ala Gly Arg Asp Thr Thr Ala Gly
305                 310                 315                 320

Leu Leu Ser Phe Ala Val Phe Glu Leu Ala Arg Asn Pro His Ile Trp
                325                 330                 335

Ala Lys Leu Arg Glu Glu Ile Glu Gln Gln Phe Gly Leu Gly Glu Asp
            340                 345                 350

Ser Arg Val Glu Glu Ile Thr Phe Glu Ser Leu Lys Arg Cys Glu Tyr
        355                 360                 365

Leu Lys Ala Phe Leu Asn Glu Thr Leu Arg Val Tyr Pro Ser Val Pro
    370                 375                 380

Arg Asn Phe Arg Ile Ala Thr Lys Asn Thr Thr Leu Pro Arg Gly Gly
385                 390                 395                 400

Gly Pro Asp Gly Thr Gln Pro Ile Leu Ile Gln Lys Gly Glu Gly Val
                405                 410                 415

Ser Tyr Gly Ile Asn Ser Thr His Leu Asp Pro Val Tyr Tyr Gly Pro
            420                 425                 430

Asp Ala Ala Glu Phe Arg Pro Glu Arg Trp Phe Glu Pro Ser Thr Arg
        435                 440                 445

Lys Leu Gly Trp Ala Tyr Leu Pro Phe Asn Gly Gly Pro Arg Ile Cys
    450                 455                 460

Leu Gly Gln Gln Phe Ala Leu Thr Glu Ala Gly Tyr Val Leu Val Arg
465                 470                 475                 480

Leu Val Gln Glu Phe Ser His Ile Arg Leu Asp Pro Asp Glu Val Tyr
                485                 490                 495

Pro Pro Lys Arg Leu Thr Asn Leu Thr Met Cys Leu Gln Asp Gly Ala
            500                 505                 510

Ile Val Lys Phe Asp
        515

<210>  17
<211>  1539
<212>  DNA
<213>  Candida tropicalis

<220>
<221>  CDS
<222>  (1)..(1539)

<400>  17
atg ctc gat cag atc tta cat tac tgg tac att gtc ttg cca ttg ttg        48
Met Leu Asp Gln Ile Leu His Tyr Trp Tyr Ile Val Leu Pro Leu Leu
1               5                   10                  15

gcc att atc aac cag atc gtg gct cat gtc agg acc aat tat ttg atg        96
Ala Ile Ile Asn Gln Ile Val Ala His Val Arg Thr Asn Tyr Leu Met
            20                  25                  30

aag aaa ttg ggt gct aag cca ttc aca cac gtc caa cgt gac ggg tgg 144
Lys Lys Leu Gly Ala Lys Pro Phe Thr His Val Gln Arg Asp Gly Trp
        35                  40                  45

ttg ggc ttc aaa ttc ggc cgt gaa ttc ctc aaa gca aaa agt gct ggg 192
Leu Gly Phe Lys Phe Gly Arg Glu Phe Leu Lys Ala Lys Ser Ala Gly
    50                  55                  60

aga ctg gtt gat tta atc atc tcc cgt ttc cac gat aat gag gac act 240
Arg Leu Val Asp Leu Ile Ile Ser Arg Phe His Asp Asn Glu Asp Thr
65                  70                  75                  80

ttc tcc agc tat gct ttt ggc aac cat gtg gtg ttc acc agg gac ccc 288
Phe Ser Ser Tyr Ala Phe Gly Asn His Val Val Phe Thr Arg Asp Pro
                85                  90                  95

gag aat atc aag gcg ctt ttg gca acc cag ttt ggt gat ttt tca ttg 336
Glu Asn Ile Lys Ala Leu Leu Ala Thr Gln Phe Gly Asp Phe Ser Leu
            100                 105                 110

ggc agc agg gtc aag ttc ttc aaa cca tta ttg ggg tac ggt atc ttc 384
Gly Ser Arg Val Lys Phe Phe Lys Pro Leu Leu Gly Tyr Gly Ile Phe
        115                 120                 125

aca ttg gac gcc gaa ggc tgg aag cac agc aga gcc atg ttg aga cca 432
Thr Leu Asp Ala Glu Gly Trp Lys His Ser Arg Ala Met Leu Arg Pro
    130                 135                 140

cag ttt gcc aga gaa caa gtt gct cat gtg acg tcg ttg gaa cca cac 480
Gln Phe Ala Arg Glu Gln Val Ala His Val Thr Ser Leu Glu Pro His
145                 150                 155                 160

ttc cag ttg ttg aag aag cat atc ctt aaa cac aag ggt gag tac ttt 528
Phe Gln Leu Leu Lys Lys His Ile Leu Lys His Lys Gly Glu Tyr Phe
                165                 170                 175

gat atc cag gaa ttg ttc ttt aga ttt act gtc gac tcg gcc acg gag 576
Asp Ile Gln Glu Leu Phe Phe Arg Phe Thr Val Asp Ser Ala Thr Glu
            180                 185                 190

ttc tta ttt ggt gag tcc gtg cac tcc tta aag gac gag gaa att ggc 624
Phe Leu Phe Gly Glu Ser Val His Ser Leu Lys Asp Glu Glu Ile Gly
        195                 200                 205

tac gac acg aaa gac atg tct gaa gaa aga cgc aga ttt gcc gac gcg 672
Tyr Asp Thr Lys Asp Met Ser Glu Glu Arg Arg Arg Phe Ala Asp Ala
    210                 215                 220

ttc aac aag tcg caa gtc tac gtg gcc acc aga gtt gct tta cag aac 720
Phe Asn Lys Ser Gln Val Tyr Val Ala Thr Arg Val Ala Leu Gln Asn
225                 230                 235                 240

ttg tac tgg ttg gtc aac aac aaa gag ttc aag gag tgc aat gac att 768
Leu Tyr Trp Leu Val Asn Asn Lys Glu Phe Lys Glu Cys Asn Asp Ile
                245                 250                 255

gtc cac aag ttt acc aac tac tat gtt cag aaa gcc ttg gat gct acc 816
Val His Lys Phe Thr Asn Tyr Tyr Val Gln Lys Ala Leu Asp Ala Thr
            260                 265                 270

cca gag gaa ctt gaa aag caa ggc ggg tat gtg ttc ttg tat gag ctt 864
Pro Glu Glu Leu Glu Lys Gln Gly Gly Tyr Val Phe Leu Tyr Glu Leu
        275                 280                 285

gtc aag cag acg aga gac ccc aag gtg ttg cgt gac cag tct ttg aac      912
Val Lys Gln Thr Arg Asp Pro Lys Val Leu Arg Asp Gln Ser Leu Asn
    290                 295                 300

atc ttg ttg gca gga aga gac acc act gct ggg ttg ttg tcc ttt gct      960
Ile Leu Leu Ala Gly Arg Asp Thr Thr Ala Gly Leu Leu Ser Phe Ala
305                 310                 315                 320

gtg ttt gag ttg gcc aga aac cca cac atc tgg gcc aag ttg aga gag      1008
Val Phe Glu Leu Ala Arg Asn Pro His Ile Trp Ala Lys Leu Arg Glu
                325                 330                 335

gaa att gaa cag cag ttt ggt ctt gga gaa gac tct cgt gtt gaa gag      1056
Glu Ile Glu Gln Gln Phe Gly Leu Gly Glu Asp Ser Arg Val Glu Glu
            340                 345                 350

att acc ttt gag agc ttg aag aga tgt gag tac ttg aag gcc gtg ttg      1104
Ile Thr Phe Glu Ser Leu Lys Arg Cys Glu Tyr Leu Lys Ala Val Leu
        355                 360                 365

aac gaa act ttg aga tta cac cca agt gtc cca aga aac gca aga ttt      1152
Asn Glu Thr Leu Arg Leu His Pro Ser Val Pro Arg Asn Ala Arg Phe
    370                 375                 380

gcg att aaa gac acg act tta cca aga ggc ggt ggc ccc aac ggc aag      1200
Ala Ile Lys Asp Thr Thr Leu Pro Arg Gly Gly Gly Pro Asn Gly Lys
385                 390                 395                 400

gat cct atc ttg atc agg aag gat gag gtg gtg cag tac tcc atc tcg      1248
Asp Pro Ile Leu Ile Arg Lys Asp Glu Val Val Gln Tyr Ser Ile Ser
                405                 410                 415

gca act cag aca aat cct gct tat tat ggc gcc gat gct gct gat ttt      1296
Ala Thr Gln Thr Asn Pro Ala Tyr Tyr Gly Ala Asp Ala Ala Asp Phe
            420                 425                 430

aga ccg gaa aga tgg ttt gaa cca tca act aga aac ttg gga tgg gct      1344
Arg Pro Glu Arg Trp Phe Glu Pro Ser Thr Arg Asn Leu Gly Trp Ala
        435                 440                 445

ttc ttg cca ttc aac ggt ggt cca aga atc tgt ttg gga caa cag ttt      1392
Phe Leu Pro Phe Asn Gly Gly Pro Arg Ile Cys Leu Gly Gln Gln Phe
    450                 455                 460

gct ttg act gaa gcc ggt tac gtt ttg gtt aga ctt gtt cag gag ttt      1440
Ala Leu Thr Glu Ala Gly Tyr Val Leu Val Arg Leu Val Gln Glu Phe
465                 470                 475                 480

cca aac ttg tca caa gac ccc gaa acc aag tac cca cca cct aga ttg      1488
Pro Asn Leu Ser Gln Asp Pro Glu Thr Lys Tyr Pro Pro Pro Arg Leu
                485                 490                 495

gca cac ttg acg atg tgc ttg ttt gac ggt gca cac gtc aag atg tca      1536
Ala His Leu Thr Met Cys Leu Phe Asp Gly Ala His Val Lys Met Ser
            500                 505                 510

tag                                                                  1539

<210>  18
<211>  512
<212>  PRT
<213>  Candida tropicalis

<400>  18

Met Leu Asp Gln Ile Leu His Tyr Trp Tyr Ile Val Leu Pro Leu Leu
1               5                   10                  15

Ala Ile Ile Asn Gln Ile Val Ala His Val Arg Thr Asn Tyr Leu Met
            20                  25                  30

Lys Lys Leu Gly Ala Lys Pro Phe Thr His Val Gln Arg Asp Gly Trp
        35                  40                  45

Leu Gly Phe Lys Phe Gly Arg Glu Phe Leu Lys Ala Lys Ser Ala Gly
    50                  55                  60

Arg Leu Val Asp Leu Ile Ile Ser Arg Phe His Asp Asn Glu Asp Thr
65                  70                  75                  80

Phe Ser Ser Tyr Ala Phe Gly Asn His Val Val Phe Thr Arg Asp Pro
                85                  90                  95

Glu Asn Ile Lys Ala Leu Leu Ala Thr Gln Phe Gly Asp Phe Ser Leu
            100                 105                 110

Gly Ser Arg Val Lys Phe Phe Lys Pro Leu Leu Gly Tyr Gly Ile Phe
        115                 120                 125

Thr Leu Asp Ala Glu Gly Trp Lys His Ser Arg Ala Met Leu Arg Pro
    130                 135                 140

Gln Phe Ala Arg Glu Gln Val Ala His Val Thr Ser Leu Glu Pro His
145                 150                 155                 160

Phe Gln Leu Leu Lys Lys His Ile Leu Lys His Lys Gly Glu Tyr Phe
                165                 170                 175

Asp Ile Gln Glu Leu Phe Phe Arg Phe Thr Val Asp Ser Ala Thr Glu
            180                 185                 190

Phe Leu Phe Gly Glu Ser Val His Ser Leu Lys Asp Glu Glu Ile Gly
        195                 200                 205

Tyr Asp Thr Lys Asp Met Ser Glu Glu Arg Arg Arg Phe Ala Asp Ala
    210                 215                 220

Phe Asn Lys Ser Gln Val Tyr Val Ala Thr Arg Val Ala Leu Gln Asn
225                 230                 235                 240

Leu Tyr Trp Leu Val Asn Asn Lys Glu Phe Lys Glu Cys Asn Asp Ile

                245                 250                 255

Val His Lys Phe Thr Asn Tyr Tyr Val Gln Lys Ala Leu Asp Ala Thr
            260                 265                 270

Pro Glu Glu Leu Glu Lys Gln Gly Gly Tyr Val Phe Leu Tyr Glu Leu
        275                 280                 285

Val Lys Gln Thr Arg Asp Pro Lys Val Leu Arg Asp Gln Ser Leu Asn
    290                 295                 300

Ile Leu Leu Ala Gly Arg Asp Thr Thr Ala Gly Leu Leu Ser Phe Ala
305                 310                 315                 320

Val Phe Glu Leu Ala Arg Asn Pro His Ile Trp Ala Lys Leu Arg Glu
                325                 330                 335

Glu Ile Glu Gln Gln Phe Gly Leu Gly Glu Asp Ser Arg Val Glu Glu
            340                 345                 350

Ile Thr Phe Glu Ser Leu Lys Arg Cys Glu Tyr Leu Lys Ala Val Leu
        355                 360                 365

Asn Glu Thr Leu Arg Leu His Pro Ser Val Pro Arg Asn Ala Arg Phe
    370                 375                 380

Ala Ile Lys Asp Thr Thr Leu Pro Arg Gly Gly Gly Pro Asn Gly Lys
385                 390                 395                 400

Asp Pro Ile Leu Ile Arg Lys Asp Glu Val Val Gln Tyr Ser Ile Ser
                405                 410                 415

Ala Thr Gln Thr Asn Pro Ala Tyr Tyr Gly Ala Asp Ala Ala Asp Phe
            420                 425                 430

Arg Pro Glu Arg Trp Phe Glu Pro Ser Thr Arg Asn Leu Gly Trp Ala
        435                 440                 445

Phe Leu Pro Phe Asn Gly Gly Pro Arg Ile Cys Leu Gly Gln Gln Phe
    450                 455                 460

Ala Leu Thr Glu Ala Gly Tyr Val Leu Val Arg Leu Val Gln Glu Phe
465                 470                 475                 480

Pro Asn Leu Ser Gln Asp Pro Glu Thr Lys Tyr Pro Pro Pro Arg Leu
                485                 490                 495

Ala His Leu Thr Met Cys Leu Phe Asp Gly Ala His Val Lys Met Ser

500                 505                 510

<210>  19
<211>  1539
<212>  DNA
<213>  Candida tropicalis

<220>
<221>  CDS
<222>  (1)..(1539)

<400>  19
atg ctc gac cag atc ttc cat tac tgg tac att gtc ttg cca ttg ttg       48
Met Leu Asp Gln Ile Phe His Tyr Trp Tyr Ile Val Leu Pro Leu Leu
1               5                   10                  15

gtc att atc aag cag atc gtg gct cat gcc agg acc aat tat ttg atg       96
Val Ile Ile Lys Gln Ile Val Ala His Ala Arg Thr Asn Tyr Leu Met
            20                  25                  30

aag aag ttg ggc gct aag cca ttc aca cat gtc caa cta gac ggg tgg      144
Lys Lys Leu Gly Ala Lys Pro Phe Thr His Val Gln Leu Asp Gly Trp
        35                  40                  45

ttt ggc ttc aaa ttt ggc cgt gaa ttc ctc aaa gct aaa agt gct ggg      192
Phe Gly Phe Lys Phe Gly Arg Glu Phe Leu Lys Ala Lys Ser Ala Gly
    50                  55                  60

agg cag gtt gat tta atc atc tcc cgt ttc cac gat aat gag gac act      240
Arg Gln Val Asp Leu Ile Ile Ser Arg Phe His Asp Asn Glu Asp Thr
65                  70                  75                  80

ttc tcc agc tat gct ttt ggc aac cat gtg gtg ttc acc agg gac ccc      288
Phe Ser Ser Tyr Ala Phe Gly Asn His Val Val Phe Thr Arg Asp Pro
                85                  90                  95

gag aat atc aag gcg ctt ttg gca acc cag ttt ggt gat ttt tca ttg      336
Glu Asn Ile Lys Ala Leu Leu Ala Thr Gln Phe Gly Asp Phe Ser Leu
            100                 105                 110

gga agc agg gtc aaa ttc ttc aaa cca ttg ttg ggg tac ggt atc ttc      384
Gly Ser Arg Val Lys Phe Phe Lys Pro Leu Leu Gly Tyr Gly Ile Phe
        115                 120                 125

acc ttg gac ggc gaa ggc tgg aag cac agc aga gcc atg ttg aga cca      432
Thr Leu Asp Gly Glu Gly Trp Lys His Ser Arg Ala Met Leu Arg Pro
    130                 135                 140

cag ttt gcc aga gag caa gtt gct cat gtg acg tcg ttg gaa cca cat      480
Gln Phe Ala Arg Glu Gln Val Ala His Val Thr Ser Leu Glu Pro His
145                 150                 155                 160

ttc cag ttg ttg aag aag cat att ctt aag cac aag ggt gaa tac ttt      528
Phe Gln Leu Leu Lys Lys His Ile Leu Lys His Lys Gly Glu Tyr Phe
                165                 170                 175

gat atc cag gaa ttg ttc ttt aga ttt acc gtt gat tca gcg acg gag      576
Asp Ile Gln Glu Leu Phe Phe Arg Phe Thr Val Asp Ser Ala Thr Glu
            180                 185                 190

ttc tta ttt ggt gag tcc gtg cac tcc tta agg gac gag gaa att ggc      624
Phe Leu Phe Gly Glu Ser Val His Ser Leu Arg Asp Glu Glu Ile Gly

        195                 200                 205

tac gat acg aag gac atg gct gaa gaa aga cgc aaa ttt gcc gac gcg 672
Tyr Asp Thr Lys Asp Met Ala Glu Glu Arg Arg Lys Phe Ala Asp Ala
    210                 215                 220

ttc aac aag tcg caa gtc tat ttg tcc acc aga gtt gct tta cag aca 720
Phe Asn Lys Ser Gln Val Tyr Leu Ser Thr Arg Val Ala Leu Gln Thr
225                 230                 235                 240

ttg tac tgg ttg gtc aac aac aaa gag ttc aag gag tgc aac gac att 768
Leu Tyr Trp Leu Val Asn Asn Lys Glu Phe Lys Glu Cys Asn Asp Ile
                245                 250                 255

gtc cac aag ttc acc aac tac tat gtt cag aaa gcc ttg gat gct acc 816
Val His Lys Phe Thr Asn Tyr Tyr Val Gln Lys Ala Leu Asp Ala Thr
            260                 265                 270

cca gag gaa ctt gaa aaa caa ggc ggg tat gtg ttc ttg tac gag ctt 864
Pro Glu Glu Leu Glu Lys Gln Gly Gly Tyr Val Phe Leu Tyr Glu Leu
        275                 280                 285

gcc aag cag acg aaa gac ccc aat gtg ttg cgt gac cag tct ttg aac 912
Ala Lys Gln Thr Lys Asp Pro Asn Val Leu Arg Asp Gln Ser Leu Asn
    290                 295                 300

atc ttg ttg gct gga agg gac acc act gct ggg ttg ttg tcc ttt gct 960
Ile Leu Leu Ala Gly Arg Asp Thr Thr Ala Gly Leu Leu Ser Phe Ala
305                 310                 315                 320

gtg ttt gag ttg gcc agg aac cca cac atc tgg gcc aag ttg aga gag 1008
Val Phe Glu Leu Ala Arg Asn Pro His Ile Trp Ala Lys Leu Arg Glu
                325                 330                 335

gaa att gaa tca cac ttt ggg ctg ggt gag gac tct cgt gtt gaa gag 1056
Glu Ile Glu Ser His Phe Gly Leu Gly Glu Asp Ser Arg Val Glu Glu
            340                 345                 350

att acc ttt gag agc ttg aag aga tgt gag tac ttg aaa gcc gtg ttg 1104
Ile Thr Phe Glu Ser Leu Lys Arg Cys Glu Tyr Leu Lys Ala Val Leu
        355                 360                 365

aac gaa acg ttg aga tta cac cca agt gtc cca aga aac gca aga ttt 1152
Asn Glu Thr Leu Arg Leu His Pro Ser Val Pro Arg Asn Ala Arg Phe
    370                 375                 380

gcg att aaa gac acg act tta cca aga ggc ggt ggc ccc aac ggc aag 1200
Ala Ile Lys Asp Thr Thr Leu Pro Arg Gly Gly Gly Pro Asn Gly Lys
385                 390                 395                 400

gat cct atc ttg atc aga aag aat gag gtg gtg caa tac tcc atc tcg 1248
Asp Pro Ile Leu Ile Arg Lys Asn Glu Val Val Gln Tyr Ser Ile Ser
                405                 410                 415

gca act cag aca aat cct gct tat tat ggc gcc gat gct gct gat ttt 1296
Ala Thr Gln Thr Asn Pro Ala Tyr Tyr Gly Ala Asp Ala Ala Asp Phe
            420                 425                 430

aga ccg gaa aga tgg ttt gag cca tca act aga aac ttg gga tgg gct 1344
Arg Pro Glu Arg Trp Phe Glu Pro Ser Thr Arg Asn Leu Gly Trp Ala
        435                 440                 445

tac ttg cca ttc aac ggt ggt cca aga atc tgc ttg gga caa cag ttt 1392
Tyr Leu Pro Phe Asn Gly Gly Pro Arg Ile Cys Leu Gly Gln Gln Phe

450                 455                 460

gct ttg acc gaa gcc ggt tac gtt ttg gtt aga ctt gtt cag gaa ttc      1440
Ala Leu Thr Glu Ala Gly Tyr Val Leu Val Arg Leu Val Gln Glu Phe
465                 470                 475                 480

cct agc ttg tca cag gac ccc gaa act gag tac cca cca cct aga ttg      1488
Pro Ser Leu Ser Gln Asp Pro Glu Thr Glu Tyr Pro Pro Pro Arg Leu
                485                 490                 495

gca cac ttg acg atg tgc ttg ttt gac ggg gca tac gtc aag atg caa      1536
Ala His Leu Thr Met Cys Leu Phe Asp Gly Ala Tyr Val Lys Met Gln
            500                 505                 510

tag                                                                  1539

<210>  20
<211>  512
<212>  PRT
<213>  Candida tropicalis

<400>  20

Met Leu Asp Gln Ile Phe His Tyr Trp Tyr Ile Val Leu Pro Leu Leu
1               5                   10                  15

Val Ile Ile Lys Gln Ile Val Ala His Ala Arg Thr Asn Tyr Leu Met
            20                  25                  30

Lys Lys Leu Gly Ala Lys Pro Phe Thr His Val Gln Leu Asp Gly Trp
        35                  40                  45

Phe Gly Phe Lys Phe Gly Arg Glu Phe Leu Lys Ala Lys Ser Ala Gly
    50                  55                  60

Arg Gln Val Asp Leu Ile Ile Ser Arg Phe His Asp Asn Glu Asp Thr
65                  70                  75                  80

Phe Ser Ser Tyr Ala Phe Gly Asn His Val Val Phe Thr Arg Asp Pro
                85                  90                  95

Glu Asn Ile Lys Ala Leu Leu Ala Thr Gln Phe Gly Asp Phe Ser Leu
            100                 105                 110

Gly Ser Arg Val Lys Phe Phe Lys Pro Leu Leu Gly Tyr Gly Ile Phe
        115                 120                 125

Thr Leu Asp Gly Glu Gly Trp Lys His Ser Arg Ala Met Leu Arg Pro
    130                 135                 140

Gln Phe Ala Arg Glu Gln Val Ala His Val Thr Ser Leu Glu Pro His
145                 150                 155                 160

Phe Gln Leu Leu Lys Lys His Ile Leu Lys His Lys Gly Glu Tyr Phe

                165                 170                 175

Asp Ile Gln Glu Leu Phe Phe Arg Phe Thr Val Asp Ser Ala Thr Glu
            180                 185                 190

Phe Leu Phe Gly Glu Ser Val His Ser Leu Arg Asp Glu Glu Ile Gly
        195                 200                 205

Tyr Asp Thr Lys Asp Met Ala Glu Glu Arg Arg Lys Phe Ala Asp Ala
    210                 215                 220

Phe Asn Lys Ser Gln Val Tyr Leu Ser Thr Arg Val Ala Leu Gln Thr
225                 230                 235                 240

Leu Tyr Trp Leu Val Asn Asn Lys Glu Phe Lys Glu Cys Asn Asp Ile
                245                 250                 255

Val His Lys Phe Thr Asn Tyr Tyr Val Gln Lys Ala Leu Asp Ala Thr
            260                 265                 270

Pro Glu Glu Leu Glu Lys Gln Gly Gly Tyr Val Phe Leu Tyr Glu Leu
        275                 280                 285

Ala Lys Gln Thr Lys Asp Pro Asn Val Leu Arg Asp Gln Ser Leu Asn
    290                 295                 300

Ile Leu Leu Ala Gly Arg Asp Thr Thr Ala Gly Leu Leu Ser Phe Ala
305                 310                 315                 320

Val Phe Glu Leu Ala Arg Asn Pro His Ile Trp Ala Lys Leu Arg Glu
                325                 330                 335

Glu Ile Glu Ser His Phe Gly Leu Gly Glu Asp Ser Arg Val Glu Glu
            340                 345                 350

Ile Thr Phe Glu Ser Leu Lys Arg Cys Glu Tyr Leu Lys Ala Val Leu
        355                 360                 365

Asn Glu Thr Leu Arg Leu His Pro Ser Val Pro Arg Asn Ala Arg Phe
    370                 375                 380

Ala Ile Lys Asp Thr Thr Leu Pro Arg Gly Gly Gly Pro Asn Gly Lys
385                 390                 395                 400

Asp Pro Ile Leu Ile Arg Lys Asn Glu Val Val Gln Tyr Ser Ile Ser
                405                 410                 415

Ala Thr Gln Thr Asn Pro Ala Tyr Tyr Gly Ala Asp Ala Ala Asp Phe

420                 425                 430

Arg Pro Glu Arg Trp Phe Glu Pro Ser Thr Arg Asn Leu Gly Trp Ala
        435                 440                 445

Tyr Leu Pro Phe Asn Gly Gly Pro Arg Ile Cys Leu Gly Gln Gln Phe
    450                 455                 460

Ala Leu Thr Glu Ala Gly Tyr Val Leu Val Arg Leu Val Gln Glu Phe
465                 470                 475                 480

Pro Ser Leu Ser Gln Asp Pro Glu Thr Glu Tyr Pro Pro Pro Arg Leu
                485                 490                 495

Ala His Leu Thr Met Cys Leu Phe Asp Gly Ala Tyr Val Lys Met Gln
            500                 505                 510

<210>  21
<211>  390
<212>  DNA
<213>  Candida tropicalis

<220>
<221>  CDS
<222>  (1)..(390)

<400>  21
atg acc gac aca gac acc acg acc acc atc tac acc cac gaa gag gtt       48
Met Thr Asp Thr Asp Thr Thr Thr Thr Ile Tyr Thr His Glu Glu Val
1               5                   10                  15

gcc cag cac acc acc cac gac gac ttg tgg gtt att ctc aat ggt aag       96
Ala Gln His Thr Thr His Asp Asp Leu Trp Val Ile Leu Asn Gly Lys
            20                  25                  30

gtc tac aac atc tcc aac tat ata gac gag cac cca ggt ggt gaa gaa      144
Val Tyr Asn Ile Ser Asn Tyr Ile Asp Glu His Pro Gly Gly Glu Glu
        35                  40                  45

gtc att ctt gat tgc gcc ggc aca gac gcc act gaa gcc ttt gac gac      192
Val Ile Leu Asp Cys Ala Gly Thr Asp Ala Thr Glu Ala Phe Asp Asp
    50                  55                  60

att ggc cac tcc gac gag gcc cac gag atc ttg gaa aag ttg tac att      240
Ile Gly His Ser Asp Glu Ala His Glu Ile Leu Glu Lys Leu Tyr Ile
65                  70                  75                  80

ggt aac ttg aag ggc gct aag att gtt gag gcc aag cac gcg cag tcg      288
Gly Asn Leu Lys Gly Ala Lys Ile Val Glu Ala Lys His Ala Gln Ser
                85                  90                  95

ttc agc acg gaa gaa gac tcg ggt atc aac ttc cca ttg att gct gtt      336
Phe Ser Thr Glu Glu Asp Ser Gly Ile Asn Phe Pro Leu Ile Ala Val
            100                 105                 110

ggt gtg ttt ttg gct gct ttc ggt gtc tac tac tac aag acc aac ttt      384
Gly Val Phe Leu Ala Ala Phe Gly Val Tyr Tyr Tyr Lys Thr Asn Phe

115                 120                 125

gcc taa                                                           390
Ala

<210>  22
<211>  129
<212>  PRT
<213>  Candida tropicalis

<400>  22

Met Thr Asp Thr Asp Thr Thr Thr Thr Ile Tyr Thr His Glu Glu Val
1               5                   10                  15

Ala Gln His Thr Thr His Asp Asp Leu Trp Val Ile Leu Asn Gly Lys
            20                  25                  30

Val Tyr Asn Ile Ser Asn Tyr Ile Asp Glu His Pro Gly Gly Glu Glu
        35                  40                  45

Val Ile Leu Asp Cys Ala Gly Thr Asp Ala Thr Glu Ala Phe Asp Asp
    50                  55                  60

Ile Gly His Ser Asp Glu Ala His Glu Ile Leu Glu Lys Leu Tyr Ile
65                  70                  75                  80

Gly Asn Leu Lys Gly Ala Lys Ile Val Glu Ala Lys His Ala Gln Ser
                85                  90                  95

Phe Ser Thr Glu Glu Asp Ser Gly Ile Asn Phe Pro Leu Ile Ala Val
            100                 105                 110

Gly Val Phe Leu Ala Ala Phe Gly Val Tyr Tyr Tyr Lys Thr Asn Phe
        115                 120                 125

Ala

<210>  23
<211>  2115
<212>  DNA
<213>  Candida tropicalis

<220>
<221>  CDS
<222>  (1)..(2115)

<400>  23
atg gct cca ttt ttg ccc gac cag gtc gac tac aaa cac gtc gac acc      48
Met Ala Pro Phe Leu Pro Asp Gln Val Asp Tyr Lys His Val Asp Thr
1               5                   10                  15

ctt atg tta tta tgt gac ggg atc atc cac gaa acc acc gtg gac gaa 96
Leu Met Leu Leu Cys Asp Gly Ile Ile His Glu Thr Thr Val Asp Glu
            20                  25                  30

atc aaa gac gtc att gcc cct gac ttc ccc gcc gac aaa tac gag gag 144
Ile Lys Asp Val Ile Ala Pro Asp Phe Pro Ala Asp Lys Tyr Glu Glu
        35                  40                  45

tac gtc agg aca ttc acc aaa ccc tcc gaa acc cca ggg ttc agg gaa 192
Tyr Val Arg Thr Phe Thr Lys Pro Ser Glu Thr Pro Gly Phe Arg Glu
    50                  55                  60

acc gtc tac aac acc gtc aac gca aac acc atg gat gca atc cac cag 240
Thr Val Tyr Asn Thr Val Asn Ala Asn Thr Met Asp Ala Ile His Gln
65                  70                  75                  80

ttc att atc ttg acc aat gtt ttg gga tca agg gtc ttg gca cca gct 288
Phe Ile Ile Leu Thr Asn Val Leu Gly Ser Arg Val Leu Ala Pro Ala
                85                  90                  95

ttg acc aac tcg ttg act cct atc aag gac atg agc ttg gaa gac cgt 336
Leu Thr Asn Ser Leu Thr Pro Ile Lys Asp Met Ser Leu Glu Asp Arg
            100                 105                 110

gaa aag ttg tta gcc tcg tgg cgt gac tcc cct att gct gct aaa agg 384
Glu Lys Leu Leu Ala Ser Trp Arg Asp Ser Pro Ile Ala Ala Lys Arg
        115                 120                 125

aag ttg ttc agg ttg gtt tct acg ctt acc ttg gtc acg ttc acg aga 432
Lys Leu Phe Arg Leu Val Ser Thr Leu Thr Leu Val Thr Phe Thr Arg
    130                 135                 140

ttg gcc aat gag ttg cat ttg aaa gcc att cat tat cca gga aga gaa 480
Leu Ala Asn Glu Leu His Leu Lys Ala Ile His Tyr Pro Gly Arg Glu
145                 150                 155                 160

gac cgt gaa aag gct tat gaa acc cag gag att gac cct ttt aag tac 528
Asp Arg Glu Lys Ala Tyr Glu Thr Gln Glu Ile Asp Pro Phe Lys Tyr
                165                 170                 175

cag ttt ttg gaa aaa ccg aag ttt tac ggc gct gag ttg tac ttg cca 576
Gln Phe Leu Glu Lys Pro Lys Phe Tyr Gly Ala Glu Leu Tyr Leu Pro
            180                 185                 190

gat att gat gtg atc att att gga tct ggg gcc ggt gct ggt gtc gtg 624
Asp Ile Asp Val Ile Ile Ile Gly Ser Gly Ala Gly Ala Gly Val Val
        195                 200                 205

gcc cac act ttg acc aac gac ggc ttc aag agt ttg gtt ttg gaa aag 672
Ala His Thr Leu Thr Asn Asp Gly Phe Lys Ser Leu Val Leu Glu Lys
    210                 215                 220

ggc aga tac ttt agc aac tcc gag ttg aac ttt gat gac aag gac ggg 720
Gly Arg Tyr Phe Ser Asn Ser Glu Leu Asn Phe Asp Asp Lys Asp Gly
225                 230                 235                 240

gtt caa gaa tta tac caa agt gga ggt act ttg acc acc gtc aac cag 768
Val Gln Glu Leu Tyr Gln Ser Gly Gly Thr Leu Thr Thr Val Asn Gln
                245                 250                 255

cag ttg ttt gtt ctt gct ggt tcc act ttt ggt ggt ggt acc act gtc 816
Gln Leu Phe Val Leu Ala Gly Ser Thr Phe Gly Gly Gly Thr Thr Val
            260                 265                 270

aat tgg tcg gcc tgt ctt aaa acg cca ttc aag gtg cgt aag gaa tgg 864
Asn Trp Ser Ala Cys Leu Lys Thr Pro Phe Lys Val Arg Lys Glu Trp
        275                 280                 285

tat gat gag ttt ggc gtt gac ttt gct gcc gat gaa gcc tac gac aaa 912
Tyr Asp Glu Phe Gly Val Asp Phe Ala Ala Asp Glu Ala Tyr Asp Lys
    290                 295                 300

gca cag gat tat gtt tgg cag caa atg gga gct tct acc gaa ggc atc 960
Ala Gln Asp Tyr Val Trp Gln Gln Met Gly Ala Ser Thr Glu Gly Ile
305                 310                 315                 320

acc cac tct ttg gct aac gag att att att gaa ggt ggc aag aaa tta 1008
Thr His Ser Leu Ala Asn Glu Ile Ile Ile Glu Gly Gly Lys Lys Leu
                325                 330                 335

ggt tac aag gcc aag gta tta gac caa aac agc ggt ggt cat cct cat 1056
Gly Tyr Lys Ala Lys Val Leu Asp Gln Asn Ser Gly Gly His Pro His
            340                 345                 350

cac aga tgc ggt ttc tgt tat ttg ggt tgt aag cac ggt atc aag cag 1104
His Arg Cys Gly Phe Cys Tyr Leu Gly Cys Lys His Gly Ile Lys Gln
        355                 360                 365

ggc tct gtt aat aac tgg ttt aga gac gca gct gcc cac ggt tct cag 1152
Gly Ser Val Asn Asn Trp Phe Arg Asp Ala Ala Ala His Gly Ser Gln
    370                 375                 380

ttc atg caa cag gtt aga gtt ttg caa atc ctt aac aag aag ggc atc 1200
Phe Met Gln Gln Val Arg Val Leu Gln Ile Leu Asn Lys Lys Gly Ile
385                 390                 395                 400

gct tat ggt atc ttg tgt gag gat gtt gta acc ggt gcc aag ttc acc 1248
Ala Tyr Gly Ile Leu Cys Glu Asp Val Val Thr Gly Ala Lys Phe Thr
                405                 410                 415

att act ggc ccc aaa aag ttt gtt gtt gcc gcc ggc gcc tta aac act 1296
Ile Thr Gly Pro Lys Lys Phe Val Val Ala Ala Gly Ala Leu Asn Thr
            420                 425                 430

cca tct gtg ttg gtc aac tcc gga ttc aag aac aag aac atc ggt aag 1344
Pro Ser Val Leu Val Asn Ser Gly Phe Lys Asn Lys Asn Ile Gly Lys
        435                 440                 445

aac tta act ttg cat cca gtt tct gtc gtg ttt ggt gat ttt ggc aaa 1392
Asn Leu Thr Leu His Pro Val Ser Val Val Phe Gly Asp Phe Gly Lys
    450                 455                 460

gac gtt caa gca gat cac ttc cac aac tcc atc atg act gct ctt tgt 1440
Asp Val Gln Ala Asp His Phe His Asn Ser Ile Met Thr Ala Leu Cys
465                 470                 475                 480

tca gaa gcc gct gat tta gac ggc aag ggt cat gga tgc aga att gaa 1488
Ser Glu Ala Ala Asp Leu Asp Gly Lys Gly His Gly Cys Arg Ile Glu
                485                 490                 495

acc atc ttg aac gct cca ttc atc cag gct tca ttc tta cca tgg aga 1536
Thr Ile Leu Asn Ala Pro Phe Ile Gln Ala Ser Phe Leu Pro Trp Arg
            500                 505                 510

ggt agt aac gag gct aga cga gac ttg ttg cgt tac aac aac atg gtg 1584
Gly Ser Asn Glu Ala Arg Arg Asp Leu Leu Arg Tyr Asn Asn Met Val
        515                 520                 525

gcc atg tta ctt ctt agt cgt gat acc acc agt ggt tcc gtt tcg tcc     1632
Ala Met Leu Leu Leu Ser Arg Asp Thr Thr Ser Gly Ser Val Ser Ser
    530                 535                 540

cat cca act aaa cct gaa gca tta gtt gtc gag tac gac gtg aac aag     1680
His Pro Thr Lys Pro Glu Ala Leu Val Val Glu Tyr Asp Val Asn Lys
545                 550                 555                 560

ttt gac aga aac tcc atc ttg cag gca ttg ttg gtc act gct gac ttg     1728
Phe Asp Arg Asn Ser Ile Leu Gln Ala Leu Leu Val Thr Ala Asp Leu
                565                 570                 575

ttg tac att caa ggt gcc aag aga atc ctt agt ccc caa cca tgg gtg     1776
Leu Tyr Ile Gln Gly Ala Lys Arg Ile Leu Ser Pro Gln Pro Trp Val
            580                 585                 590

cca att ttt gaa tcc gac aag cca aag gat aag aga tca atc aag gac     1824
Pro Ile Phe Glu Ser Asp Lys Pro Lys Asp Lys Arg Ser Ile Lys Asp
        595                 600                 605

gag gac tat gtc gaa tgg aga gcc aag gtt gcc aag att cct ttt gac     1872
Glu Asp Tyr Val Glu Trp Arg Ala Lys Val Ala Lys Ile Pro Phe Asp
    610                 615                 620

acc tac ggc tcg cct tat ggt tcg gcg cat caa atg tct tct tgt cgt     1920
Thr Tyr Gly Ser Pro Tyr Gly Ser Ala His Gln Met Ser Ser Cys Arg
625                 630                 635                 640

atg tca ggt aag ggt cct aaa tac ggt gct gtt gat acc gat ggt aga     1968
Met Ser Gly Lys Gly Pro Lys Tyr Gly Ala Val Asp Thr Asp Gly Arg
                645                 650                 655

ttg ttt gaa tgt tcg aat gtt tat gtt gct gac gct agt ctt ttg cca     2016
Leu Phe Glu Cys Ser Asn Val Tyr Val Ala Asp Ala Ser Leu Leu Pro
            660                 665                 670

act gct agc ggt gct aat cct atg gtc acc acc atg act ctt gca aga     2064
Thr Ala Ser Gly Ala Asn Pro Met Val Thr Thr Met Thr Leu Ala Arg
        675                 680                 685

cat gtt gcg tta ggt ttg gca gac tcc ttg aag acc aag gcc aag ttg     2112
His Val Ala Leu Gly Leu Ala Asp Ser Leu Lys Thr Lys Ala Lys Leu
    690                 695                 700

tag                                                                 2115


<210>  24
<211>  704
<212>  PRT
<213>  Candida tropicalis

<400>  24

Met Ala Pro Phe Leu Pro Asp Gln Val Asp Tyr Lys His Val Asp Thr
1               5                   10                  15


Leu Met Leu Leu Cys Asp Gly Ile Ile His Glu Thr Thr Val Asp Glu
            20                  25                  30


Ile Lys Asp Val Ile Ala Pro Asp Phe Pro Ala Asp Lys Tyr Glu Glu

        35                  40                  45

Tyr Val Arg Thr Phe Thr Lys Pro Ser Glu Thr Pro Gly Phe Arg Glu
    50                  55                  60

Thr Val Tyr Asn Thr Val Asn Ala Asn Thr Met Asp Ala Ile His Gln
65                  70                  75                  80

Phe Ile Ile Leu Thr Asn Val Leu Gly Ser Arg Val Leu Ala Pro Ala
                85                  90                  95

Leu Thr Asn Ser Leu Thr Pro Ile Lys Asp Met Ser Leu Glu Asp Arg
            100                 105                 110

Glu Lys Leu Leu Ala Ser Trp Arg Asp Ser Pro Ile Ala Ala Lys Arg
        115                 120                 125

Lys Leu Phe Arg Leu Val Ser Thr Leu Thr Leu Val Thr Phe Thr Arg
    130                 135                 140

Leu Ala Asn Glu Leu His Leu Lys Ala Ile His Tyr Pro Gly Arg Glu
145                 150                 155                 160

Asp Arg Glu Lys Ala Tyr Glu Thr Gln Glu Ile Asp Pro Phe Lys Tyr
                165                 170                 175

Gln Phe Leu Glu Lys Pro Lys Phe Tyr Gly Ala Glu Leu Tyr Leu Pro
            180                 185                 190

Asp Ile Asp Val Ile Ile Ile Gly Ser Gly Ala Gly Ala Gly Val Val
        195                 200                 205

Ala His Thr Leu Thr Asn Asp Gly Phe Lys Ser Leu Val Leu Glu Lys
    210                 215                 220

Gly Arg Tyr Phe Ser Asn Ser Glu Leu Asn Phe Asp Asp Lys Asp Gly
225                 230                 235                 240

Val Gln Glu Leu Tyr Gln Ser Gly Gly Thr Leu Thr Thr Val Asn Gln
                245                 250                 255

Gln Leu Phe Val Leu Ala Gly Ser Thr Phe Gly Gly Gly Thr Thr Val
            260                 265                 270

Asn Trp Ser Ala Cys Leu Lys Thr Pro Phe Lys Val Arg Lys Glu Trp
        275                 280                 285

Tyr Asp Glu Phe Gly Val Asp Phe Ala Ala Asp Glu Ala Tyr Asp Lys

290                 295                 300

Ala Gln Asp Tyr Val Trp Gln Gln Met Gly Ala Ser Thr Glu Gly Ile
305                 310                 315                 320

Thr His Ser Leu Ala Asn Glu Ile Ile Ile Glu Gly Gly Lys Lys Leu
                325                 330                 335

Gly Tyr Lys Ala Lys Val Leu Asp Gln Asn Ser Gly Gly His Pro His
            340                 345                 350

His Arg Cys Gly Phe Cys Tyr Leu Gly Cys Lys His Gly Ile Lys Gln
        355                 360                 365

Gly Ser Val Asn Asn Trp Phe Arg Asp Ala Ala Ala His Gly Ser Gln
    370                 375                 380

Phe Met Gln Gln Val Arg Val Leu Gln Ile Leu Asn Lys Lys Gly Ile
385                 390                 395                 400

Ala Tyr Gly Ile Leu Cys Glu Asp Val Val Thr Gly Ala Lys Phe Thr
                405                 410                 415

Ile Thr Gly Pro Lys Lys Phe Val Val Ala Ala Gly Ala Leu Asn Thr
            420                 425                 430

Pro Ser Val Leu Val Asn Ser Gly Phe Lys Asn Lys Asn Ile Gly Lys
        435                 440                 445

Asn Leu Thr Leu His Pro Val Ser Val Val Phe Gly Asp Phe Gly Lys
    450                 455                 460

Asp Val Gln Ala Asp His Phe His Asn Ser Ile Met Thr Ala Leu Cys
465                 470                 475                 480

Ser Glu Ala Ala Asp Leu Asp Gly Lys Gly His Gly Cys Arg Ile Glu
                485                 490                 495

Thr Ile Leu Asn Ala Pro Phe Ile Gln Ala Ser Phe Leu Pro Trp Arg
            500                 505                 510

Gly Ser Asn Glu Ala Arg Arg Asp Leu Leu Arg Tyr Asn Asn Met Val
        515                 520                 525

Ala Met Leu Leu Leu Ser Arg Asp Thr Thr Ser Gly Ser Val Ser Ser
    530                 535                 540

His Pro Thr Lys Pro Glu Ala Leu Val Val Glu Tyr Asp Val Asn Lys

545                 550                 555                 560

Phe Asp Arg Asn Ser Ile Leu Gln Ala Leu Leu Val Thr Ala Asp Leu
                565                 570                 575

Leu Tyr Ile Gln Gly Ala Lys Arg Ile Leu Ser Pro Gln Pro Trp Val
            580                 585                 590

Pro Ile Phe Glu Ser Asp Lys Pro Lys Asp Lys Arg Ser Ile Lys Asp
        595                 600                 605

Glu Asp Tyr Val Glu Trp Arg Ala Lys Val Ala Lys Ile Pro Phe Asp
    610                 615                 620

Thr Tyr Gly Ser Pro Tyr Gly Ser Ala His Gln Met Ser Ser Cys Arg
625                 630                 635                 640

Met Ser Gly Lys Gly Pro Lys Tyr Gly Ala Val Asp Thr Asp Gly Arg
                645                 650                 655

Leu Phe Glu Cys Ser Asn Val Tyr Val Ala Asp Ala Ser Leu Leu Pro
            660                 665                 670

Thr Ala Ser Gly Ala Asn Pro Met Val Thr Thr Met Thr Leu Ala Arg
        675                 680                 685

His Val Ala Leu Gly Leu Ala Asp Ser Leu Lys Thr Lys Ala Lys Leu
    690                 695                 700

<210>  25
<211>  2115
<212>  DNA
<213>  Candida tropicalis

<220>
<221>  CDS
<222>  (1)..(2115)

<400>  25
atg aat acc ttc ttg cca gac gtg ctc gaa tac aaa cac gtc gac acc       48
Met Asn Thr Phe Leu Pro Asp Val Leu Glu Tyr Lys His Val Asp Thr
1               5                   10                  15

ctt ttg tta ttg tgt gac ggg atc atc cac gaa acc aca gtc gat cag       96
Leu Leu Leu Leu Cys Asp Gly Ile Ile His Glu Thr Thr Val Asp Gln
            20                  25                  30

atc aag gac gcc att gct ccc gac ttc cct gag gac cag tac gag gag      144
Ile Lys Asp Ala Ile Ala Pro Asp Phe Pro Glu Asp Gln Tyr Glu Glu
        35                  40                  45

tat ctc aag acc ttc acc aag cca tct gag acc cct ggg ttc aga gaa      192
Tyr Leu Lys Thr Phe Thr Lys Pro Ser Glu Thr Pro Gly Phe Arg Glu

    50                  55                  60

gcc gtc tac gac acg atc aac gcc acc cca acc gat gcc gtg cac atg 240
Ala Val Tyr Asp Thr Ile Asn Ala Thr Pro Thr Asp Ala Val His Met
65                  70                  75                  80

tgt att gtc ttg acc acc gca ttg gac tcc aga atc ttg gcc ccc acg 288
Cys Ile Val Leu Thr Thr Ala Leu Asp Ser Arg Ile Leu Ala Pro Thr
                85                  90                  95

ttg acc aac tcg ttg acg cct atc aag gat atg acc ttg aag gag cgt 336
Leu Thr Asn Ser Leu Thr Pro Ile Lys Asp Met Thr Leu Lys Glu Arg
            100                 105                 110

gaa caa ttg ttg gcc tct tgg cgt gat tcc ccg att gcg gca aag aga 384
Glu Gln Leu Leu Ala Ser Trp Arg Asp Ser Pro Ile Ala Ala Lys Arg
        115                 120                 125

aga ttg ttc aga ttg att tcc tcg ctt acc ttg acg acg ttt acg aga 432
Arg Leu Phe Arg Leu Ile Ser Ser Leu Thr Leu Thr Thr Phe Thr Arg
    130                 135                 140

ttg gcc agc gaa ttg cac ttg aaa gcc atc cac tac cct ggc aga gac 480
Leu Ala Ser Glu Leu His Leu Lys Ala Ile His Tyr Pro Gly Arg Asp
145                 150                 155                 160

ttg cgt gaa aag gcg tat gaa acc cag gtg gtt gac cct ttc agg tac 528
Leu Arg Glu Lys Ala Tyr Glu Thr Gln Val Val Asp Pro Phe Arg Tyr
                165                 170                 175

ctg ttt atg gag aaa cca aag ttt gac ggc gcc gaa ttg tac ttg cca 576
Leu Phe Met Glu Lys Pro Lys Phe Asp Gly Ala Glu Leu Tyr Leu Pro
            180                 185                 190

gat atc gac gtc atc atc att gga tca ggc gcc ggt gct ggt gtc atg 624
Asp Ile Asp Val Ile Ile Ile Gly Ser Gly Ala Gly Ala Gly Val Met
        195                 200                 205

gcc cac act ctc gcc aac gac ggg ttc aag acc ttg gtt ttg gaa aag 672
Ala His Thr Leu Ala Asn Asp Gly Phe Lys Thr Leu Val Leu Glu Lys
    210                 215                 220

gga aag tat ttc agc aac tcc gag ttg aac ttt aat gac gct gat ggc 720
Gly Lys Tyr Phe Ser Asn Ser Glu Leu Asn Phe Asn Asp Ala Asp Gly
225                 230                 235                 240

gtg aaa gag ttg tac caa ggt aaa ggt gct ttg gcc acc acc aat cag 768
Val Lys Glu Leu Tyr Gln Gly Lys Gly Ala Leu Ala Thr Thr Asn Gln
                245                 250                 255

cag atg ttt att ctt gcc ggt tcc act ttg ggc ggt ggt acc act gtc 816
Gln Met Phe Ile Leu Ala Gly Ser Thr Leu Gly Gly Gly Thr Thr Val
            260                 265                 270

aac tgg tct gct tgc ctt aaa aca cca ttt aaa gtg cgt aag gag tgg 864
Asn Trp Ser Ala Cys Leu Lys Thr Pro Phe Lys Val Arg Lys Glu Trp
        275                 280                 285

tac gac gag ttt ggt ctt gaa ttt gct gcc gat gaa gcc tac gac aaa 912
Tyr Asp Glu Phe Gly Leu Glu Phe Ala Ala Asp Glu Ala Tyr Asp Lys
    290                 295                 300

gcg cag gat tat gtt tgg aaa caa atg ggt gct tca aca gat gga atc 960
Ala Gln Asp Tyr Val Trp Lys Gln Met Gly Ala Ser Thr Asp Gly Ile

305                 310                 315                 320

act cac tcc ttg gcc aac gaa gtt gtg gtt gaa gga ggt aag aag ttg      1008
Thr His Ser Leu Ala Asn Glu Val Val Val Glu Gly Gly Lys Lys Leu
                325                 330                 335

ggc tac aag agc aag gaa att gag cag aac aac ggt ggc cac cct gac      1056
Gly Tyr Lys Ser Lys Glu Ile Glu Gln Asn Asn Gly Gly His Pro Asp
            340                 345                 350

cac cca tgt ggt ttc tgt tac ttg ggc tgt aag tac ggt att aaa cag      1104
His Pro Cys Gly Phe Cys Tyr Leu Gly Cys Lys Tyr Gly Ile Lys Gln
        355                 360                 365

ggt tct gtg aat aac tgg ttt aga gac gca gct gcc cac ggg tcc aag      1152
Gly Ser Val Asn Asn Trp Phe Arg Asp Ala Ala Ala His Gly Ser Lys
    370                 375                 380

ttc atg caa caa gtc aga gtt gtg caa atc ctc aac aag aat ggc gtc      1200
Phe Met Gln Gln Val Arg Val Val Gln Ile Leu Asn Lys Asn Gly Val
385                 390                 395                 400

gct tat ggt atc ttg tgt gag gat gtc gaa acc gga gtc agg ttc act      1248
Ala Tyr Gly Ile Leu Cys Glu Asp Val Glu Thr Gly Val Arg Phe Thr
                405                 410                 415

att agt ggc ccc aaa aag ttt gtt gtt tct gct ggt tct ttg aac acg      1296
Ile Ser Gly Pro Lys Lys Phe Val Val Ser Ala Gly Ser Leu Asn Thr
            420                 425                 430

cca act gtg ttg acc aac tcc gga ttc aag aac aag cac att ggt aag      1344
Pro Thr Val Leu Thr Asn Ser Gly Phe Lys Asn Lys His Ile Gly Lys
        435                 440                 445

aac ttg acg ttg cac cca gtt tcc acc gtg ttt ggt gac ttt ggc aga      1392
Asn Leu Thr Leu His Pro Val Ser Thr Val Phe Gly Asp Phe Gly Arg
    450                 455                 460

gac gtg caa gcc gac cat ttc cac aaa tct att atg act tcg ctt tgt      1440
Asp Val Gln Ala Asp His Phe His Lys Ser Ile Met Thr Ser Leu Cys
465                 470                 475                 480

tac gag gtt gct gac ttg gac ggc aag ggc cac gga tgc aga atc gaa      1488
Tyr Glu Val Ala Asp Leu Asp Gly Lys Gly His Gly Cys Arg Ile Glu
                485                 490                 495

acc atc ttg aac gct cca ttc atc caa gct tct ttg ttg cca tgg aga      1536
Thr Ile Leu Asn Ala Pro Phe Ile Gln Ala Ser Leu Leu Pro Trp Arg
            500                 505                 510

gga agt gac gag gtc aga aga gac ttg ttg cgt tac aac aac atg gtg      1584
Gly Ser Asp Glu Val Arg Arg Asp Leu Leu Arg Tyr Asn Asn Met Val
        515                 520                 525

gcc atg ttg ctt atc acg cgt gat acc acc agt ggt tca gtt tct gct      1632
Ala Met Leu Leu Ile Thr Arg Asp Thr Thr Ser Gly Ser Val Ser Ala
    530                 535                 540

gac cca aag aag ccc gac gct ttg att gtc gac tat gag att aac aag      1680
Asp Pro Lys Lys Pro Asp Ala Leu Ile Val Asp Tyr Glu Ile Asn Lys
545                 550                 555                 560

ttt gac aag aat gcc atc ttg caa gct ttc ttg atc act tcc gac atg      1728
Phe Asp Lys Asn Ala Ile Leu Gln Ala Phe Leu Ile Thr Ser Asp Met

565                 570                 575

ttg tac att gaa ggt gcc aag aga atc ctc agt cca cag cca tgg gtg      1776
Leu Tyr Ile Glu Gly Ala Lys Arg Ile Leu Ser Pro Gln Pro Trp Val
            580                 585                 590

cca atc ttt gag tcg aac aag cca aag gag caa aga acg atc aag gac      1824
Pro Ile Phe Glu Ser Asn Lys Pro Lys Glu Gln Arg Thr Ile Lys Asp
        595                 600                 605

aag gac tat gtt gag tgg aga gcc aag gct gct aag ata cct ttc gac      1872
Lys Asp Tyr Val Glu Trp Arg Ala Lys Ala Ala Lys Ile Pro Phe Asp
    610                 615                 620

acc tac ggt tct gca tat ggg tcc gca cat caa atg tcc acc tgt cgt      1920
Thr Tyr Gly Ser Ala Tyr Gly Ser Ala His Gln Met Ser Thr Cys Arg
625                 630                 635                 640

atg tcc gga aag ggt cct aaa tac ggt gct gtt gat act gat ggt aga      1968
Met Ser Gly Lys Gly Pro Lys Tyr Gly Ala Val Asp Thr Asp Gly Arg
                645                 650                 655

ttg ttt gaa tgt tcg aat gtc tat gtt gct gat gct agt gtt ttg cct      2016
Leu Phe Glu Cys Ser Asn Val Tyr Val Ala Asp Ala Ser Val Leu Pro
            660                 665                 670

act gcc agc ggt gcc aac cca atg ata tcc acc atg acc ttt gct aga      2064
Thr Ala Ser Gly Ala Asn Pro Met Ile Ser Thr Met Thr Phe Ala Arg
        675                 680                 685

cag att gcg tta ggt ttg gct gac tcc ttg aag acc aaa ccc aag ttg      2112
Gln Ile Ala Leu Gly Leu Ala Asp Ser Leu Lys Thr Lys Pro Lys Leu
    690                 695                 700

tag                                                                  2115


<210>  26
<211>  704
<212>  PRT
<213>  Candida tropicalis

<400>  26

Met Asn Thr Phe Leu Pro Asp Val Leu Glu Tyr Lys His Val Asp Thr
1               5                   10                  15


Leu Leu Leu Leu Cys Asp Gly Ile Ile His Glu Thr Thr Val Asp Gln
            20                  25                  30


Ile Lys Asp Ala Ile Ala Pro Asp Phe Pro Glu Asp Gln Tyr Glu Glu
        35                  40                  45


Tyr Leu Lys Thr Phe Thr Lys Pro Ser Glu Thr Pro Gly Phe Arg Glu
    50                  55                  60


Ala Val Tyr Asp Thr Ile Asn Ala Thr Pro Thr Asp Ala Val His Met
65                  70                  75                  80


Cys Ile Val Leu Thr Thr Ala Leu Asp Ser Arg Ile Leu Ala Pro Thr

85                  90                  95

Leu Thr Asn Ser Leu Thr Pro Ile Lys Asp Met Thr Leu Lys Glu Arg
            100                 105                 110

Glu Gln Leu Leu Ala Ser Trp Arg Asp Ser Pro Ile Ala Ala Lys Arg
        115                 120                 125

Arg Leu Phe Arg Leu Ile Ser Ser Leu Thr Leu Thr Thr Phe Thr Arg
    130                 135                 140

Leu Ala Ser Glu Leu His Leu Lys Ala Ile His Tyr Pro Gly Arg Asp
145                 150                 155                 160

Leu Arg Glu Lys Ala Tyr Glu Thr Gln Val Val Asp Pro Phe Arg Tyr
                165                 170                 175

Leu Phe Met Glu Lys Pro Lys Phe Asp Gly Ala Glu Leu Tyr Leu Pro
            180                 185                 190

Asp Ile Asp Val Ile Ile Ile Gly Ser Gly Ala Gly Ala Gly Val Met
        195                 200                 205

Ala His Thr Leu Ala Asn Asp Gly Phe Lys Thr Leu Val Leu Glu Lys
    210                 215                 220

Gly Lys Tyr Phe Ser Asn Ser Glu Leu Asn Phe Asn Asp Ala Asp Gly
225                 230                 235                 240

Val Lys Glu Leu Tyr Gln Gly Lys Gly Ala Leu Ala Thr Thr Asn Gln
                245                 250                 255

Gln Met Phe Ile Leu Ala Gly Ser Thr Leu Gly Gly Gly Thr Thr Val
            260                 265                 270

Asn Trp Ser Ala Cys Leu Lys Thr Pro Phe Lys Val Arg Lys Glu Trp
        275                 280                 285

Tyr Asp Glu Phe Gly Leu Glu Phe Ala Ala Asp Glu Ala Tyr Asp Lys
    290                 295                 300

Ala Gln Asp Tyr Val Trp Lys Gln Met Gly Ala Ser Thr Asp Gly Ile
305                 310                 315                 320

Thr His Ser Leu Ala Asn Glu Val Val Val Glu Gly Gly Lys Lys Leu
                325                 330                 335

Gly Tyr Lys Ser Lys Glu Ile Glu Gln Asn Asn Gly Gly His Pro Asp

            340                 345                 350

His Pro Cys Gly Phe Cys Tyr Leu Gly Cys Lys Tyr Gly Ile Lys Gln
        355                 360                 365

Gly Ser Val Asn Asn Trp Phe Arg Asp Ala Ala Ala His Gly Ser Lys
    370                 375                 380

Phe Met Gln Gln Val Arg Val Val Gln Ile Leu Asn Lys Asn Gly Val
385                 390                 395                 400

Ala Tyr Gly Ile Leu Cys Glu Asp Val Glu Thr Gly Val Arg Phe Thr
                405                 410                 415

Ile Ser Gly Pro Lys Lys Phe Val Val Ser Ala Gly Ser Leu Asn Thr
            420                 425                 430

Pro Thr Val Leu Thr Asn Ser Gly Phe Lys Asn Lys His Ile Gly Lys
        435                 440                 445

Asn Leu Thr Leu His Pro Val Ser Thr Val Phe Gly Asp Phe Gly Arg
    450                 455                 460

Asp Val Gln Ala Asp His Phe His Lys Ser Ile Met Thr Ser Leu Cys
465                 470                 475                 480

Tyr Glu Val Ala Asp Leu Asp Gly Lys Gly His Gly Cys Arg Ile Glu
                485                 490                 495

Thr Ile Leu Asn Ala Pro Phe Ile Gln Ala Ser Leu Leu Pro Trp Arg
            500                 505                 510

Gly Ser Asp Glu Val Arg Arg Asp Leu Leu Arg Tyr Asn Asn Met Val
        515                 520                 525

Ala Met Leu Leu Ile Thr Arg Asp Thr Thr Ser Gly Ser Val Ser Ala
    530                 535                 540

Asp Pro Lys Lys Pro Asp Ala Leu Ile Val Asp Tyr Glu Ile Asn Lys
545                 550                 555                 560

Phe Asp Lys Asn Ala Ile Leu Gln Ala Phe Leu Ile Thr Ser Asp Met
                565                 570                 575

Leu Tyr Ile Glu Gly Ala Lys Arg Ile Leu Ser Pro Gln Pro Trp Val
            580                 585                 590

Pro Ile Phe Glu Ser Asn Lys Pro Lys Glu Gln Arg Thr Ile Lys Asp

595                 600                 605

Lys Asp Tyr Val Glu Trp Arg Ala Lys Ala Ala Lys Ile Pro Phe Asp
    610                 615                 620

Thr Tyr Gly Ser Ala Tyr Gly Ser Ala His Gln Met Ser Thr Cys Arg
625                 630                 635                 640

Met Ser Gly Lys Gly Pro Lys Tyr Gly Ala Val Asp Thr Asp Gly Arg
                645                 650                 655

Leu Phe Glu Cys Ser Asn Val Tyr Val Ala Asp Ala Ser Val Leu Pro
            660                 665                 670

Thr Ala Ser Gly Ala Asn Pro Met Ile Ser Thr Met Thr Phe Ala Arg
        675                 680                 685

Gln Ile Ala Leu Gly Leu Ala Asp Ser Leu Lys Thr Lys Pro Lys Leu
    690                 695                 700

<210>  27
<211>  2115
<212>  DNA
<213>  Candida tropicalis

<220>
<221>  CDS
<222>  (1)..(2115)

<400>  27
atg aat acc ttc ttg cca gac gtg ctc gaa tac aaa cac gtc gat acc       48
Met Asn Thr Phe Leu Pro Asp Val Leu Glu Tyr Lys His Val Asp Thr
1               5                   10                  15

ctt ttg tta tta tgt gac ggg atc atc cac gaa acc aca gtc gac cag       96
Leu Leu Leu Leu Cys Asp Gly Ile Ile His Glu Thr Thr Val Asp Gln
            20                  25                  30

atc agg gac gcc att gct ccc gac ttc cct gaa gac cag tac gag gag      144
Ile Arg Asp Ala Ile Ala Pro Asp Phe Pro Glu Asp Gln Tyr Glu Glu
        35                  40                  45

tat ctc aag acc ttc acc aag cca tct gag acc cct ggg ttc aga gaa      192
Tyr Leu Lys Thr Phe Thr Lys Pro Ser Glu Thr Pro Gly Phe Arg Glu
    50                  55                  60

gcc gtc tac gac acg atc aac agc acc cca acc gag gct gtg cac atg      240
Ala Val Tyr Asp Thr Ile Asn Ser Thr Pro Thr Glu Ala Val His Met
65                  70                  75                  80

tgt att gta ttg acc acc gca ttg gac tcg aga atc ttg gcc ccc acg      288
Cys Ile Val Leu Thr Thr Ala Leu Asp Ser Arg Ile Leu Ala Pro Thr
                85                  90                  95

ttg acc aac tcg ttg acg cct atc aag gat atg acc ttg aaa gag cgt      336
Leu Thr Asn Ser Leu Thr Pro Ile Lys Asp Met Thr Leu Lys Glu Arg

            100                 105                 110

gaa caa ttg ttg gct gcc tgg cgt gat tcc ccg atc gcg gcc aag aga 384
Glu Gln Leu Leu Ala Ala Trp Arg Asp Ser Pro Ile Ala Ala Lys Arg
        115                 120                 125

aga ttg ttc aga ttg att tcc tca ctt acc ttg acg acc ttt acg aga 432
Arg Leu Phe Arg Leu Ile Ser Ser Leu Thr Leu Thr Thr Phe Thr Arg
    130                 135                 140

ttg gcc agc gac ttg cac ttg aga gcc atc cac tac cct ggc aga gac 480
Leu Ala Ser Asp Leu His Leu Arg Ala Ile His Tyr Pro Gly Arg Asp
145                 150                 155                 160

ttg cgt gaa aag gca tat gaa acc cag gtg gtt gac cct ttc agg tac 528
Leu Arg Glu Lys Ala Tyr Glu Thr Gln Val Val Asp Pro Phe Arg Tyr
                165                 170                 175

ctg ttt atg gaa aaa cca aag ttt gac ggc acc gag ttg tac ttg cca 576
Leu Phe Met Glu Lys Pro Lys Phe Asp Gly Thr Glu Leu Tyr Leu Pro
            180                 185                 190

gat atc gac gtc atc atc att gga tcc ggt gcc ggt gct ggt gtc atg 624
Asp Ile Asp Val Ile Ile Ile Gly Ser Gly Ala Gly Ala Gly Val Met
        195                 200                 205

gcc cac act tta gcc aac gac ggg tac aag acc ttg gtt ttg gaa aag 672
Ala His Thr Leu Ala Asn Asp Gly Tyr Lys Thr Leu Val Leu Glu Lys
    210                 215                 220

gga aag tat ttc agc aac tcc gag ttg aac ttt aat gat gcc gat ggt 720
Gly Lys Tyr Phe Ser Asn Ser Glu Leu Asn Phe Asn Asp Ala Asp Gly
225                 230                 235                 240

atg aaa gag ttg tac caa ggt aaa tgt gcg ttg acc acc acg aac cag 768
Met Lys Glu Leu Tyr Gln Gly Lys Cys Ala Leu Thr Thr Thr Asn Gln
                245                 250                 255

cag atg ttt att ctt gcc ggt tcc act ttg ggc ggt ggt acc act gtt 816
Gln Met Phe Ile Leu Ala Gly Ser Thr Leu Gly Gly Gly Thr Thr Val
            260                 265                 270

aac tgg tct gct tgt ctt aaa aca cca ttt aaa gtg cgt aag gag tgg 864
Asn Trp Ser Ala Cys Leu Lys Thr Pro Phe Lys Val Arg Lys Glu Trp
        275                 280                 285

tac gac gag ttt ggt ctt gaa ttt gct gcc gac gaa gcc tac gac aaa 912
Tyr Asp Glu Phe Gly Leu Glu Phe Ala Ala Asp Glu Ala Tyr Asp Lys
    290                 295                 300

gca caa gac tat gtt tgg aaa caa atg ggc gct tct acc gaa gga atc 960
Ala Gln Asp Tyr Val Trp Lys Gln Met Gly Ala Ser Thr Glu Gly Ile
305                 310                 315                 320

act cac tct ttg gcg aac gcg gtt gtg gtt gaa gga ggt aag aag ttg 1008
Thr His Ser Leu Ala Asn Ala Val Val Val Glu Gly Gly Lys Lys Leu
                325                 330                 335

ggt tac aag agc aag gaa atc gag cag aac aat ggt ggc cat cct gac 1056
Gly Tyr Lys Ser Lys Glu Ile Glu Gln Asn Asn Gly Gly His Pro Asp
            340                 345                 350

cac ccc tgt ggt ttc tgt tac ttg ggc tgt aag tac ggt att aag cag 1104
His Pro Cys Gly Phe Cys Tyr Leu Gly Cys Lys Tyr Gly Ile Lys Gln

        355                 360                 365

ggt tct gtg aat aac tgg ttt aga gac gca gct gcc cac ggg tcc aag 1152
Gly Ser Val Asn Asn Trp Phe Arg Asp Ala Ala Ala His Gly Ser Lys
    370                 375                 380

ttc atg caa caa gtc aga gtt gtg caa atc ctc cac aat aaa ggc gtc 1200
Phe Met Gln Gln Val Arg Val Val Gln Ile Leu His Asn Lys Gly Val
385                 390                 395                 400

gct tat ggc atc ttg tgt gag gat gtc gag acc gga gtc aaa ttc act 1248
Ala Tyr Gly Ile Leu Cys Glu Asp Val Glu Thr Gly Val Lys Phe Thr
                405                 410                 415

atc agt ggc ccc aaa aag ttt gtt gtt tct gca ggt tct ttg aac acg 1296
Ile Ser Gly Pro Lys Lys Phe Val Val Ser Ala Gly Ser Leu Asn Thr
            420                 425                 430

cca acg gtg ttg acc aac tcc gga ttc aag aac aaa cac atc ggt aag 1344
Pro Thr Val Leu Thr Asn Ser Gly Phe Lys Asn Lys His Ile Gly Lys
        435                 440                 445

aac ttg acg ttg cac cca gtt tcg acc gtg ttt ggt gac ttt ggc aga 1392
Asn Leu Thr Leu His Pro Val Ser Thr Val Phe Gly Asp Phe Gly Arg
    450                 455                 460

gac gtg caa gcc gac cat ttc cac aaa tct att atg act tcg ctc tgt 1440
Asp Val Gln Ala Asp His Phe His Lys Ser Ile Met Thr Ser Leu Cys
465                 470                 475                 480

tac gaa gtc gct gac ttg gac ggc aag ggc cac gga tgc aga atc gag 1488
Tyr Glu Val Ala Asp Leu Asp Gly Lys Gly His Gly Cys Arg Ile Glu
                485                 490                 495

acc atc ttg aac gct cca ttc atc caa gct tct ttg ttg cca tgg aga 1536
Thr Ile Leu Asn Ala Pro Phe Ile Gln Ala Ser Leu Leu Pro Trp Arg
            500                 505                 510

gga agc gac gag gtc aga aga gac ttg ttg cgt tac aac aac atg gtg 1584
Gly Ser Asp Glu Val Arg Arg Asp Leu Leu Arg Tyr Asn Asn Met Val
        515                 520                 525

gcc atg ttg ctt atc acc cgt gac acc acc agt ggt tca gtt tct gct 1632
Ala Met Leu Leu Ile Thr Arg Asp Thr Thr Ser Gly Ser Val Ser Ala
    530                 535                 540

gac cca aag aag ccc gac gct ttg att gtc gac tat gac atc aac aag 1680
Asp Pro Lys Lys Pro Asp Ala Leu Ile Val Asp Tyr Asp Ile Asn Lys
545                 550                 555                 560

ttt gac aag aat gcc atc ttg caa gct ttc ttg atc acc tcc gac atg 1728
Phe Asp Lys Asn Ala Ile Leu Gln Ala Phe Leu Ile Thr Ser Asp Met
                565                 570                 575

ttg tac atc gaa ggt gcc aag aga atc ctc agt cca cag gca tgg gtg 1776
Leu Tyr Ile Glu Gly Ala Lys Arg Ile Leu Ser Pro Gln Ala Trp Val
            580                 585                 590

cca atc ttt gag tcg aac aag cca aag gag caa aga aca atc aag gac 1824
Pro Ile Phe Glu Ser Asn Lys Pro Lys Glu Gln Arg Thr Ile Lys Asp
        595                 600                 605

aag gac tat gtc gaa tgg aga gcc aag gct gcc aag ata cct ttc gac 1872
Lys Asp Tyr Val Glu Trp Arg Ala Lys Ala Ala Lys Ile Pro Phe Asp

610                 615                 620

acc tac ggt tct gcc tat ggg tcc gca cat caa atg tcc acc tgt cgt      1920
Thr Tyr Gly Ser Ala Tyr Gly Ser Ala His Gln Met Ser Thr Cys Arg
625                 630                 635                 640

atg tcc gga aag ggt cct aaa tac ggc gcc gtt gat acc gat ggt aga      1968
Met Ser Gly Lys Gly Pro Lys Tyr Gly Ala Val Asp Thr Asp Gly Arg
                645                 650                 655

ttg ttt gaa tgt tcg aat gtc tat gtt gct gat gct agt gtt ttg cct      2016
Leu Phe Glu Cys Ser Asn Val Tyr Val Ala Asp Ala Ser Val Leu Pro
            660                 665                 670

act gcc agc ggt gcc aac cca atg atc tcc acc atg acg ttt gct aga      2064
Thr Ala Ser Gly Ala Asn Pro Met Ile Ser Thr Met Thr Phe Ala Arg
        675                 680                 685

cag att gcg tta ggt ttg gct gac tct ttg aag acc aaa ccc aag ttg      2112
Gln Ile Ala Leu Gly Leu Ala Asp Ser Leu Lys Thr Lys Pro Lys Leu
    690                 695                 700

tag                                                                  2115


<210>  28
<211>  704
<212>  PRT
<213>  Candida tropicalis

<400>  28

Met Asn Thr Phe Leu Pro Asp Val Leu Glu Tyr Lys His Val Asp Thr
1               5                   10                  15


Leu Leu Leu Leu Cys Asp Gly Ile Ile His Glu Thr Thr Val Asp Gln
            20                  25                  30


Ile Arg Asp Ala Ile Ala Pro Asp Phe Pro Glu Asp Gln Tyr Glu Glu
        35                  40                  45


Tyr Leu Lys Thr Phe Thr Lys Pro Ser Glu Thr Pro Gly Phe Arg Glu
    50                  55                  60


Ala Val Tyr Asp Thr Ile Asn Ser Thr Pro Thr Glu Ala Val His Met
65                  70                  75                  80


Cys Ile Val Leu Thr Thr Ala Leu Asp Ser Arg Ile Leu Ala Pro Thr
                85                  90                  95


Leu Thr Asn Ser Leu Thr Pro Ile Lys Asp Met Thr Leu Lys Glu Arg
            100                 105                 110


Glu Gln Leu Leu Ala Ala Trp Arg Asp Ser Pro Ile Ala Ala Lys Arg
        115                 120                 125


Arg Leu Phe Arg Leu Ile Ser Ser Leu Thr Leu Thr Thr Phe Thr Arg

130                 135                 140

Leu Ala Ser Asp Leu His Leu Arg Ala Ile His Tyr Pro Gly Arg Asp
145                 150                 155                 160

Leu Arg Glu Lys Ala Tyr Glu Thr Gln Val Val Asp Pro Phe Arg Tyr
                165                 170                 175

Leu Phe Met Glu Lys Pro Lys Phe Asp Gly Thr Glu Leu Tyr Leu Pro
            180                 185                 190

Asp Ile Asp Val Ile Ile Ile Gly Ser Gly Ala Gly Ala Gly Val Met
        195                 200                 205

Ala His Thr Leu Ala Asn Asp Gly Tyr Lys Thr Leu Val Leu Glu Lys
    210                 215                 220

Gly Lys Tyr Phe Ser Asn Ser Glu Leu Asn Phe Asn Asp Ala Asp Gly
225                 230                 235                 240

Met Lys Glu Leu Tyr Gln Gly Lys Cys Ala Leu Thr Thr Thr Asn Gln
                245                 250                 255

Gln Met Phe Ile Leu Ala Gly Ser Thr Leu Gly Gly Gly Thr Thr Val
            260                 265                 270

Asn Trp Ser Ala Cys Leu Lys Thr Pro Phe Lys Val Arg Lys Glu Trp
        275                 280                 285

Tyr Asp Glu Phe Gly Leu Glu Phe Ala Ala Asp Glu Ala Tyr Asp Lys
    290                 295                 300

Ala Gln Asp Tyr Val Trp Lys Gln Met Gly Ala Ser Thr Glu Gly Ile
305                 310                 315                 320

Thr His Ser Leu Ala Asn Ala Val Val Val Glu Gly Gly Lys Lys Leu
                325                 330                 335

Gly Tyr Lys Ser Lys Glu Ile Glu Gln Asn Asn Gly Gly His Pro Asp
            340                 345                 350

His Pro Cys Gly Phe Cys Tyr Leu Gly Cys Lys Tyr Gly Ile Lys Gln
        355                 360                 365

Gly Ser Val Asn Asn Trp Phe Arg Asp Ala Ala Ala His Gly Ser Lys
    370                 375                 380

Phe Met Gln Gln Val Arg Val Val Gln Ile Leu His Asn Lys Gly Val

385                 390                 395                 400

Ala Tyr Gly Ile Leu Cys Glu Asp Val Glu Thr Gly Val Lys Phe Thr
                405                 410                 415

Ile Ser Gly Pro Lys Lys Phe Val Val Ser Ala Gly Ser Leu Asn Thr
            420                 425                 430

Pro Thr Val Leu Thr Asn Ser Gly Phe Lys Asn Lys His Ile Gly Lys
        435                 440                 445

Asn Leu Thr Leu His Pro Val Ser Thr Val Phe Gly Asp Phe Gly Arg
    450                 455                 460

Asp Val Gln Ala Asp His Phe His Lys Ser Ile Met Thr Ser Leu Cys
465                 470                 475                 480

Tyr Glu Val Ala Asp Leu Asp Gly Lys Gly His Gly Cys Arg Ile Glu
                485                 490                 495

Thr Ile Leu Asn Ala Pro Phe Ile Gln Ala Ser Leu Leu Pro Trp Arg
            500                 505                 510

Gly Ser Asp Glu Val Arg Arg Asp Leu Leu Arg Tyr Asn Asn Met Val
        515                 520                 525

Ala Met Leu Leu Ile Thr Arg Asp Thr Thr Ser Gly Ser Val Ser Ala
    530                 535                 540

Asp Pro Lys Lys Pro Asp Ala Leu Ile Val Asp Tyr Asp Ile Asn Lys
545                 550                 555                 560

Phe Asp Lys Asn Ala Ile Leu Gln Ala Phe Leu Ile Thr Ser Asp Met
                565                 570                 575

Leu Tyr Ile Glu Gly Ala Lys Arg Ile Leu Ser Pro Gln Ala Trp Val
            580                 585                 590

Pro Ile Phe Glu Ser Asn Lys Pro Lys Glu Gln Arg Thr Ile Lys Asp
        595                 600                 605

Lys Asp Tyr Val Glu Trp Arg Ala Lys Ala Ala Lys Ile Pro Phe Asp
    610                 615                 620

Thr Tyr Gly Ser Ala Tyr Gly Ser Ala His Gln Met Ser Thr Cys Arg
625                 630                 635                 640

Met Ser Gly Lys Gly Pro Lys Tyr Gly Ala Val Asp Thr Asp Gly Arg

645                 650                 655

Leu Phe Glu Cys Ser Asn Val Tyr Val Ala Asp Ala Ser Val Leu Pro
            660                 665                 670

Thr Ala Ser Gly Ala Asn Pro Met Ile Ser Thr Met Thr Phe Ala Arg
        675                 680                 685

Gln Ile Ala Leu Gly Leu Ala Asp Ser Leu Lys Thr Lys Pro Lys Leu
    690                 695                 700

<210>  29
<211>  1446
<212>  DNA
<213>  Candida tropicalis

<220>
<221>  CDS
<222>  (1)..(1446)

<400>  29
atg ata atg ttt gat atc gtc aaa tac acc tta att ggg tta ttt agc       48
Met Ile Met Phe Asp Ile Val Lys Tyr Thr Leu Ile Gly Leu Phe Ser
1               5                   10                  15

tat ttg cta tat gta att ctc gat ata gta cta cca cca ttc aat ttt       96
Tyr Leu Leu Tyr Val Ile Leu Asp Ile Val Leu Pro Pro Phe Asn Phe
            20                  25                  30

ccc aaa aat atc cca aca atc cca ttc tat gtt tcc ttc tta ggt gct      144
Pro Lys Asn Ile Pro Thr Ile Pro Phe Tyr Val Ser Phe Leu Gly Ala
        35                  40                  45

tac act aac ttg gat cag agg gat att tac aat ttg tat ttg aga gag      192
Tyr Thr Asn Leu Asp Gln Arg Asp Ile Tyr Asn Leu Tyr Leu Arg Glu
    50                  55                  60

aag ttg gaa aag tac ggg gca gta aag ata tat ttt gct tca aga tgg      240
Lys Leu Glu Lys Tyr Gly Ala Val Lys Ile Tyr Phe Ala Ser Arg Trp
65                  70                  75                  80

aac ata ctc att act agg cca gaa tat ctt ctt gaa atg ttt aga aat      288
Asn Ile Leu Ile Thr Arg Pro Glu Tyr Leu Leu Glu Met Phe Arg Asn
                85                  90                  95

gaa gat gtg tac tca aaa cgg gga aac cac cta aaa atc cct ggt tca      336
Glu Asp Val Tyr Ser Lys Arg Gly Asn His Leu Lys Ile Pro Gly Ser
            100                 105                 110

gtg atg gct aca tac act ggt gat aat atc att agt gct cat gga gaa      384
Val Met Ala Thr Tyr Thr Gly Asp Asn Ile Ile Ser Ala His Gly Glu
        115                 120                 125

tta tgg aaa tta tat cga gaa gtt att gcc aaa agt att caa ttt ccc      432
Leu Trp Lys Leu Tyr Arg Glu Val Ile Ala Lys Ser Ile Gln Phe Pro
    130                 135                 140

gat ttt gaa cca att acg aaa aat act aaa tca tta ctt gaa att att      480
Asp Phe Glu Pro Ile Thr Lys Asn Thr Lys Ser Leu Leu Glu Ile Ile

145                 150                 155                 160

gat ggt atg att gac agt gat aaa aat cat gca att att cca atc aca       528
Asp Gly Met Ile Asp Ser Asp Lys Asn His Ala Ile Ile Pro Ile Thr
                165                 170                 175

gat tta ttt caa aaa tat tca tta gca aac gtt aca gaa tct ata ctt       576
Asp Leu Phe Gln Lys Tyr Ser Leu Ala Asn Val Thr Glu Ser Ile Leu
            180                 185                 190

gga gta aat ttt aag gtt ctt gaa ggc gat caa tca atc atg cat caa       624
Gly Val Asn Phe Lys Val Leu Glu Gly Asp Gln Ser Ile Met His Gln
        195                 200                 205

aaa ata aag tac gtc aag ctg caa ata ttc aaa cct ttt ttc ttg aac       672
Lys Ile Lys Tyr Val Lys Leu Gln Ile Phe Lys Pro Phe Phe Leu Asn
    210                 215                 220

ttt cct tat ttt gat agc ttt cct att cca agt agg tta caa gca aga       720
Phe Pro Tyr Phe Asp Ser Phe Pro Ile Pro Ser Arg Leu Gln Ala Arg
225                 230                 235                 240

aag gaa gtt att aat ttt agg aat tgg tat ggt caa agt att att gac       768
Lys Glu Val Ile Asn Phe Arg Asn Trp Tyr Gly Gln Ser Ile Ile Asp
                245                 250                 255

aag cat gat cca caa ctt cct aat agt gca gct aca aaa tta gtt gat       816
Lys His Asp Pro Gln Leu Pro Asn Ser Ala Ala Thr Lys Leu Val Asp
            260                 265                 270

ggc ttg atg caa gaa aaa ctc act gaa aaa caa ttt ttg gat aat gcc       864
Gly Leu Met Gln Glu Lys Leu Thr Glu Lys Gln Phe Leu Asp Asn Ala
        275                 280                 285

att att gtg atg att gct gga cat gaa aat cca ctt ttg tta atg tta       912
Ile Ile Val Met Ile Ala Gly His Glu Asn Pro Leu Leu Leu Met Leu
    290                 295                 300

tcg ttg atg ttt gtt gct gca aaa tat cca aaa gtt cag gag gcc ata       960
Ser Leu Met Phe Val Ala Ala Lys Tyr Pro Lys Val Gln Glu Ala Ile
305                 310                 315                 320

cgt tca gaa ata gat cca aca aaa cct tat cta cac tct gtt att tat      1008
Arg Ser Glu Ile Asp Pro Thr Lys Pro Tyr Leu His Ser Val Ile Tyr
                325                 330                 335

gaa act tta aga atg tat cca cca ttg gga tta atc att aat cgt tat      1056
Glu Thr Leu Arg Met Tyr Pro Pro Leu Gly Leu Ile Ile Asn Arg Tyr
            340                 345                 350

acc acc aga cct act aaa cta ggc aac ata gta att ccc aaa ggt gtt      1104
Thr Thr Arg Pro Thr Lys Leu Gly Asn Ile Val Ile Pro Lys Gly Val
        355                 360                 365

tac tgt ggc tat aat aat ttt ggt acg ggt aga gac aga aat gtt tgg      1152
Tyr Cys Gly Tyr Asn Asn Phe Gly Thr Gly Arg Asp Arg Asn Val Trp
    370                 375                 380

gga cca gac tcg gat gag ttt aaa cca gag aga tgg gga agg gat aat      1200
Gly Pro Asp Ser Asp Glu Phe Lys Pro Glu Arg Trp Gly Arg Asp Asn
385                 390                 395                 400

att gaa gaa ata aat cgc aat tat gct aat gcc aaa aga tca gct gaa      1248
Ile Glu Glu Ile Asn Arg Asn Tyr Ala Asn Ala Lys Arg Ser Ala Glu

                405                 410                 415

tta cct gcg ttt cat ggc aga aag aga gct tgt tta gga gaa aag tat 1296
Leu Pro Ala Phe His Gly Arg Lys Arg Ala Cys Leu Gly Glu Lys Tyr
            420                 425                 430

gcc tta tat gaa gtt aaa gaa ttg cta act agt att tta gga cat tac 1344
Ala Leu Tyr Glu Val Lys Glu Leu Leu Thr Ser Ile Leu Gly His Tyr
        435                 440                 445

aaa gtt act tta gat gca agt tgg aaa gag aaa ata acc cct gct gga 1392
Lys Val Thr Leu Asp Ala Ser Trp Lys Glu Lys Ile Thr Pro Ala Gly
    450                 455                 460

cct att agt cca ttt ggt ttg aag gtg aaa ttt gaa aag ctt att gtt 1440
Pro Ile Ser Pro Phe Gly Leu Lys Val Lys Phe Glu Lys Leu Ile Val
465                 470                 475                 480

gca taa 1446
Ala

<210> 30
<211> 481
<212> PRT
<213> Candida tropicalis

<400> 30

Met Ile Met Phe Asp Ile Val Lys Tyr Thr Leu Ile Gly Leu Phe Ser
1               5                   10                  15

Tyr Leu Leu Tyr Val Ile Leu Asp Ile Val Leu Pro Pro Phe Asn Phe
            20                  25                  30

Pro Lys Asn Ile Pro Thr Ile Pro Phe Tyr Val Ser Phe Leu Gly Ala
        35                  40                  45

Tyr Thr Asn Leu Asp Gln Arg Asp Ile Tyr Asn Leu Tyr Leu Arg Glu
    50                  55                  60

Lys Leu Glu Lys Tyr Gly Ala Val Lys Ile Tyr Phe Ala Ser Arg Trp
65                  70                  75                  80

Asn Ile Leu Ile Thr Arg Pro Glu Tyr Leu Leu Glu Met Phe Arg Asn
                85                  90                  95

Glu Asp Val Tyr Ser Lys Arg Gly Asn His Leu Lys Ile Pro Gly Ser
            100                 105                 110

Val Met Ala Thr Tyr Thr Gly Asp Asn Ile Ile Ser Ala His Gly Glu
        115                 120                 125

Leu Trp Lys Leu Tyr Arg Glu Val Ile Ala Lys Ser Ile Gln Phe Pro
    130                 135                 140

Asp Phe Glu Pro Ile Thr Lys Asn Thr Lys Ser Leu Leu Glu Ile Ile
145                 150                 155                 160

Asp Gly Met Ile Asp Ser Asp Lys Asn His Ala Ile Ile Pro Ile Thr
                165                 170                 175

Asp Leu Phe Gln Lys Tyr Ser Leu Ala Asn Val Thr Glu Ser Ile Leu
            180                 185                 190

Gly Val Asn Phe Lys Val Leu Glu Gly Asp Gln Ser Ile Met His Gln
        195                 200                 205

Lys Ile Lys Tyr Val Lys Leu Gln Ile Phe Lys Pro Phe Phe Leu Asn
    210                 215                 220

Phe Pro Tyr Phe Asp Ser Phe Pro Ile Pro Ser Arg Leu Gln Ala Arg
225                 230                 235                 240

Lys Glu Val Ile Asn Phe Arg Asn Trp Tyr Gly Gln Ser Ile Ile Asp
                245                 250                 255

Lys His Asp Pro Gln Leu Pro Asn Ser Ala Ala Thr Lys Leu Val Asp
            260                 265                 270

Gly Leu Met Gln Glu Lys Leu Thr Glu Lys Gln Phe Leu Asp Asn Ala
        275                 280                 285

Ile Ile Val Met Ile Ala Gly His Glu Asn Pro Leu Leu Leu Met Leu
    290                 295                 300

Ser Leu Met Phe Val Ala Ala Lys Tyr Pro Lys Val Gln Glu Ala Ile
305                 310                 315                 320

Arg Ser Glu Ile Asp Pro Thr Lys Pro Tyr Leu His Ser Val Ile Tyr
                325                 330                 335

Glu Thr Leu Arg Met Tyr Pro Pro Leu Gly Leu Ile Ile Asn Arg Tyr
            340                 345                 350

Thr Thr Arg Pro Thr Lys Leu Gly Asn Ile Val Ile Pro Lys Gly Val
        355                 360                 365

Tyr Cys Gly Tyr Asn Asn Phe Gly Thr Gly Arg Asp Arg Asn Val Trp
    370                 375                 380

Gly Pro Asp Ser Asp Glu Phe Lys Pro Glu Arg Trp Gly Arg Asp Asn
385                 390                 395                 400

Ile Glu Glu Ile Asn Arg Asn Tyr Ala Asn Ala Lys Arg Ser Ala Glu
                405                 410                 415

Leu Pro Ala Phe His Gly Arg Lys Arg Ala Cys Leu Gly Glu Lys Tyr
            420                 425                 430

Ala Leu Tyr Glu Val Lys Glu Leu Leu Thr Ser Ile Leu Gly His Tyr
        435                 440                 445

Lys Val Thr Leu Asp Ala Ser Trp Lys Glu Lys Ile Thr Pro Ala Gly
    450                 455                 460

Pro Ile Ser Pro Phe Gly Leu Lys Val Lys Phe Glu Lys Leu Ile Val
465                 470                 475                 480

Ala

<210>  31
<211>  1623
<212>  DNA
<213>  Candida tropicalis

<220>
<221>  CDS
<222>  (1)..(1623)

<400>  31
atg tcg tcg tct cca tct att gct caa gaa ttt ctc gca acc att act       48
Met Ser Ser Ser Pro Ser Ile Ala Gln Glu Phe Leu Ala Thr Ile Thr
1               5                   10                  15

cca tat gtt gag tat tgt caa gag aat tat acc aaa tgg tac tac ttc       96
Pro Tyr Val Glu Tyr Cys Gln Glu Asn Tyr Thr Lys Trp Tyr Tyr Phe
            20                  25                  30

att cca ttg gtg att ctt tcg ttg aat ctt atc tcc atg ctt cat aca      144
Ile Pro Leu Val Ile Leu Ser Leu Asn Leu Ile Ser Met Leu His Thr
        35                  40                  45

aag tat ttg gaa cgt aaa ttt aag gct aaa ccg ctt gct gtc tat gtt      192
Lys Tyr Leu Glu Arg Lys Phe Lys Ala Lys Pro Leu Ala Val Tyr Val
    50                  55                  60

caa gat tat acc ttt ggt ctt att act cca ctt gtt ttg atc tac tac      240
Gln Asp Tyr Thr Phe Gly Leu Ile Thr Pro Leu Val Leu Ile Tyr Tyr
65                  70                  75                  80

aag tct aaa ggt acc gtg atg caa ttt gcc tgt gat tta tgg gac aag      288
Lys Ser Lys Gly Thr Val Met Gln Phe Ala Cys Asp Leu Trp Asp Lys
                85                  90                  95

aaa ctc att gtc agt gat cca aag gca aag act att ggt ctt aag att      336
Lys Leu Ile Val Ser Asp Pro Lys Ala Lys Thr Ile Gly Leu Lys Ile
            100                 105                 110

tta ggt att cca ttg att gaa act aaa gac cca gag aat gtc aag gct 384
Leu Gly Ile Pro Leu Ile Glu Thr Lys Asp Pro Glu Asn Val Lys Ala
        115                 120                 125

att tta gcc acc cag ttt aat gat ttt tct ttg gga act aga cat gat 432
Ile Leu Ala Thr Gln Phe Asn Asp Phe Ser Leu Gly Thr Arg His Asp
    130                 135                 140

ttc ttg tat tca ttg tta gga gat ggt att ttc act tta gat ggt gct 480
Phe Leu Tyr Ser Leu Leu Gly Asp Gly Ile Phe Thr Leu Asp Gly Ala
145                 150                 155                 160

ggc tgg aaa cac agc aga acc atg ttg aga cca caa ttc gct aga gag 528
Gly Trp Lys His Ser Arg Thr Met Leu Arg Pro Gln Phe Ala Arg Glu
                165                 170                 175

caa gtt tcg cat gta aag ttg ttg gaa ccc cat atg caa gtt tta ttt 576
Gln Val Ser His Val Lys Leu Leu Glu Pro His Met Gln Val Leu Phe
            180                 185                 190

aaa cac att aga aaa cat cat ggc caa act ttt gat atc caa gaa ttg 624
Lys His Ile Arg Lys His His Gly Gln Thr Phe Asp Ile Gln Glu Leu
        195                 200                 205

ttt ttc aga tta act gtt gat tcg gct act gag ttt ttg ttt ggt gaa 672
Phe Phe Arg Leu Thr Val Asp Ser Ala Thr Glu Phe Leu Phe Gly Glu
    210                 215                 220

tct gct gaa tct tta aga gac gag tct gtc ggt tta act cca aca acc 720
Ser Ala Glu Ser Leu Arg Asp Glu Ser Val Gly Leu Thr Pro Thr Thr
225                 230                 235                 240

aaa gat ttt gat ggt aga aat gag ttt gcc gat gcg ttc aac tat tct 768
Lys Asp Phe Asp Gly Arg Asn Glu Phe Ala Asp Ala Phe Asn Tyr Ser
                245                 250                 255

caa act tac caa gcg tac aga ttc ttg ttg caa caa atg tat tgg att 816
Gln Thr Tyr Gln Ala Tyr Arg Phe Leu Leu Gln Gln Met Tyr Trp Ile
            260                 265                 270

ttg aat ggt tct gaa ttt aga aaa tct att gct att gtc cac aag ttt 864
Leu Asn Gly Ser Glu Phe Arg Lys Ser Ile Ala Ile Val His Lys Phe
        275                 280                 285

gct gac cac tat gtt caa aag gca ttg gaa tta act gat gaa gat ttg 912
Ala Asp His Tyr Val Gln Lys Ala Leu Glu Leu Thr Asp Glu Asp Leu
    290                 295                 300

gaa aag aaa gaa ggc tat gta ttt tta ttc gag tta gcc aaa caa acc 960
Glu Lys Lys Glu Gly Tyr Val Phe Leu Phe Glu Leu Ala Lys Gln Thr
305                 310                 315                 320

aga gat cca aag gtt ttg aga gat caa ttg tta aat atc ttg gtt gct 1008
Arg Asp Pro Lys Val Leu Arg Asp Gln Leu Leu Asn Ile Leu Val Ala
                325                 330                 335

ggt aga gat acc aca gct ggc ttg ttg tcg ttt ctt ttc ttt gaa ttg 1056
Gly Arg Asp Thr Thr Ala Gly Leu Leu Ser Phe Leu Phe Phe Glu Leu
            340                 345                 350

tct aga aac cca gaa ata ttt gca aaa ttg aga gaa gaa atc gaa aac 1104
Ser Arg Asn Pro Glu Ile Phe Ala Lys Leu Arg Glu Glu Ile Glu Asn
        355                 360                 365

aag ttt ggt ctt gga caa gat gct cgt gtt gaa gag att tct ttt gaa     1152
Lys Phe Gly Leu Gly Gln Asp Ala Arg Val Glu Glu Ile Ser Phe Glu
    370                 375                 380

aca ttg aaa tct tgt gaa tac ttg aag gct gtt atc aat gaa act ttg     1200
Thr Leu Lys Ser Cys Glu Tyr Leu Lys Ala Val Ile Asn Glu Thr Leu
385                 390                 395                 400

aga att tat cct tcc gtc cca cat aat ttt aga gtt gct act aga aac     1248
Arg Ile Tyr Pro Ser Val Pro His Asn Phe Arg Val Ala Thr Arg Asn
                405                 410                 415

aca act tta cca aga ggt ggt ggt gaa ggt ggt tta tcc cca att gct     1296
Thr Thr Leu Pro Arg Gly Gly Gly Glu Gly Gly Leu Ser Pro Ile Ala
            420                 425                 430

att aag aag ggc caa gtt gtt atg tac acg att ctt gct act cac aga     1344
Ile Lys Lys Gly Gln Val Val Met Tyr Thr Ile Leu Ala Thr His Arg
        435                 440                 445

gat aaa gac att tat ggt gaa gat gct tat gtt ttc agg cca gaa aga     1392
Asp Lys Asp Ile Tyr Gly Glu Asp Ala Tyr Val Phe Arg Pro Glu Arg
    450                 455                 460

tgg ttt gaa cct gaa acc aga aaa ttg ggc tgg gca tat gtt cca ttc     1440
Trp Phe Glu Pro Glu Thr Arg Lys Leu Gly Trp Ala Tyr Val Pro Phe
465                 470                 475                 480

aat ggc ggt cca aga att tgt ttg ggt caa cag ttt gct tta act gaa     1488
Asn Gly Gly Pro Arg Ile Cys Leu Gly Gln Gln Phe Ala Leu Thr Glu
                485                 490                 495

gca tca tat gtc act gtt aga ttg ctt caa gaa ttt ggt aac ttg aaa     1536
Ala Ser Tyr Val Thr Val Arg Leu Leu Gln Glu Phe Gly Asn Leu Lys
            500                 505                 510

caa gat cca aat act gaa tat cca cca aaa tta caa aac aca ttg act     1584
Gln Asp Pro Asn Thr Glu Tyr Pro Pro Lys Leu Gln Asn Thr Leu Thr
        515                 520                 525

ttg tct ctt ttt gaa ggt gct gaa gta caa atg tat taa                 1623
Leu Ser Leu Phe Glu Gly Ala Glu Val Gln Met Tyr
    530                 535                 540

<210>  32
<211>  540
<212>  PRT
<213>  Candida tropicalis

<400>  32

Met Ser Ser Ser Pro Ser Ile Ala Gln Glu Phe Leu Ala Thr Ile Thr
1               5                   10                  15

Pro Tyr Val Glu Tyr Cys Gln Glu Asn Tyr Thr Lys Trp Tyr Tyr Phe
            20                  25                  30

Ile Pro Leu Val Ile Leu Ser Leu Asn Leu Ile Ser Met Leu His Thr
        35                  40                  45

50                  55                  60

Gln Asp Tyr Thr Phe Gly Leu Ile Thr Pro Leu Val Leu Ile Tyr Tyr
65                  70                  75                  80

Lys Ser Lys Gly Thr Val Met Gln Phe Ala Cys Asp Leu Trp Asp Lys
                85                  90                  95

Lys Leu Ile Val Ser Asp Pro Lys Ala Lys Thr Ile Gly Leu Lys Ile
            100                 105                 110

Leu Gly Ile Pro Leu Ile Glu Thr Lys Asp Pro Glu Asn Val Lys Ala
        115                 120                 125

Ile Leu Ala Thr Gln Phe Asn Asp Phe Ser Leu Gly Thr Arg His Asp
    130                 135                 140

Phe Leu Tyr Ser Leu Leu Gly Asp Gly Ile Phe Thr Leu Asp Gly Ala
145                 150                 155                 160

Gly Trp Lys His Ser Arg Thr Met Leu Arg Pro Gln Phe Ala Arg Glu
                165                 170                 175

Gln Val Ser His Val Lys Leu Leu Glu Pro His Met Gln Val Leu Phe
            180                 185                 190

Lys His Ile Arg Lys His His Gly Gln Thr Phe Asp Ile Gln Glu Leu
        195                 200                 205

Phe Phe Arg Leu Thr Val Asp Ser Ala Thr Glu Phe Leu Phe Gly Glu
    210                 215                 220

Ser Ala Glu Ser Leu Arg Asp Glu Ser Val Gly Leu Thr Pro Thr Thr
225                 230                 235                 240

Lys Asp Phe Asp Gly Arg Asn Glu Phe Ala Asp Ala Phe Asn Tyr Ser
                245                 250                 255

Gln Thr Tyr Gln Ala Tyr Arg Phe Leu Leu Gln Gln Met Tyr Trp Ile
            260                 265                 270

Leu Asn Gly Ser Glu Phe Arg Lys Ser Ile Ala Ile Val His Lys Phe
        275                 280                 285

Ala Asp His Tyr Val Gln Lys Ala Leu Glu Leu Thr Asp Glu Asp Leu
    290                 295                 300

Glu Lys Lys Glu Gly Tyr Val Phe Leu Phe Glu Leu Ala Lys Gln Thr

305                 310                 315                 320

Arg Asp Pro Lys Val Leu Arg Asp Gln Leu Leu Asn Ile Leu Val Ala
                325                 330                 335

Gly Arg Asp Thr Thr Ala Gly Leu Leu Ser Phe Leu Phe Phe Glu Leu
            340                 345                 350

Ser Arg Asn Pro Glu Ile Phe Ala Lys Leu Arg Glu Glu Ile Glu Asn
        355                 360                 365

Lys Phe Gly Leu Gly Gln Asp Ala Arg Val Glu Glu Ile Ser Phe Glu
    370                 375                 380

Thr Leu Lys Ser Cys Glu Tyr Leu Lys Ala Val Ile Asn Glu Thr Leu
385                 390                 395                 400

Arg Ile Tyr Pro Ser Val Pro His Asn Phe Arg Val Ala Thr Arg Asn
                405                 410                 415

Thr Thr Leu Pro Arg Gly Gly Gly Glu Gly Gly Leu Ser Pro Ile Ala
            420                 425                 430

Ile Lys Lys Gly Gln Val Val Met Tyr Thr Ile Leu Ala Thr His Arg
        435                 440                 445

Asp Lys Asp Ile Tyr Gly Glu Asp Ala Tyr Val Phe Arg Pro Glu Arg
    450                 455                 460

Trp Phe Glu Pro Glu Thr Arg Lys Leu Gly Trp Ala Tyr Val Pro Phe
465                 470                 475                 480

Asn Gly Gly Pro Arg Ile Cys Leu Gly Gln Gln Phe Ala Leu Thr Glu
                485                 490                 495

Ala Ser Tyr Val Thr Val Arg Leu Leu Gln Glu Phe Gly Asn Leu Lys
            500                 505                 510

Gln Asp Pro Asn Thr Glu Tyr Pro Pro Lys Leu Gln Asn Thr Leu Thr
        515                 520                 525

Leu Ser Leu Phe Glu Gly Ala Glu Val Gln Met Tyr
    530                 535                 540

<210>  33
<211>  1569
<212>  DNA
<213>  Candida tropicalis

<221>  CDS
<222>  (1)..(1569)

<400>  33
atg agt att caa gat att gtt gaa act tac agt acc aaa tgg tac gtt       48
Met Ser Ile Gln Asp Ile Val Glu Thr Tyr Ser Thr Lys Trp Tyr Val
1               5                   10                  15

gtt gta ctg gtt gct ttg att gta tac aag gtt ttt gat ttc ttt tat       96
Val Val Leu Val Ala Leu Ile Val Tyr Lys Val Phe Asp Phe Phe Tyr
            20                  25                  30

gct aga tat ttg atg tat aag ctt ggt gct aag cca ttc ctt caa agt      144
Ala Arg Tyr Leu Met Tyr Lys Leu Gly Ala Lys Pro Phe Leu Gln Ser
        35                  40                  45

caa acc gac ggt tat ctt ggt ttc aga gtt cca ttt gaa ttg atg gga      192
Gln Thr Asp Gly Tyr Leu Gly Phe Arg Val Pro Phe Glu Leu Met Gly
    50                  55                  60

aag aag agt gaa ggt aca ctt ata gac ttt aca tat caa cgt act ttg      240
Lys Lys Ser Glu Gly Thr Leu Ile Asp Phe Thr Tyr Gln Arg Thr Leu
65                  70                  75                  80

gag ctt gac aat ccc gat att cca aca ttt aca ttc cca ata ttt tct      288
Glu Leu Asp Asn Pro Asp Ile Pro Thr Phe Thr Phe Pro Ile Phe Ser
                85                  90                  95

gtt ctg att atc tca act ctt gaa cca gac aac atc aaa gct att ttg      336
Val Leu Ile Ile Ser Thr Leu Glu Pro Asp Asn Ile Lys Ala Ile Leu
            100                 105                 110

gcc aca caa ttc aat gac ttt tca tta ggt aca aga cat tca cat ttt      384
Ala Thr Gln Phe Asn Asp Phe Ser Leu Gly Thr Arg His Ser His Phe
        115                 120                 125

gct cct tta tta ggt gac ggt att ttc act tta gat ggt gct ggt tgg      432
Ala Pro Leu Leu Gly Asp Gly Ile Phe Thr Leu Asp Gly Ala Gly Trp
    130                 135                 140

aaa cat agt aga tct atg ttg aga cca caa ttt gca aga gaa cag gtt      480
Lys His Ser Arg Ser Met Leu Arg Pro Gln Phe Ala Arg Glu Gln Val
145                 150                 155                 160

tct cac gtt aag ttg ttg gaa cca cat atg caa gtt ttt ttc aaa cat      528
Ser His Val Lys Leu Leu Glu Pro His Met Gln Val Phe Phe Lys His
                165                 170                 175

att aga aaa cat cat ggc caa acc ttt gat ata caa gaa ttg ttt ttc      576
Ile Arg Lys His His Gly Gln Thr Phe Asp Ile Gln Glu Leu Phe Phe
            180                 185                 190

aga tta act gtt gat tct gcc act gag ttt ttg ttt ggt gaa tct gtt      624
Arg Leu Thr Val Asp Ser Ala Thr Glu Phe Leu Phe Gly Glu Ser Val
        195                 200                 205

gag tct tta aga gac gag tcc atc ggt atg tta aac gat gct ctt gat      672
Glu Ser Leu Arg Asp Glu Ser Ile Gly Met Leu Asn Asp Ala Leu Asp
    210                 215                 220

ttt gat ggt aag gct gga ttt gct gat gcc ttt aac tat tct caa aac      720
Phe Asp Gly Lys Ala Gly Phe Ala Asp Ala Phe Asn Tyr Ser Gln Asn
225                 230                 235                 240

tat ttg gct tct cga gct ctt atg caa caa atg tac tgg att ttg aac      768

Tyr Leu Ala Ser Arg Ala Leu Met Gln Gln Met Tyr Trp Ile Leu Asn
                245                 250                 255

gga aaa aag ttt aaa gaa tgt aat gcc aag gtt cac aag ttt gct gat      816
Gly Lys Lys Phe Lys Glu Cys Asn Ala Lys Val His Lys Phe Ala Asp
            260                 265                 270

tat tat gtt gaa aaa gca ttg gaa tta act ccg gac caa ttg gaa aaa      864
Tyr Tyr Val Glu Lys Ala Leu Glu Leu Thr Pro Asp Gln Leu Glu Lys
        275                 280                 285

caa gat ggg tat gtt ttc ttg tat gaa ttg gta aaa caa acc aga gac      912
Gln Asp Gly Tyr Val Phe Leu Tyr Glu Leu Val Lys Gln Thr Arg Asp
    290                 295                 300

aga caa gtc ttg aga gat cag tta ttg aat ata tta gtt gct ggt aga      960
Arg Gln Val Leu Arg Asp Gln Leu Leu Asn Ile Leu Val Ala Gly Arg
305                 310                 315                 320

gat acc act gct ggt tta tta tca ttt gtg ttt ttt gaa ttg gca aga     1008
Asp Thr Thr Ala Gly Leu Leu Ser Phe Val Phe Phe Glu Leu Ala Arg
                325                 330                 335

act cca aga gta gca aat aaa tta aga gaa gaa atc gaa gac aaa ttt     1056
Thr Pro Arg Val Ala Asn Lys Leu Arg Glu Glu Ile Glu Asp Lys Phe
            340                 345                 350

ggc ctt gga caa gat gct cgt gtt gaa gaa att tcc ttt gaa tct tta     1104
Gly Leu Gly Gln Asp Ala Arg Val Glu Glu Ile Ser Phe Glu Ser Leu
        355                 360                 365

aaa tca tgt gaa tat ttg aag gca gtg ctt aat gaa tgt tta aga tta     1152
Lys Ser Cys Glu Tyr Leu Lys Ala Val Leu Asn Glu Cys Leu Arg Leu
    370                 375                 380

tac cca tct gtt cca caa aac ttt aga gtt gct acc aga aat acc aca     1200
Tyr Pro Ser Val Pro Gln Asn Phe Arg Val Ala Thr Arg Asn Thr Thr
385                 390                 395                 400

tta cca aga ggt ggt ggc aag gat ggt tta tca cca gta tta gtt aga     1248
Leu Pro Arg Gly Gly Gly Lys Asp Gly Leu Ser Pro Val Leu Val Arg
                405                 410                 415

aag ggt caa act gtg atg tac agt gtg tat gct gcc cat aga aac aaa     1296
Lys Gly Gln Thr Val Met Tyr Ser Val Tyr Ala Ala His Arg Asn Lys
            420                 425                 430

caa att tat ggt gaa gac gca ctt gaa ttc agg cca gaa aga tgg ttt     1344
Gln Ile Tyr Gly Glu Asp Ala Leu Glu Phe Arg Pro Glu Arg Trp Phe
        435                 440                 445

gaa cca gag aca aag aaa ttg ggc tgg gcc ttc tta cct ttt aat ggt     1392
Glu Pro Glu Thr Lys Lys Leu Gly Trp Ala Phe Leu Pro Phe Asn Gly
    450                 455                 460

ggt cca aga att tgt ttg ggt caa caa ttt gct ttg act gaa gct tct     1440
Gly Pro Arg Ile Cys Leu Gly Gln Gln Phe Ala Leu Thr Glu Ala Ser
465                 470                 475                 480

tat gtt act gtt aga tta ctt caa gag ttt agc cac ttg aca atg gat     1488
Tyr Val Thr Val Arg Leu Leu Gln Glu Phe Ser His Leu Thr Met Asp
                485                 490                 495

cca aac act gaa tac ccg cca aag aaa atg tcc cat ttg aca atg tct     1536

Pro Asn Thr Glu Tyr Pro Pro Lys Lys Met Ser His Leu Thr Met Ser
            500                 505                 510

cta ttt gat ggt gcc aac att caa atg tat tag 1569
Leu Phe Asp Gly Ala Asn Ile Gln Met Tyr
        515                 520

<210> 34
<211> 522
<212> PRT
<213> Candida tropicalis

<400> 34

Met Ser Ile Gln Asp Ile Val Glu Thr Tyr Ser Thr Lys Trp Tyr Val
1               5                   10                  15

Val Val Leu Val Ala Leu Ile Val Tyr Lys Val Phe Asp Phe Phe Tyr
            20                  25                  30

Ala Arg Tyr Leu Met Tyr Lys Leu Gly Ala Lys Pro Phe Leu Gln Ser
        35                  40                  45

Gln Thr Asp Gly Tyr Leu Gly Phe Arg Val Pro Phe Glu Leu Met Gly
    50                  55                  60

Lys Lys Ser Glu Gly Thr Leu Ile Asp Phe Thr Tyr Gln Arg Thr Leu
65                  70                  75                  80

Glu Leu Asp Asn Pro Asp Ile Pro Thr Phe Thr Phe Pro Ile Phe Ser
                85                  90                  95

Val Leu Ile Ile Ser Thr Leu Glu Pro Asp Asn Ile Lys Ala Ile Leu
            100                 105                 110

Ala Thr Gln Phe Asn Asp Phe Ser Leu Gly Thr Arg His Ser His Phe
        115                 120                 125

Ala Pro Leu Leu Gly Asp Gly Ile Phe Thr Leu Asp Gly Ala Gly Trp
    130                 135                 140

Lys His Ser Arg Ser Met Leu Arg Pro Gln Phe Ala Arg Glu Gln Val
145                 150                 155                 160

Ser His Val Lys Leu Leu Glu Pro His Met Gln Val Phe Phe Lys His
                165                 170                 175

Ile Arg Lys His His Gly Gln Thr Phe Asp Ile Gln Glu Leu Phe Phe
            180                 185                 190

Arg Leu Thr Val Asp Ser Ala Thr Glu Phe Leu Phe Gly Glu Ser Val

195                 200                 205

Glu Ser Leu Arg Asp Glu Ser Ile Gly Met Leu Asn Asp Ala Leu Asp
    210                 215                 220

Phe Asp Gly Lys Ala Gly Phe Ala Asp Ala Phe Asn Tyr Ser Gln Asn
225                 230                 235                 240

Tyr Leu Ala Ser Arg Ala Leu Met Gln Gln Met Tyr Trp Ile Leu Asn
                245                 250                 255

Gly Lys Lys Phe Lys Glu Cys Asn Ala Lys Val His Lys Phe Ala Asp
            260                 265                 270

Tyr Tyr Val Glu Lys Ala Leu Glu Leu Thr Pro Asp Gln Leu Glu Lys
        275                 280                 285

Gln Asp Gly Tyr Val Phe Leu Tyr Glu Leu Val Lys Gln Thr Arg Asp
    290                 295                 300

Arg Gln Val Leu Arg Asp Gln Leu Leu Asn Ile Leu Val Ala Gly Arg
305                 310                 315                 320

Asp Thr Thr Ala Gly Leu Leu Ser Phe Val Phe Phe Glu Leu Ala Arg
                325                 330                 335

Thr Pro Arg Val Ala Asn Lys Leu Arg Glu Glu Ile Glu Asp Lys Phe
            340                 345                 350

Gly Leu Gly Gln Asp Ala Arg Val Glu Glu Ile Ser Phe Glu Ser Leu
        355                 360                 365

Lys Ser Cys Glu Tyr Leu Lys Ala Val Leu Asn Glu Cys Leu Arg Leu
    370                 375                 380

Tyr Pro Ser Val Pro Gln Asn Phe Arg Val Ala Thr Arg Asn Thr Thr
385                 390                 395                 400

Leu Pro Arg Gly Gly Gly Lys Asp Gly Leu Ser Pro Val Leu Val Arg
                405                 410                 415

Lys Gly Gln Thr Val Met Tyr Ser Val Tyr Ala Ala His Arg Asn Lys
            420                 425                 430

Gln Ile Tyr Gly Glu Asp Ala Leu Glu Phe Arg Pro Glu Arg Trp Phe
        435                 440                 445

Glu Pro Glu Thr Lys Lys Leu Gly Trp Ala Phe Leu Pro Phe Asn Gly

450                 455                 460

Gly Pro Arg Ile Cys Leu Gly Gln Gln Phe Ala Leu Thr Glu Ala Ser
465                 470                 475                 480

Tyr Val Thr Val Arg Leu Leu Gln Glu Phe Ser His Leu Thr Met Asp
                485                 490                 495

Pro Asn Thr Glu Tyr Pro Pro Lys Lys Met Ser His Leu Thr Met Ser
            500                 505                 510

Leu Phe Asp Gly Ala Asn Ile Gln Met Tyr
        515                 520

<210>  35
<211>  1572
<212>  DNA
<213>  Candida tropicalis

<220>
<221>  CDS
<222>  (1)..(1572)

<400>  35
atg gcc aca caa gaa att att gat tct gca ctt ccg tac ttg aca aag       48
Met Ala Thr Gln Glu Ile Ile Asp Ser Ala Leu Pro Tyr Leu Thr Lys
1               5                   10                  15

tgg tat act gtt atc act tta gca gct ttg gtt ttc tta att tca tct       96
Trp Tyr Thr Val Ile Thr Leu Ala Ala Leu Val Phe Leu Ile Ser Ser
            20                  25                  30

aat att aaa aat tac gtc aag gct aag aag ttg aaa tgc aga gat cct      144
Asn Ile Lys Asn Tyr Val Lys Ala Lys Lys Leu Lys Cys Arg Asp Pro
        35                  40                  45

cca tat ttc aaa gga gcc ggt tgg aca ggt att agt cca tta att gaa      192
Pro Tyr Phe Lys Gly Ala Gly Trp Thr Gly Ile Ser Pro Leu Ile Glu
    50                  55                  60

att att aaa gtt aaa ggt aat ggt aga ttg gca gat ttt gcc gat aaa      240
Ile Ile Lys Val Lys Gly Asn Gly Arg Leu Ala Asp Phe Ala Asp Lys
65                  70                  75                  80

aca ttc gac gac tat cca aac cat act ttt tac atg tct att att ggt      288
Thr Phe Asp Asp Tyr Pro Asn His Thr Phe Tyr Met Ser Ile Ile Gly
                85                  90                  95

gct ttg aaa atc gtc ttg act gtt gat cca gaa aat att aaa gct gtt      336
Ala Leu Lys Ile Val Leu Thr Val Asp Pro Glu Asn Ile Lys Ala Val
            100                 105                 110

ttg gct act caa ttt act gat ttc tcc tta ggt acc aga cat gct cat      384
Leu Ala Thr Gln Phe Thr Asp Phe Ser Leu Gly Thr Arg His Ala His
        115                 120                 125

ttc tat cca tta tta ggt gat ggt att ttt act ttg gat ggt gaa ggt      432
Phe Tyr Pro Leu Leu Gly Asp Gly Ile Phe Thr Leu Asp Gly Glu Gly

130                 135                 140

tgg aaa cat agt aga gct atg ttg aga cca caa ttt gct aga gat caa       480
Trp Lys His Ser Arg Ala Met Leu Arg Pro Gln Phe Ala Arg Asp Gln
145                 150                 155                 160

att ggt cat gtt aaa gct ttg gaa cca cat att caa atc ttg gcc aaa       528
Ile Gly His Val Lys Ala Leu Glu Pro His Ile Gln Ile Leu Ala Lys
                165                 170                 175

caa att aaa ttg aat aaa ggt aaa act ttt gat att caa gaa ttg ttt       576
Gln Ile Lys Leu Asn Lys Gly Lys Thr Phe Asp Ile Gln Glu Leu Phe
            180                 185                 190

ttc aga ttt act gtt gat act gct act gaa ttc ttg ttt ggt gaa tct       624
Phe Arg Phe Thr Val Asp Thr Ala Thr Glu Phe Leu Phe Gly Glu Ser
        195                 200                 205

gtt cac tct ttg tat gat gaa aaa tta ggt att cct act cca aat gaa       672
Val His Ser Leu Tyr Asp Glu Lys Leu Gly Ile Pro Thr Pro Asn Glu
    210                 215                 220

att cca ggt aga gat aat ttt gca act gct ttt aac act tct caa cat       720
Ile Pro Gly Arg Asp Asn Phe Ala Thr Ala Phe Asn Thr Ser Gln His
225                 230                 235                 240

tat ttg gct acc aga aca tac tcc caa act ttc tac ttt tta act aac       768
Tyr Leu Ala Thr Arg Thr Tyr Ser Gln Thr Phe Tyr Phe Leu Thr Asn
                245                 250                 255

cct aag gaa ttt aga gac tgt aat gct aaa gtt cat tac ttg gct aaa       816
Pro Lys Glu Phe Arg Asp Cys Asn Ala Lys Val His Tyr Leu Ala Lys
            260                 265                 270

tat ttt gtc aat aaa gct ttg aat ttc act ccg gaa gaa att gaa gaa       864
Tyr Phe Val Asn Lys Ala Leu Asn Phe Thr Pro Glu Glu Ile Glu Glu
        275                 280                 285

aag tcc aaa tct ggt tat gtt ttc ttg tat gaa ttg gtt aaa caa acc       912
Lys Ser Lys Ser Gly Tyr Val Phe Leu Tyr Glu Leu Val Lys Gln Thr
    290                 295                 300

aga gat cca aaa gtt tta caa gat caa tta ttg aac att atg gtt gcc       960
Arg Asp Pro Lys Val Leu Gln Asp Gln Leu Leu Asn Ile Met Val Ala
305                 310                 315                 320

ggt aga gat acc act gct ggt tta tta tca ttt gca atg ttt gaa tta      1008
Gly Arg Asp Thr Thr Ala Gly Leu Leu Ser Phe Ala Met Phe Glu Leu
                325                 330                 335

gct aga cat cca gaa att tgg tct aaa tta aga gaa gaa att gaa gtt      1056
Ala Arg His Pro Glu Ile Trp Ser Lys Leu Arg Glu Glu Ile Glu Val
            340                 345                 350

aac ttt ggt gtt ggt gaa gaa tct cgt gtt gaa gaa att act ttt gaa      1104
Asn Phe Gly Val Gly Glu Glu Ser Arg Val Glu Glu Ile Thr Phe Glu
        355                 360                 365

tct ttg aag aga tgt gaa tac ttg aaa gct att ctt aat gaa act ttg      1152
Ser Leu Lys Arg Cys Glu Tyr Leu Lys Ala Ile Leu Asn Glu Thr Leu
    370                 375                 380

cgt atg tat cct tct gtt cca gtc aat tcc aga aca gcc act aga gat      1200
Arg Met Tyr Pro Ser Val Pro Val Asn Ser Arg Thr Ala Thr Arg Asp

385                 390                 395                 400

acc aca tta cca aga ggt ggt ggt cca aat ggt act gat cca att ttt      1248
Thr Thr Leu Pro Arg Gly Gly Gly Pro Asn Gly Thr Asp Pro Ile Phe
                405                 410                 415

att cca aag ggt tcc act gtt gct tat att gtt tac aaa act cat cgt      1296
Ile Pro Lys Gly Ser Thr Val Ala Tyr Ile Val Tyr Lys Thr His Arg
            420                 425                 430

tta gaa gaa tat tat ggt aaa gat gct gat gat ttc aga cca gaa aga      1344
Leu Glu Glu Tyr Tyr Gly Lys Asp Ala Asp Asp Phe Arg Pro Glu Arg
        435                 440                 445

tgg ttt gaa cca tca act aaa aag tta ggt tgg gct tat gtt cca ttt      1392
Trp Phe Glu Pro Ser Thr Lys Lys Leu Gly Trp Ala Tyr Val Pro Phe
    450                 455                 460

aat ggt ggt cca aga att tgt tta ggc caa caa ttt gct tta act gaa      1440
Asn Gly Gly Pro Arg Ile Cys Leu Gly Gln Gln Phe Ala Leu Thr Glu
465                 470                 475                 480

gct tct tat gtt att acc aga ttg gta caa atg ttt gaa act gtt tct      1488
Ala Ser Tyr Val Ile Thr Arg Leu Val Gln Met Phe Glu Thr Val Ser
                485                 490                 495

tct tcc cca gat gtt gaa tac cct cca cca aaa tgt att cat ttg act      1536
Ser Ser Pro Asp Val Glu Tyr Pro Pro Pro Lys Cys Ile His Leu Thr
            500                 505                 510

atg agt cat gat gat ggt gtt ttc gtt aaa atg taa                      1572
Met Ser His Asp Asp Gly Val Phe Val Lys Met
        515                 520


<210>  36
<211>  523
<212>  PRT
<213>  Candida tropicalis

<400>  36

Met Ala Thr Gln Glu Ile Ile Asp Ser Ala Leu Pro Tyr Leu Thr Lys
1               5                   10                  15


Trp Tyr Thr Val Ile Thr Leu Ala Ala Leu Val Phe Leu Ile Ser Ser
            20                  25                  30


Asn Ile Lys Asn Tyr Val Lys Ala Lys Lys Leu Lys Cys Arg Asp Pro
        35                  40                  45


Pro Tyr Phe Lys Gly Ala Gly Trp Thr Gly Ile Ser Pro Leu Ile Glu
    50                  55                  60


Ile Ile Lys Val Lys Gly Asn Gly Arg Leu Ala Asp Phe Ala Asp Lys
65                  70                  75                  80


Thr Phe Asp Asp Tyr Pro Asn His Thr Phe Tyr Met Ser Ile Ile Gly
                85                  90                  95

Ala Leu Lys Ile Val Leu Thr Val Asp Pro Glu Asn Ile Lys Ala Val
            100                 105                 110

Leu Ala Thr Gln Phe Thr Asp Phe Ser Leu Gly Thr Arg His Ala His
        115                 120                 125

Phe Tyr Pro Leu Leu Gly Asp Gly Ile Phe Thr Leu Asp Gly Glu Gly
    130                 135                 140

Trp Lys His Ser Arg Ala Met Leu Arg Pro Gln Phe Ala Arg Asp Gln
145                 150                 155                 160

Ile Gly His Val Lys Ala Leu Glu Pro His Ile Gln Ile Leu Ala Lys
                165                 170                 175

Gln Ile Lys Leu Asn Lys Gly Lys Thr Phe Asp Ile Gln Glu Leu Phe
            180                 185                 190

Phe Arg Phe Thr Val Asp Thr Ala Thr Glu Phe Leu Phe Gly Glu Ser
        195                 200                 205

Val His Ser Leu Tyr Asp Glu Lys Leu Gly Ile Pro Thr Pro Asn Glu
    210                 215                 220

Ile Pro Gly Arg Asp Asn Phe Ala Thr Ala Phe Asn Thr Ser Gln His
225                 230                 235                 240

Tyr Leu Ala Thr Arg Thr Tyr Ser Gln Thr Phe Tyr Phe Leu Thr Asn
                245                 250                 255

Pro Lys Glu Phe Arg Asp Cys Asn Ala Lys Val His Tyr Leu Ala Lys
            260                 265                 270

Tyr Phe Val Asn Lys Ala Leu Asn Phe Thr Pro Glu Glu Ile Glu Glu
        275                 280                 285

Lys Ser Lys Ser Gly Tyr Val Phe Leu Tyr Glu Leu Val Lys Gln Thr
    290                 295                 300

Arg Asp Pro Lys Val Leu Gln Asp Gln Leu Leu Asn Ile Met Val Ala
305                 310                 315                 320

Gly Arg Asp Thr Thr Ala Gly Leu Leu Ser Phe Ala Met Phe Glu Leu
                325                 330                 335

Ala Arg His Pro Glu Ile Trp Ser Lys Leu Arg Glu Glu Ile Glu Val
            340                 345                 350

Asn Phe Gly Val Gly Glu Glu Ser Arg Val Glu Glu Ile Thr Phe Glu
        355                 360                 365

Ser Leu Lys Arg Cys Glu Tyr Leu Lys Ala Ile Leu Asn Glu Thr Leu
    370                 375                 380

Arg Met Tyr Pro Ser Val Pro Val Asn Ser Arg Thr Ala Thr Arg Asp
385                 390                 395                 400

Thr Thr Leu Pro Arg Gly Gly Gly Pro Asn Gly Thr Asp Pro Ile Phe
                405                 410                 415

Ile Pro Lys Gly Ser Thr Val Ala Tyr Ile Val Tyr Lys Thr His Arg
            420                 425                 430

Leu Glu Glu Tyr Tyr Gly Lys Asp Ala Asp Asp Phe Arg Pro Glu Arg
        435                 440                 445

Trp Phe Glu Pro Ser Thr Lys Lys Leu Gly Trp Ala Tyr Val Pro Phe
    450                 455                 460

Asn Gly Gly Pro Arg Ile Cys Leu Gly Gln Gln Phe Ala Leu Thr Glu
465                 470                 475                 480

Ala Ser Tyr Val Ile Thr Arg Leu Val Gln Met Phe Glu Thr Val Ser
                485                 490                 495

Ser Ser Pro Asp Val Glu Tyr Pro Pro Pro Lys Cys Ile His Leu Thr
            500                 505                 510

Met Ser His Asp Asp Gly Val Phe Val Lys Met
        515                 520

<210>  37
<211>  1512
<212>  DNA
<213>  Candida tropicalis

<220>
<221>  CDS
<222>  (1)..(1512)

<400>  37
atg tca tta aca gaa aca act gct acg ttt att tac aat tat tgg tac        48
Met Ser Leu Thr Glu Thr Thr Ala Thr Phe Ile Tyr Asn Tyr Trp Tyr
1               5                   10                  15

atc ata ttc att ata ttc tac aca acc agc aaa atc atc aag tat cat        96
Ile Ile Phe Ile Ile Phe Tyr Thr Thr Ser Lys Ile Ile Lys Tyr His
            20                  25                  30

cac acg aca tat ctt atg ata aag ttc aaa gct tct ccg cct ttg aat 144
His Thr Thr Tyr Leu Met Ile Lys Phe Lys Ala Ser Pro Pro Leu Asn
        35                  40                  45

tac ata aat aaa ggt ttt ttt gga att cag gcg acg ttc acg gaa ttg 192
Tyr Ile Asn Lys Gly Phe Phe Gly Ile Gln Ala Thr Phe Thr Glu Leu
    50                  55                  60

aaa cat ctt ata tgt cac aca tcg att gat tac gcc atc gat caa ttc 240
Lys His Leu Ile Cys His Thr Ser Ile Asp Tyr Ala Ile Asp Gln Phe
65                  70                  75                  80

aat aac gtc cca ttc cca cat gtt cat act ttt gta acc aaa gtt ctt 288
Asn Asn Val Pro Phe Pro His Val His Thr Phe Val Thr Lys Val Leu
                85                  90                  95

ggt aat gag tta atc atg aca aaa gat cct gaa aat att aaa gtt tta 336
Gly Asn Glu Leu Ile Met Thr Lys Asp Pro Glu Asn Ile Lys Val Leu
            100                 105                 110

ttg agt tcc agt ttt gat aag ttt gat tat gga aca cgt tca agt gcc 384
Leu Ser Ser Ser Phe Asp Lys Phe Asp Tyr Gly Thr Arg Ser Ser Ala
        115                 120                 125

gtg caa cca tct tta gga atg ggg ata ttc act ctt gaa gga gaa aat 432
Val Gln Pro Ser Leu Gly Met Gly Ile Phe Thr Leu Glu Gly Glu Asn
    130                 135                 140

tgg aaa gca aca aga agt gtt tta agg aac atg ttt gat aga aaa tca 480
Trp Lys Ala Thr Arg Ser Val Leu Arg Asn Met Phe Asp Arg Lys Ser
145                 150                 155                 160

att gac aag gta cat gat ttt gaa cca cat ttc aaa acc ctt cag aaa 528
Ile Asp Lys Val His Asp Phe Glu Pro His Phe Lys Thr Leu Gln Lys
                165                 170                 175

aga ata gat ggg aag gtt gga tat ttt gac atc caa cag gag ttt tta 576
Arg Ile Asp Gly Lys Val Gly Tyr Phe Asp Ile Gln Gln Glu Phe Leu
            180                 185                 190

aaa tta gga ttg gaa ttg agt att gag ttt att ttt ggt caa gtt gta 624
Lys Leu Gly Leu Glu Leu Ser Ile Glu Phe Ile Phe Gly Gln Val Val
        195                 200                 205

tcg gaa gat gtc cca cat tat gat gat ttt acc cag gct tgg gat aga 672
Ser Glu Asp Val Pro His Tyr Asp Asp Phe Thr Gln Ala Trp Asp Arg
    210                 215                 220

tgt caa gac tat atg atg cta aga tta ttg ttg ggg gat ttt tat tgg 720
Cys Gln Asp Tyr Met Met Leu Arg Leu Leu Leu Gly Asp Phe Tyr Trp
225                 230                 235                 240

ata gct aat gac tgg aga tat aaa cag tcc aat caa att gtg caa gct 768
Ile Ala Asn Asp Trp Arg Tyr Lys Gln Ser Asn Gln Ile Val Gln Ala
                245                 250                 255

ttt tgt gat tat ttg gtg caa aaa tca ctt gaa aat aca tgc aac gac 816
Phe Cys Asp Tyr Leu Val Gln Lys Ser Leu Glu Asn Thr Cys Asn Asp
            260                 265                 270

aaa ttt gtc ttt gta cag gaa ctt gca aaa cac acg acc aac aaa acg 864
Lys Phe Val Phe Val Gln Glu Leu Ala Lys His Thr Thr Asn Lys Thr
        275                 280                 285

ttc att aga gat caa gca tta agc ttg att atg gct tca aga gac aca 912
Phe Ile Arg Asp Gln Ala Leu Ser Leu Ile Met Ala Ser Arg Asp Thr
    290                 295                 300

act gct gag ttg atg gca ttc acc ata cta gaa tta tcc aga aat ccc 960
Thr Ala Glu Leu Met Ala Phe Thr Ile Leu Glu Leu Ser Arg Asn Pro
305                 310                 315                 320

act atc tgg gaa aga tta aga gag gaa ata gat gcc aat ttt gga ttg 1008
Thr Ile Trp Glu Arg Leu Arg Glu Glu Ile Asp Ala Asn Phe Gly Leu
                325                 330                 335

gaa tca ccc gac ttg ctt aca ttt gat tca ctt cgc aag ttc aaa tat 1056
Glu Ser Pro Asp Leu Leu Thr Phe Asp Ser Leu Arg Lys Phe Lys Tyr
            340                 345                 350

gtt caa gct atc ttg aat gag act ctt agg atg tac cct gga gtt cca 1104
Val Gln Ala Ile Leu Asn Glu Thr Leu Arg Met Tyr Pro Gly Val Pro
        355                 360                 365

aga aat atg aaa act gct aaa tgt act act aca tta cca aaa gga gga 1152
Arg Asn Met Lys Thr Ala Lys Cys Thr Thr Thr Leu Pro Lys Gly Gly
    370                 375                 380

ggg aaa gat ggt caa gat cca att cta gtt aaa aag ggt caa tct gtt 1200
Gly Lys Asp Gly Gln Asp Pro Ile Leu Val Lys Lys Gly Gln Ser Val
385                 390                 395                 400

ggg ttt att tcc ata gct act cat ttg gat ccg gtt tat ttt ggt agt 1248
Gly Phe Ile Ser Ile Ala Thr His Leu Asp Pro Val Tyr Phe Gly Ser
                405                 410                 415

gat gcc cat gtg ttt aga cca gac cgc tgg ttt gat tct agt atg aaa 1296
Asp Ala His Val Phe Arg Pro Asp Arg Trp Phe Asp Ser Ser Met Lys
            420                 425                 430

aat ttg ggg tgt aaa tac ttg ccc ttt aac gtc ggt cca aga acg tgt 1344
Asn Leu Gly Cys Lys Tyr Leu Pro Phe Asn Val Gly Pro Arg Thr Cys
        435                 440                 445

ttg gga caa cag tac act ttg att gag gca agc tac ttg tta gtt cgt 1392
Leu Gly Gln Gln Tyr Thr Leu Ile Glu Ala Ser Tyr Leu Leu Val Arg
    450                 455                 460

cta gcg caa aca tat gaa aca gtt gaa tca cat ccc gat tca gtt tat 1440
Leu Ala Gln Thr Tyr Glu Thr Val Glu Ser His Pro Asp Ser Val Tyr
465                 470                 475                 480

cca cca agg aag aaa gcg ttg atc aat atg tgt gct gca gac ggt gtt 1488
Pro Pro Arg Lys Lys Ala Leu Ile Asn Met Cys Ala Ala Asp Gly Val
                485                 490                 495

gat gtc aag ttc cat aga tta taa 1512
Asp Val Lys Phe His Arg Leu
            500

<210> 38
<211> 503
<212> PRT
<213> Candida tropicalis

<400> 38

Met Ser Leu Thr Glu Thr Thr Ala Thr Phe Ile Tyr Asn Tyr Trp Tyr
1               5                   10                  15

Ile Ile Phe Ile Ile Phe Tyr Thr Thr Ser Lys Ile Ile Lys Tyr His
            20                  25                  30

His Thr Thr Tyr Leu Met Ile Lys Phe Lys Ala Ser Pro Pro Leu Asn
        35                  40                  45

Tyr Ile Asn Lys Gly Phe Phe Gly Ile Gln Ala Thr Phe Thr Glu Leu
    50                  55                  60

Lys His Leu Ile Cys His Thr Ser Ile Asp Tyr Ala Ile Asp Gln Phe
65                  70                  75                  80

Asn Asn Val Pro Phe Pro His Val His Thr Phe Val Thr Lys Val Leu
                85                  90                  95

Gly Asn Glu Leu Ile Met Thr Lys Asp Pro Glu Asn Ile Lys Val Leu
            100                 105                 110

Leu Ser Ser Ser Phe Asp Lys Phe Asp Tyr Gly Thr Arg Ser Ser Ala
        115                 120                 125

Val Gln Pro Ser Leu Gly Met Gly Ile Phe Thr Leu Glu Gly Glu Asn
    130                 135                 140

Trp Lys Ala Thr Arg Ser Val Leu Arg Asn Met Phe Asp Arg Lys Ser
145                 150                 155                 160

Ile Asp Lys Val His Asp Phe Glu Pro His Phe Lys Thr Leu Gln Lys
                165                 170                 175

Arg Ile Asp Gly Lys Val Gly Tyr Phe Asp Ile Gln Gln Glu Phe Leu
            180                 185                 190

Lys Leu Gly Leu Glu Leu Ser Ile Glu Phe Ile Phe Gly Gln Val Val
        195                 200                 205

Ser Glu Asp Val Pro His Tyr Asp Asp Phe Thr Gln Ala Trp Asp Arg
    210                 215                 220

Cys Gln Asp Tyr Met Met Leu Arg Leu Leu Leu Gly Asp Phe Tyr Trp
225                 230                 235                 240

Ile Ala Asn Asp Trp Arg Tyr Lys Gln Ser Asn Gln Ile Val Gln Ala
                245                 250                 255

Phe Cys Asp Tyr Leu Val Gln Lys Ser Leu Glu Asn Thr Cys Asn Asp

260                 265                 270

Lys Phe Val Phe Val Gln Glu Leu Ala Lys His Thr Thr Asn Lys Thr
        275                 280                 285

Phe Ile Arg Asp Gln Ala Leu Ser Leu Ile Met Ala Ser Arg Asp Thr
    290                 295                 300

Thr Ala Glu Leu Met Ala Phe Thr Ile Leu Glu Leu Ser Arg Asn Pro
305                 310                 315                 320

Thr Ile Trp Glu Arg Leu Arg Glu Glu Ile Asp Ala Asn Phe Gly Leu
                325                 330                 335

Glu Ser Pro Asp Leu Leu Thr Phe Asp Ser Leu Arg Lys Phe Lys Tyr
            340                 345                 350

Val Gln Ala Ile Leu Asn Glu Thr Leu Arg Met Tyr Pro Gly Val Pro
        355                 360                 365

Arg Asn Met Lys Thr Ala Lys Cys Thr Thr Thr Leu Pro Lys Gly Gly
    370                 375                 380

Gly Lys Asp Gly Gln Asp Pro Ile Leu Val Lys Lys Gly Gln Ser Val
385                 390                 395                 400

Gly Phe Ile Ser Ile Ala Thr His Leu Asp Pro Val Tyr Phe Gly Ser
                405                 410                 415

Asp Ala His Val Phe Arg Pro Asp Arg Trp Phe Asp Ser Ser Met Lys
            420                 425                 430

Asn Leu Gly Cys Lys Tyr Leu Pro Phe Asn Val Gly Pro Arg Thr Cys
        435                 440                 445

Leu Gly Gln Gln Tyr Thr Leu Ile Glu Ala Ser Tyr Leu Leu Val Arg
    450                 455                 460

Leu Ala Gln Thr Tyr Glu Thr Val Glu Ser His Pro Asp Ser Val Tyr
465                 470                 475                 480

Pro Pro Arg Lys Lys Ala Leu Ile Asn Met Cys Ala Ala Asp Gly Val
                485                 490                 495

Asp Val Lys Phe His Arg Leu
            500

<210>  39

<211>  1554
<212>  DNA
<213>  Candida tropicalis

<220>
<221>  CDS
<222>  (1)..(1554)

<400>  39
atg atc gaa caa gtt gtt gaa tac tgg tac gtg gtc tta cca ttg gta        48
Met Ile Glu Gln Val Val Glu Tyr Trp Tyr Val Val Leu Pro Leu Val
1               5                   10                  15

ttt atc ctt cat aaa gta ttt gac atg tgg cac act cgt cgg ttg atg        96
Phe Ile Leu His Lys Val Phe Asp Met Trp His Thr Arg Arg Leu Met
            20                  25                  30

aag caa ttg ggc gct gct cct gtc aca aac caa tta cac gac aat ttt       144
Lys Gln Leu Gly Ala Ala Pro Val Thr Asn Gln Leu His Asp Asn Phe
        35                  40                  45

ttt ggt att atc aac gga tgg aaa gca ctt aag ttc aag aaa gaa ggt       192
Phe Gly Ile Ile Asn Gly Trp Lys Ala Leu Lys Phe Lys Lys Glu Gly
    50                  55                  60

aga gct caa gaa tat aat gat tat aaa ttt gcc aat tcg aaa att cca       240
Arg Ala Gln Glu Tyr Asn Asp Tyr Lys Phe Ala Asn Ser Lys Ile Pro
65                  70                  75                  80

agt gtg ggt act tat gtt agt acc atc ttt gga aca aag ctc ctc gtc       288
Ser Val Gly Thr Tyr Val Ser Thr Ile Phe Gly Thr Lys Leu Leu Val
                85                  90                  95

aca aaa gat ccg gag aat atc aaa gct tta tta gca acc caa ttc agt       336
Thr Lys Asp Pro Glu Asn Ile Lys Ala Leu Leu Ala Thr Gln Phe Ser
            100                 105                 110

gat ttt tcc ttg ggt aag agg cat aca ctt ttc aaa cca tta tta ggt       384
Asp Phe Ser Leu Gly Lys Arg His Thr Leu Phe Lys Pro Leu Leu Gly
        115                 120                 125

gac ggt att ttc act ttg gat gga gaa ggt tgg aaa cat agt cga gct       432
Asp Gly Ile Phe Thr Leu Asp Gly Glu Gly Trp Lys His Ser Arg Ala
    130                 135                 140

atg ttg aga cca cag ttt gca aga gaa caa gtt gcc cat gtc act tct       480
Met Leu Arg Pro Gln Phe Ala Arg Glu Gln Val Ala His Val Thr Ser
145                 150                 155                 160

tta gag cca cat ttc caa ttg ttg aaa aaa cat atc ctc aag aac aaa       528
Leu Glu Pro His Phe Gln Leu Leu Lys Lys His Ile Leu Lys Asn Lys
                165                 170                 175

gga ggt ttt ttt gat atc cag gaa ttg ttt ttc cga ttc act gtt gat       576
Gly Gly Phe Phe Asp Ile Gln Glu Leu Phe Phe Arg Phe Thr Val Asp
            180                 185                 190

tca gct act gag ttt ttg ttt ggt gaa tca gta cac tct ttg aag gat       624
Ser Ala Thr Glu Phe Leu Phe Gly Glu Ser Val His Ser Leu Lys Asp
        195                 200                 205

gaa aca att ggg tat aac caa gat gat atc gac ttt gtt ggt aga aag       672
Glu Thr Ile Gly Tyr Asn Gln Asp Asp Ile Asp Phe Val Gly Arg Lys

210                 215                 220

gat ttt gcg gaa tcg ttc aac aag gca caa gag tat ctt gct att aga         720
Asp Phe Ala Glu Ser Phe Asn Lys Ala Gln Glu Tyr Leu Ala Ile Arg
225                 230                 235                 240

act ttg gtg caa gat ttt tat tat ctt gta aac aat cag gaa ttc aga         768
Thr Leu Val Gln Asp Phe Tyr Tyr Leu Val Asn Asn Gln Glu Phe Arg
                245                 250                 255

gat tgc aat aaa ctg gta cac aag ttt acc aac tac tat gtc caa aga         816
Asp Cys Asn Lys Leu Val His Lys Phe Thr Asn Tyr Tyr Val Gln Arg
            260                 265                 270

gca ttg gat gcc act cct gaa gag ctt gaa aag caa agt gga tat gtt         864
Ala Leu Asp Ala Thr Pro Glu Glu Leu Glu Lys Gln Ser Gly Tyr Val
        275                 280                 285

ttc ttg tat gaa ttg gtt aaa caa acc aga gac cct aat gta ttg aga         912
Phe Leu Tyr Glu Leu Val Lys Gln Thr Arg Asp Pro Asn Val Leu Arg
    290                 295                 300

gat caa tca ttg aac atc tta tta gct ggt aga gat acc act gct ggg         960
Asp Gln Ser Leu Asn Ile Leu Leu Ala Gly Arg Asp Thr Thr Ala Gly
305                 310                 315                 320

ttg ttg tca ttt gcg gta ttt gaa ctt gct agg aat cca cat att tgg        1008
Leu Leu Ser Phe Ala Val Phe Glu Leu Ala Arg Asn Pro His Ile Trp
                325                 330                 335

gcc aaa tta aga gaa gat gtt gaa tcc caa ttt ggt ctt ggt gaa gaa        1056
Ala Lys Leu Arg Glu Asp Val Glu Ser Gln Phe Gly Leu Gly Glu Glu
            340                 345                 350

tct cgc att gaa gag att acc ttt gaa agt tta aaa cga tgt gaa tac        1104
Ser Arg Ile Glu Glu Ile Thr Phe Glu Ser Leu Lys Arg Cys Glu Tyr
        355                 360                 365

ttg aaa gct ttc ctt aac gaa aca tta cgt gtt tat cca agt gtt cca        1152
Leu Lys Ala Phe Leu Asn Glu Thr Leu Arg Val Tyr Pro Ser Val Pro
    370                 375                 380

aga aat ttc aga att gct acc aaa aac acc act tta cca aga ggt ggt        1200
Arg Asn Phe Arg Ile Ala Thr Lys Asn Thr Thr Leu Pro Arg Gly Gly
385                 390                 395                 400

ggt tca gac ggc aat tct cct gtt ttg gtc aaa aag ggc gag gct gtt        1248
Gly Ser Asp Gly Asn Ser Pro Val Leu Val Lys Lys Gly Glu Ala Val
                405                 410                 415

tca tat ggt ata aat tct act cac tta gat cct gtc tat tat ggt gac        1296
Ser Tyr Gly Ile Asn Ser Thr His Leu Asp Pro Val Tyr Tyr Gly Asp
            420                 425                 430

gat gct gca gaa ttt aga cca gaa aga tgg aac gag cca tca aca aga        1344
Asp Ala Ala Glu Phe Arg Pro Glu Arg Trp Asn Glu Pro Ser Thr Arg
        435                 440                 445

aaa ttg gga tgg gca tat tta ccg ttc aac gga ggc cca aga att tgt        1392
Lys Leu Gly Trp Ala Tyr Leu Pro Phe Asn Gly Gly Pro Arg Ile Cys
    450                 455                 460

tta ggt caa caa ttt gct tta acc gaa gcg ggt tat gta ttg gtt aga        1440
Leu Gly Gln Gln Phe Ala Leu Thr Glu Ala Gly Tyr Val Leu Val Arg

465                 470                 475                 480

ttg gcc caa agt ttt gac acc ttg gaa ttg aag cca cca gtt gtg tat      1488
Leu Ala Gln Ser Phe Asp Thr Leu Glu Leu Lys Pro Pro Val Val Tyr
                485                 490                 495

cca cca aag aga tta aca aac ttg act atg tct tta caa gac gga act      1536
Pro Pro Lys Arg Leu Thr Asn Leu Thr Met Ser Leu Gln Asp Gly Thr
            500                 505                 510

att gtc aag atc gat tag                                              1554
Ile Val Lys Ile Asp
        515

<210>  40
<211>  517
<212>  PRT
<213>  Candida tropicalis

<400>  40

Met Ile Glu Gln Val Val Glu Tyr Trp Tyr Val Val Leu Pro Leu Val
1               5                   10                  15

Phe Ile Leu His Lys Val Phe Asp Met Trp His Thr Arg Arg Leu Met
            20                  25                  30

Lys Gln Leu Gly Ala Ala Pro Val Thr Asn Gln Leu His Asp Asn Phe
        35                  40                  45

Phe Gly Ile Ile Asn Gly Trp Lys Ala Leu Lys Phe Lys Lys Glu Gly
    50                  55                  60

Arg Ala Gln Glu Tyr Asn Asp Tyr Lys Phe Ala Asn Ser Lys Ile Pro
65                  70                  75                  80

Ser Val Gly Thr Tyr Val Ser Thr Ile Phe Gly Thr Lys Leu Leu Val
                85                  90                  95

Thr Lys Asp Pro Glu Asn Ile Lys Ala Leu Leu Ala Thr Gln Phe Ser
            100                 105                 110

Asp Phe Ser Leu Gly Lys Arg His Thr Leu Phe Lys Pro Leu Leu Gly
        115                 120                 125

Asp Gly Ile Phe Thr Leu Asp Gly Glu Gly Trp Lys His Ser Arg Ala
    130                 135                 140

Met Leu Arg Pro Gln Phe Ala Arg Glu Gln Val Ala His Val Thr Ser
145                 150                 155                 160

Leu Glu Pro His Phe Gln Leu Leu Lys Lys His Ile Leu Lys Asn Lys
                165                 170                 175

Gly Gly Phe Phe Asp Ile Gln Glu Leu Phe Phe Arg Phe Thr Val Asp
            180                 185                 190

Ser Ala Thr Glu Phe Leu Phe Gly Glu Ser Val His Ser Leu Lys Asp
        195                 200                 205

Glu Thr Ile Gly Tyr Asn Gln Asp Asp Ile Asp Phe Val Gly Arg Lys
    210                 215                 220

Asp Phe Ala Glu Ser Phe Asn Lys Ala Gln Glu Tyr Leu Ala Ile Arg
225                 230                 235                 240

Thr Leu Val Gln Asp Phe Tyr Tyr Leu Val Asn Asn Gln Glu Phe Arg
                245                 250                 255

Asp Cys Asn Lys Leu Val His Lys Phe Thr Asn Tyr Tyr Val Gln Arg
            260                 265                 270

Ala Leu Asp Ala Thr Pro Glu Glu Leu Glu Lys Gln Ser Gly Tyr Val
        275                 280                 285

Phe Leu Tyr Glu Leu Val Lys Gln Thr Arg Asp Pro Asn Val Leu Arg
    290                 295                 300

Asp Gln Ser Leu Asn Ile Leu Leu Ala Gly Arg Asp Thr Thr Ala Gly
305                 310                 315                 320

Leu Leu Ser Phe Ala Val Phe Glu Leu Ala Arg Asn Pro His Ile Trp
                325                 330                 335

Ala Lys Leu Arg Glu Asp Val Glu Ser Gln Phe Gly Leu Gly Glu Glu
            340                 345                 350

Ser Arg Ile Glu Glu Ile Thr Phe Glu Ser Leu Lys Arg Cys Glu Tyr
        355                 360                 365

Leu Lys Ala Phe Leu Asn Glu Thr Leu Arg Val Tyr Pro Ser Val Pro
    370                 375                 380

Arg Asn Phe Arg Ile Ala Thr Lys Asn Thr Thr Leu Pro Arg Gly Gly
385                 390                 395                 400

Gly Ser Asp Gly Asn Ser Pro Val Leu Val Lys Lys Gly Glu Ala Val
                405                 410                 415

Ser Tyr Gly Ile Asn Ser Thr His Leu Asp Pro Val Tyr Tyr Gly Asp
            420                 425                 430

Asp Ala Ala Glu Phe Arg Pro Glu Arg Trp Asn Glu Pro Ser Thr Arg
        435                 440                 445

Lys Leu Gly Trp Ala Tyr Leu Pro Phe Asn Gly Gly Pro Arg Ile Cys
    450                 455                 460

Leu Gly Gln Gln Phe Ala Leu Thr Glu Ala Gly Tyr Val Leu Val Arg
465                 470                 475                 480

Leu Ala Gln Ser Phe Asp Thr Leu Glu Leu Lys Pro Pro Val Val Tyr
                485                 490                 495

Pro Pro Lys Arg Leu Thr Asn Leu Thr Met Ser Leu Gln Asp Gly Thr
            500                 505                 510

Ile Val Lys Ile Asp
        515

<210>  41
<211>  1539
<212>  DNA
<213>  Candida tropicalis

<220>
<221>  CDS
<222>  (1)..(1539)

<400>  41
atg att gaa cag gtt tta cat tat tgg tat att gtt tta cct gca ttt       48
Met Ile Glu Gln Val Leu His Tyr Trp Tyr Ile Val Leu Pro Ala Phe
1               5                   10                  15

ata att ttt cat tgg att gta tct gca att cat aca aat tcc ttg cgt       96
Ile Ile Phe His Trp Ile Val Ser Ala Ile His Thr Asn Ser Leu Arg
            20                  25                  30

aga aaa cta ggt gcc aaa cct ttc act cat aca caa ctt gat ggt ttt      144
Arg Lys Leu Gly Ala Lys Pro Phe Thr His Thr Gln Leu Asp Gly Phe
        35                  40                  45

tat gga ttt aaa ttt ggc cgt gat ttt ctt aaa gct aaa agg att ggt      192
Tyr Gly Phe Lys Phe Gly Arg Asp Phe Leu Lys Ala Lys Arg Ile Gly
    50                  55                  60

agg caa gtt gat tta atc aat tcc cgt ttc cca gac gat att gac aca      240
Arg Gln Val Asp Leu Ile Asn Ser Arg Phe Pro Asp Asp Ile Asp Thr
65                  70                  75                  80

ttt tca agt tat act ttc ggc aac cac gtg att ttt aca cgt gat cca      288
Phe Ser Ser Tyr Thr Phe Gly Asn His Val Ile Phe Thr Arg Asp Pro
                85                  90                  95

gaa aat atc aaa gct tta ttg gca aca caa ttt aat gat ttt tca tta      336
Glu Asn Ile Lys Ala Leu Leu Ala Thr Gln Phe Asn Asp Phe Ser Leu
            100                 105                 110

ggt ggt aga att aag ttt ttc aaa cca ttg ttg gga tat gga atc ttt 384
Gly Gly Arg Ile Lys Phe Phe Lys Pro Leu Leu Gly Tyr Gly Ile Phe
        115                 120                 125

act ttg gat gga gaa ggt tgg aaa cat agt cga gct atg ttg aga cca 432
Thr Leu Asp Gly Glu Gly Trp Lys His Ser Arg Ala Met Leu Arg Pro
    130                 135                 140

cag ttt gca aga gaa caa gtt gcc cat gtc act tct tta gaa cca cat 480
Gln Phe Ala Arg Glu Gln Val Ala His Val Thr Ser Leu Glu Pro His
145                 150                 155                 160

ttc caa ttg ttg aaa aag cat atc ctc aag aac aaa ggt ggg ttt ttt 528
Phe Gln Leu Leu Lys Lys His Ile Leu Lys Asn Lys Gly Gly Phe Phe
                165                 170                 175

gat atc cag gaa ttg ttt ttc cga ttc acc gtt gat tca gct act gag 576
Asp Ile Gln Glu Leu Phe Phe Arg Phe Thr Val Asp Ser Ala Thr Glu
            180                 185                 190

ttt ttg ttt ggt gaa tca gtg aac tct ttg aaa agt gca tca att ggt 624
Phe Leu Phe Gly Glu Ser Val Asn Ser Leu Lys Ser Ala Ser Ile Gly
        195                 200                 205

tgt gac gag gaa acc gag ctt gag gaa aga aag aaa ttt gcg gaa gca 672
Cys Asp Glu Glu Thr Glu Leu Glu Glu Arg Lys Lys Phe Ala Glu Ala
    210                 215                 220

ttc aat aaa gcg caa gag tat att tct act cga gtt gct ttg caa caa 720
Phe Asn Lys Ala Gln Glu Tyr Ile Ser Thr Arg Val Ala Leu Gln Gln
225                 230                 235                 240

tta tat tgg ttt gtt aat aat agc gaa ttc aag gaa tgt aac gaa att 768
Leu Tyr Trp Phe Val Asn Asn Ser Glu Phe Lys Glu Cys Asn Glu Ile
                245                 250                 255

gtt cat aag ttt acc aat tat tat gta caa aag gca ttg gat gct act 816
Val His Lys Phe Thr Asn Tyr Tyr Val Gln Lys Ala Leu Asp Ala Thr
            260                 265                 270

cct gaa gag ctt gaa aag caa agt gga tat gtt ttc ttg tat gaa ttg 864
Pro Glu Glu Leu Glu Lys Gln Ser Gly Tyr Val Phe Leu Tyr Glu Leu
        275                 280                 285

gtt aaa caa acc aga gac cct aat gta ttg aga gat caa tca ttg aat 912
Val Lys Gln Thr Arg Asp Pro Asn Val Leu Arg Asp Gln Ser Leu Asn
    290                 295                 300

atc tta tta gct ggt aga gat acc act gct ggg ttg ttg tca ttt gcg 960
Ile Leu Leu Ala Gly Arg Asp Thr Thr Ala Gly Leu Leu Ser Phe Ala
305                 310                 315                 320

gta ttt gaa ctt gct agg aat cca cat att tgg gcc aaa tta aga gaa 1008
Val Phe Glu Leu Ala Arg Asn Pro His Ile Trp Ala Lys Leu Arg Glu
                325                 330                 335

gat gtc gaa tcc caa ttt ggt ctt ggt gaa gaa tct cgc att gaa gag 1056
Asp Val Glu Ser Gln Phe Gly Leu Gly Glu Glu Ser Arg Ile Glu Glu
            340                 345                 350

att acc ttt gaa agt tta aaa cga tgt gaa tat ttg aaa gcc gtg atg 1104
Ile Thr Phe Glu Ser Leu Lys Arg Cys Glu Tyr Leu Lys Ala Val Met
        355                 360                 365

aat gaa aca ttg aga ttg cat cca agt gtt cca aga aat gct aga ttt      1152
Asn Glu Thr Leu Arg Leu His Pro Ser Val Pro Arg Asn Ala Arg Phe
    370                 375                 380

gca ctt aag gat aca act tta cct aga ggt gga ggt cca gat gga aaa      1200
Ala Leu Lys Asp Thr Thr Leu Pro Arg Gly Gly Gly Pro Asp Gly Lys
385                 390                 395                 400

gac ccg att tta gtt aga aaa aat gaa gtt gtt caa tat tcc att tct      1248
Asp Pro Ile Leu Val Arg Lys Asn Glu Val Val Gln Tyr Ser Ile Ser
                405                 410                 415

ggc aca caa att gat cca aaa cat tat ggc aaa gat gct aaa ttg ttt      1296
Gly Thr Gln Ile Asp Pro Lys His Tyr Gly Lys Asp Ala Lys Leu Phe
            420                 425                 430

aga cca gaa aga tgg ttt gaa tca agt aca aga aat tta ggt tgg gca      1344
Arg Pro Glu Arg Trp Phe Glu Ser Ser Thr Arg Asn Leu Gly Trp Ala
        435                 440                 445

tac tta cca ttc aac ggg ggt ccg aga att tgt tta ggt caa caa ttt      1392
Tyr Leu Pro Phe Asn Gly Gly Pro Arg Ile Cys Leu Gly Gln Gln Phe
    450                 455                 460

gct tta acc gaa gca ggt tac ata ttg gtt aga ttg gct caa agt ttt      1440
Ala Leu Thr Glu Ala Gly Tyr Ile Leu Val Arg Leu Ala Gln Ser Phe
465                 470                 475                 480

gac acc ttg gaa ttg aaa cca gat aca gaa tac cct cca cca aga tta      1488
Asp Thr Leu Glu Leu Lys Pro Asp Thr Glu Tyr Pro Pro Pro Arg Leu
                485                 490                 495

gcc cat ttg act atg tgt ttg ttt gat ggt gcg ctt gtc aag atg gat      1536
Ala His Leu Thr Met Cys Leu Phe Asp Gly Ala Leu Val Lys Met Asp
            500                 505                 510

taa                                                                  1539


<210>  42
<211>  512
<212>  PRT
<213>  Candida tropicalis

<400>  42

Met Ile Glu Gln Val Leu His Tyr Trp Tyr Ile Val Leu Pro Ala Phe
1               5                   10                  15

Ile Ile Phe His Trp Ile Val Ser Ala Ile His Thr Asn Ser Leu Arg
            20                  25                  30

Arg Lys Leu Gly Ala Lys Pro Phe Thr His Thr Gln Leu Asp Gly Phe
        35                  40                  45

Tyr Gly Phe Lys Phe Gly Arg Asp Phe Leu Lys Ala Lys Arg Ile Gly
    50                  55                  60

Arg Gln Val Asp Leu Ile Asn Ser Arg Phe Pro Asp Asp Ile Asp Thr

65                  70                  75                  80

Phe Ser Ser Tyr Thr Phe Gly Asn His Val Ile Phe Thr Arg Asp Pro
                85                  90                  95

Glu Asn Ile Lys Ala Leu Leu Ala Thr Gln Phe Asn Asp Phe Ser Leu
            100                 105                 110

Gly Gly Arg Ile Lys Phe Phe Lys Pro Leu Leu Gly Tyr Gly Ile Phe
        115                 120                 125

Thr Leu Asp Gly Glu Gly Trp Lys His Ser Arg Ala Met Leu Arg Pro
    130                 135                 140

Gln Phe Ala Arg Glu Gln Val Ala His Val Thr Ser Leu Glu Pro His
145                 150                 155                 160

Phe Gln Leu Leu Lys Lys His Ile Leu Lys Asn Lys Gly Gly Phe Phe
                165                 170                 175

Asp Ile Gln Glu Leu Phe Phe Arg Phe Thr Val Asp Ser Ala Thr Glu
            180                 185                 190

Phe Leu Phe Gly Glu Ser Val Asn Ser Leu Lys Ser Ala Ser Ile Gly
        195                 200                 205

Cys Asp Glu Glu Thr Glu Leu Glu Glu Arg Lys Lys Phe Ala Glu Ala
    210                 215                 220

Phe Asn Lys Ala Gln Glu Tyr Ile Ser Thr Arg Val Ala Leu Gln Gln
225                 230                 235                 240

Leu Tyr Trp Phe Val Asn Asn Ser Glu Phe Lys Glu Cys Asn Glu Ile
                245                 250                 255

Val His Lys Phe Thr Asn Tyr Tyr Val Gln Lys Ala Leu Asp Ala Thr
            260                 265                 270

Pro Glu Glu Leu Glu Lys Gln Ser Gly Tyr Val Phe Leu Tyr Glu Leu
        275                 280                 285

Val Lys Gln Thr Arg Asp Pro Asn Val Leu Arg Asp Gln Ser Leu Asn
    290                 295                 300

Ile Leu Leu Ala Gly Arg Asp Thr Thr Ala Gly Leu Leu Ser Phe Ala
305                 310                 315                 320

Val Phe Glu Leu Ala Arg Asn Pro His Ile Trp Ala Lys Leu Arg Glu

                325                 330                 335

Asp Val Glu Ser Gln Phe Gly Leu Gly Glu Glu Ser Arg Ile Glu Glu
            340                 345                 350

Ile Thr Phe Glu Ser Leu Lys Arg Cys Glu Tyr Leu Lys Ala Val Met
        355                 360                 365

Asn Glu Thr Leu Arg Leu His Pro Ser Val Pro Arg Asn Ala Arg Phe
    370                 375                 380

Ala Leu Lys Asp Thr Thr Leu Pro Arg Gly Gly Gly Pro Asp Gly Lys
385                 390                 395                 400

Asp Pro Ile Leu Val Arg Lys Asn Glu Val Val Gln Tyr Ser Ile Ser
                405                 410                 415

Gly Thr Gln Ile Asp Pro Lys His Tyr Gly Lys Asp Ala Lys Leu Phe
            420                 425                 430

Arg Pro Glu Arg Trp Phe Glu Ser Ser Thr Arg Asn Leu Gly Trp Ala
        435                 440                 445

Tyr Leu Pro Phe Asn Gly Gly Pro Arg Ile Cys Leu Gly Gln Gln Phe
    450                 455                 460

Ala Leu Thr Glu Ala Gly Tyr Ile Leu Val Arg Leu Ala Gln Ser Phe
465                 470                 475                 480

Asp Thr Leu Glu Leu Lys Pro Asp Thr Glu Tyr Pro Pro Pro Arg Leu
                485                 490                 495

Ala His Leu Thr Met Cys Leu Phe Asp Gly Ala Leu Val Lys Met Asp
            500                 505                 510

<210> 43
<211> 1572
<212> DNA
<213> Candida tropicalis

<220>
<221> CDS
<222> (1)..(1572)

<400> 43
atg att aaa gaa ata gtg tat ttt gta tat tat tat gga att acc cac 48
Met Ile Lys Glu Ile Val Tyr Phe Val Tyr Tyr Tyr Gly Ile Thr His
1               5                   10                  15

tca gtt tca gtg cag gtc acc gct ctt gtc ctt atc gtg aca tac ttt 96
Ser Val Ser Val Gln Val Thr Ala Leu Val Leu Ile Val Thr Tyr Phe

20                  25                  30

ttt gtt att cgt cca att aac tca cct tta tgg aga gtt ccc gga cca       144
Phe Val Ile Arg Pro Ile Asn Ser Pro Leu Trp Arg Val Pro Gly Pro
        35                  40                  45

tat tta cat cgg gta act tac ttt cca tgt tta aat gcc caa cga aag       192
Tyr Leu His Arg Val Thr Tyr Phe Pro Cys Leu Asn Ala Gln Arg Lys
    50                  55                  60

gga gaa tgg atc tcc aaa gtt tat gat ttg cac aag aaa tat ggt gat       240
Gly Glu Trp Ile Ser Lys Val Tyr Asp Leu His Lys Lys Tyr Gly Asp
65                  70                  75                  80

gta gtt tta ctt tct cca aat gaa atc agc gtc aat ggt gac cca aaa       288
Val Val Leu Leu Ser Pro Asn Glu Ile Ser Val Asn Gly Asp Pro Lys
                85                  90                  95

tat ttg act gat att tat gta aag aac ctc cca aag tca aag ttt tat       336
Tyr Leu Thr Asp Ile Tyr Val Lys Asn Leu Pro Lys Ser Lys Phe Tyr
            100                 105                 110

gaa aac ttt aga aat cat gga ttc cag gat aat att ttt gcc agt ttg       384
Glu Asn Phe Arg Asn His Gly Phe Gln Asp Asn Ile Phe Ala Ser Leu
        115                 120                 125

gaa aat gat aga cat atc aag tat aaa aga atg ata aat aac ttg tac       432
Glu Asn Asp Arg His Ile Lys Tyr Lys Arg Met Ile Asn Asn Leu Tyr
    130                 135                 140

agt aaa tct tcc atc ttc tcc aaa gaa aac cac aca aga tca gtt tta       480
Ser Lys Ser Ser Ile Phe Ser Lys Glu Asn His Thr Arg Ser Val Leu
145                 150                 155                 160

ttt gac acc aca aaa aca tta gtc gat gca gtt gct aga gaa tct ccg       528
Phe Asp Thr Thr Lys Thr Leu Val Asp Ala Val Ala Arg Glu Ser Pro
                165                 170                 175

tca att gat gtg ttt acg tta ttt ggt tcc ttg gcc atg gat gtg gtt       576
Ser Ile Asp Val Phe Thr Leu Phe Gly Ser Leu Ala Met Asp Val Val
            180                 185                 190

tca agg ttt gaa ctt ggt aga gac aat gga acg gat tta ttg aat cat       624
Ser Arg Phe Glu Leu Gly Arg Asp Asn Gly Thr Asp Leu Leu Asn His
        195                 200                 205

cca caa gaa agg cat att att gaa tct cat aga aag gtg tct tgt atg       672
Pro Gln Glu Arg His Ile Ile Glu Ser His Arg Lys Val Ser Cys Met
    210                 215                 220

ggg ttc tgg aca aca ttg atg cca agc ttc ttt tgg aat ttg gca gca       720
Gly Phe Trp Thr Thr Leu Met Pro Ser Phe Phe Trp Asn Leu Ala Ala
225                 230                 235                 240

acc aag gcc acc ttg caa gct gtt gat gat att tgc aat ttc caa ttg       768
Thr Lys Ala Thr Leu Gln Ala Val Asp Asp Ile Cys Asn Phe Gln Leu
                245                 250                 255

ggt tta tat aaa att gca gaa tcc aat ctt gtg tcc aat ggt aaa aac       816
Gly Leu Tyr Lys Ile Ala Glu Ser Asn Leu Val Ser Asn Gly Lys Asn
            260                 265                 270

cta act acg ata caa aca ctc aag aag tac gga ttg gaa gga aat tct       864
Leu Thr Thr Ile Gln Thr Leu Lys Lys Tyr Gly Leu Glu Gly Asn Ser

275                 280                 285

gcg tat tct ttt ctc acg gat aat tta ttt gct gga cat gaa act aca         912
Ala Tyr Ser Phe Leu Thr Asp Asn Leu Phe Ala Gly His Glu Thr Thr
    290                 295                 300

gct gtt caa ttg aca tat ttg tgt tat gaa tta tca aga cca gct aat         960
Ala Val Gln Leu Thr Tyr Leu Cys Tyr Glu Leu Ser Arg Pro Ala Asn
305                 310                 315                 320

tac aaa att cag aat aga tta aga tat gag ctt caa gaa gca ttt cca        1008
Tyr Lys Ile Gln Asn Arg Leu Arg Tyr Glu Leu Gln Glu Ala Phe Pro
                325                 330                 335

agt ggt caa att gaa gat ttg gaa gtg gtt gat aat ctt ccg tat ctt        1056
Ser Gly Gln Ile Glu Asp Leu Glu Val Val Asp Asn Leu Pro Tyr Leu
            340                 345                 350

aat gcg ttg ttg tcc gaa aat ggt cga att cac acc tca att cct gga        1104
Asn Ala Leu Leu Ser Glu Asn Gly Arg Ile His Thr Ser Ile Pro Gly
        355                 360                 365

gct gag cca cgt gtg gtt gca aaa ccg tat act att ggc aaa ttg ctt        1152
Ala Glu Pro Arg Val Val Ala Lys Pro Tyr Thr Ile Gly Lys Leu Leu
    370                 375                 380

att cca gtt ggt acg gtt atc tct tgt ctt cct tat gcg tat cat aga        1200
Ile Pro Val Gly Thr Val Ile Ser Cys Leu Pro Tyr Ala Tyr His Arg
385                 390                 395                 400

aat ccg tcg gta ttt acc aat cct gat aaa ttt atc ccc gaa aga tgg        1248
Asn Pro Ser Val Phe Thr Asn Pro Asp Lys Phe Ile Pro Glu Arg Trp
                405                 410                 415

tta gtt gac aac gaa gaa gac aag aaa cga gtc aaa caa caa gcg aag        1296
Leu Val Asp Asn Glu Glu Asp Lys Lys Arg Val Lys Gln Gln Ala Lys
            420                 425                 430

tat atg atg cca ttt ggt aaa ggt gta aga atg tgt ctt ggg atg aac        1344
Tyr Met Met Pro Phe Gly Lys Gly Val Arg Met Cys Leu Gly Met Asn
        435                 440                 445

ttg gca ctc att gaa atg aag ttg gcc att gca agt ttg tat tta aac        1392
Leu Ala Leu Ile Glu Met Lys Leu Ala Ile Ala Ser Leu Tyr Leu Asn
    450                 455                 460

ttt tct tct tcc att gac gaa gac tgg tgt ggc aaa gta tta gaa aat        1440
Phe Ser Ser Ser Ile Asp Glu Asp Trp Cys Gly Lys Val Leu Glu Asn
465                 470                 475                 480

gat gac ccc ata ggt atc ggg aat tca tgc act cat gag act gat cag        1488
Asp Asp Pro Ile Gly Ile Gly Asn Ser Cys Thr His Glu Thr Asp Gln
                485                 490                 495

gat aaa atg aaa atg tac gat gct tat act acg aga cca atg cta gat        1536
Asp Lys Met Lys Met Tyr Asp Ala Tyr Thr Thr Arg Pro Met Leu Asp
            500                 505                 510

gaa tgt tac ttg aag tgg aca aga tta act cct tga                        1572
Glu Cys Tyr Leu Lys Trp Thr Arg Leu Thr Pro
        515                 520

<210>  44

<211>  523
<212>  PRT
<213>  Candida tropicalis

<400>  44

Met Ile Lys Glu Ile Val Tyr Phe Val Tyr Tyr Tyr Gly Ile Thr His
1               5                   10                  15

Ser Val Ser Val Gln Val Thr Ala Leu Val Leu Ile Val Thr Tyr Phe
            20                  25                  30

Phe Val Ile Arg Pro Ile Asn Ser Pro Leu Trp Arg Val Pro Gly Pro
        35                  40                  45

Tyr Leu His Arg Val Thr Tyr Phe Pro Cys Leu Asn Ala Gln Arg Lys
    50                  55                  60

Gly Glu Trp Ile Ser Lys Val Tyr Asp Leu His Lys Lys Tyr Gly Asp
65                  70                  75                  80

Val Val Leu Leu Ser Pro Asn Glu Ile Ser Val Asn Gly Asp Pro Lys
                85                  90                  95

Tyr Leu Thr Asp Ile Tyr Val Lys Asn Leu Pro Lys Ser Lys Phe Tyr
            100                 105                 110

Glu Asn Phe Arg Asn His Gly Phe Gln Asp Asn Ile Phe Ala Ser Leu
        115                 120                 125

Glu Asn Asp Arg His Ile Lys Tyr Lys Arg Met Ile Asn Asn Leu Tyr
    130                 135                 140

Ser Lys Ser Ser Ile Phe Ser Lys Glu Asn His Thr Arg Ser Val Leu
145                 150                 155                 160

Phe Asp Thr Thr Lys Thr Leu Val Asp Ala Val Ala Arg Glu Ser Pro
                165                 170                 175

Ser Ile Asp Val Phe Thr Leu Phe Gly Ser Leu Ala Met Asp Val Val
            180                 185                 190

Ser Arg Phe Glu Leu Gly Arg Asp Asn Gly Thr Asp Leu Leu Asn His
        195                 200                 205

Pro Gln Glu Arg His Ile Ile Glu Ser His Arg Lys Val Ser Cys Met
    210                 215                 220

Gly Phe Trp Thr Thr Leu Met Pro Ser Phe Phe Trp Asn Leu Ala Ala
225                 230                 235                 240

Thr Lys Ala Thr Leu Gln Ala Val Asp Asp Ile Cys Asn Phe Gln Leu
                245                 250                 255

Gly Leu Tyr Lys Ile Ala Glu Ser Asn Leu Val Ser Asn Gly Lys Asn
            260                 265                 270

Leu Thr Thr Ile Gln Thr Leu Lys Lys Tyr Gly Leu Glu Gly Asn Ser
        275                 280                 285

Ala Tyr Ser Phe Leu Thr Asp Asn Leu Phe Ala Gly His Glu Thr Thr
    290                 295                 300

Ala Val Gln Leu Thr Tyr Leu Cys Tyr Glu Leu Ser Arg Pro Ala Asn
305                 310                 315                 320

Tyr Lys Ile Gln Asn Arg Leu Arg Tyr Glu Leu Gln Glu Ala Phe Pro
                325                 330                 335

Ser Gly Gln Ile Glu Asp Leu Glu Val Val Asp Asn Leu Pro Tyr Leu
            340                 345                 350

Asn Ala Leu Leu Ser Glu Asn Gly Arg Ile His Thr Ser Ile Pro Gly
        355                 360                 365

Ala Glu Pro Arg Val Val Ala Lys Pro Tyr Thr Ile Gly Lys Leu Leu
    370                 375                 380

Ile Pro Val Gly Thr Val Ile Ser Cys Leu Pro Tyr Ala Tyr His Arg
385                 390                 395                 400

Asn Pro Ser Val Phe Thr Asn Pro Asp Lys Phe Ile Pro Glu Arg Trp
                405                 410                 415

Leu Val Asp Asn Glu Glu Asp Lys Lys Arg Val Lys Gln Gln Ala Lys
            420                 425                 430

Tyr Met Met Pro Phe Gly Lys Gly Val Arg Met Cys Leu Gly Met Asn
        435                 440                 445

Leu Ala Leu Ile Glu Met Lys Leu Ala Ile Ala Ser Leu Tyr Leu Asn
    450                 455                 460

Phe Ser Ser Ser Ile Asp Glu Asp Trp Cys Gly Lys Val Leu Glu Asn
465                 470                 475                 480

Asp Asp Pro Ile Gly Ile Gly Asn Ser Cys Thr His Glu Thr Asp Gln
                485                 490                 495

Asp Lys Met Lys Met Tyr Asp Ala Tyr Thr Thr Arg Pro Met Leu Asp
            500                 505                 510

Glu Cys Tyr Leu Lys Trp Thr Arg Leu Thr Pro
        515                 520

<210>  45
<211>  1728
<212>  DNA
<213>  Candida tropicalis

<220>
<221>  CDS
<222>  (1)..(1728)

<400>  45
atg ctt ctg agc ata cca tgg gat caa tca ttg cta acg ttg cta aca       48
Met Leu Leu Ser Ile Pro Trp Asp Gln Ser Leu Leu Thr Leu Leu Thr
1               5                   10                  15

tat ctt gat acc cat cca ata gct acg ata ttc acc att att tta acc       96
Tyr Leu Asp Thr His Pro Ile Ala Thr Ile Phe Thr Ile Ile Leu Thr
            20                  25                  30

att tta aca att gga att tta ttt gat tat tgt cta tca cct aaa gag      144
Ile Leu Thr Ile Gly Ile Leu Phe Asp Tyr Cys Leu Ser Pro Lys Glu
        35                  40                  45

att gcc aat att ttc agt att cct gga gat ttg cca ttt att ggt cat      192
Ile Ala Asn Ile Phe Ser Ile Pro Gly Asp Leu Pro Phe Ile Gly His
    50                  55                  60

tta cat tta ata cta gat aat cca gca ttg att tat tta aca tgg tat      240
Leu His Leu Ile Leu Asp Asn Pro Ala Leu Ile Tyr Leu Thr Trp Tyr
65                  70                  75                  80

aaa tta tat aat aaa ctg gtt ttt caa att cgt att gga aat aaa cgt      288
Lys Leu Tyr Asn Lys Leu Val Phe Gln Ile Arg Ile Gly Asn Lys Arg
                85                  90                  95

gta gtt gtt gtt aat tca ttt gat gat gtt gtt gga tta tgg ata aat      336
Val Val Val Val Asn Ser Phe Asp Asp Val Val Gly Leu Trp Ile Asn
            100                 105                 110

cat agt tgt caa aat aat tca aga cct tta agt tat act ttt cat gga      384
His Ser Cys Gln Asn Asn Ser Arg Pro Leu Ser Tyr Thr Phe His Gly
        115                 120                 125

tta gtt tca gca tta caa ggt ttt aca gtt ggt tct act cct gca agt      432
Leu Val Ser Ala Leu Gln Gly Phe Thr Val Gly Ser Thr Pro Ala Ser
    130                 135                 140

tta aca ttt ctg aga aag aag aaa gtt att tca ctg tgt tta aga aag      480
Leu Thr Phe Leu Arg Lys Lys Lys Val Ile Ser Leu Cys Leu Arg Lys
145                 150                 155                 160

aaa gaa att gat gaa aaa gta tgt ctc ata gat aat gaa att tgt gtt      528
Lys Glu Ile Asp Glu Lys Val Cys Leu Ile Asp Asn Glu Ile Cys Val
                165                 170                 175

atg att aaa gaa ata att aaa aag aag gat ata tca act gat gta aat      576
Met Ile Lys Glu Ile Ile Lys Lys Lys Asp Ile Ser Thr Asp Val Asn
        180                 185                 190

atg tta cct tat tta caa aaa ttc ata tta aaa aca gct att ctt atg      624
Met Leu Pro Tyr Leu Gln Lys Phe Ile Leu Lys Thr Ala Ile Leu Met
        195                 200                 205

agt tat gga att gaa ttg gat tgt tat aat aaa gat gtt aaa tta tgt      672
Ser Tyr Gly Ile Glu Leu Asp Cys Tyr Asn Lys Asp Val Lys Leu Cys
    210                 215                 220

caa gag att atc acc gtg gag aat aat atc ata aga tta aga tca cca      720
Gln Glu Ile Ile Thr Val Glu Asn Asn Ile Ile Arg Leu Arg Ser Pro
225                 230                 235                 240

ata tct aat ctt caa gat tct gta cct ttc tta aga cta ata cca tgg      768
Ile Ser Asn Leu Gln Asp Ser Val Pro Phe Leu Arg Leu Ile Pro Trp
                245                 250                 255

ttt aat aat cga gaa ttt gcc ctt cgt tgt gga aat aga agg aat aaa      816
Phe Asn Asn Arg Glu Phe Ala Leu Arg Cys Gly Asn Arg Arg Asn Lys
            260                 265                 270

tat atg gat caa tta tat aat agg tta caa aat gga tta gct gaa aat      864
Tyr Met Asp Gln Leu Tyr Asn Arg Leu Gln Asn Gly Leu Ala Glu Asn
        275                 280                 285

gat cca aat ata gct aat agt att ctt gga caa tta att ctt aat aat      912
Asp Pro Asn Ile Ala Asn Ser Ile Leu Gly Gln Leu Ile Leu Asn Asn
    290                 295                 300

gat aat aat aat tct aac agc tta act agt caa gaa ata caa agt att      960
Asp Asn Asn Asn Ser Asn Ser Leu Thr Ser Gln Glu Ile Gln Ser Ile
305                 310                 315                 320

tgt tta aca tta gta agt gct gga tta gat aat acc cca ctt aat ttg      1008
Cys Leu Thr Leu Val Ser Ala Gly Leu Asp Asn Thr Pro Leu Asn Leu
                325                 330                 335

aat tat ctt att gga ata tta tca caa cca aga ata ggt aag ata ttt      1056
Asn Tyr Leu Ile Gly Ile Leu Ser Gln Pro Arg Ile Gly Lys Ile Phe
            340                 345                 350

caa gat aaa gct ata aaa gat att tta aat cat gca aat gga gat att      1104
Gln Asp Lys Ala Ile Lys Asp Ile Leu Asn His Ala Asn Gly Asp Ile
        355                 360                 365

att caa gca tgg aat caa ctg aat gaa gaa aat cga gat tgt aaa tat      1152
Ile Gln Ala Trp Asn Gln Leu Asn Glu Glu Asn Arg Asp Cys Lys Tyr
    370                 375                 380

att caa gct tta att ctt gaa act tta aga cat ttt aca gta tta cca      1200
Ile Gln Ala Leu Ile Leu Glu Thr Leu Arg His Phe Thr Val Leu Pro
385                 390                 395                 400

tta agt tta cct aga tta act aca aaa cca ata tat tat aaa aat ttt      1248
Leu Ser Leu Pro Arg Leu Thr Thr Lys Pro Ile Tyr Tyr Lys Asn Phe
                405                 410                 415

atg att cct aaa aat act cat atg ttt atg aat gca tat tct gca aat      1296
Met Ile Pro Lys Asn Thr His Met Phe Met Asn Ala Tyr Ser Ala Asn
            420                 425                 430

cat gat gaa tta ata ttc aaa aat cca ttt aaa ttt gat cca gaa aga      1344
His Asp Glu Leu Ile Phe Lys Asn Pro Phe Lys Phe Asp Pro Glu Arg
        435                 440                 445

tgg tta gat tca gaa act aat gaa att aaa tca aaa ata ctt gct act      1392
Trp Leu Asp Ser Glu Thr Asn Glu Ile Lys Ser Lys Ile Leu Ala Thr
    450                 455                 460

act tct tcg tct tct tct tcg aca cat cat ggt ggt gga aat gga ata      1440
Thr Ser Ser Ser Ser Ser Ser Thr His His Gly Gly Gly Asn Gly Ile
465                 470                 475                 480

aat gta cag aat ttt cat ttt gca ttt ggt gct gga tca aga atg tgt      1488
Asn Val Gln Asn Phe His Phe Ala Phe Gly Ala Gly Ser Arg Met Cys
                485                 490                 495

tca ggt tat aat cta gtt atg aaa gaa atg tat atg atg ata att aaa      1536
Ser Gly Tyr Asn Leu Val Met Lys Glu Met Tyr Met Met Ile Ile Lys
            500                 505                 510

tta tta tta tta ttt gaa att aat cct cct gat aat aat aat aat aat      1584
Leu Leu Leu Leu Phe Glu Ile Asn Pro Pro Asp Asn Asn Asn Asn Asn
        515                 520                 525

ggg aaa tat tta atg gaa atg aat cct ttt gtt aat aat ctg aat cct      1632
Gly Lys Tyr Leu Met Glu Met Asn Pro Phe Val Asn Asn Leu Asn Pro
    530                 535                 540

aga ggt act tca ttt gaa cca cga ata cat aat att aaa tta caa tat      1680
Arg Gly Thr Ser Phe Glu Pro Arg Ile His Asn Ile Lys Leu Gln Tyr
545                 550                 555                 560

aga aaa cta cct aat tat gaa act cta cac gaa ata gtt ctc aaa tag      1728
Arg Lys Leu Pro Asn Tyr Glu Thr Leu His Glu Ile Val Leu Lys
                565                 570                 575


<210>  46
<211>  575
<212>  PRT
<213>  Candida tropicalis

<400>  46

Met Leu Leu Ser Ile Pro Trp Asp Gln Ser Leu Leu Thr Leu Leu Thr
1               5                   10                  15


Tyr Leu Asp Thr His Pro Ile Ala Thr Ile Phe Thr Ile Ile Leu Thr
            20                  25                  30


Ile Leu Thr Ile Gly Ile Leu Phe Asp Tyr Cys Leu Ser Pro Lys Glu
        35                  40                  45


Ile Ala Asn Ile Phe Ser Ile Pro Gly Asp Leu Pro Phe Ile Gly His
    50                  55                  60


Leu His Leu Ile Leu Asp Asn Pro Ala Leu Ile Tyr Leu Thr Trp Tyr
65                  70                  75                  80


Lys Leu Tyr Asn Lys Leu Val Phe Gln Ile Arg Ile Gly Asn Lys Arg

85                  90                  95

Val Val Val Val Asn Ser Phe Asp Asp Val Val Gly Leu Trp Ile Asn
            100                 105                 110

His Ser Cys Gln Asn Asn Ser Arg Pro Leu Ser Tyr Thr Phe His Gly
        115                 120                 125

Leu Val Ser Ala Leu Gln Gly Phe Thr Val Gly Ser Thr Pro Ala Ser
    130                 135                 140

Leu Thr Phe Leu Arg Lys Lys Lys Val Ile Ser Leu Cys Leu Arg Lys
145                 150                 155                 160

Lys Glu Ile Asp Glu Lys Val Cys Leu Ile Asp Asn Glu Ile Cys Val
                165                 170                 175

Met Ile Lys Glu Ile Ile Lys Lys Lys Asp Ile Ser Thr Asp Val Asn
            180                 185                 190

Met Leu Pro Tyr Leu Gln Lys Phe Ile Leu Lys Thr Ala Ile Leu Met
        195                 200                 205

Ser Tyr Gly Ile Glu Leu Asp Cys Tyr Asn Lys Asp Val Lys Leu Cys
    210                 215                 220

Gln Glu Ile Ile Thr Val Glu Asn Asn Ile Ile Arg Leu Arg Ser Pro
225                 230                 235                 240

Ile Ser Asn Leu Gln Asp Ser Val Pro Phe Leu Arg Leu Ile Pro Trp
                245                 250                 255

Phe Asn Asn Arg Glu Phe Ala Leu Arg Cys Gly Asn Arg Arg Asn Lys
            260                 265                 270

Tyr Met Asp Gln Leu Tyr Asn Arg Leu Gln Asn Gly Leu Ala Glu Asn
        275                 280                 285

Asp Pro Asn Ile Ala Asn Ser Ile Leu Gly Gln Leu Ile Leu Asn Asn
    290                 295                 300

Asp Asn Asn Asn Ser Asn Ser Leu Thr Ser Gln Glu Ile Gln Ser Ile
305                 310                 315                 320

Cys Leu Thr Leu Val Ser Ala Gly Leu Asp Asn Thr Pro Leu Asn Leu
                325                 330                 335

Asn Tyr Leu Ile Gly Ile Leu Ser Gln Pro Arg Ile Gly Lys Ile Phe

340                 345                 350

Gln Asp Lys Ala Ile Lys Asp Ile Leu Asn His Ala Asn Gly Asp Ile
        355                 360                 365

Ile Gln Ala Trp Asn Gln Leu Asn Glu Glu Asn Arg Asp Cys Lys Tyr
    370                 375                 380

Ile Gln Ala Leu Ile Leu Glu Thr Leu Arg His Phe Thr Val Leu Pro
385                 390                 395                 400

Leu Ser Leu Pro Arg Leu Thr Thr Lys Pro Ile Tyr Tyr Lys Asn Phe
                405                 410                 415

Met Ile Pro Lys Asn Thr His Met Phe Met Asn Ala Tyr Ser Ala Asn
            420                 425                 430

His Asp Glu Leu Ile Phe Lys Asn Pro Phe Lys Phe Asp Pro Glu Arg
        435                 440                 445

Trp Leu Asp Ser Glu Thr Asn Glu Ile Lys Ser Lys Ile Leu Ala Thr
    450                 455                 460

Thr Ser Ser Ser Ser Ser Ser Thr His His Gly Gly Gly Asn Gly Ile
465                 470                 475                 480

Asn Val Gln Asn Phe His Phe Ala Phe Gly Ala Gly Ser Arg Met Cys
                485                 490                 495

Ser Gly Tyr Asn Leu Val Met Lys Glu Met Tyr Met Met Ile Ile Lys
            500                 505                 510

Leu Leu Leu Leu Phe Glu Ile Asn Pro Pro Asp Asn Asn Asn Asn Asn
        515                 520                 525

Gly Lys Tyr Leu Met Glu Met Asn Pro Phe Val Asn Asn Leu Asn Pro
    530                 535                 540

Arg Gly Thr Ser Phe Glu Pro Arg Ile His Asn Ile Lys Leu Gln Tyr
545                 550                 555                 560

Arg Lys Leu Pro Asn Tyr Glu Thr Leu His Glu Ile Val Leu Lys
                565                 570                 575

<210>  47
<211>  1518
<212>  DNA
<213>  Candida tropicalis

<220>

<221>  CDS
<222>  (1)..(1518)

<400>  47
atg tat caa tta ttt tgt ttt ctt gct ggt ata att gtt gta tat aaa      48
Met Tyr Gln Leu Phe Cys Phe Leu Ala Gly Ile Ile Val Val Tyr Lys
1               5                   10                  15

gca gca caa tac tac aag aga agg aca ctt gtg act aaa ttt cat tgc      96
Ala Ala Gln Tyr Tyr Lys Arg Arg Thr Leu Val Thr Lys Phe His Cys
            20                  25                  30

aaa caa gca cgt att tcc cca aac aag tca tgg ttg gaa tat tta ggc      144
Lys Gln Ala Arg Ile Ser Pro Asn Lys Ser Trp Leu Glu Tyr Leu Gly
        35                  40                  45

att gcc tcc gtt gta cat gcc aat gaa atg att aga aaa gga gga ttg      192
Ile Ala Ser Val Val His Ala Asn Glu Met Ile Arg Lys Gly Gly Leu
    50                  55                  60

tat tca gaa att gat gga aga ttt aaa tcc ctt gat gtt tca aca ttc      240
Tyr Ser Glu Ile Asp Gly Arg Phe Lys Ser Leu Asp Val Ser Thr Phe
65                  70                  75                  80

aag tct ata act ttg gga aag aca acg tat gtt acc aaa gat att gaa      288
Lys Ser Ile Thr Leu Gly Lys Thr Thr Tyr Val Thr Lys Asp Ile Glu
                85                  90                  95

aac atc cgt cat atc ttg agt gca acg gaa atg aac tca tgg aat ctt      336
Asn Ile Arg His Ile Leu Ser Ala Thr Glu Met Asn Ser Trp Asn Leu
            100                 105                 110

ggt gcc cgt cca att gcg tta aga ccc ttg att ggt gat gga att ttt      384
Gly Ala Arg Pro Ile Ala Leu Arg Pro Leu Ile Gly Asp Gly Ile Phe
        115                 120                 125

gct agt gaa ggt caa tct tgg aaa cat agt cga atc atg ctt aga cca      432
Ala Ser Glu Gly Gln Ser Trp Lys His Ser Arg Ile Met Leu Arg Pro
    130                 135                 140

gta ttt gca aaa gaa cac gtt aaa caa atc act tca atg gaa cca tat      480
Val Phe Ala Lys Glu His Val Lys Gln Ile Thr Ser Met Glu Pro Tyr
145                 150                 155                 160

gta caa ctg ttg atc aaa atc atc aag aac cat gaa ggg gaa cca tta      528
Val Gln Leu Leu Ile Lys Ile Ile Lys Asn His Glu Gly Glu Pro Leu
                165                 170                 175

gag ttt caa acc tta gcc cat ctt ttt aca ata gat tat tct act gat      576
Glu Phe Gln Thr Leu Ala His Leu Phe Thr Ile Asp Tyr Ser Thr Asp
            180                 185                 190

ttc cta tta ggt gaa agt tgt gat agt ttg aag gat ttc cta gga gaa      624
Phe Leu Leu Gly Glu Ser Cys Asp Ser Leu Lys Asp Phe Leu Gly Glu
        195                 200                 205

gag tcc aat tcc aca tta gat aca tcg ttg aga ctg gca ttt gca tca      672
Glu Ser Asn Ser Thr Leu Asp Thr Ser Leu Arg Leu Ala Phe Ala Ser
    210                 215                 220

cag ttt aat aaa acc cag cag caa atg aca att cga ttc atg ttg gga      720
Gln Phe Asn Lys Thr Gln Gln Gln Met Thr Ile Arg Phe Met Leu Gly
225                 230                 235                 240

aaa ttg gcc ttt ctc atg tat cca aag agt ttt caa aat agt att caa      768

Lys Leu Ala Phe Leu Met Tyr Pro Lys Ser Phe Gln Asn Ser Ile Gln
                245                 250                 255

atg caa aag gac ttt gtt gat gaa tat atc gac aga gta gta ggt atg      816
Met Gln Lys Asp Phe Val Asp Glu Tyr Ile Asp Arg Val Val Gly Met
            260                 265                 270

tcc gaa gaa gaa ttg aac aat cat cca aag agc tat gtt ttg ttg tac      864
Ser Glu Glu Glu Leu Asn Asn His Pro Lys Ser Tyr Val Leu Leu Tyr
        275                 280                 285

caa tta gca aga caa act aag aat cgt gat ata tta caa gat gaa ttg      912
Gln Leu Ala Arg Gln Thr Lys Asn Arg Asp Ile Leu Gln Asp Glu Leu
    290                 295                 300

atg tcc att tta ctt gca ggt aga gac acc act gcc agt ttg ttg act      960
Met Ser Ile Leu Leu Ala Gly Arg Asp Thr Thr Ala Ser Leu Leu Thr
305                 310                 315                 320

ttt ttg ttt ttc gaa tta agt cac cat cca gaa gta ttt aac aaa tta     1008
Phe Leu Phe Phe Glu Leu Ser His His Pro Glu Val Phe Asn Lys Leu
                325                 330                 335

aaa gag gaa atc gaa aga cac ttt cct gat gtt gaa tcc gtt aca ttt     1056
Lys Glu Glu Ile Glu Arg His Phe Pro Asp Val Glu Ser Val Thr Phe
            340                 345                 350

gga act atc cag aga tgc gac tat ctt caa tgg tgt att aac gaa act     1104
Gly Thr Ile Gln Arg Cys Asp Tyr Leu Gln Trp Cys Ile Asn Glu Thr
        355                 360                 365

atg aga ctc cat cca tca gtt cct ttt aat ttc aga act gca gcc aat     1152
Met Arg Leu His Pro Ser Val Pro Phe Asn Phe Arg Thr Ala Ala Asn
    370                 375                 380

gac aca gta ata cca aga ggt gga ggt aaa tcc tgt aca gat cct att     1200
Asp Thr Val Ile Pro Arg Gly Gly Gly Lys Ser Cys Thr Asp Pro Ile
385                 390                 395                 400

ctt gtc cat aag ggt gaa caa gta tta ttc agt ttc tat tct gta aac     1248
Leu Val His Lys Gly Glu Gln Val Leu Phe Ser Phe Tyr Ser Val Asn
                405                 410                 415

aga gaa gaa aag tat ttt ggt aca aat acc gac aag ttt gct cca gaa     1296
Arg Glu Glu Lys Tyr Phe Gly Thr Asn Thr Asp Lys Phe Ala Pro Glu
            420                 425                 430

aga tgg agt gaa tca tta agg aga act gag ttc ata cca ttt tct gct     1344
Arg Trp Ser Glu Ser Leu Arg Arg Thr Glu Phe Ile Pro Phe Ser Ala
        435                 440                 445

gga cct cgt gcc tgt ttg ggt caa cag tta gct aga gtt gaa gct tca     1392
Gly Pro Arg Ala Cys Leu Gly Gln Gln Leu Ala Arg Val Glu Ala Ser
    450                 455                 460

tat gtt act att aga ttg ctt caa acc ttt cat ggg ttg cat aat gcc     1440
Tyr Val Thr Ile Arg Leu Leu Gln Thr Phe His Gly Leu His Asn Ala
465                 470                 475                 480

agt aaa caa tac cca cca aat aga gtg gtt gca gct aca atg aga ttg     1488
Ser Lys Gln Tyr Pro Pro Asn Arg Val Val Ala Ala Thr Met Arg Leu
                485                 490                 495

act gac ggt tgt aac gtt tgt ttt atc tag                             1518

Thr Asp Gly Cys Asn Val Cys Phe Ile
            500                 505

<210>  48
<211>  505
<212>  PRT
<213>  Candida tropicalis

<400>  48

Met Tyr Gln Leu Phe Cys Phe Leu Ala Gly Ile Ile Val Val Tyr Lys
1               5                   10                  15

Ala Ala Gln Tyr Tyr Lys Arg Arg Thr Leu Val Thr Lys Phe His Cys
            20                  25                  30

Lys Gln Ala Arg Ile Ser Pro Asn Lys Ser Trp Leu Glu Tyr Leu Gly
        35                  40                  45

Ile Ala Ser Val Val His Ala Asn Glu Met Ile Arg Lys Gly Gly Leu
    50                  55                  60

Tyr Ser Glu Ile Asp Gly Arg Phe Lys Ser Leu Asp Val Ser Thr Phe
65                  70                  75                  80

Lys Ser Ile Thr Leu Gly Lys Thr Thr Tyr Val Thr Lys Asp Ile Glu
                85                  90                  95

Asn Ile Arg His Ile Leu Ser Ala Thr Glu Met Asn Ser Trp Asn Leu
            100                 105                 110

Gly Ala Arg Pro Ile Ala Leu Arg Pro Leu Ile Gly Asp Gly Ile Phe
        115                 120                 125

Ala Ser Glu Gly Gln Ser Trp Lys His Ser Arg Ile Met Leu Arg Pro
    130                 135                 140

Val Phe Ala Lys Glu His Val Lys Gln Ile Thr Ser Met Glu Pro Tyr
145                 150                 155                 160

Val Gln Leu Leu Ile Lys Ile Ile Lys Asn His Glu Gly Glu Pro Leu
                165                 170                 175

Glu Phe Gln Thr Leu Ala His Leu Phe Thr Ile Asp Tyr Ser Thr Asp
            180                 185                 190

Phe Leu Leu Gly Glu Ser Cys Asp Ser Leu Lys Asp Phe Leu Gly Glu
        195                 200                 205

Glu Ser Asn Ser Thr Leu Asp Thr Ser Leu Arg Leu Ala Phe Ala Ser

210                 215                 220

Gln Phe Asn Lys Thr Gln Gln Gln Met Thr Ile Arg Phe Met Leu Gly
225                 230                 235                 240

Lys Leu Ala Phe Leu Met Tyr Pro Lys Ser Phe Gln Asn Ser Ile Gln
                245                 250                 255

Met Gln Lys Asp Phe Val Asp Glu Tyr Ile Asp Arg Val Val Gly Met
            260                 265                 270

Ser Glu Glu Glu Leu Asn Asn His Pro Lys Ser Tyr Val Leu Leu Tyr
        275                 280                 285

Gln Leu Ala Arg Gln Thr Lys Asn Arg Asp Ile Leu Gln Asp Glu Leu
    290                 295                 300

Met Ser Ile Leu Leu Ala Gly Arg Asp Thr Thr Ala Ser Leu Leu Thr
305                 310                 315                 320

Phe Leu Phe Phe Glu Leu Ser His His Pro Glu Val Phe Asn Lys Leu
                325                 330                 335

Lys Glu Glu Ile Glu Arg His Phe Pro Asp Val Glu Ser Val Thr Phe
            340                 345                 350

Gly Thr Ile Gln Arg Cys Asp Tyr Leu Gln Trp Cys Ile Asn Glu Thr
        355                 360                 365

Met Arg Leu His Pro Ser Val Pro Phe Asn Phe Arg Thr Ala Ala Asn
    370                 375                 380

Asp Thr Val Ile Pro Arg Gly Gly Gly Lys Ser Cys Thr Asp Pro Ile
385                 390                 395                 400

Leu Val His Lys Gly Glu Gln Val Leu Phe Ser Phe Tyr Ser Val Asn
                405                 410                 415

Arg Glu Glu Lys Tyr Phe Gly Thr Asn Thr Asp Lys Phe Ala Pro Glu
            420                 425                 430

Arg Trp Ser Glu Ser Leu Arg Arg Thr Glu Phe Ile Pro Phe Ser Ala
        435                 440                 445

Gly Pro Arg Ala Cys Leu Gly Gln Gln Leu Ala Arg Val Glu Ala Ser
    450                 455                 460

Tyr Val Thr Ile Arg Leu Leu Gln Thr Phe His Gly Leu His Asn Ala

465                 470                 475                 480

Ser Lys Gln Tyr Pro Pro Asn Arg Val Val Ala Ala Thr Met Arg Leu
                485                 490                 495

Thr Asp Gly Cys Asn Val Cys Phe Ile
            500                 505

<210>  49
<211>  1584
<212>  DNA
<213>  Candida tropicalis

<220>
<221>  CDS
<222>  (1)..(1584)

<400>  49
atg agt gtc aat act act tct cca gtt gtg gat agt att agt gat aat       48
Met Ser Val Asn Thr Thr Ser Pro Val Val Asp Ser Ile Ser Asp Asn
1               5                   10                  15

gct act aca tat ttg agt aca aaa tat atc atc gat agt tta tat tca       96
Ala Thr Thr Tyr Leu Ser Thr Lys Tyr Ile Ile Asp Ser Leu Tyr Ser
            20                  25                  30

act tat caa caa gct tca tgg tta caa atc att tta acc tca atc att      144
Thr Tyr Gln Gln Ala Ser Trp Leu Gln Ile Ile Leu Thr Ser Ile Ile
        35                  40                  45

ctc att ttg act tat gat caa atc ctg tat caa atc aat aaa ggt tca      192
Leu Ile Leu Thr Tyr Asp Gln Ile Leu Tyr Gln Ile Asn Lys Gly Ser
    50                  55                  60

att gct ggt cca aaa ttc aaa ttt tgg cca att att ggt cca ttc ttg      240
Ile Ala Gly Pro Lys Phe Lys Phe Trp Pro Ile Ile Gly Pro Phe Leu
65                  70                  75                  80

gaa tct tta gat cca aaa ttc gaa gaa tat aaa gct aaa tgg gat tct      288
Glu Ser Leu Asp Pro Lys Phe Glu Glu Tyr Lys Ala Lys Trp Asp Ser
                85                  90                  95

ggt gaa ttg agt tgt gtt tcc att ttc cat aaa ttc gtt gtt att gct      336
Gly Glu Leu Ser Cys Val Ser Ile Phe His Lys Phe Val Val Ile Ala
            100                 105                 110

tct tct cgt gat tta gct aga aag att tta gca tct cca aaa tac gtt      384
Ser Ser Arg Asp Leu Ala Arg Lys Ile Leu Ala Ser Pro Lys Tyr Val
        115                 120                 125

aaa cct tgt gtt gtt gat gtt gct gtt aaa att tta aga cct tca aat      432
Lys Pro Cys Val Val Asp Val Ala Val Lys Ile Leu Arg Pro Ser Asn
    130                 135                 140

tgg gtt ttc tta gac ggt aaa gca cat act gat tac cgt cgt tct ttg      480
Trp Val Phe Leu Asp Gly Lys Ala His Thr Asp Tyr Arg Arg Ser Leu
145                 150                 155                 160

aat ggt tta ttc tcc caa aga gct ttg gaa att tac att cca gtt caa      528
Asn Gly Leu Phe Ser Gln Arg Ala Leu Glu Ile Tyr Ile Pro Val Gln

                165                 170                 175

gaa aaa tac atg gat att tat tta gat aga ttc tgt aag tac gac ggt 576
Glu Lys Tyr Met Asp Ile Tyr Leu Asp Arg Phe Cys Lys Tyr Asp Gly
            180                 185                 190

cca cgt gaa ttc ttc cca gaa ttt aga gaa ttg ttg tgt gct tta tct 624
Pro Arg Glu Phe Phe Pro Glu Phe Arg Glu Leu Leu Cys Ala Leu Ser
        195                 200                 205

ttg aga act ttc tgt ggg gat tac atc act gaa gat caa att gct tta 672
Leu Arg Thr Phe Cys Gly Asp Tyr Ile Thr Glu Asp Gln Ile Ala Leu
    210                 215                 220

gtt gct gat aac tat tac aga gtc act gct gct ttg gaa ttg gtc aat 720
Val Ala Asp Asn Tyr Tyr Arg Val Thr Ala Ala Leu Glu Leu Val Asn
225                 230                 235                 240

ttc cca atc att att cct tac act aaa act tgg tac ggt aag aag att 768
Phe Pro Ile Ile Ile Pro Tyr Thr Lys Thr Trp Tyr Gly Lys Lys Ile
                245                 250                 255

gct gat gat acc atg aag att ttt gaa aat tgt gct gct atg tcc aag 816
Ala Asp Asp Thr Met Lys Ile Phe Glu Asn Cys Ala Ala Met Ser Lys
            260                 265                 270

aaa cac att aat gaa aat aat ggt act cca ggt tgt gtt atg gat gaa 864
Lys His Ile Asn Glu Asn Asn Gly Thr Pro Gly Cys Val Met Asp Glu
        275                 280                 285

tgg att tac ttg atg aaa gaa gct aaa gaa aaa cac tct gat gat cca 912
Trp Ile Tyr Leu Met Lys Glu Ala Lys Glu Lys His Ser Asp Asp Pro
    290                 295                 300

gat tcc aaa tta ttg att aga gaa ttc tcc aac cgt gaa att tcc gaa 960
Asp Ser Lys Leu Leu Ile Arg Glu Phe Ser Asn Arg Glu Ile Ser Glu
305                 310                 315                 320

gcc att ttc act ttc ttg ttt gct tct caa gat gcc tct tct tct ttg 1008
Ala Ile Phe Thr Phe Leu Phe Ala Ser Gln Asp Ala Ser Ser Ser Leu
                325                 330                 335

gct tgt tgg tta ttc caa att gtc gcc gac aga cca gat gtt gtt gcc 1056
Ala Cys Trp Leu Phe Gln Ile Val Ala Asp Arg Pro Asp Val Val Ala
            340                 345                 350

aag att aga gaa gaa caa ttg aga gtt aga aac aat gac cca tct gtt 1104
Lys Ile Arg Glu Glu Gln Leu Arg Val Arg Asn Asn Asp Pro Ser Val
        355                 360                 365

aaa ttg tcc ttg gat ttg att aat gaa atg act tac acc aat gat gtt 1152
Lys Leu Ser Leu Asp Leu Ile Asn Glu Met Thr Tyr Thr Asn Asp Val
    370                 375                 380

gtt aaa gaa tct ttg aga tac cgt cca cca gtc ttg atg gtt cca tat 1200
Val Lys Glu Ser Leu Arg Tyr Arg Pro Pro Val Leu Met Val Pro Tyr
385                 390                 395                 400

gtt gtt aag aaa gct ttc cca gtt act gaa aaa tac act gct cca aag 1248
Val Val Lys Lys Ala Phe Pro Val Thr Glu Lys Tyr Thr Ala Pro Lys
                405                 410                 415

ggt tct atg ctt atc cca act ttg tac cct gct tta cat gat cct gaa 1296
Gly Ser Met Leu Ile Pro Thr Leu Tyr Pro Ala Leu His Asp Pro Glu

420                 425                 430

gtt tat gat gaa cca gat tct ttc att cca gaa aga tgg gcc act gct      1344
Val Tyr Asp Glu Pro Asp Ser Phe Ile Pro Glu Arg Trp Ala Thr Ala
        435                 440                 445

tct ggt gat atg tac aaa cgt aac tgg ttg gtc ttc ggt act ggt cca      1392
Ser Gly Asp Met Tyr Lys Arg Asn Trp Leu Val Phe Gly Thr Gly Pro
    450                 455                 460

cac gtt tgt ttg ggt aag aac tat gtc atg atg ttg ttt act ggt atg      1440
His Val Cys Leu Gly Lys Asn Tyr Val Met Met Leu Phe Thr Gly Met
465                 470                 475                 480

ttg ggt aaa ttt gtc atg aac tct gat atc att cat cac aaa act gca      1488
Leu Gly Lys Phe Val Met Asn Ser Asp Ile Ile His His Lys Thr Ala
                485                 490                 495

tta tct gaa gaa atc aaa gtt ttc gct act att ttc cct aag gac gat      1536
Leu Ser Glu Glu Ile Lys Val Phe Ala Thr Ile Phe Pro Lys Asp Asp
            500                 505                 510

gtt att tta gaa tgg aaa aag aga gat ccg tta gct gct tct aat taa      1584
Val Ile Leu Glu Trp Lys Lys Arg Asp Pro Leu Ala Ala Ser Asn
        515                 520                 525


<210>  50
<211>  527
<212>  PRT
<213>  Candida tropicalis

<400>  50

Met Ser Val Asn Thr Thr Ser Pro Val Val Asp Ser Ile Ser Asp Asn
1               5                   10                  15


Ala Thr Thr Tyr Leu Ser Thr Lys Tyr Ile Ile Asp Ser Leu Tyr Ser
            20                  25                  30


Thr Tyr Gln Gln Ala Ser Trp Leu Gln Ile Ile Leu Thr Ser Ile Ile
        35                  40                  45


Leu Ile Leu Thr Tyr Asp Gln Ile Leu Tyr Gln Ile Asn Lys Gly Ser
    50                  55                  60


Ile Ala Gly Pro Lys Phe Lys Phe Trp Pro Ile Ile Gly Pro Phe Leu
65                  70                  75                  80


Glu Ser Leu Asp Pro Lys Phe Glu Glu Tyr Lys Ala Lys Trp Asp Ser
                85                  90                  95


Gly Glu Leu Ser Cys Val Ser Ile Phe His Lys Phe Val Val Ile Ala
            100                 105                 110


Ser Ser Arg Asp Leu Ala Arg Lys Ile Leu Ala Ser Pro Lys Tyr Val
        115                 120                 125

Lys Pro Cys Val Val Asp Val Ala Val Lys Ile Leu Arg Pro Ser Asn
    130                 135                 140

Trp Val Phe Leu Asp Gly Lys Ala His Thr Asp Tyr Arg Arg Ser Leu
145                 150                 155                 160

Asn Gly Leu Phe Ser Gln Arg Ala Leu Glu Ile Tyr Ile Pro Val Gln
                165                 170                 175

Glu Lys Tyr Met Asp Ile Tyr Leu Asp Arg Phe Cys Lys Tyr Asp Gly
            180                 185                 190

Pro Arg Glu Phe Phe Pro Glu Phe Arg Glu Leu Leu Cys Ala Leu Ser
        195                 200                 205

Leu Arg Thr Phe Cys Gly Asp Tyr Ile Thr Glu Asp Gln Ile Ala Leu
    210                 215                 220

Val Ala Asp Asn Tyr Tyr Arg Val Thr Ala Ala Leu Glu Leu Val Asn
225                 230                 235                 240

Phe Pro Ile Ile Ile Pro Tyr Thr Lys Thr Trp Tyr Gly Lys Lys Ile
                245                 250                 255

Ala Asp Asp Thr Met Lys Ile Phe Glu Asn Cys Ala Ala Met Ser Lys
            260                 265                 270

Lys His Ile Asn Glu Asn Asn Gly Thr Pro Gly Cys Val Met Asp Glu
        275                 280                 285

Trp Ile Tyr Leu Met Lys Glu Ala Lys Glu Lys His Ser Asp Asp Pro
    290                 295                 300

Asp Ser Lys Leu Leu Ile Arg Glu Phe Ser Asn Arg Glu Ile Ser Glu
305                 310                 315                 320

Ala Ile Phe Thr Phe Leu Phe Ala Ser Gln Asp Ala Ser Ser Ser Leu
                325                 330                 335

Ala Cys Trp Leu Phe Gln Ile Val Ala Asp Arg Pro Asp Val Val Ala
            340                 345                 350

Lys Ile Arg Glu Glu Gln Leu Arg Val Arg Asn Asn Asp Pro Ser Val
        355                 360                 365

Lys Leu Ser Leu Asp Leu Ile Asn Glu Met Thr Tyr Thr Asn Asp Val
    370                 375                 380

Val Lys Glu Ser Leu Arg Tyr Arg Pro Pro Val Leu Met Val Pro Tyr
385                 390                 395                 400

Val Val Lys Lys Ala Phe Pro Val Thr Glu Lys Tyr Thr Ala Pro Lys
                405                 410                 415

Gly Ser Met Leu Ile Pro Thr Leu Tyr Pro Ala Leu His Asp Pro Glu
            420                 425                 430

Val Tyr Asp Glu Pro Asp Ser Phe Ile Pro Glu Arg Trp Ala Thr Ala
        435                 440                 445

Ser Gly Asp Met Tyr Lys Arg Asn Trp Leu Val Phe Gly Thr Gly Pro
    450                 455                 460

His Val Cys Leu Gly Lys Asn Tyr Val Met Met Leu Phe Thr Gly Met
465                 470                 475                 480

Leu Gly Lys Phe Val Met Asn Ser Asp Ile Ile His His Lys Thr Ala
                485                 490                 495

Leu Ser Glu Glu Ile Lys Val Phe Ala Thr Ile Phe Pro Lys Asp Asp
            500                 505                 510

Val Ile Leu Glu Trp Lys Lys Arg Asp Pro Leu Ala Ala Ser Asn
        515                 520                 525

<210>  51
<211>  1587
<212>  DNA
<213>  Candida tropicalis

<220>
<221>  CDS
<222>  (1)..(1587)

<400>  51
atg gct att gtt gat act gcc att gat ggc atc aat tat ttc tta tcc       48
Met Ala Ile Val Asp Thr Ala Ile Asp Gly Ile Asn Tyr Phe Leu Ser
1               5                   10                  15

tta tca tta act caa caa atc acc atc ttg gtt gtt ttc cca ttc atc       96
Leu Ser Leu Thr Gln Gln Ile Thr Ile Leu Val Val Phe Pro Phe Ile
            20                  25                  30

tac aac ata gca tgg caa tta ctt tac tcc tta aga aaa gat aga gtt      144
Tyr Asn Ile Ala Trp Gln Leu Leu Tyr Ser Leu Arg Lys Asp Arg Val
        35                  40                  45

cca atg gtt ttc tac tgg atc cca tgg ttt ggt tct gct gct agt tat      192
Pro Met Val Phe Tyr Trp Ile Pro Trp Phe Gly Ser Ala Ala Ser Tyr
    50                  55                  60

ggt atg caa cca tac gaa ttc ttt gaa aag tgc aga ttg aaa tat ggt        240
Gly Met Gln Pro Tyr Glu Phe Phe Glu Lys Cys Arg Leu Lys Tyr Gly
65                  70                  75                  80

gat gtt ttt tca ttt atg tta tta ggt aaa gtt atg act gtt tat ttg        288
Asp Val Phe Ser Phe Met Leu Leu Gly Lys Val Met Thr Val Tyr Leu
                85                  90                  95

ggt cca aaa ggt cac gaa ttc att tac aat gct aaa tta tcc gat gtt        336
Gly Pro Lys Gly His Glu Phe Ile Tyr Asn Ala Lys Leu Ser Asp Val
            100                 105                 110

tct gct gaa gaa gct tat acc cat ttg act act cct gtt ttt ggt aaa        384
Ser Ala Glu Glu Ala Tyr Thr His Leu Thr Thr Pro Val Phe Gly Lys
        115                 120                 125

ggt gtt att tat gat tgt cca aac tct aga tta atg gaa caa aag aag        432
Gly Val Ile Tyr Asp Cys Pro Asn Ser Arg Leu Met Glu Gln Lys Lys
    130                 135                 140

ttt gct aaa ttt gct ttg act act gat tct ttc aaa acc tat gtt cca        480
Phe Ala Lys Phe Ala Leu Thr Thr Asp Ser Phe Lys Thr Tyr Val Pro
145                 150                 155                 160

aag atc aga gaa gaa gtt ttg aat tat ttt gtt aac gat gtt agt ttc        528
Lys Ile Arg Glu Glu Val Leu Asn Tyr Phe Val Asn Asp Val Ser Phe
                165                 170                 175

aaa acc aag gaa aga gac cat ggt gtt gct agt gtt atg aaa act caa        576
Lys Thr Lys Glu Arg Asp His Gly Val Ala Ser Val Met Lys Thr Gln
            180                 185                 190

cca gaa atc act att ttc act gct tct cgt tgt tta ttt ggt gat gaa        624
Pro Glu Ile Thr Ile Phe Thr Ala Ser Arg Cys Leu Phe Gly Asp Glu
        195                 200                 205

atg aga aag agt ttc gac aga tca ttt gct caa ttg tat gct gac ttg        672
Met Arg Lys Ser Phe Asp Arg Ser Phe Ala Gln Leu Tyr Ala Asp Leu
    210                 215                 220

gat aaa ggt ttc acc cca atc aac ttt gtt ttc cca aac ttg cca tta        720
Asp Lys Gly Phe Thr Pro Ile Asn Phe Val Phe Pro Asn Leu Pro Leu
225                 230                 235                 240

cct cat tac tgg aga cgt gac gct gct caa aga aag ata tct gct cat        768
Pro His Tyr Trp Arg Arg Asp Ala Ala Gln Arg Lys Ile Ser Ala His
                245                 250                 255

tac atg aag gaa att aag aga aga aga gaa agc ggt gat att gat cca        816
Tyr Met Lys Glu Ile Lys Arg Arg Arg Glu Ser Gly Asp Ile Asp Pro
            260                 265                 270

aag aga gat ttg att gat tcc ttg ttg gtt aac tct act tat aaa gat        864
Lys Arg Asp Leu Ile Asp Ser Leu Leu Val Asn Ser Thr Tyr Lys Asp
        275                 280                 285

ggt gtt aaa atg act gat caa gaa att gct aac ctt tta att ggt gtt        912
Gly Val Lys Met Thr Asp Gln Glu Ile Ala Asn Leu Leu Ile Gly Val
    290                 295                 300

ttg atg ggt ggt caa cat act tct gct tcc act tct gcc tgg ttc ttg        960
Leu Met Gly Gly Gln His Thr Ser Ala Ser Thr Ser Ala Trp Phe Leu
305                 310                 315                 320

ttg cat ttg gct gaa caa cca caa tta caa gat gat ctt tac gaa gaa     1008
Leu His Leu Ala Glu Gln Pro Gln Leu Gln Asp Asp Leu Tyr Glu Glu
                325                 330                 335

ttg acc aac ttg ttg aaa gaa aag ggt ggt gac ttg aac gat ttg act     1056
Leu Thr Asn Leu Leu Lys Glu Lys Gly Gly Asp Leu Asn Asp Leu Thr
            340                 345                 350

tac gaa gac ttg caa aaa tta cca ttg gtt aac aac act att aaa gaa     1104
Tyr Glu Asp Leu Gln Lys Leu Pro Leu Val Asn Asn Thr Ile Lys Glu
        355                 360                 365

act ttg aga atg cac atg cca ttg cat tct att ttc aga aaa gtt atg     1152
Thr Leu Arg Met His Met Pro Leu His Ser Ile Phe Arg Lys Val Met
    370                 375                 380

aac cca ttg aga gtc cca aat acc aaa tat gtt att cca aaa ggt cac     1200
Asn Pro Leu Arg Val Pro Asn Thr Lys Tyr Val Ile Pro Lys Gly His
385                 390                 395                 400

tat gtc tta gtt tct gcc ggt tat gct cat acc agt gat aga tgg ttt     1248
Tyr Val Leu Val Ser Ala Gly Tyr Ala His Thr Ser Asp Arg Trp Phe
                405                 410                 415

gaa cac cca gaa cat ttc aac cca aga aga tgg gaa tct gat gat acc     1296
Glu His Pro Glu His Phe Asn Pro Arg Arg Trp Glu Ser Asp Asp Thr
            420                 425                 430

aag gct agt gct gtt tct ttc aat tct gaa gat act gtt gat tat ggt     1344
Lys Ala Ser Ala Val Ser Phe Asn Ser Glu Asp Thr Val Asp Tyr Gly
        435                 440                 445

ttc ggt aaa att tcc aaa ggt gtc tcc tct cca tac ttg cca ttc ggt     1392
Phe Gly Lys Ile Ser Lys Gly Val Ser Ser Pro Tyr Leu Pro Phe Gly
    450                 455                 460

ggt ggt aga cac aga tgt att ggt gaa caa ttt gct tat gtt caa ttg     1440
Gly Gly Arg His Arg Cys Ile Gly Glu Gln Phe Ala Tyr Val Gln Leu
465                 470                 475                 480

gga act att ttg acc act tat atc tac aac ttc aaa tgg aga tta aac     1488
Gly Thr Ile Leu Thr Thr Tyr Ile Tyr Asn Phe Lys Trp Arg Leu Asn
                485                 490                 495

ggt gat aag gtt cca gat gtt gat tac caa tcc atg gtt acc tta cca     1536
Gly Asp Lys Val Pro Asp Val Asp Tyr Gln Ser Met Val Thr Leu Pro
            500                 505                 510

tta gaa cct gct gaa atc gtt tgg gaa aag aga gat act tgt atg gtt     1584
Leu Glu Pro Ala Glu Ile Val Trp Glu Lys Arg Asp Thr Cys Met Val
        515                 520                 525

tag                                                                 1587


<210>  52
<211>  528
<212>  PRT
<213>  Candida tropicalis

<400>  52

Met Ala Ile Val Asp Thr Ala Ile Asp Gly Ile Asn Tyr Phe Leu Ser

1               5                   10                  15

Leu Ser Leu Thr Gln Gln Ile Thr Ile Leu Val Val Phe Pro Phe Ile
            20                  25                  30

Tyr Asn Ile Ala Trp Gln Leu Leu Tyr Ser Leu Arg Lys Asp Arg Val
        35                  40                  45

Pro Met Val Phe Tyr Trp Ile Pro Trp Phe Gly Ser Ala Ala Ser Tyr
    50                  55                  60

Gly Met Gln Pro Tyr Glu Phe Phe Glu Lys Cys Arg Leu Lys Tyr Gly
65                  70                  75                  80

Asp Val Phe Ser Phe Met Leu Leu Gly Lys Val Met Thr Val Tyr Leu
                85                  90                  95

Gly Pro Lys Gly His Glu Phe Ile Tyr Asn Ala Lys Leu Ser Asp Val
            100                 105                 110

Ser Ala Glu Glu Ala Tyr Thr His Leu Thr Thr Pro Val Phe Gly Lys
        115                 120                 125

Gly Val Ile Tyr Asp Cys Pro Asn Ser Arg Leu Met Glu Gln Lys Lys
    130                 135                 140

Phe Ala Lys Phe Ala Leu Thr Thr Asp Ser Phe Lys Thr Tyr Val Pro
145                 150                 155                 160

Lys Ile Arg Glu Glu Val Leu Asn Tyr Phe Val Asn Asp Val Ser Phe
                165                 170                 175

Lys Thr Lys Glu Arg Asp His Gly Val Ala Ser Val Met Lys Thr Gln
            180                 185                 190

Pro Glu Ile Thr Ile Phe Thr Ala Ser Arg Cys Leu Phe Gly Asp Glu
        195                 200                 205

Met Arg Lys Ser Phe Asp Arg Ser Phe Ala Gln Leu Tyr Ala Asp Leu
    210                 215                 220

Asp Lys Gly Phe Thr Pro Ile Asn Phe Val Phe Pro Asn Leu Pro Leu
225                 230                 235                 240

Pro His Tyr Trp Arg Arg Asp Ala Ala Gln Arg Lys Ile Ser Ala His
                245                 250                 255

Tyr Met Lys Glu Ile Lys Arg Arg Arg Glu Ser Gly Asp Ile Asp Pro

            260                 265                 270

Lys Arg Asp Leu Ile Asp Ser Leu Leu Val Asn Ser Thr Tyr Lys Asp
        275                 280                 285

Gly Val Lys Met Thr Asp Gln Glu Ile Ala Asn Leu Leu Ile Gly Val
    290                 295                 300

Leu Met Gly Gly Gln His Thr Ser Ala Ser Thr Ser Ala Trp Phe Leu
305                 310                 315                 320

Leu His Leu Ala Glu Gln Pro Gln Leu Gln Asp Asp Leu Tyr Glu Glu
                325                 330                 335

Leu Thr Asn Leu Leu Lys Glu Lys Gly Gly Asp Leu Asn Asp Leu Thr
            340                 345                 350

Tyr Glu Asp Leu Gln Lys Leu Pro Leu Val Asn Asn Thr Ile Lys Glu
        355                 360                 365

Thr Leu Arg Met His Met Pro Leu His Ser Ile Phe Arg Lys Val Met
    370                 375                 380

Asn Pro Leu Arg Val Pro Asn Thr Lys Tyr Val Ile Pro Lys Gly His
385                 390                 395                 400

Tyr Val Leu Val Ser Ala Gly Tyr Ala His Thr Ser Asp Arg Trp Phe
                405                 410                 415

Glu His Pro Glu His Phe Asn Pro Arg Arg Trp Glu Ser Asp Asp Thr
            420                 425                 430

Lys Ala Ser Ala Val Ser Phe Asn Ser Glu Asp Thr Val Asp Tyr Gly
        435                 440                 445

Phe Gly Lys Ile Ser Lys Gly Val Ser Ser Pro Tyr Leu Pro Phe Gly
    450                 455                 460

Gly Gly Arg His Arg Cys Ile Gly Glu Gln Phe Ala Tyr Val Gln Leu
465                 470                 475                 480

Gly Thr Ile Leu Thr Thr Tyr Ile Tyr Asn Phe Lys Trp Arg Leu Asn
                485                 490                 495

Gly Asp Lys Val Pro Asp Val Asp Tyr Gln Ser Met Val Thr Leu Pro
            500                 505                 510

Leu Glu Pro Ala Glu Ile Val Trp Glu Lys Arg Asp Thr Cys Met Val

515                 520                 525

<210>  53
<211>  2097
<212>  DNA
<213>  Candida cloacae

<220>
<221>  CDS
<222>  (1)..(2097)

<400>  53
atg tcc cat caa gtt gaa gac cac gac tta gac gtg ttc tgt tta ttg        48
Met Ser His Gln Val Glu Asp His Asp Leu Asp Val Phe Cys Leu Leu
1               5                   10                  15

gcc gat gcc gtg ctc cat gaa att cct ccc agc gaa atc gtg gag tac        96
Ala Asp Ala Val Leu His Glu Ile Pro Pro Ser Glu Ile Val Glu Tyr
            20                  25                  30

ctt cat cct gac ttc ccc aaa gat aag atc gaa gag tat ttg aca ggc       144
Leu His Pro Asp Phe Pro Lys Asp Lys Ile Glu Glu Tyr Leu Thr Gly
        35                  40                  45

ttt tcc cgt ccg tct gct gtt cct cag ttt aga caa tgt gcc aag aag       192
Phe Ser Arg Pro Ser Ala Val Pro Gln Phe Arg Gln Cys Ala Lys Lys
    50                  55                  60

ctt atc aac aga ggc tcc gag ctg tcg atc aag ttg ttt ttg tac ttg       240
Leu Ile Asn Arg Gly Ser Glu Leu Ser Ile Lys Leu Phe Leu Tyr Leu
65                  70                  75                  80

acc act gcg ttg gac tca aga atc ctt gca cct gcc ttg acc aat tcg       288
Thr Thr Ala Leu Asp Ser Arg Ile Leu Ala Pro Ala Leu Thr Asn Ser
                85                  90                  95

ttg act ttg atc agg gat atg gat ctt tcc caa aga gag gag ttg ttg       336
Leu Thr Leu Ile Arg Asp Met Asp Leu Ser Gln Arg Glu Glu Leu Leu
            100                 105                 110

aga tca tgg aga gac tct cct tta act gca aaa aga aga tta ttt aga       384
Arg Ser Trp Arg Asp Ser Pro Leu Thr Ala Lys Arg Arg Leu Phe Arg
        115                 120                 125

gtg tat gcc tct ttt acc ttg tct act ttt aac aag ttg gga aca gac       432
Val Tyr Ala Ser Phe Thr Leu Ser Thr Phe Asn Lys Leu Gly Thr Asp
    130                 135                 140

ttg cac ttt aag gcg ttg ggc tac cca ggt aga gag ctc aga acg caa       480
Leu His Phe Lys Ala Leu Gly Tyr Pro Gly Arg Glu Leu Arg Thr Gln
145                 150                 155                 160

att caa gac tac gaa gtc gac cct ttt aga tat tcg ttt atg gag aaa       528
Ile Gln Asp Tyr Glu Val Asp Pro Phe Arg Tyr Ser Phe Met Glu Lys
                165                 170                 175

ctt aaa cac gag ggc cac gaa ttg ttc ctt cct gat att gac gtt tta       576
Leu Lys His Glu Gly His Glu Leu Phe Leu Pro Asp Ile Asp Val Leu
            180                 185                 190

atc atc ggg tcg gga tca gga gca ggt gtg gtt gca caa act ctt act       624
Ile Ile Gly Ser Gly Ser Gly Ala Gly Val Val Ala Gln Thr Leu Thr

195                 200                 205

gaa agt ggc ctc aaa tca ttg gtt ttg gaa aag ggc aaa tac ttt gcc       672
Glu Ser Gly Leu Lys Ser Leu Val Leu Glu Lys Gly Lys Tyr Phe Ala
    210                 215                 220

agt gaa gaa ttg tgc atg acg gac ttg gac ggt aac gag gca tta ttc       720
Ser Glu Glu Leu Cys Met Thr Asp Leu Asp Gly Asn Glu Ala Leu Phe
225                 230                 235                 240

gaa agt gga gga aca att cct tcc acc aac caa caa ttg ttc atg att       768
Glu Ser Gly Gly Thr Ile Pro Ser Thr Asn Gln Gln Leu Phe Met Ile
                245                 250                 255

gca ggt tcg act ttt ggt ggt ggt tct aca gtt aat tgg tct gcc tgt       816
Ala Gly Ser Thr Phe Gly Gly Gly Ser Thr Val Asn Trp Ser Ala Cys
            260                 265                 270

ttg aag acc cca ttc aaa gta aga aag gaa tgg tat gac gat ttc gga       864
Leu Lys Thr Pro Phe Lys Val Arg Lys Glu Trp Tyr Asp Asp Phe Gly
        275                 280                 285

ctt gat ttt gtc gct act caa caa tac gac gat tgt atg gat tac gtg       912
Leu Asp Phe Val Ala Thr Gln Gln Tyr Asp Asp Cys Met Asp Tyr Val
    290                 295                 300

tgg aag aaa atg ggt gct tcg acc gaa cat atc gaa cat tct gct gca       960
Trp Lys Lys Met Gly Ala Ser Thr Glu His Ile Glu His Ser Ala Ala
305                 310                 315                 320

aat gcc gtc atc atg gac ggg gca gca aaa ctt ggc tac gca cac aga      1008
Asn Ala Val Ile Met Asp Gly Ala Ala Lys Leu Gly Tyr Ala His Arg
                325                 330                 335

gca ctt gag cag aat acc ggg ggc cat gtt cac gac tgt ggg atg tgc      1056
Ala Leu Glu Gln Asn Thr Gly Gly His Val His Asp Cys Gly Met Cys
            340                 345                 350

cac ttg gga tgt aga ttc ggt atc aaa caa ggt ggt gta aat tgc tgg      1104
His Leu Gly Cys Arg Phe Gly Ile Lys Gln Gly Gly Val Asn Cys Trp
        355                 360                 365

ttc cgt gaa cct agt gaa aag ggt tct aag ttc atg gaa caa gtt gtt      1152
Phe Arg Glu Pro Ser Glu Lys Gly Ser Lys Phe Met Glu Gln Val Val
    370                 375                 380

gtt gaa aag att ttg cag cac aag ggt aaa gct act ggg att ttg tgt      1200
Val Glu Lys Ile Leu Gln His Lys Gly Lys Ala Thr Gly Ile Leu Cys
385                 390                 395                 400

aga gat act gaa agt ggg att aaa ttc aaa atc act gga cca aag aaa      1248
Arg Asp Thr Glu Ser Gly Ile Lys Phe Lys Ile Thr Gly Pro Lys Lys
                405                 410                 415

tac gtt gtt tcc ggt ggt tct ttg caa acc cca gtt ttg tta caa aaa      1296
Tyr Val Val Ser Gly Gly Ser Leu Gln Thr Pro Val Leu Leu Gln Lys
            420                 425                 430

tct ggt ttc aag aat aaa cat att gga gct aac tta aaa ctt cac cca      1344
Ser Gly Phe Lys Asn Lys His Ile Gly Ala Asn Leu Lys Leu His Pro
        435                 440                 445

gtc tcg gtt gcc ctt ggg gac ttt ggt aat gaa gtg gac ttt gaa gcc      1392
Val Ser Val Ala Leu Gly Asp Phe Gly Asn Glu Val Asp Phe Glu Ala

450                 455                 460

tac aag aga cca ctt atg acc gcc gtt tgt aat gcc gtc gat gat tta      1440
Tyr Lys Arg Pro Leu Met Thr Ala Val Cys Asn Ala Val Asp Asp Leu
465                 470                 475                 480

gat ggc aag gcc cat gga aca aga att gaa gcc att ttg cat gct cca      1488
Asp Gly Lys Ala His Gly Thr Arg Ile Glu Ala Ile Leu His Ala Pro
                485                 490                 495

tac gtc act gcc cca ttt tac cca tgg caa tca ggt gct caa gca aga      1536
Tyr Val Thr Ala Pro Phe Tyr Pro Trp Gln Ser Gly Ala Gln Ala Arg
            500                 505                 510

aag aac ctc ttg aaa tat aaa caa act gtg ccg tta tta ctt ctt tct      1584
Lys Asn Leu Leu Lys Tyr Lys Gln Thr Val Pro Leu Leu Leu Leu Ser
        515                 520                 525

aga gat aca tca tca ggt acc gtt aca tat gat aaa caa aag cct gac      1632
Arg Asp Thr Ser Ser Gly Thr Val Thr Tyr Asp Lys Gln Lys Pro Asp
    530                 535                 540

gta ttg gta gtt gac tac act gtt aac aag ttt gac aga aat tcg att      1680
Val Leu Val Val Asp Tyr Thr Val Asn Lys Phe Asp Arg Asn Ser Ile
545                 550                 555                 560

tta caa ggg ttt ttg gtt gct tcc gac atc ttg tat att gaa ggt gct      1728
Leu Gln Gly Phe Leu Val Ala Ser Asp Ile Leu Tyr Ile Glu Gly Ala
                565                 570                 575

aaa gag att ttg tca cca caa gct tgg gta cca acc ttc aag agc aac      1776
Lys Glu Ile Leu Ser Pro Gln Ala Trp Val Pro Thr Phe Lys Ser Asn
            580                 585                 590

aaa cca aaa cat gct aga tcg atc aaa gac gaa gat tac gtc aaa tgg      1824
Lys Pro Lys His Ala Arg Ser Ile Lys Asp Glu Asp Tyr Val Lys Trp
        595                 600                 605

aga gaa acc gtg gcc aag atc cca ttt gac tcc tac ggt tcg cca tac      1872
Arg Glu Thr Val Ala Lys Ile Pro Phe Asp Ser Tyr Gly Ser Pro Tyr
    610                 615                 620

ggt tct gct cat caa atg agt tcg tgt aga atg tct ggt aag gga cca      1920
Gly Ser Ala His Gln Met Ser Ser Cys Arg Met Ser Gly Lys Gly Pro
625                 630                 635                 640

gga tac ggt gct tgt gac act aaa gga aga tta ttt gaa tgt aac aac      1968
Gly Tyr Gly Ala Cys Asp Thr Lys Gly Arg Leu Phe Glu Cys Asn Asn
                645                 650                 655

gtt tac gtt gct gat gct tcg gtt atg cct act gca tcg gga gtc aat      2016
Val Tyr Val Ala Asp Ala Ser Val Met Pro Thr Ala Ser Gly Val Asn
            660                 665                 670

cct atg atc act aca atg gct ttt gca aga cat gtg gcc tta tgt ctt      2064
Pro Met Ile Thr Thr Met Ala Phe Ala Arg His Val Ala Leu Cys Leu
        675                 680                 685

gct aaa gac ttg caa cca caa act aaa ctt tag                          2097
Ala Lys Asp Leu Gln Pro Gln Thr Lys Leu
    690                 695

<210>  54

<211>  698
<212>  PRT
<213>  Candida cloacae

<400>  54

Met Ser His Gln Val Glu Asp His Asp Leu Asp Val Phe Cys Leu Leu
1               5                   10                  15

Ala Asp Ala Val Leu His Glu Ile Pro Pro Ser Glu Ile Val Glu Tyr
            20                  25                  30

Leu His Pro Asp Phe Pro Lys Asp Lys Ile Glu Glu Tyr Leu Thr Gly
        35                  40                  45

Phe Ser Arg Pro Ser Ala Val Pro Gln Phe Arg Gln Cys Ala Lys Lys
    50                  55                  60

Leu Ile Asn Arg Gly Ser Glu Leu Ser Ile Lys Leu Phe Leu Tyr Leu
65                  70                  75                  80

Thr Thr Ala Leu Asp Ser Arg Ile Leu Ala Pro Ala Leu Thr Asn Ser
                85                  90                  95

Leu Thr Leu Ile Arg Asp Met Asp Leu Ser Gln Arg Glu Glu Leu Leu
            100                 105                 110

Arg Ser Trp Arg Asp Ser Pro Leu Thr Ala Lys Arg Arg Leu Phe Arg
        115                 120                 125

Val Tyr Ala Ser Phe Thr Leu Ser Thr Phe Asn Lys Leu Gly Thr Asp
    130                 135                 140

Leu His Phe Lys Ala Leu Gly Tyr Pro Gly Arg Glu Leu Arg Thr Gln
145                 150                 155                 160

Ile Gln Asp Tyr Glu Val Asp Pro Phe Arg Tyr Ser Phe Met Glu Lys
                165                 170                 175

Leu Lys His Glu Gly His Glu Leu Phe Leu Pro Asp Ile Asp Val Leu
            180                 185                 190

Ile Ile Gly Ser Gly Ser Gly Ala Gly Val Val Ala Gln Thr Leu Thr
        195                 200                 205

Glu Ser Gly Leu Lys Ser Leu Val Leu Glu Lys Gly Lys Tyr Phe Ala
    210                 215                 220

Ser Glu Glu Leu Cys Met Thr Asp Leu Asp Gly Asn Glu Ala Leu Phe
225                 230                 235                 240

Glu Ser Gly Gly Thr Ile Pro Ser Thr Asn Gln Gln Leu Phe Met Ile
                245                 250                 255

Ala Gly Ser Thr Phe Gly Gly Gly Ser Thr Val Asn Trp Ser Ala Cys
            260                 265                 270

Leu Lys Thr Pro Phe Lys Val Arg Lys Glu Trp Tyr Asp Asp Phe Gly
        275                 280                 285

Leu Asp Phe Val Ala Thr Gln Gln Tyr Asp Asp Cys Met Asp Tyr Val
    290                 295                 300

Trp Lys Lys Met Gly Ala Ser Thr Glu His Ile Glu His Ser Ala Ala
305                 310                 315                 320

Asn Ala Val Ile Met Asp Gly Ala Ala Lys Leu Gly Tyr Ala His Arg
                325                 330                 335

Ala Leu Glu Gln Asn Thr Gly Gly His Val His Asp Cys Gly Met Cys
            340                 345                 350

His Leu Gly Cys Arg Phe Gly Ile Lys Gln Gly Gly Val Asn Cys Trp
        355                 360                 365

Phe Arg Glu Pro Ser Glu Lys Gly Ser Lys Phe Met Glu Gln Val Val
    370                 375                 380

Val Glu Lys Ile Leu Gln His Lys Gly Lys Ala Thr Gly Ile Leu Cys
385                 390                 395                 400

Arg Asp Thr Glu Ser Gly Ile Lys Phe Lys Ile Thr Gly Pro Lys Lys
                405                 410                 415

Tyr Val Val Ser Gly Gly Ser Leu Gln Thr Pro Val Leu Leu Gln Lys
            420                 425                 430

Ser Gly Phe Lys Asn Lys His Ile Gly Ala Asn Leu Lys Leu His Pro
        435                 440                 445

Val Ser Val Ala Leu Gly Asp Phe Gly Asn Glu Val Asp Phe Glu Ala
    450                 455                 460

Tyr Lys Arg Pro Leu Met Thr Ala Val Cys Asn Ala Val Asp Asp Leu
465                 470                 475                 480

Asp Gly Lys Ala His Gly Thr Arg Ile Glu Ala Ile Leu His Ala Pro
                485                 490                 495

Tyr Val Thr Ala Pro Phe Tyr Pro Trp Gln Ser Gly Ala Gln Ala Arg
            500                 505                 510

Lys Asn Leu Leu Lys Tyr Lys Gln Thr Val Pro Leu Leu Leu Leu Ser
        515                 520                 525

Arg Asp Thr Ser Ser Gly Thr Val Thr Tyr Asp Lys Gln Lys Pro Asp
    530                 535                 540

Val Leu Val Val Asp Tyr Thr Val Asn Lys Phe Asp Arg Asn Ser Ile
545                 550                 555                 560

Leu Gln Gly Phe Leu Val Ala Ser Asp Ile Leu Tyr Ile Glu Gly Ala
                565                 570                 575

Lys Glu Ile Leu Ser Pro Gln Ala Trp Val Pro Thr Phe Lys Ser Asn
            580                 585                 590

Lys Pro Lys His Ala Arg Ser Ile Lys Asp Glu Asp Tyr Val Lys Trp
        595                 600                 605

Arg Glu Thr Val Ala Lys Ile Pro Phe Asp Ser Tyr Gly Ser Pro Tyr
    610                 615                 620

Gly Ser Ala His Gln Met Ser Ser Cys Arg Met Ser Gly Lys Gly Pro
625                 630                 635                 640

Gly Tyr Gly Ala Cys Asp Thr Lys Gly Arg Leu Phe Glu Cys Asn Asn
                645                 650                 655

Val Tyr Val Ala Asp Ala Ser Val Met Pro Thr Ala Ser Gly Val Asn
            660                 665                 670

Pro Met Ile Thr Thr Met Ala Phe Ala Arg His Val Ala Leu Cys Leu
        675                 680                 685

Ala Lys Asp Leu Gln Pro Gln Thr Lys Leu
    690                 695

<210>  55
<211>  2097
<212>  DNA
<213>  Candida cloacae

<220>
<221>  CDS
<222>  (1)..(2097)

<400>  55

atg aat ccc gtt gtt gaa gac agc cat tta gat gtg ttc tgc ttg tta      48
Met Asn Pro Val Val Glu Asp Ser His Leu Asp Val Phe Cys Leu Leu
1               5                   10                  15

gcc gat gct gtg gtc cat gag ata cct cct agt gag atc gta gag tac      96
Ala Asp Ala Val Val His Glu Ile Pro Pro Ser Glu Ile Val Glu Tyr
            20                  25                  30

tta cat cct gat ttc cca aag gac aag gtt gaa gag tat ttg gct gag      144
Leu His Pro Asp Phe Pro Lys Asp Lys Val Glu Glu Tyr Leu Ala Glu
        35                  40                  45

ttt tct cat cct tca gca att ccc gaa ttt aga gaa gtt gca aaa aga      192
Phe Ser His Pro Ser Ala Ile Pro Glu Phe Arg Glu Val Ala Lys Arg
    50                  55                  60

att att aac aaa ggg act gtg ctg tca ata aag ttg ttt ttg ctc ttg      240
Ile Ile Asn Lys Gly Thr Val Leu Ser Ile Lys Leu Phe Leu Leu Leu
65                  70                  75                  80

gca act gct cta gac tcg aga atc ctt gct cct gcg ttg acc aac tcg      288
Ala Thr Ala Leu Asp Ser Arg Ile Leu Ala Pro Ala Leu Thr Asn Ser
                85                  90                  95

acg acg tta atc aga gat atg gat ctt tct caa aga gaa gaa tta ttg      336
Thr Thr Leu Ile Arg Asp Met Asp Leu Ser Gln Arg Glu Glu Leu Leu
            100                 105                 110

aga tca tgg aga gac tct cca ttc act aca aaa agg aaa ttg ttc agg      384
Arg Ser Trp Arg Asp Ser Pro Phe Thr Thr Lys Arg Lys Leu Phe Arg
        115                 120                 125

gtg tat aat tca ttc acc ttg aac gcg ttt agt aag acc gca aca gat      432
Val Tyr Asn Ser Phe Thr Leu Asn Ala Phe Ser Lys Thr Ala Thr Asp
    130                 135                 140

ttg cac ttc aaa gcg ttg gga tat cct ggt aga gag ctc agg act caa      480
Leu His Phe Lys Ala Leu Gly Tyr Pro Gly Arg Glu Leu Arg Thr Gln
145                 150                 155                 160

att cag gac tat gag gtc gat cct ttc aga tat acg ttc ttg gaa aaa      528
Ile Gln Asp Tyr Glu Val Asp Pro Phe Arg Tyr Thr Phe Leu Glu Lys
                165                 170                 175

ccc caa caa gac ggc cag gag tta cat ttt ccc gac att gat gtc ttg      576
Pro Gln Gln Asp Gly Gln Glu Leu His Phe Pro Asp Ile Asp Val Leu
            180                 185                 190

att atc ggg tct ggt tcg gga gca gga gtg gtt gct caa act ctt tcg      624
Ile Ile Gly Ser Gly Ser Gly Ala Gly Val Val Ala Gln Thr Leu Ser
        195                 200                 205

gaa aac gga ctt aaa tca ttg gtg ttg gaa aaa ggt aaa tac ttt tcc      672
Glu Asn Gly Leu Lys Ser Leu Val Leu Glu Lys Gly Lys Tyr Phe Ser
    210                 215                 220

aat gat gag ttg acc atg aat gat ttg gaa ggt agt gag gca tta ttc      720
Asn Asp Glu Leu Thr Met Asn Asp Leu Glu Gly Ser Glu Ala Leu Phe
225                 230                 235                 240

gaa aat gga ggt gcc ctc agt agt acc aac caa cag ata ttt ata att      768
Glu Asn Gly Gly Ala Leu Ser Ser Thr Asn Gln Gln Ile Phe Ile Ile
                245                 250                 255

gca ggt tcg act ttt ggg ggt ggt tct aca gtt aat tgg tct gcc tgt      816

Ala Gly Ser Thr Phe Gly Gly Gly Ser Thr Val Asn Trp Ser Ala Cys
            260                 265                 270

tta aaa act ccg ttc aaa gta aga aaa gag tgg tat gac aac ttc gga      864
Leu Lys Thr Pro Phe Lys Val Arg Lys Glu Trp Tyr Asp Asn Phe Gly
        275                 280                 285

ctt gat ttc gtc gca acc caa tac tac gaa gat tgt atg gat tat gtt      912
Leu Asp Phe Val Ala Thr Gln Tyr Tyr Glu Asp Cys Met Asp Tyr Val
    290                 295                 300

tgg aag aaa atg ggt gct tcg aac gaa aat atc gac cat tct ggt gct      960
Trp Lys Lys Met Gly Ala Ser Asn Glu Asn Ile Asp His Ser Gly Ala
305                 310                 315                 320

aat agt gtt ata ttg gaa ggg tcc aaa aaa ctt ggc tac cct cac agg     1008
Asn Ser Val Ile Leu Glu Gly Ser Lys Lys Leu Gly Tyr Pro His Arg
                325                 330                 335

gcc gtt gaa caa aat aat ggg ggc aaa att cat gac tgt ggt atg tgt     1056
Ala Val Glu Gln Asn Asn Gly Gly Lys Ile His Asp Cys Gly Met Cys
            340                 345                 350

cac ttg ggt tgt aga ttt ggt att aaa cag gga agt gta aat tgc tgg     1104
His Leu Gly Cys Arg Phe Gly Ile Lys Gln Gly Ser Val Asn Cys Trp
        355                 360                 365

ttc cgt ggt cca agt gaa aac gga tcc aag ttt atg caa caa gtt ctc     1152
Phe Arg Gly Pro Ser Glu Asn Gly Ser Lys Phe Met Gln Gln Val Leu
    370                 375                 380

gtg gat aag ata ttg caa cgt gac ggt aaa gca gtc ggt gtt ttg tgt     1200
Val Asp Lys Ile Leu Gln Arg Asp Gly Lys Ala Val Gly Val Leu Cys
385                 390                 395                 400

aga gat gtt gta acc ggt gtt aag ttc aag atc act gga cca aag aaa     1248
Arg Asp Val Val Thr Gly Val Lys Phe Lys Ile Thr Gly Pro Lys Lys
                405                 410                 415

att gtt gtt ttc tgg tgg ttc ttt gca aac tcc ggt ttt gtt aca aaa     1296
Ile Val Val Phe Trp Trp Phe Phe Ala Asn Ser Gly Phe Val Thr Lys
            420                 425                 430

tca ggt ttc aag aat aaa cac att ggt gct aac ttg aag ctc cat cca     1344
Ser Gly Phe Lys Asn Lys His Ile Gly Ala Asn Leu Lys Leu His Pro
        435                 440                 445

gtt tca ctt acg ctt gga gac ttc ggt aac aac gtg gat ttc gaa gct     1392
Val Ser Leu Thr Leu Gly Asp Phe Gly Asn Asn Val Asp Phe Glu Ala
    450                 455                 460

tac agg aaa cca att atg aca tca att tgt aat aaa gtc gaa gat tta     1440
Tyr Arg Lys Pro Ile Met Thr Ser Ile Cys Asn Lys Val Glu Asp Leu
465                 470                 475                 480

gat gga aag gcc cat ggt aca aga att gaa gct atg ttg aat gct cca     1488
Asp Gly Lys Ala His Gly Thr Arg Ile Glu Ala Met Leu Asn Ala Pro
                485                 490                 495

tat ggt gtt gca cca ttt ttc ccc tgg aaa tca ggc gct gaa tca aga     1536
Tyr Gly Val Ala Pro Phe Phe Pro Trp Lys Ser Gly Ala Glu Ser Arg
            500                 505                 510

aag gat ctc ttg aga tac aaa caa act gtg cct ata tta ctt ctt tcc     1584

Lys Asp Leu Leu Arg Tyr Lys Gln Thr Val Pro Ile Leu Leu Leu Ser
        515                 520                 525

aga gat act act tca ggg tct gtc aca tat gat aaa caa aaa cca gat      1632
Arg Asp Thr Thr Ser Gly Ser Val Thr Tyr Asp Lys Gln Lys Pro Asp
    530                 535                 540

gca ttg gtg att gat tac ctg tta aac aag ttt gac aga aac tca att      1680
Ala Leu Val Ile Asp Tyr Leu Leu Asn Lys Phe Asp Arg Asn Ser Ile
545                 550                 555                 560

tta caa ggg ttt ttg ata gct tca gac ctt tta tat att gaa ggt gcc      1728
Leu Gln Gly Phe Leu Ile Ala Ser Asp Leu Leu Tyr Ile Glu Gly Ala
                565                 570                 575

agt aga gat cat gtc acc tac aag ctt ggg tac caa tgg ttc aag agt      1776
Ser Arg Asp His Val Thr Tyr Lys Leu Gly Tyr Gln Trp Phe Lys Ser
            580                 585                 590

tct aaa cct aaa cat gcc aga tcg att gaa gat gaa gac tac gta aac      1824
Ser Lys Pro Lys His Ala Arg Ser Ile Glu Asp Glu Asp Tyr Val Asn
        595                 600                 605

tgg aga gct aaa gtt gca aag att cca ttt gat tcc tac gga tca cca      1872
Trp Arg Ala Lys Val Ala Lys Ile Pro Phe Asp Ser Tyr Gly Ser Pro
    610                 615                 620

tac ggt tca gct cac caa atg agt aca tgc aga atg tca ggt aag gga      1920
Tyr Gly Ser Ala His Gln Met Ser Thr Cys Arg Met Ser Gly Lys Gly
625                 630                 635                 640

cca gga tat gga gct tgt gat acc aaa gga aaa tta ttt gaa tgt agc      1968
Pro Gly Tyr Gly Ala Cys Asp Thr Lys Gly Lys Leu Phe Glu Cys Ser
                645                 650                 655

aat gtt tat gtt gca gat gct tcc act ttg cct act gca tca ggg gct      2016
Asn Val Tyr Val Ala Asp Ala Ser Thr Leu Pro Thr Ala Ser Gly Ala
            660                 665                 670

aat cca atg gtt agt acc atg tca ttt gca agg cac gtg tcc tta gga      2064
Asn Pro Met Val Ser Thr Met Ser Phe Ala Arg His Val Ser Leu Gly
        675                 680                 685

att gtt aaa gaa ttg caa caa agt aaa ctt tag                          2097
Ile Val Lys Glu Leu Gln Gln Ser Lys Leu
    690                 695

<210>  56
<211>  698
<212>  PRT
<213>  Candida cloacae

<400>  56

Met Asn Pro Val Val Glu Asp Ser His Leu Asp Val Phe Cys Leu Leu
1               5                   10                  15

Ala Asp Ala Val Val His Glu Ile Pro Pro Ser Glu Ile Val Glu Tyr
            20                  25                  30

Leu His Pro Asp Phe Pro Lys Asp Lys Val Glu Glu Tyr Leu Ala Glu

        35                  40                  45

Phe Ser His Pro Ser Ala Ile Pro Glu Phe Arg Glu Val Ala Lys Arg
    50                  55                  60

Ile Ile Asn Lys Gly Thr Val Leu Ser Ile Lys Leu Phe Leu Leu Leu
65                  70                  75                  80

Ala Thr Ala Leu Asp Ser Arg Ile Leu Ala Pro Ala Leu Thr Asn Ser
                85                  90                  95

Thr Thr Leu Ile Arg Asp Met Asp Leu Ser Gln Arg Glu Glu Leu Leu
            100                 105                 110

Arg Ser Trp Arg Asp Ser Pro Phe Thr Thr Lys Arg Lys Leu Phe Arg
        115                 120                 125

Val Tyr Asn Ser Phe Thr Leu Asn Ala Phe Ser Lys Thr Ala Thr Asp
    130                 135                 140

Leu His Phe Lys Ala Leu Gly Tyr Pro Gly Arg Glu Leu Arg Thr Gln
145                 150                 155                 160

Ile Gln Asp Tyr Glu Val Asp Pro Phe Arg Tyr Thr Phe Leu Glu Lys
                165                 170                 175

Pro Gln Gln Asp Gly Gln Glu Leu His Phe Pro Asp Ile Asp Val Leu
            180                 185                 190

Ile Ile Gly Ser Gly Ser Gly Ala Gly Val Val Ala Gln Thr Leu Ser
        195                 200                 205

Glu Asn Gly Leu Lys Ser Leu Val Leu Glu Lys Gly Lys Tyr Phe Ser
    210                 215                 220

Asn Asp Glu Leu Thr Met Asn Asp Leu Glu Gly Ser Glu Ala Leu Phe
225                 230                 235                 240

Glu Asn Gly Gly Ala Leu Ser Ser Thr Asn Gln Gln Ile Phe Ile Ile
                245                 250                 255

Ala Gly Ser Thr Phe Gly Gly Gly Ser Thr Val Asn Trp Ser Ala Cys
            260                 265                 270

Leu Lys Thr Pro Phe Lys Val Arg Lys Glu Trp Tyr Asp Asn Phe Gly
        275                 280                 285

Leu Asp Phe Val Ala Thr Gln Tyr Tyr Glu Asp Cys Met Asp Tyr Val

290                 295                 300

Trp Lys Lys Met Gly Ala Ser Asn Glu Asn Ile Asp His Ser Gly Ala
305                 310                 315                 320

Asn Ser Val Ile Leu Glu Gly Ser Lys Lys Leu Gly Tyr Pro His Arg
                325                 330                 335

Ala Val Glu Gln Asn Asn Gly Gly Lys Ile His Asp Cys Gly Met Cys
            340                 345                 350

His Leu Gly Cys Arg Phe Gly Ile Lys Gln Gly Ser Val Asn Cys Trp
        355                 360                 365

Phe Arg Gly Pro Ser Glu Asn Gly Ser Lys Phe Met Gln Gln Val Leu
    370                 375                 380

Val Asp Lys Ile Leu Gln Arg Asp Gly Lys Ala Val Gly Val Leu Cys
385                 390                 395                 400

Arg Asp Val Val Thr Gly Val Lys Phe Lys Ile Thr Gly Pro Lys Lys
                405                 410                 415

Ile Val Val Phe Trp Trp Phe Phe Ala Asn Ser Gly Phe Val Thr Lys
            420                 425                 430

Ser Gly Phe Lys Asn Lys His Ile Gly Ala Asn Leu Lys Leu His Pro
        435                 440                 445

Val Ser Leu Thr Leu Gly Asp Phe Gly Asn Asn Val Asp Phe Glu Ala
    450                 455                 460

Tyr Arg Lys Pro Ile Met Thr Ser Ile Cys Asn Lys Val Glu Asp Leu
465                 470                 475                 480

Asp Gly Lys Ala His Gly Thr Arg Ile Glu Ala Met Leu Asn Ala Pro
                485                 490                 495

Tyr Gly Val Ala Pro Phe Phe Pro Trp Lys Ser Gly Ala Glu Ser Arg
            500                 505                 510

Lys Asp Leu Leu Arg Tyr Lys Gln Thr Val Pro Ile Leu Leu Leu Ser
        515                 520                 525

Arg Asp Thr Thr Ser Gly Ser Val Thr Tyr Asp Lys Gln Lys Pro Asp
    530                 535                 540

Ala Leu Val Ile Asp Tyr Leu Leu Asn Lys Phe Asp Arg Asn Ser Ile

545                 550                 555                 560

Leu Gln Gly Phe Leu Ile Ala Ser Asp Leu Leu Tyr Ile Glu Gly Ala
                565                 570                 575

Ser Arg Asp His Val Thr Tyr Lys Leu Gly Tyr Gln Trp Phe Lys Ser
            580                 585                 590

Ser Lys Pro Lys His Ala Arg Ser Ile Glu Asp Glu Asp Tyr Val Asn
        595                 600                 605

Trp Arg Ala Lys Val Ala Lys Ile Pro Phe Asp Ser Tyr Gly Ser Pro
    610                 615                 620

Tyr Gly Ser Ala His Gln Met Ser Thr Cys Arg Met Ser Gly Lys Gly
625                 630                 635                 640

Pro Gly Tyr Gly Ala Cys Asp Thr Lys Gly Lys Leu Phe Glu Cys Ser
                645                 650                 655

Asn Val Tyr Val Ala Asp Ala Ser Thr Leu Pro Thr Ala Ser Gly Ala
            660                 665                 670

Asn Pro Met Val Ser Thr Met Ser Phe Ala Arg His Val Ser Leu Gly
        675                 680                 685

Ile Val Lys Glu Leu Gln Gln Ser Lys Leu
    690                 695

<210>  57
<211>  948
<212>  DNA
<213>  Candida tropicalis

<220>
<221>  CDS
<222>  (1)..(948)

<400>  57
cca ttg caa tac acc gat atc cca gtt cca gtc cct aag cca aac gaa       48
Pro Leu Gln Tyr Thr Asp Ile Pro Val Pro Val Pro Lys Pro Asn Glu
1               5                   10                  15

ttg ctc gtc cac gtc aaa tac tcc ggt gtt tgt cac tca gat ata cac       96
Leu Leu Val His Val Lys Tyr Ser Gly Val Cys His Ser Asp Ile His
            20                  25                  30

gtc tgg aag ggt gac tgg ttc cca gca tcg aaa ttg ccc gtt gtt ggt      144
Val Trp Lys Gly Asp Trp Phe Pro Ala Ser Lys Leu Pro Val Val Gly
        35                  40                  45

ggt cac gaa ggt gcc ggt gtt gtc gtt gcc att ggt gaa aac gtc caa      192
Gly His Glu Gly Ala Gly Val Val Val Ala Ile Gly Glu Asn Val Gln

50                  55                  60

ggc tgg aaa gta ggt gac ttg gca ggt ata aag atg ttg aat ggt tcc      240
Gly Trp Lys Val Gly Asp Leu Ala Gly Ile Lys Met Leu Asn Gly Ser
65                  70                  75                  80

tgt atg aac tgt gaa tac tgt caa caa ggt gct gaa cca aac tgt ccc      288
Cys Met Asn Cys Glu Tyr Cys Gln Gln Gly Ala Glu Pro Asn Cys Pro
                85                  90                  95

cac gct gat gtc tcg ggt tac tcc cac gac ggt act ttc caa cag tac      336
His Ala Asp Val Ser Gly Tyr Ser His Asp Gly Thr Phe Gln Gln Tyr
            100                 105                 110

gct acc gcc gat gct gtt caa gct gct aaa ttc cca gct ggt tct gat      384
Ala Thr Ala Asp Ala Val Gln Ala Ala Lys Phe Pro Ala Gly Ser Asp
        115                 120                 125

tta gct agc atc gca cct ata tcc tgc gcc ggt gtt act gtt tac aaa      432
Leu Ala Ser Ile Ala Pro Ile Ser Cys Ala Gly Val Thr Val Tyr Lys
    130                 135                 140

gca ttg aaa act gca ggc ttg cag cca ggt caa tgg gtt gcc atc tct      480
Ala Leu Lys Thr Ala Gly Leu Gln Pro Gly Gln Trp Val Ala Ile Ser
145                 150                 155                 160

ggt gca gct ggt ggt ttg ggt tct ttg gct gtg caa tac gcc aag gcc      528
Gly Ala Ala Gly Gly Leu Gly Ser Leu Ala Val Gln Tyr Ala Lys Ala
                165                 170                 175

atg ggt ttg aga gtc gtg gcc att gac ggt ggt gac gaa aga gga gtg      576
Met Gly Leu Arg Val Val Ala Ile Asp Gly Gly Asp Glu Arg Gly Val
            180                 185                 190

ttt gtc aaa tcg ttg ggt gct gaa gtt ttc gtt gat ttc acc aaa gag      624
Phe Val Lys Ser Leu Gly Ala Glu Val Phe Val Asp Phe Thr Lys Glu
        195                 200                 205

gcc aat gtc tct gag gct atc atc aag gct acc gac ggt ggt gcc cat      672
Ala Asn Val Ser Glu Ala Ile Ile Lys Ala Thr Asp Gly Gly Ala His
    210                 215                 220

ggc gtc atc aac gtt tcc att tct gaa aaa gcc atc aac cag tct gtt      720
Gly Val Ile Asn Val Ser Ile Ser Glu Lys Ala Ile Asn Gln Ser Val
225                 230                 235                 240

gaa tat gtt aga act ttg gga act gtt gtc ttg gtt ggt ttg cca gct      768
Glu Tyr Val Arg Thr Leu Gly Thr Val Val Leu Val Gly Leu Pro Ala
                245                 250                 255

ggt gca aag ctc gaa gct cct atc ttc aat gcc gtt gcc aaa tcc atc      816
Gly Ala Lys Leu Glu Ala Pro Ile Phe Asn Ala Val Ala Lys Ser Ile
            260                 265                 270

caa atc aaa ggt tct tac gtg gga aac aga aga gac act gct gag gct      864
Gln Ile Lys Gly Ser Tyr Val Gly Asn Arg Arg Asp Thr Ala Glu Ala
        275                 280                 285

gtt gat ttc ttc gct aga ggt ttg gtc aaa tgt cca att aag gtt gtt      912
Val Asp Phe Phe Ala Arg Gly Leu Val Lys Cys Pro Ile Lys Val Val
    290                 295                 300

ggg ttg agt gaa ttg cca gag att ttc aaa ttg ttg                      948
Gly Leu Ser Glu Leu Pro Glu Ile Phe Lys Leu Leu

305                 310                 315

<210>  58
<211>  316
<212>  PRT
<213>  Candida tropicalis

<400>  58

Pro Leu Gln Tyr Thr Asp Ile Pro Val Pro Val Pro Lys Pro Asn Glu
1               5                   10                  15

Leu Leu Val His Val Lys Tyr Ser Gly Val Cys His Ser Asp Ile His
            20                  25                  30

Val Trp Lys Gly Asp Trp Phe Pro Ala Ser Lys Leu Pro Val Val Gly
        35                  40                  45

Gly His Glu Gly Ala Gly Val Val Val Ala Ile Gly Glu Asn Val Gln
    50                  55                  60

Gly Trp Lys Val Gly Asp Leu Ala Gly Ile Lys Met Leu Asn Gly Ser
65                  70                  75                  80

Cys Met Asn Cys Glu Tyr Cys Gln Gln Gly Ala Glu Pro Asn Cys Pro
                85                  90                  95

His Ala Asp Val Ser Gly Tyr Ser His Asp Gly Thr Phe Gln Gln Tyr
            100                 105                 110

Ala Thr Ala Asp Ala Val Gln Ala Ala Lys Phe Pro Ala Gly Ser Asp
        115                 120                 125

Leu Ala Ser Ile Ala Pro Ile Ser Cys Ala Gly Val Thr Val Tyr Lys
    130                 135                 140

Ala Leu Lys Thr Ala Gly Leu Gln Pro Gly Gln Trp Val Ala Ile Ser
145                 150                 155                 160

Gly Ala Ala Gly Gly Leu Gly Ser Leu Ala Val Gln Tyr Ala Lys Ala
                165                 170                 175

Met Gly Leu Arg Val Val Ala Ile Asp Gly Gly Asp Glu Arg Gly Val
            180                 185                 190

Phe Val Lys Ser Leu Gly Ala Glu Val Phe Val Asp Phe Thr Lys Glu
        195                 200                 205

Ala Asn Val Ser Glu Ala Ile Ile Lys Ala Thr Asp Gly Gly Ala His
    210                 215                 220

Gly Val Ile Asn Val Ser Ile Ser Glu Lys Ala Ile Asn Gln Ser Val
225                 230                 235                 240

Glu Tyr Val Arg Thr Leu Gly Thr Val Val Leu Val Gly Leu Pro Ala
                245                 250                 255

Gly Ala Lys Leu Glu Ala Pro Ile Phe Asn Ala Val Ala Lys Ser Ile
            260                 265                 270

Gln Ile Lys Gly Ser Tyr Val Gly Asn Arg Arg Asp Thr Ala Glu Ala
        275                 280                 285

Val Asp Phe Phe Ala Arg Gly Leu Val Lys Cys Pro Ile Lys Val Val
    290                 295                 300

Gly Leu Ser Glu Leu Pro Glu Ile Phe Lys Leu Leu
305                 310                 315

<210>  59
<211>  948
<212>  DNA
<213>  Candida tropicalis

<220>
<221>  CDS
<222>  (1)..(948)

<400>  59
aaa tta gaa tac aag gac atc cca gtt cca aag cca aag cca aac gaa        48
Lys Leu Glu Tyr Lys Asp Ile Pro Val Pro Lys Pro Lys Pro Asn Glu
1               5                   10                  15

ttg ctc atc aac gtc aag tac tcc ggt gtc tgc cac act gat tta cac        96
Leu Leu Ile Asn Val Lys Tyr Ser Gly Val Cys His Thr Asp Leu His
            20                  25                  30

gcc tgg aag ggt gac tgg cca ttg gac acc aag ttg cca ttg gtg ggt       144
Ala Trp Lys Gly Asp Trp Pro Leu Asp Thr Lys Leu Pro Leu Val Gly
        35                  40                  45

ggt cac gaa ggt gct ggt gtt gtt gtt gcc att ggt gac aat gtc aag       192
Gly His Glu Gly Ala Gly Val Val Val Ala Ile Gly Asp Asn Val Lys
    50                  55                  60

gga tgg aag gtc ggt gat ttg gcc ggt gtc aag tgg ttg aac ggt tcc       240
Gly Trp Lys Val Gly Asp Leu Ala Gly Val Lys Trp Leu Asn Gly Ser
65                  70                  75                  80

tgt atg aac tgt gag tac tgt caa cag ggt gcc gaa cca aac tgt cca       288
Cys Met Asn Cys Glu Tyr Cys Gln Gln Gly Ala Glu Pro Asn Cys Pro
                85                  90                  95

cag gct gac ttg tct ggt tac acc cac gac ggt tct ttc cag caa tac       336
Gln Ala Asp Leu Ser Gly Tyr Thr His Asp Gly Ser Phe Gln Gln Tyr
            100                 105                 110

gcc act gca gat gcc gtg caa gcc gct aga att cca gct ggt act gat        384
Ala Thr Ala Asp Ala Val Gln Ala Ala Arg Ile Pro Ala Gly Thr Asp
        115                 120                 125

tta gcc aac gtt gcc ccc atc ttg tgt gct ggt gtc act gtt tac aag        432
Leu Ala Asn Val Ala Pro Ile Leu Cys Ala Gly Val Thr Val Tyr Lys
    130                 135                 140

gcc ttg aag acc gcc gac ttg cag cca ggt caa tgg gtc gcc att tcc        480
Ala Leu Lys Thr Ala Asp Leu Gln Pro Gly Gln Trp Val Ala Ile Ser
145                 150                 155                 160

ggt gcc gct ggt ggt ttg ggt tct ttg gcc gtt caa tac gcc aag gcc        528
Gly Ala Ala Gly Gly Leu Gly Ser Leu Ala Val Gln Tyr Ala Lys Ala
                165                 170                 175

atg ggc tac aga gtt gtc gcc atc gat ggt ggt gcc gac aag ggt gag        576
Met Gly Tyr Arg Val Val Ala Ile Asp Gly Gly Ala Asp Lys Gly Glu
            180                 185                 190

ttc gtc aag tct ttg ggc gct gag gtc ttt gtt gat ttc ctc aag gaa        624
Phe Val Lys Ser Leu Gly Ala Glu Val Phe Val Asp Phe Leu Lys Glu
        195                 200                 205

aag gac att gtt ggt gct gtc aag aag gca acc gat ggt ggc cca cac        672
Lys Asp Ile Val Gly Ala Val Lys Lys Ala Thr Asp Gly Gly Pro His
    210                 215                 220

ggt gcc gtt aac gtt tcc atc tcc gaa aag gcc atc aac caa tct gtc        720
Gly Ala Val Asn Val Ser Ile Ser Glu Lys Ala Ile Asn Gln Ser Val
225                 230                 235                 240

gac tac gtt aga acc ttg ggt aag gtt gtc ttg gtc ggt ttg cca gct        768
Asp Tyr Val Arg Thr Leu Gly Lys Val Val Leu Val Gly Leu Pro Ala
                245                 250                 255

ggc tcc aag gtt tct gct cca gtc ttt gac tcc gtc gtc aag tcc atc        816
Gly Ser Lys Val Ser Ala Pro Val Phe Asp Ser Val Val Lys Ser Ile
            260                 265                 270

caa atc aag ggt tcc tat gtc ggt aac aga aag gac act gcc gaa gct        864
Gln Ile Lys Gly Ser Tyr Val Gly Asn Arg Lys Asp Thr Ala Glu Ala
        275                 280                 285

gtt gac ttt ttc tcc aga ggc ttg atc aag tgt cca atc aag gtt gtc        912
Val Asp Phe Phe Ser Arg Gly Leu Ile Lys Cys Pro Ile Lys Val Val
    290                 295                 300

ggt ttg agt gaa ttg cca gaa gtc tac aag ttg atg                        948
Gly Leu Ser Glu Leu Pro Glu Val Tyr Lys Leu Met
305                 310                 315

<210>  60
<211>  316
<212>  PRT
<213>  Candida tropicalis

<400>  60

Lys Leu Glu Tyr Lys Asp Ile Pro Val Pro Lys Pro Lys Pro Asn Glu
1               5                   10                  15

Leu Leu Ile Asn Val Lys Tyr Ser Gly Val Cys His Thr Asp Leu His

            20                  25                  30

Ala Trp Lys Gly Asp Trp Pro Leu Asp Thr Lys Leu Pro Leu Val Gly
        35                  40                  45

Gly His Glu Gly Ala Gly Val Val Val Ala Ile Gly Asp Asn Val Lys
    50                  55                  60

Gly Trp Lys Val Gly Asp Leu Ala Gly Val Lys Trp Leu Asn Gly Ser
65                  70                  75                  80

Cys Met Asn Cys Glu Tyr Cys Gln Gln Gly Ala Glu Pro Asn Cys Pro
                85                  90                  95

Gln Ala Asp Leu Ser Gly Tyr Thr His Asp Gly Ser Phe Gln Gln Tyr
            100                 105                 110

Ala Thr Ala Asp Ala Val Gln Ala Ala Arg Ile Pro Ala Gly Thr Asp
        115                 120                 125

Leu Ala Asn Val Ala Pro Ile Leu Cys Ala Gly Val Thr Val Tyr Lys
    130                 135                 140

Ala Leu Lys Thr Ala Asp Leu Gln Pro Gly Gln Trp Val Ala Ile Ser
145                 150                 155                 160

Gly Ala Ala Gly Gly Leu Gly Ser Leu Ala Val Gln Tyr Ala Lys Ala
                165                 170                 175

Met Gly Tyr Arg Val Val Ala Ile Asp Gly Gly Ala Asp Lys Gly Glu
            180                 185                 190

Phe Val Lys Ser Leu Gly Ala Glu Val Phe Val Asp Phe Leu Lys Glu
        195                 200                 205

Lys Asp Ile Val Gly Ala Val Lys Lys Ala Thr Asp Gly Gly Pro His
    210                 215                 220

Gly Ala Val Asn Val Ser Ile Ser Glu Lys Ala Ile Asn Gln Ser Val
225                 230                 235                 240

Asp Tyr Val Arg Thr Leu Gly Lys Val Val Leu Val Gly Leu Pro Ala
                245                 250                 255

Gly Ser Lys Val Ser Ala Pro Val Phe Asp Ser Val Val Lys Ser Ile
            260                 265                 270

Gln Ile Lys Gly Ser Tyr Val Gly Asn Arg Lys Asp Thr Ala Glu Ala

275                 280                 285

Val Asp Phe Phe Ser Arg Gly Leu Ile Lys Cys Pro Ile Lys Val Val
    290                 295                 300

Gly Leu Ser Glu Leu Pro Glu Val Tyr Lys Leu Met
305                 310                 315

<210>  61
<211>  456
<212>  DNA
<213>  Candida tropicalis

<220>
<221>  CDS
<222>  (1)..(456)

<400>  61
aac tgt gag ttt tgc caa cag ggc gct gaa cct aat tgt cca aga gcc       48
Asn Cys Glu Phe Cys Gln Gln Gly Ala Glu Pro Asn Cys Pro Arg Ala
1               5                   10                  15

gac atg tct gga tat acc cac gat ggg act ttc caa caa tat gct acc       96
Asp Met Ser Gly Tyr Thr His Asp Gly Thr Phe Gln Gln Tyr Ala Thr
            20                  25                  30

gcc gat gcc gtc caa gct gcc aag atc cca gaa ggc gca gac atg gct      144
Ala Asp Ala Val Gln Ala Ala Lys Ile Pro Glu Gly Ala Asp Met Ala
        35                  40                  45

agt atc gcc ccg atc ttg tgt gct ggt gtg acc gtg tac aag gct ttg      192
Ser Ile Ala Pro Ile Leu Cys Ala Gly Val Thr Val Tyr Lys Ala Leu
    50                  55                  60

aag aac gcc gac ttg ttg gct ggc caa tgg gtg gct atc tct ggt gct      240
Lys Asn Ala Asp Leu Leu Ala Gly Gln Trp Val Ala Ile Ser Gly Ala
65                  70                  75                  80

ggt ggt ggt ttg ggc tcc ttg ggt gtg cag tac gct aaa gcc atg ggt      288
Gly Gly Gly Leu Gly Ser Leu Gly Val Gln Tyr Ala Lys Ala Met Gly
                85                  90                  95

tac aga gtg tta gcc atc gat ggt ggt gat gag aga gga gag ttt gtc      336
Tyr Arg Val Leu Ala Ile Asp Gly Gly Asp Glu Arg Gly Glu Phe Val
            100                 105                 110

aag tca ttg ggc gcc gaa gtg tac att gac ttc ctt aag gaa cag gac      384
Lys Ser Leu Gly Ala Glu Val Tyr Ile Asp Phe Leu Lys Glu Gln Asp
        115                 120                 125

att gtt agt gcc att aga aag gca act ggt ggt ggc cca cac ggt gtt      432
Ile Val Ser Ala Ile Arg Lys Ala Thr Gly Gly Gly Pro His Gly Val
    130                 135                 140

att aac gtc tcg gtg tcc gaa aag                                      456
Ile Asn Val Ser Val Ser Glu Lys
145                 150

<210>  62

<211>  152
<212>  PRT
<213>  Candida tropicalis

<400>  62

Asn Cys Glu Phe Cys Gln Gln Gly Ala Glu Pro Asn Cys Pro Arg Ala
1               5                   10                  15

Asp Met Ser Gly Tyr Thr His Asp Gly Thr Phe Gln Gln Tyr Ala Thr
            20                  25                  30

Ala Asp Ala Val Gln Ala Ala Lys Ile Pro Glu Gly Ala Asp Met Ala
        35                  40                  45

Ser Ile Ala Pro Ile Leu Cys Ala Gly Val Thr Val Tyr Lys Ala Leu
    50                  55                  60

Lys Asn Ala Asp Leu Leu Ala Gly Gln Trp Val Ala Ile Ser Gly Ala
65                  70                  75                  80

Gly Gly Gly Leu Gly Ser Leu Gly Val Gln Tyr Ala Lys Ala Met Gly
                85                  90                  95

Tyr Arg Val Leu Ala Ile Asp Gly Gly Asp Glu Arg Gly Glu Phe Val
            100                 105                 110

Lys Ser Leu Gly Ala Glu Val Tyr Ile Asp Phe Leu Lys Glu Gln Asp
        115                 120                 125

Ile Val Ser Ala Ile Arg Lys Ala Thr Gly Gly Gly Pro His Gly Val
    130                 135                 140

Ile Asn Val Ser Val Ser Glu Lys
145                 150

<210>  63
<211>  948
<212>  DNA
<213>  Candida tropicalis

<220>
<221>  CDS
<222>  (1)..(948)

<400>  63
aag tta gaa tac aaa gac gtg ccg gtc cct gtc cct aaa ccc aac gaa       48
Lys Leu Glu Tyr Lys Asp Val Pro Val Pro Val Pro Lys Pro Asn Glu
1               5                   10                  15

ttg ctt gtc aac gtc aag tac tcg ggt gtg tgt cat tct gac ttg cat       96
Leu Leu Val Asn Val Lys Tyr Ser Gly Val Cys His Ser Asp Leu His
            20                  25                  30

gtc tgg aaa ggc gac tgg ccc att cct gcc aag ttg ccc ttg gtg gga 144
Val Trp Lys Gly Asp Trp Pro Ile Pro Ala Lys Leu Pro Leu Val Gly
        35                  40                  45

ggt cac gaa ggt gct ggt gtc gtt gtc ggc atg ggt gac aac gtc aag 192
Gly His Glu Gly Ala Gly Val Val Val Gly Met Gly Asp Asn Val Lys
    50                  55                  60

ggc tgg aag gtg ggg gac ttg gct ggt atc aag tgg ttg aat ggt tcg 240
Gly Trp Lys Val Gly Asp Leu Ala Gly Ile Lys Trp Leu Asn Gly Ser
65                  70                  75                  80

tgt atg aac tgt gag ttt tgc caa cag ggc gca gaa cct aac tgt tca 288
Cys Met Asn Cys Glu Phe Cys Gln Gln Gly Ala Glu Pro Asn Cys Ser
                85                  90                  95

aga gcc gac atg tct ggg tat acc cac gat gga act ttc caa caa tac 336
Arg Ala Asp Met Ser Gly Tyr Thr His Asp Gly Thr Phe Gln Gln Tyr
            100                 105                 110

gcc act gct gat gct gtc caa gct gcc aag atc cca gaa ggc gcc gac 384
Ala Thr Ala Asp Ala Val Gln Ala Ala Lys Ile Pro Glu Gly Ala Asp
        115                 120                 125

atg gct agt atc gcc ccg atc ttg tgc gct ggt gtg acc gtg tac aag 432
Met Ala Ser Ile Ala Pro Ile Leu Cys Ala Gly Val Thr Val Tyr Lys
    130                 135                 140

gct ttg aag aac gcc gac ttg ttg gct ggc caa tgg gtg gct atc tct 480
Ala Leu Lys Asn Ala Asp Leu Leu Ala Gly Gln Trp Val Ala Ile Ser
145                 150                 155                 160

ggt gct ggt ggt ggt ttg ggc tcc ttg ggt gtg cag tac gct aaa gcc 528
Gly Ala Gly Gly Gly Leu Gly Ser Leu Gly Val Gln Tyr Ala Lys Ala
                165                 170                 175

atg ggt tac aga gtg ttg gct atc gac ggt ggt gac gag aga gga gag 576
Met Gly Tyr Arg Val Leu Ala Ile Asp Gly Gly Asp Glu Arg Gly Glu
            180                 185                 190

ttt gtc aag tcc ttg ggc gcc gaa gtg tac att gac ttc ctt aag gaa 624
Phe Val Lys Ser Leu Gly Ala Glu Val Tyr Ile Asp Phe Leu Lys Glu
        195                 200                 205

cag gac atc gtt agt gct atc aga aag gca act ggt ggt ggt cca cac 672
Gln Asp Ile Val Ser Ala Ile Arg Lys Ala Thr Gly Gly Gly Pro His
    210                 215                 220

ggt gtt att aac gtg tca gtg tcc gaa aag gca atc aac cag tcg gtg 720
Gly Val Ile Asn Val Ser Val Ser Glu Lys Ala Ile Asn Gln Ser Val
225                 230                 235                 240

gag tac gtc aga act ttg ggg aaa gtg gtt tta gtt agc ttg ccg gca 768
Glu Tyr Val Arg Thr Leu Gly Lys Val Val Leu Val Ser Leu Pro Ala
                245                 250                 255

ggt ggt aaa ctc act gct cct ctt ttc gag tct gtt gct aga tca atc 816
Gly Gly Lys Leu Thr Ala Pro Leu Phe Glu Ser Val Ala Arg Ser Ile
            260                 265                 270

cag att aga act acg tgt gtt ggc aac aga aag gat act act gaa gct 864
Gln Ile Arg Thr Thr Cys Val Gly Asn Arg Lys Asp Thr Thr Glu Ala
        275                 280                 285

att gat ttc ttt gtt aga ggg ttg atc gat tgc cca att aaa gtc gct        912
Ile Asp Phe Phe Val Arg Gly Leu Ile Asp Cys Pro Ile Lys Val Ala
    290                 295                 300

ggt tta agt gaa gtg cca gag att ttt gac ttg atg                        948
Gly Leu Ser Glu Val Pro Glu Ile Phe Asp Leu Met
305                 310                 315


<210>  64
<211>  316
<212>  PRT
<213>  Candida tropicalis

<400>  64

Lys Leu Glu Tyr Lys Asp Val Pro Val Pro Val Pro Lys Pro Asn Glu
1               5                   10                  15

Leu Leu Val Asn Val Lys Tyr Ser Gly Val Cys His Ser Asp Leu His
            20                  25                  30

Val Trp Lys Gly Asp Trp Pro Ile Pro Ala Lys Leu Pro Leu Val Gly
        35                  40                  45

Gly His Glu Gly Ala Gly Val Val Val Gly Met Gly Asp Asn Val Lys
    50                  55                  60

Gly Trp Lys Val Gly Asp Leu Ala Gly Ile Lys Trp Leu Asn Gly Ser
65                  70                  75                  80

Cys Met Asn Cys Glu Phe Cys Gln Gln Gly Ala Glu Pro Asn Cys Ser
                85                  90                  95

Arg Ala Asp Met Ser Gly Tyr Thr His Asp Gly Thr Phe Gln Gln Tyr
            100                 105                 110

Ala Thr Ala Asp Ala Val Gln Ala Ala Lys Ile Pro Glu Gly Ala Asp
        115                 120                 125

Met Ala Ser Ile Ala Pro Ile Leu Cys Ala Gly Val Thr Val Tyr Lys
    130                 135                 140

Ala Leu Lys Asn Ala Asp Leu Leu Ala Gly Gln Trp Val Ala Ile Ser
145                 150                 155                 160

Gly Ala Gly Gly Gly Leu Gly Ser Leu Gly Val Gln Tyr Ala Lys Ala
                165                 170                 175

Met Gly Tyr Arg Val Leu Ala Ile Asp Gly Gly Asp Glu Arg Gly Glu
            180                 185                 190

Phe Val Lys Ser Leu Gly Ala Glu Val Tyr Ile Asp Phe Leu Lys Glu

195                 200                 205

Gln Asp Ile Val Ser Ala Ile Arg Lys Ala Thr Gly Gly Gly Pro His
    210                 215                 220

Gly Val Ile Asn Val Ser Val Ser Glu Lys Ala Ile Asn Gln Ser Val
225                 230                 235                 240

Glu Tyr Val Arg Thr Leu Gly Lys Val Val Leu Val Ser Leu Pro Ala
                245                 250                 255

Gly Gly Lys Leu Thr Ala Pro Leu Phe Glu Ser Val Ala Arg Ser Ile
            260                 265                 270

Gln Ile Arg Thr Thr Cys Val Gly Asn Arg Lys Asp Thr Thr Glu Ala
        275                 280                 285

Ile Asp Phe Phe Val Arg Gly Leu Ile Asp Cys Pro Ile Lys Val Ala
    290                 295                 300

Gly Leu Ser Glu Val Pro Glu Ile Phe Asp Leu Met
305                 310                 315

<210>  65
<211>  948
<212>  DNA
<213>  Candida tropicalis

<220>
<221>  CDS
<222>  (1)..(948)

<400>  65
gaa tta gaa tac aaa gat atc cca gtg cca acc cca aag gcc aac gaa       48
Glu Leu Glu Tyr Lys Asp Ile Pro Val Pro Thr Pro Lys Ala Asn Glu
1               5                   10                  15

ttg ctc atc aac gtc aaa tac tcg ggt gtc tgc cac act gat ttg cac       96
Leu Leu Ile Asn Val Lys Tyr Ser Gly Val Cys His Thr Asp Leu His
            20                  25                  30

gcc tgg aag ggt gac tgg cca ttg gcc acc aag ttg cca ttg gtt ggt      144
Ala Trp Lys Gly Asp Trp Pro Leu Ala Thr Lys Leu Pro Leu Val Gly
        35                  40                  45

ggt cac gaa ggt gct ggt gtc gtt gtc ggc atg ggt gaa aac gtc aag      192
Gly His Glu Gly Ala Gly Val Val Val Gly Met Gly Glu Asn Val Lys
    50                  55                  60

ggc tgg aag att ggt gac ttc gcc ggt atc aaa tgg ttg aac ggt tcc      240
Gly Trp Lys Ile Gly Asp Phe Ala Gly Ile Lys Trp Leu Asn Gly Ser
65                  70                  75                  80

tgt atg tcc tgt gag ttc tgt caa caa ggt gct gaa cca aac tgt ggt      288
Cys Met Ser Cys Glu Phe Cys Gln Gln Gly Ala Glu Pro Asn Cys Gly

85                  90                  95

gag gcc gac ttg tct ggt tac acc cac gat ggt tct ttc gaa caa tac      336
Glu Ala Asp Leu Ser Gly Tyr Thr His Asp Gly Ser Phe Glu Gln Tyr
            100                 105                 110

gcc act gct gat gct gtt caa gcc gcc aga atc cca gct ggt act gat      384
Ala Thr Ala Asp Ala Val Gln Ala Ala Arg Ile Pro Ala Gly Thr Asp
        115                 120                 125

ttg gcc gaa gtt gcc cca atc ttg tgt gcg ggt gtc acc gtc tac aaa      432
Leu Ala Glu Val Ala Pro Ile Leu Cys Ala Gly Val Thr Val Tyr Lys
    130                 135                 140

gcc ttg aag act gcc gac ttg gcc gct ggt caa tgg gtc gct atc tcc      480
Ala Leu Lys Thr Ala Asp Leu Ala Ala Gly Gln Trp Val Ala Ile Ser
145                 150                 155                 160

ggt gct ggt ggt ggt ttg ggt tcc ttg gct gtc caa tac gcc gtc gcc      528
Gly Ala Gly Gly Gly Leu Gly Ser Leu Ala Val Gln Tyr Ala Val Ala
                165                 170                 175

atg ggc ttg aga gtc gtt gcc att gac ggt ggt gac gaa aag ggt gcc      576
Met Gly Leu Arg Val Val Ala Ile Asp Gly Gly Asp Glu Lys Gly Ala
            180                 185                 190

ttt gtc aag tcc ttg ggt gct gaa gcc tac att gat ttc ctc aag gaa      624
Phe Val Lys Ser Leu Gly Ala Glu Ala Tyr Ile Asp Phe Leu Lys Glu
        195                 200                 205

aag gac att gtc tct gct gtc aag aag gcc acc gat gga ggt cca cac      672
Lys Asp Ile Val Ser Ala Val Lys Lys Ala Thr Asp Gly Gly Pro His
    210                 215                 220

ggt gct atc aat gtt tcc gtt tcc gaa aaa gcc att gac caa tcc gtc      720
Gly Ala Ile Asn Val Ser Val Ser Glu Lys Ala Ile Asp Gln Ser Val
225                 230                 235                 240

gag tac gtt aga cca ttg ggt aag gtt gtt ttg gtt ggt ttg cca gct      768
Glu Tyr Val Arg Pro Leu Gly Lys Val Val Leu Val Gly Leu Pro Ala
                245                 250                 255

ggc tcc aag gtc act gct ggt gtt ttc gaa gcc gtt gtc aag tcc att      816
Gly Ser Lys Val Thr Ala Gly Val Phe Glu Ala Val Val Lys Ser Ile
            260                 265                 270

gaa atc aag ggt tcc tat gtc ggt aac aga aag gat acc gcc gaa gcc      864
Glu Ile Lys Gly Ser Tyr Val Gly Asn Arg Lys Asp Thr Ala Glu Ala
        275                 280                 285

gtt gac ttt ttc tcc aga ggc ttg atc aag tgt cca atc aag att gtt      912
Val Asp Phe Phe Ser Arg Gly Leu Ile Lys Cys Pro Ile Lys Ile Val
    290                 295                 300

ggc ttg agt gaa ttg cca cag gtc ttc aag ttg atg                      948
Gly Leu Ser Glu Leu Pro Gln Val Phe Lys Leu Met
305                 310                 315

<210>  66
<211>  316
<212>  PRT
<213>  Candida tropicalis

<400>  66

Glu Leu Glu Tyr Lys Asp Ile Pro Val Pro Thr Pro Lys Ala Asn Glu
1               5                   10                  15

Leu Leu Ile Asn Val Lys Tyr Ser Gly Val Cys His Thr Asp Leu His
            20                  25                  30

Ala Trp Lys Gly Asp Trp Pro Leu Ala Thr Lys Leu Pro Leu Val Gly
        35                  40                  45

Gly His Glu Gly Ala Gly Val Val Val Gly Met Gly Glu Asn Val Lys
    50                  55                  60

Gly Trp Lys Ile Gly Asp Phe Ala Gly Ile Lys Trp Leu Asn Gly Ser
65                  70                  75                  80

Cys Met Ser Cys Glu Phe Cys Gln Gln Gly Ala Glu Pro Asn Cys Gly
                85                  90                  95

Glu Ala Asp Leu Ser Gly Tyr Thr His Asp Gly Ser Phe Glu Gln Tyr
            100                 105                 110

Ala Thr Ala Asp Ala Val Gln Ala Ala Arg Ile Pro Ala Gly Thr Asp
        115                 120                 125

Leu Ala Glu Val Ala Pro Ile Leu Cys Ala Gly Val Thr Val Tyr Lys
    130                 135                 140

Ala Leu Lys Thr Ala Asp Leu Ala Ala Gly Gln Trp Val Ala Ile Ser
145                 150                 155                 160

Gly Ala Gly Gly Gly Leu Gly Ser Leu Ala Val Gln Tyr Ala Val Ala
                165                 170                 175

Met Gly Leu Arg Val Val Ala Ile Asp Gly Gly Asp Glu Lys Gly Ala
            180                 185                 190

Phe Val Lys Ser Leu Gly Ala Glu Ala Tyr Ile Asp Phe Leu Lys Glu
        195                 200                 205

Lys Asp Ile Val Ser Ala Val Lys Lys Ala Thr Asp Gly Gly Pro His
    210                 215                 220

Gly Ala Ile Asn Val Ser Val Ser Glu Lys Ala Ile Asp Gln Ser Val
225                 230                 235                 240

Glu Tyr Val Arg Pro Leu Gly Lys Val Val Leu Val Gly Leu Pro Ala
                245                 250                 255

Gly Ser Lys Val Thr Ala Gly Val Phe Glu Ala Val Val Lys Ser Ile

260                 265                 270

Glu Ile Lys Gly Ser Tyr Val Gly Asn Arg Lys Asp Thr Ala Glu Ala
        275                 280                 285

Val Asp Phe Phe Ser Arg Gly Leu Ile Lys Cys Pro Ile Lys Ile Val
    290                 295                 300

Gly Leu Ser Glu Leu Pro Gln Val Phe Lys Leu Met
305                 310                 315

<210>  67
<211>  1992
<212>  DNA
<213>  Candida tropicalis

<220>
<221>  CDS
<222>  (1)..(1992)

<400>  67
ctt atg tta tta tgt gac ggg atc atc cac gaa acc acc gtc gac caa       48
Leu Met Leu Leu Cys Asp Gly Ile Ile His Glu Thr Thr Val Asp Gln
1               5                   10                  15

atc aaa gac gtt att gct cct gac ttc cct gct gac aag tac gaa gag       96
Ile Lys Asp Val Ile Ala Pro Asp Phe Pro Ala Asp Lys Tyr Glu Glu
            20                  25                  30

tac gtc agg aca ttc acc aaa ccc tcc gaa acc cca ggg ttc agg gaa      144
Tyr Val Arg Thr Phe Thr Lys Pro Ser Glu Thr Pro Gly Phe Arg Glu
        35                  40                  45

acc gtc tac aac aca gtc aac gca aac acc acg gac gca atc cac cag      192
Thr Val Tyr Asn Thr Val Asn Ala Asn Thr Thr Asp Ala Ile His Gln
    50                  55                  60

ttc att atc ttg acc aat gtt ttg gca tcc agg gtc ttg gct cca gct      240
Phe Ile Ile Leu Thr Asn Val Leu Ala Ser Arg Val Leu Ala Pro Ala
65                  70                  75                  80

ttg acc aac tcg ttg acg cct atc aag gac atg agc ttg gaa gac cgt      288
Leu Thr Asn Ser Leu Thr Pro Ile Lys Asp Met Ser Leu Glu Asp Arg
                85                  90                  95

gaa aaa ttg ttg gcc tcg tgg cgc gac tcc cca atc gct gcc aaa agg      336
Glu Lys Leu Leu Ala Ser Trp Arg Asp Ser Pro Ile Ala Ala Lys Arg
            100                 105                 110

aaa ttg ttc agg ttg gtt tcc acg ctt acc ttg gtt act ttc acg aga      384
Lys Leu Phe Arg Leu Val Ser Thr Leu Thr Leu Val Thr Phe Thr Arg
        115                 120                 125

ttg gcc aat gag ttg cat ttg aaa gcc att cac tat cca gga aga gaa      432
Leu Ala Asn Glu Leu His Leu Lys Ala Ile His Tyr Pro Gly Arg Glu
    130                 135                 140

gac cgt gaa aag gct tat gaa acc cag gag att gac cct ttc aag tac      480
Asp Arg Glu Lys Ala Tyr Glu Thr Gln Glu Ile Asp Pro Phe Lys Tyr

145                 150                 155                 160

cag ttt atg gaa aag cca aag ttt gac ggc gct gag ttg tac ttg cca       528
Gln Phe Met Glu Lys Pro Lys Phe Asp Gly Ala Glu Leu Tyr Leu Pro
                165                 170                 175

gat att gat gtt atc att att gga tct ggt gcc ggt gct ggt gtt gtg       576
Asp Ile Asp Val Ile Ile Ile Gly Ser Gly Ala Gly Ala Gly Val Val
            180                 185                 190

gcc cac act ttg gcc aac gat ggc ttc aag agt ttg gtt ttg gaa aag       624
Ala His Thr Leu Ala Asn Asp Gly Phe Lys Ser Leu Val Leu Glu Lys
        195                 200                 205

ggc aaa tac ttt agc aac tcc gag ttg aac ttt gat gac aag gac ggc       672
Gly Lys Tyr Phe Ser Asn Ser Glu Leu Asn Phe Asp Asp Lys Asp Gly
    210                 215                 220

gtt caa gaa tta tac caa agt gga ggt act ttg act aca gtc aac caa       720
Val Gln Glu Leu Tyr Gln Ser Gly Gly Thr Leu Thr Thr Val Asn Gln
225                 230                 235                 240

cag ttg ttt gtt ctt gct ggt tcc act ttt ggt ggc ggt acc act gtc       768
Gln Leu Phe Val Leu Ala Gly Ser Thr Phe Gly Gly Gly Thr Thr Val
                245                 250                 255

aat tgg tca gcc tgt ctt aag acg cca ttc aag gtg cgt aag gaa tgg       816
Asn Trp Ser Ala Cys Leu Lys Thr Pro Phe Lys Val Arg Lys Glu Trp
            260                 265                 270

tat gat gag ttt ggt gtt gac ttt gct gct gat gaa gca tac gat aaa       864
Tyr Asp Glu Phe Gly Val Asp Phe Ala Ala Asp Glu Ala Tyr Asp Lys
        275                 280                 285

gcg cag gat tat gtt tgg cag caa atg gga gct tct acc gaa ggc atc       912
Ala Gln Asp Tyr Val Trp Gln Gln Met Gly Ala Ser Thr Glu Gly Ile
    290                 295                 300

acc cac tct ttg gct aac gag att att att gaa ggt ggt aag aaa tta       960
Thr His Ser Leu Ala Asn Glu Ile Ile Ile Glu Gly Gly Lys Lys Leu
305                 310                 315                 320

ggt tac aag gcc aag gta tta gac caa aac agc ggt ggt cat cct cag      1008
Gly Tyr Lys Ala Lys Val Leu Asp Gln Asn Ser Gly Gly His Pro Gln
                325                 330                 335

cac aga tgc ggt ttc tgt tat ttg ggc tgt aag cac ggt atc aag cag      1056
His Arg Cys Gly Phe Cys Tyr Leu Gly Cys Lys His Gly Ile Lys Gln
            340                 345                 350

ggt tct gtt aat aac tgg ttt aga gac gca gct gcc cac ggt tcc cag      1104
Gly Ser Val Asn Asn Trp Phe Arg Asp Ala Ala Ala His Gly Ser Gln
        355                 360                 365

ttc atg caa cag gtt aga gtt ttg caa ata ctt aac aag aag ggg atc      1152
Phe Met Gln Gln Val Arg Val Leu Gln Ile Leu Asn Lys Lys Gly Ile
    370                 375                 380

gct tac ggt atc ttg tgt gag gat gtt gta acc ggc gcc aag ttc acc      1200
Ala Tyr Gly Ile Leu Cys Glu Asp Val Val Thr Gly Ala Lys Phe Thr
385                 390                 395                 400

att act ggc ccc aaa aag ttt gtt gtt gct gcc ggt gct ttg aac act      1248
Ile Thr Gly Pro Lys Lys Phe Val Val Ala Ala Gly Ala Leu Asn Thr

405                 410                 415

cca tct gtg ttg gtc aac tcc ggc ttc aag aac aag aac atc ggt aag      1296
Pro Ser Val Leu Val Asn Ser Gly Phe Lys Asn Lys Asn Ile Gly Lys
        420                 425                 430

aac tta act ttg cac cca gtt tct gtc gtg ttt ggt gat ttt ggc aaa      1344
Asn Leu Thr Leu His Pro Val Ser Val Val Phe Gly Asp Phe Gly Lys
        435                 440                 445

gac gtt caa gca gac cac ttc cac aac tcc atc atg act gcc ctt tgt      1392
Asp Val Gln Ala Asp His Phe His Asn Ser Ile Met Thr Ala Leu Cys
    450                 455                 460

tca gaa gcc gct gat tta gac ggc aag ggc cat gga tgc aga att gaa      1440
Ser Glu Ala Ala Asp Leu Asp Gly Lys Gly His Gly Cys Arg Ile Glu
465                 470                 475                 480

acc atc ttg aac gct cca ttc atc cag gct tca ttc tta cca tgg aga      1488
Thr Ile Leu Asn Ala Pro Phe Ile Gln Ala Ser Phe Leu Pro Trp Arg
                485                 490                 495

ggt agt aac gag gct aga cga gac ttg ttg cgt tac aac aac atg gtg      1536
Gly Ser Asn Glu Ala Arg Arg Asp Leu Leu Arg Tyr Asn Asn Met Val
            500                 505                 510

gcg atg ttg ctc ctt agt cgt gac acc acc agt ggt tcc gtt tct gct      1584
Ala Met Leu Leu Leu Ser Arg Asp Thr Thr Ser Gly Ser Val Ser Ala
        515                 520                 525

cat cca acc aaa cct gaa gct ttg gtt gtc gag tac gac gtg aac aag      1632
His Pro Thr Lys Pro Glu Ala Leu Val Val Glu Tyr Asp Val Asn Lys
    530                 535                 540

ttt gac aga aac tcg atc ttg cag gca ttg ttg gtc act gct gac ttg      1680
Phe Asp Arg Asn Ser Ile Leu Gln Ala Leu Leu Val Thr Ala Asp Leu
545                 550                 555                 560

ttg tat atc caa ggt gcc aag aga atc ctt agt cca cag gca tgg gtg      1728
Leu Tyr Ile Gln Gly Ala Lys Arg Ile Leu Ser Pro Gln Ala Trp Val
                565                 570                 575

cca att ttt gaa tcc gac aag cca aag gat aag aga tca atc aag gac      1776
Pro Ile Phe Glu Ser Asp Lys Pro Lys Asp Lys Arg Ser Ile Lys Asp
            580                 585                 590

gag gac tat gtc gaa tgg aga gcc aag gtt gcc aag att cct ttc gac      1824
Glu Asp Tyr Val Glu Trp Arg Ala Lys Val Ala Lys Ile Pro Phe Asp
        595                 600                 605

acc tac ggc tca cct tat ggt tcg gca cat caa atg tct tct tgc cgt      1872
Thr Tyr Gly Ser Pro Tyr Gly Ser Ala His Gln Met Ser Ser Cys Arg
    610                 615                 620

atg tca ggt aag ggt cct aaa tac ggt gct gtt gac acc gat ggt aga      1920
Met Ser Gly Lys Gly Pro Lys Tyr Gly Ala Val Asp Thr Asp Gly Arg
625                 630                 635                 640

ttg ttt gaa tgt tcg aat gtt tat gtt gcc gat gca agt ctt ttg cca      1968
Leu Phe Glu Cys Ser Asn Val Tyr Val Ala Asp Ala Ser Leu Leu Pro
                645                 650                 655

act gca agc ggt gct aat cct atg                                      1992
Thr Ala Ser Gly Ala Asn Pro Met

            660

<210>  68
<211>  664
<212>  PRT
<213>  Candida tropicalis

<400>  68

Leu Met Leu Leu Cys Asp Gly Ile Ile His Glu Thr Thr Val Asp Gln
1               5                   10                  15

Ile Lys Asp Val Ile Ala Pro Asp Phe Pro Ala Asp Lys Tyr Glu Glu
            20                  25                  30

Tyr Val Arg Thr Phe Thr Lys Pro Ser Glu Thr Pro Gly Phe Arg Glu
        35                  40                  45

Thr Val Tyr Asn Thr Val Asn Ala Asn Thr Thr Asp Ala Ile His Gln
    50                  55                  60

Phe Ile Ile Leu Thr Asn Val Leu Ala Ser Arg Val Leu Ala Pro Ala
65                  70                  75                  80

Leu Thr Asn Ser Leu Thr Pro Ile Lys Asp Met Ser Leu Glu Asp Arg
                85                  90                  95

Glu Lys Leu Leu Ala Ser Trp Arg Asp Ser Pro Ile Ala Ala Lys Arg
            100                 105                 110

Lys Leu Phe Arg Leu Val Ser Thr Leu Thr Leu Val Thr Phe Thr Arg
        115                 120                 125

Leu Ala Asn Glu Leu His Leu Lys Ala Ile His Tyr Pro Gly Arg Glu
    130                 135                 140

Asp Arg Glu Lys Ala Tyr Glu Thr Gln Glu Ile Asp Pro Phe Lys Tyr
145                 150                 155                 160

Gln Phe Met Glu Lys Pro Lys Phe Asp Gly Ala Glu Leu Tyr Leu Pro
                165                 170                 175

Asp Ile Asp Val Ile Ile Ile Gly Ser Gly Ala Gly Ala Gly Val Val
            180                 185                 190

Ala His Thr Leu Ala Asn Asp Gly Phe Lys Ser Leu Val Leu Glu Lys
        195                 200                 205

Gly Lys Tyr Phe Ser Asn Ser Glu Leu Asn Phe Asp Asp Lys Asp Gly
    210                 215                 220

Val Gln Glu Leu Tyr Gln Ser Gly Gly Thr Leu Thr Thr Val Asn Gln
225                 230                 235                 240

Gln Leu Phe Val Leu Ala Gly Ser Thr Phe Gly Gly Gly Thr Thr Val
                245                 250                 255

Asn Trp Ser Ala Cys Leu Lys Thr Pro Phe Lys Val Arg Lys Glu Trp
            260                 265                 270

Tyr Asp Glu Phe Gly Val Asp Phe Ala Ala Asp Glu Ala Tyr Asp Lys
        275                 280                 285

Ala Gln Asp Tyr Val Trp Gln Gln Met Gly Ala Ser Thr Glu Gly Ile
    290                 295                 300

Thr His Ser Leu Ala Asn Glu Ile Ile Ile Glu Gly Gly Lys Lys Leu
305                 310                 315                 320

Gly Tyr Lys Ala Lys Val Leu Asp Gln Asn Ser Gly Gly His Pro Gln
                325                 330                 335

His Arg Cys Gly Phe Cys Tyr Leu Gly Cys Lys His Gly Ile Lys Gln
            340                 345                 350

Gly Ser Val Asn Asn Trp Phe Arg Asp Ala Ala Ala His Gly Ser Gln
        355                 360                 365

Phe Met Gln Gln Val Arg Val Leu Gln Ile Leu Asn Lys Lys Gly Ile
    370                 375                 380

Ala Tyr Gly Ile Leu Cys Glu Asp Val Val Thr Gly Ala Lys Phe Thr
385                 390                 395                 400

Ile Thr Gly Pro Lys Lys Phe Val Val Ala Ala Gly Ala Leu Asn Thr
                405                 410                 415

Pro Ser Val Leu Val Asn Ser Gly Phe Lys Asn Lys Asn Ile Gly Lys
            420                 425                 430

Asn Leu Thr Leu His Pro Val Ser Val Val Phe Gly Asp Phe Gly Lys
        435                 440                 445

Asp Val Gln Ala Asp His Phe His Asn Ser Ile Met Thr Ala Leu Cys
    450                 455                 460

Ser Glu Ala Ala Asp Leu Asp Gly Lys Gly His Gly Cys Arg Ile Glu
465                 470                 475                 480

Thr Ile Leu Asn Ala Pro Phe Ile Gln Ala Ser Phe Leu Pro Trp Arg
                485                 490                 495

Gly Ser Asn Glu Ala Arg Arg Asp Leu Leu Arg Tyr Asn Asn Met Val
            500                 505                 510

Ala Met Leu Leu Leu Ser Arg Asp Thr Thr Ser Gly Ser Val Ser Ala
        515                 520                 525

His Pro Thr Lys Pro Glu Ala Leu Val Val Glu Tyr Asp Val Asn Lys
    530                 535                 540

Phe Asp Arg Asn Ser Ile Leu Gln Ala Leu Leu Val Thr Ala Asp Leu
545                 550                 555                 560

Leu Tyr Ile Gln Gly Ala Lys Arg Ile Leu Ser Pro Gln Ala Trp Val
                565                 570                 575

Pro Ile Phe Glu Ser Asp Lys Pro Lys Asp Lys Arg Ser Ile Lys Asp
            580                 585                 590

Glu Asp Tyr Val Glu Trp Arg Ala Lys Val Ala Lys Ile Pro Phe Asp
        595                 600                 605

Thr Tyr Gly Ser Pro Tyr Gly Ser Ala His Gln Met Ser Ser Cys Arg
    610                 615                 620

Met Ser Gly Lys Gly Pro Lys Tyr Gly Ala Val Asp Thr Asp Gly Arg
625                 630                 635                 640

Leu Phe Glu Cys Ser Asn Val Tyr Val Ala Asp Ala Ser Leu Leu Pro
                645                 650                 655

Thr Ala Ser Gly Ala Asn Pro Met
            660